# EUROPEAN PATENT APPLICATION

(11) **EP 4 814 014 A1**
(43) Date of publication of application: **30.09.2026**
(21) Application number: 24893581.9
(22) Date of filing: 22.11.2024
(51) Int. Cl.: C07K 16/28, C07K 19/00, A61P 35/00, A61K 47/55, A61K 47/68, A61K 39/395, C12N 15/13, C12N 5/10

(54) **BINDING MOLECULE AND ANTIBODY-DRUG CONJUGATE AND USE THEREOF**

(30) Priority: 23.11.2023 CN 202311575715; 09.01.2024 CN 202410031836; 20.05.2024 CN 202410629124; 08.08.2024 CN 202411090866
(71) Applicant: Velavigo Bio, Inc., Middletown, DE 19709 (US)
(72) Inventor: WANG, Wei, Shanghai 200131 (CN); LI, Jing, Shanghai 200131 (CN); MA, Lin, Shanghai 200131 (CN); XU, Man, Shanghai 200131 (CN); YIN, Qun, Shanghai 200131 (CN)
(74) Representative: Sagittarius IP
(86) International application number: PCT/CN2024/133842
(87) International publication number: WO 2025/108427

(57) **Abstract**

Provided are an immunoglobulin single variable domain (ISVD) specifically binding to EGFR and/or cMet, and an EGFR- and/or cMet-binding molecule and an antibody-drug conjugate (ADC) comprising the immunoglobulin single variable domain. Further provided are a nucleic acid encoding the ISVD or the binding molecule, a vector comprising the nucleic acid, and the therapeutic use of the ISVD or the binding molecule and the ADC.

## Description

### TECHNICAL FIELD

The present disclosure relates to an immunoglobulin single variable domain (ISVD) specifically binding to EGFR and/or cMet, and an EGFR- and/or cMet-binding molecule and an antibody-drug conjugate (ADC) comprising the immunoglobulin single variable domain. The present disclosure also relates to a nucleic acid encoding the ISVD or the binding molecule, a vector comprising the nucleic acid, and therapeutic use of the ISVD or the binding molecule and the ADC.

### BACKGROUND

EGFR is a transmembrane receptor protein present on the cell membrane and having tyrosine kinase activity. It can transmit signals by binding to exogenous growth factors (such as EGF) to further activate downstream pathways associated with cell division, cell survival, and angiogenesis, thereby affecting cellular events such as proliferation, survival, and differentiation. High expression or mutation of EGFR has been found in many tumor types. Thus, EGFR has become an important target for treating such cancers. At present, a variety of drugs for inhibiting the function of EGFR have been developed, such as tyrosine kinase inhibitors and monoclonal antibodies. These drugs have been widely used in the treatment of multiple malignant tumors, such as lung cancer, colorectal cancer, and head and neck cancer.

cMet is a receptor expressed on epithelial cells and having tyrosine kinase (RTK) activity, and it plays a key role in cell proliferation, survival, and migration. cMet, upon binding to its binding ligand, the hepatocyte growth factor (HGF), undergoes dimerization and activates the cMet pathway, promoting cell division, angiogenesis, immunoregulation, and the like. High expression or mutation of cMet is present in many cancer types. Thus, cMet has become an important drug target, and related anti-cancer drugs are currently under research and development.

The U.S. Food and Drug Administration has approved an EGFR and cMet dual-targeting antibody, amivantamab (also known as "JNJ-61186372"), for the treatment of adult patients with locally advanced or metastatic non-small cell lung cancer (NSCLC) harboring epidermal growth factor receptor (EGFR) exon 20 insertion mutations, and this drug is used to overcome resistance to targeted therapy in patients with non-small cell lung cancer. AZD9592, an anti-EGFR/cMet bispecific antibody ADC drug under clinical study, carries a topoisomerase 1 inhibitor as a payload and is used for the treatment of advanced solid tumors (Moores, Sheri L. et al, 2016. "A Novel Bispecific Antibody Targeting EGFR and cMet Is Effective against EGFR Inhibitor-Resistant Lung Tumors." Cancer Research 76 (13): 3942-53. https://doi.org/10.1158/0008-5472.CAN-15-2833; Robert Hsu et al, "A narrative review of antibody-drug conjugates in EGFR-mutated non-small cell lung cancer", Front Oncol. 2023 Dec 1;13:1252652. doi: 10.3389/fonc.2023.1252652).

The anti-EGFR/cMet bispecific antibody JNJ-61186372 and the antibody in the ADC drug AZD9592 have a conventional four-chain antibody structure and a large molecular weight (150 KDa), which render them disadvantageous in deep tumor tissue penetration. Therefore, there still remains an urgent need in the art to develop new antibody formats targeting EGFR and/or cMet targets and EGFR- and/or cMet-binding molecules and ADC molecules with advantageous properties.

Immunoglobulin single variable domains (ISVDs), such as nanobodies, are small proteins consisting of single-chain antibody molecules. They feature high antigen specificity and affinity and have a smaller volume, higher stability, and deeper tissue penetration compared with conventional four-chain antibodies. Therefore, the application of nanobodies and drug molecules constructed based thereon in the field of tumor treatment is receiving wide attention, and they are expected to become one of the important means for future tumor treatment.

### SUMMARY

The present disclosure provides novel anti-cMet and anti-EGFR immunoglobulin single variable domains (ISVDs) on the basis of screening anti-EGFR and anti-cMet phage display libraries, and constructs an anti-EGFR binding molecule, an anti-cMet binding molecule, an anti-EGFR/cMet binding molecule, and an antibody-drug conjugate possessing excellent tumor-targeting properties, endocytic activity, and killing activity by using the ISVDs as components.

Thus, in a first aspect, the present disclosure provides an immunoglobulin single variable domain (ISVD) specifically binding to cMet, a heavy-chain antibody comprising the ISVD, and a cMet-binding molecule. In some embodiments, the cMet-binding molecule is an anti-cMet biepitopic antibody. In some embodiments, the cMet-binding molecule further comprises a binding domain binding to EGFR.

In a second aspect, the present disclosure provides an immunoglobulin single variable domain (ISVD) specifically binding to EGFR, a heavy-chain antibody comprising the ISVD, and an EGFR-binding molecule. In some embodiments, the EGFR-binding molecule further comprises a binding domain binding to cMet.

In a third aspect, the present disclosure provides an EGFR- and cMet-binding molecule comprising one or more immunoglobulin single variable domains (ISVDs) specifically binding to EGFR and cMet according to the first aspect and/or the second aspect of the present disclosure. In some embodiments, the binding molecule is a single-chain or multi-chain antibody. In some embodiments, the antibody is a bispecific antibody or a multispecific antibody.

In a fourth aspect, the present disclosure provides nucleic acids encoding the ISVD, the EGFR-binding molecule, the cMet-binding molecule, and the EGFR- and cMet-binding molecule according to the first aspect to the third aspect of the present disclosure, vectors (preferably, expression vectors) comprising the nucleic acids, and host cells comprising the nucleic acids or the vectors. In some embodiments, the host cell is prokaryotic or eukaryotic, e.g., a host cell selected from an *E. coli* cell, a yeast cell, a mammalian cell, and other cells suitable for preparing ISVDs or binding molecules. In some embodiments, the host cell is an HEK 293 cell or a CHO cell. The present disclosure also provides a method for preparing the ISVD, the EGFR-binding molecule, the cMet-binding molecule, and the EGFR- and cMet-binding molecule according to the first aspect to the third aspect of the present disclosure.

In some embodiments, the binding molecule specifically binding to EGFR and/or cMet according to the present disclosure has a molecular weight lower than that of a conventional four-chain antibody, and has better tumor tissue penetration ability.

In some embodiments, the EGFR- and/or cMet-binding molecule according to the present disclosure has a relatively low specific binding activity for the EGFR antigen expressed on the cell surface, thereby reducing the "on-target/off-tumor" toxicity of the EGFR-binding molecule, for example, significantly reducing the skin toxicity of drugs containing EGFR-binding molecules that has often been reported (Taieb, Julien, et al., 2023. "Adverse Events Associated with Encorafenib Plus Cetuximab in Patients with BRAFV600E-Mutant Metastatic Colorectal Cancer: An in-Depth Analysis of the BEACON CRC Study." Clinical Colorectal Cancer, Updates in Pancreatic Cancer, 22 (1): 59-66. https://doi.org/10.1016/j.clcc.2022.12.003; Robert, Caroline, et al., 2005. "Cutaneous Side-Effects of Kinase Inhibitors and Blocking Antibodies." The Lancet Oncology 6(7): 491-500. https://doi.org/10.1016/51470-2045(05)70243-6).

In some embodiments, the binding molecule according to the present disclosure has a structure characterized by biepitopic binding to cMet (i.e., binding to two different epitopes on cMet); the structure enables the binding molecule to have better tumor-targeting property. In some embodiments, the cMet biepitopic design of the binding molecule according to the present disclosure significantly increases the affinity of the binding molecule for target cells and endocytosis capacity compared to the design of targeting only a single epitope on cMet.

The ISVD specifically binding to EGFR and/or cMet and the EGFR- and/or cMet-binding molecule comprising the ISVD of the present disclosure have good tumor-targeting property, tumor tissue penetration ability, and/or target cell endocytosis ability, and the like. Therefore, the ISVD or the binding molecule can serve as a targeting module of a conjugate and be conjugated or coupled to, e.g., a chemotherapeutic agent, a toxin, a drug (e.g., an immunotherapeutic agent), a radioelement, a probe, or a signaling molecule to provide use in tumor killing, immunoregulation, disease detection, or the like.

In a fifth aspect, the present disclosure provides a conjugate, a fusion, and an antibody-drug conjugate (ADC), particularly an anti-EGFR/cMet multispecific antibody-drug conjugate comprising the ISVD, the EGFR-binding molecule, the cMet-binding molecule, or the EGFR- and cMet-binding molecule according to the first aspect to the third aspect of the present disclosure.

In some embodiments, the antibody-drug conjugate according to the present disclosure has the following advantages:
(1) binding to tumor cells expressing human EGFR or cMet and tumor cells co-expressing human EGFR and cMet;
(2) exhibiting endocytic activity on tumor cells expressing human EGFR or cMet and tumor cells co-expressing human EGFR and cMet;
(3) blocking the binding of HGF to cMet on tumor cells;
(4) having a bystander killing effect;
(5) having a wide anti-tumor activity spectrum, and exhibiting significant killing activity against tumors with different expression densities of EGFR and cMet;
(6) having low *in vivo* toxic and side effects, e.g., low on-target/off-tumor toxicity.

In some embodiments, the antibody-drug conjugate according to the present disclosure also has one or more advantages selected from the following:
(7) being able to target multiple antigens simultaneously and having better targeting property and reduced toxic and side effects (e.g., reduced off-target toxicity or reduced potential dose-limiting toxicity) due to the multispecific antibody contained therein;
(8) having higher endocytosis efficiency in tumor cells compared to a single-target ADC targeting EGFR or cMet;
(9) having higher affinity for tumor cells compared to a single-target ADC targeting EGFR or cMet;
(10) having a stronger killing effect on tumor cells and a stronger inhibitory effect on tumor growth compared to a single-target ADC targeting EGFR or cMet.

In some embodiments, the antibody-drug conjugate according to the present disclosure also has one or more advantages selected from the following:
(11) having good product uniformity;
(12) having good product stability; and
(13) having outstanding potential to be manufactured as a medicament.

In a sixth aspect, the present disclosure provides a pharmaceutical composition and a pharmaceutical formulation. The pharmaceutical composition and the pharmaceutical formulation comprise the ISVD, the EGFR-binding molecule, the cMet-binding molecule, or the EGFR- and cMet-binding molecule according to the first aspect to the third aspect of the present disclosure, or the ADC according to the fifth aspect of the present disclosure, and a pharmaceutically acceptable carrier; optionally, the pharmaceutical composition and the pharmaceutical formulation further comprise one or more additional pharmaceutically active polypeptides and/or compounds; for example, the pharmaceutical composition and the pharmaceutical formulation further comprise an additional therapeutic agent selected from an oncolytic drug, a cytotoxic agent, a cytokine, and an inhibitor of an immune checkpoint molecule. In this aspect, the present disclosure also provides a combination product or a kit comprising the ISVD, the EGFR-binding molecule, the cMet-binding molecule, or the EGFR- and cMet-binding molecule according to the first aspect to the third aspect of the present disclosure, or the ADC according to the fifth aspect of the present disclosure.

In a seventh aspect, the present disclosure provides use of the ISVD, the EGFR-binding molecule, the cMet-binding molecule, or the EGFR- and cMet-binding molecule according to the first aspect to the third aspect of the present disclosure, or the ADC according to the fifth aspect of the present disclosure for use as a medicament or for use in the manufacture of a medicament, wherein the medicament is for treating a cancer; for example, the cancer is selected from lung cancer (e.g., lung squamous cell carcinoma, lung adenocarcinoma, and non-small cell lung cancer), breast cancer (e.g., triple negative breast cancer), gastric cancer, colon cancer, and head and neck cancer (e.g., pharyngeal squamous cell carcinoma). In this aspect, the present disclosure also provides a method for treating a cancer, which comprises administering to a subject in need thereof an effective amount of the ISVD, the EGFR-binding molecule, the cMet-binding molecule, or the EGFR- and cMet-binding molecule according to the first aspect to the third aspect of the present disclosure, or the nucleic acid or the vector or the host cell according to the fourth aspect of the present disclosure, or the ADC according to the fifth aspect of the present disclosure, wherein the subject is a mammal; preferably, the subject is a human; wherein the cancer is, for example, lung cancer (e.g., lung squamous cell carcinoma, lung adenocarcinoma, or non-small cell lung cancer), breast cancer (e.g., triple negative breast cancer), gastric cancer, colon cancer, or head and neck cancer (e.g., pharyngeal squamous cell carcinoma).

### BRIEF DESCRIPTION OF THE DRAWINGS

The preferred embodiments of the present disclosure described in detail below will be better understood when read in conjunction with the following drawings. For the purpose of illustrating the present disclosure, currently preferred embodiments are shown in the drawings. However, it should be understood that the present disclosure is not limited to the precise arrangement and means of the embodiments shown in the drawings.
FIG. 1 shows the binding activity of anti-EGFR antibodies for target cells as assayed by FACS.
FIG. 2 shows the binding activity of anti-cMet antibodies for target cells as assayed by FACS.
FIG. 3 shows the blocking effect of anti-cMet VHH-Fc on the binding of the ligand HGF to the target cells EBC-1.
FIG. 4 shows the binding of anti-cMet antibodies V-n7A12 and V-n9A2 to different epitopes on the antigen cMet.
FIGs. 5A and 5B show the endocytosis of each VHH-Fc by cells as assayed by FACS.
FIG. 6 shows the results of whether the anti-cMet antibodies V-n7A12, V-n9A2, and V-n9A10 have cross-reactivity with the human cMet antigen and the cynomolgus monkey cMet antigen, as determined by ELISA.
FIG. 7 shows the binding of biepitopic antibodies to target cells as assayed by FACS.
FIG. 8 shows the endocytosis of biepitopic antibodies in target cells as assayed by FACS. Biepitopic cases mediate synergistic endocytic activity compared to the monoepitopic case.
FIG. 9 shows a schematic diagram of the structure of a multispecific EGFR/cMet antibody molecule in a single-chain form.
FIG. 10 shows a schematic diagram of the structure of a multispecific EGFR/cMet antibody molecule in a double-chain form.
FIG. 11 shows the binding of trispecific antibody candidate molecules on the target cells EBC-1 and NCI-H1975.
FIG. 12 shows the endocytosis of single-chain trispecific antibody candidate molecules by target cells.
FIG. 13 shows the endocytosis of double-chain trispecific antibody candidate molecules by target cells.
FIG. 14 shows trispecific anti-EGFR/cMet antibodies blocking the binding of EBC-1 cells to the ligand HGF.
FIG. 15 shows the synergistic endocytosis effects of trispecific antibodies on target cells mediated by anti-EGFR ISVDs and anti-cMet ISVDs, as assayed by FACS.
FIG. 16 shows the synergistic binding effects of trispecific antibodies on target cells mediated by anti-EGFR ISVDs and anti-cMet ISVDs, as assayed by FACS.
FIG. 17 shows the binding of ADC molecules to target cells.
FIGs. 18A and 18B show the killing of target cells by ADC molecules.
FIG. 19 shows the cell binding assay of ADC molecules having linkers with PEG and/or EVC.
FIG. 20 shows the cell killing assay of ADC molecules having linkers with PEG and/or EVC.
FIG. 21 shows the *in vivo* efficacy of V-20-Fc-PEG-EVC-MMAE, V-23-Fc-PEG-EVC-MMAE, and V-26-Fc-PEG-EVC-MMAE in CDX models.
FIG. 22 shows the *in vivo* efficacy of V-23-Fc-VA-Exd in CDX models.
FIG. 23 shows the *in vivo* efficacy of V-23-Fc-Glu-Exd in CDX models.

### DETAILED DESCRIPTION

Unless otherwise defined, all technical and scientific terms used herein have the same meaning as commonly understood by those of ordinary skill in the art to which the present disclosure belongs. All publications, patent applications, patents, and other references mentioned herein are incorporated by reference in their entirety. In addition, the materials, methods, and examples described herein are illustrative only and are not intended to be limiting. Other features, objectives, and advantages of the present disclosure will be apparent from this specification and drawings and from the appended claims.

### Definitions

In order to illustrate this specification, the following definitions will be used, and wherever appropriate, terms used in the singular form may also include the plural form, and vice versa. It should be understood that the terms used herein are for the purpose of describing specific embodiments only, and are not intended to be limiting.

The term "about" used in combination with a numerical value is intended to encompass the numerical values in a range from a lower limit less than the specified numerical value by 5%, 4%, 3%, 2%, or 1% to an upper limit greater than the specified numerical value by 5%, 4%, 3%, 2%, or 1%.

As used herein, the term "and/or" refers to any one of the options or any two or more of the options.

The term "comprise" or "include" used herein, unless indicated otherwise, also encompasses the situation where the entirety consists of the described elements, integers, or steps. For example, when referring to an antibody variable region "comprising" a specific sequence, it is also intended to encompass an antibody variable region consisting of the specific sequence.

The term "on-target/off tumor toxicity" refers to damages from binding of normal tissue cells that, in addition to tumor cells, also express tumor-associated antigens targeted by the antibodies, to the antibodies.

When used together with an antigen, the terms "binding molecule" and "antigen-binding molecule" are used interchangeably (e.g., EGFR-binding molecule, cMet-binding molecule, and EGFR- and cMet-binding molecule) and refer to a protein or a polypeptide molecule that can specifically bind to the antigen or an epitope on the antigen. The binding molecule has "affinity" and/or "specificity" for the antigen. Herein, the EGFR-binding molecule refers to a protein or a polypeptide that can specifically bind to EGFR, the cMet-binding molecule refers to a protein or a polypeptide that can specifically bind to cMet, and the EGFR- and cMet-binding molecule refers to a protein or a polypeptide that can specifically bind to both EGFR and cMet. Some examples of binding molecules include antibodies, antibody fragments, fusion proteins, and the like, as long as they exhibit the desired antigen binding activity.

Herein, the domain in the antigen-binding molecule that actually binds to the antigen is referred to as the "antigen-binding site" or "antigen-binding domain". As a folded structure in a protein or a polypeptide, a "domain" is generally responsible for a single function of the protein or the polypeptide. For example, in general, conventional antibodies and immunoglobulins form an antigen-binding domain on the surface of a VH-VL dimer through 3 complementarity-determining regions (HCDRs 1-3) in their heavy chain variable region (VH) and 3 complementarity-determining regions (LCDRs 1-3) in their light chain variable region (VL), wherein the 6 CDRs endow the antibody with specific binding to an antigen. However, in some cases, a single immunoglobulin variable domain (e.g., a heavy chain variable domain (VH) or a light chain variable domain (VL), a heavy chain variable domain (VHH) derived from a *Camelidae* heavy-chain antibody, or a VH-like single domain (v-NAR) derived from fish IgNAR) can endow antigen binding. That is, the single variable domain does not need to interact with another variable domain, and can independently serve as an "antigen-binding domain" that recognizes and binds to a target antigen. Typically, by means of engineering modification, the "antigen-binding domain" of an antibody, including the single immunoglobulin variable domain described above and the variable domain pair of a conventional antibody, may be added to, removed from, or transferred to other proteins or polypeptides and still exert its antigen binding function without impairing the functions of the rest parts and/or the rest domains of the protein or polypeptide.

The term "binding" or "specific binding" means that the binding effect is selective for antigens and can be distinguished from unwanted or non-specific interactions. The ability of an antigen-binding site to bind to a particular antigen can be determined by conventional binding assays known in the art. For example, the binding ability of an antibody for an antigen is determined by the ELISA assay described in examples, or the binding ability of an antibody for cells expressing an antigen on the surface is determined by the FACS assay described in examples, or the affinity constant K_{D} is determined by the SPR technique described in examples.

Herein, the term "antibody" is used in the broadest sense and refers to a protein comprising an antigen-binding site of an immunoglobulin and encompasses natural antibodies and artificial antibodies of various structures, including but not limited to, monoclonal antibodies, polyclonal antibodies, mono- and multi-epitopic antibodies (e.g., biepitopic antibodies), monospecific and multispecific antibodies (e.g., bispecific antibodies), single- and multi-chain antibodies, nanobodies, single-domain antibodies, heavy-chain antibodies, chimeric antibodies, humanized antibodies, intact antibodies, and antibody fragments. In some embodiments, preferably, the antibody of the present disclosure is a single-domain antibody, a nanobody, or a heavy-chain antibody. In some other embodiments, preferably, the antibody of the present disclosure is a biepitopic antibody, a bispecific antibody, or a multispecific antibody.

The terms "antibody fragment" and "antigen-binding fragment" of an antibody are used interchangeably and refer to a molecule different from an intact antibody, which comprises a portion of the intact antibody and can bind to an antigen to which the intact antibody binds. Examples of the antibody fragment include, but are not limited to, Fv, Fab, Fab', Fab'-SH, and F(ab')₂; single-chain antibody fragments (e.g., scFv and scFab); single immunoglobulin domains; variable domain fragments of *Camelidae* heavy-chain antibodies; and various monospecific, bispecific, or multispecific antibody structures formed from antibody fragments, e.g., linear antibody fragments and diabody fragments. In the present disclosure, unless otherwise stated or clearly contradicted to the context, reference to the term "antibody" is equivalent to reference to "antibodies and antibody fragments thereof". In some embodiments according to the present disclosure, the antibody fragment comprises a cysteine residue portion for forming an interchain disulfide bond between a heavy chain and another heavy chain, e.g., cysteine residues of the antibody hinge region, to provide amino acid residue sites that can be used for sulfhydryl conjugation chemistry. In some other embodiments according to the present disclosure, the antibody fragment comprises cysteine residues introduced into the Fc region, to provide amino acid residue sites that can be used for sulfhydryl conjugation chemistry.

In the present disclosure, the term "immunoglobulin single variable domain" (abbreviated as "ISVD") is used interchangeably with the term "single variable domain" to refer to an antibody polypeptide fragment that can specifically recognize and bind to an antigen of interest via a single variable domain, e.g., a single VHH domain or a single VH domain or a single VL domain, without pairing with an additional immunoglobulin variable domain. For an ISVD composed of a VHH domain or a VH or VL domain, its structure can be considered to consist of four framework regions ("FRs") referred to as "framework region 1" or "FR1", "framework region 2" or "FR2", "framework region 3" or "FR3", and "framework region 4" or "FR4", respectively, and three complementarity-determining regions ("CDRs") referred to as "complementarity-determining region 1" or "CDR1", "complementarity-determining region 2" or "CDR2", and "complementarity-determining region 3" or "CDR3", respectively, wherein the three complementarity determining region are interposed between the four framework regions. From the N-terminus to the C-terminus of the ISVD polypeptide, the four framework regions and the three complementarity-determining regions are arranged in the following order: FR1-CDR1-FR2-CDR2-FR3-CDR3-FR4. The immunoglobulin single variable domain may include a fully human sequence, a humanized sequence, a sequence-optimized sequence by other means, or a chimeric immunoglobulin sequence. The immunoglobulin single variable domain may be used alone in an isolated form or as part of a larger protein to exert an antigen binding function. In the present disclosure, an ISVD specifically binding to EGFR is also referred to as an anti-EGFR ISVD; an ISVD specifically binding to cMet is also referred to as an anti-cMet ISVD; an ISVD specifically binding to EGFR and cMet is also referred to as an anti-EGFR/cMet ISVD.

In the present disclosure, the terms "single-domain antibody" and "single domain antibody" are used interchangeably herein and generally refer to an antibody that recognizes and binds to an antigen through an ISVD.

Examples of single-domain antibodies include single-domain antibodies derived from *Camelidae* (Llama and camel) and cartilaginous fishes (e.g., nurse sharks) (WO 2005/035572).

In the present disclosure, the term "heavy-chain antibody (hcAb)" refers to an antibody having only heavy chains and no light chains. From N-terminus to C-terminus, the heavy chain of the heavy-chain antibody may comprise, e.g., VH-CH2-CH3, or may comprise VH-CH1-CH2-CH3, or may comprise VHH-CH2-CH3, and the like. The heavy chains may constitute a homodimer. In some embodiments, preferably, the heavy-chain antibody according to the present disclosure is a dimer comprising two monomers, wherein each of the monomers comprises a VHH domain linked to immunoglobulin constant regions (CH2 and CH3 domains) via an immunoglobulin hinge region.

Herein, the term "nanobody" is used to refer to an antibody comprising, consisting essentially of, or consisting of a single ISVD domain (e.g., a VHH domain) and having a molecular weight of less than 20 kDa (typically a molecular weight of about 12-15 kDa).

Herein, the term "VHH antibody" is used to refer to an antibody consisting of a VHH domain. "VHH domain", also known as VHH, VHH sequence, or VHH antibody fragment, is a single-chain antibody fragment comprising FR4-CDR3-FR3-CDR2-FR2-CDR1-FR1, from C-terminus to N-terminus. Compared to using conventional VH and VL domains, scFv, or conventional antibody fragments (such as Fab or F(ab')2 fragments), using a VHH domain (alone or as part of a larger polypeptide) to recognize and bind to a target antigen provides a variety of significant advantages:
- only a single domain is required to bind to an antigen with high affinity and high selectivity, and this eliminates the need for both the presence of two separate domains and the requirement to ensure that these two domains are present in an appropriate spatial conformation and configuration (for example, the use of specially designed linker is generally required for an scFv);
- VHH domains can be readily engineered into multivalent and multispecific formats;
- VHH domains are highly soluble and do not have a tendency to aggregate;
- VHH domains are highly stable to heat, pH, protein or polypeptide enzymes, and other denaturing agents or conditions and, thus, may be prepared, stored, or transported without the use of refrigeration equipment, so that the cost and time can be saved, and the impact on environment can be reduced;
- VHH domains are easy to prepare and relatively inexpensive, even on a manufacturing scale;
- VHH domains are relatively smaller than conventional four-peptide-chain antibodies and antigen-binding fragments thereof, and therefore exhibit relatively high tissue permeability and can be administered at relatively high doses;
- VHH domains can show so-called cavity-binding properties (compared to conventional VH domains, VHHs have extended CDR3 loops, so that they can access target epitopes not accessible for conventional four-peptide-chain antibodies and antigen-binding fragments thereof).

The VHH includes a humanized VHH, a camelized VH, or a VHH obtained by affinity maturation.

For further description of VHHs, reference may be made to WO 94/04678, WO 95/04079, and WO 96/34103. In some embodiments, the antigen-binding sites of the antigen-binding molecule and the antibody according to the present disclosure are preferably provided by VHH domains.

The term "...valent" refers to the number of antigen-binding sites present in an antigen-binding molecule (e.g., an antibody). Thus, "monovalent", "bivalent", "trivalent", and "tetravalent" antibodies refer to antibody molecules where 1 antigen-binding site, 2 antigen-binding sites, 3 antigen-binding sites, and 4 antigen-binding sites are present, respectively.

Herein, "monospecific" refers to the ability that the antigen-binding molecule can only bind to a single epitope. "Multispecific" refers to the ability that the antigen-binding molecule can bind to two or more different epitopes (e.g., different epitopes on the same antigen and/or different antigens). Correspondingly, "bispecific" refers to the ability that the antigen-binding molecule can bind to two different epitopes. The monospecific antigen-binding molecules (e.g., antibodies) may be monovalent or multivalent. The multispecific and bispecific antigen-binding molecules (e.g., antibodies) may be bivalent, trivalent, tetravalent, or of higher valency.

The term "variable region" or "variable domain" refers to a domain of a heavy chain or a light chain of an antibody involved in the binding of the antibody to an antigen. Variable domains of heavy chains and light chains generally have similar structures and comprise four conserved framework regions (FRs) and three complementarity-determining regions (CDRs). Since CDR sequences are responsible for most of the antibody-antigen interactions, antibody variants that mimic the properties of known antibodies can be constructed by engineering the variable regions. In some cases, CDR sequences from known antibodies can be grafted onto the framework regions of different antibodies with different properties, and one to several residue mutations, e.g., back mutations, may be performed as needed to refine the desired properties of the antibodies. In some other cases, the variable domains of the antibody may be engineered to construct humanized, de-immunogenic, and/or PTM (post-translational modification)-removal variants thereof. The properties of the modified antibody, including but not limited to target antigen binding properties or other desired functional properties, e.g., internalization activity, may be determined and screened *in vitro* or *in vivo* using methods known in the art and described herein. It should be understood that such functional variants of any variable regions (e.g., VH and/or VL regions, VHH regions) provided herein are contemplated by the present disclosure.

The term "complementarity-determining region" or "CDR region" or "CDR" is a region in an antibody variable domain that is highly variable in sequence and forms a structurally defined loop ("hypervariable loop") and/or comprises antigen-contacting residues ("antigen-contacting sites"). The CDRs are primarily responsible for binding to an antigenic epitope and include CDR1, CDR2, and CDR3, numbered sequentially from the N-terminus of the variable region. In a given amino acid sequence of a variable region, the exact amino acid sequence boundary of each CDR may be determined using any one or a combination of many well-known antibody CDR assignment systems including, e.g., Chothia based on the three-dimensional structure of antibodies and the topology of the CDR loops (Chothia et al., (1989) Nature, 342: 877-883; Al-Lazikani et al., "Standard conformations for the canonical structures of immunoglobulins", Journal of Molecular Biology, 273, 927-948 (1997)); Kabat based on antibody sequence variability (Kabat et al., Sequences of Proteins of Immunological Interest, 4th Ed., U.S. Department of Health and Human Services, National Institutes of Health (1987)); AbM (University of Bath); Contact (University College London), International ImMunoGeneTics database (IMGT) (http://imgt.cines.fr/); and North CDR definition based on the affinity propagation clustering using a large number of crystal structures. Unless otherwise stated, the term "CDR" or "CDR sequence" in the present disclosure encompasses CDR sequences determined by any one of the schemes described above. CDRs can also be determined based on having the same AbM or Kabat numbering positions as a reference CDR sequence (e.g., an exemplary CDR sequence of the present disclosure). In one embodiment, the CDRs of the antibody of the present disclosure are defined according to Kabat or Chothia or AbM or IMGT or Contact, or any combination thereof. In one embodiment, the CDRs of the antibody of the present disclosure are determined according to the Kabat definition scheme.

Antibodies with different specificities (i.e., targeting different antigenic epitopes) have different CDRs. However, although CDRs vary from antibody to antibody, only a limited number of amino acid positions within the CDRs are directly involved in antigen binding. The smallest overlapping region can be determined using at least two of the Kabat, Chothia, AbM, IMGT, and Contact methods, thereby providing a "minimal binding unit" for antigen binding. The minimal binding unit may be a sub-portion of the CDR. As will be clear to those skilled in the art, the residues in the remainder of the CDR sequences may be determined by the antibody structure and protein folding. Thus, variants of any of the CDRs presented herein are also contemplated by the present disclosure. For example, in a variant of one CDR, the amino acid residues of the minimal binding unit may remain unchanged, while the remaining CDR residues defined according to Kabat or Chothia or AbM or IMGT or Contact may be substituted with conservative amino acid residues.

If an amino acid sequence (e.g., an ISVD) is specific for two different antigens or antigenic determinants (e.g., EGFRs from different mammalian species, e.g., human EGFR and cynomolgus monkey EGFR, or cMets from different mammalian species, e.g., human cMet and cynomolgus monkey cMet), the sequence is referred to as "cross-reactive" for the two different antigens or antigenic determinants. It would be advantageous for the antibody to have cross-reactivity with human and monkey species, particularly to have similar antigen binding affinities for human and monkey antigens. The property may facilitate preclinical drug development of the antibody, such as toxicological detection of ADC molecules formed from antibodies. In some embodiments, the antibody of the present disclosure preferably has cross-reactivity with human and monkey species.

As used herein, the term "epitope" refers to an antigen moiety to which an antibody specifically binds. The epitope may consist of contiguous and/or noncontiguous amino acids that form conformational space units. For a noncontiguous epitope, the amino acids at different portions of the linear antigen sequence are in close proximity in a three-dimensional space by protein molecule folding. Epitope binning can be performed on different antibodies binding to the same antigen by competitive binding assays. Such competitive binding assays can be performed by methods known in the art, such as direct or indirect solid-phase radioimmunoassay (RIA), direct or indirect solid-phase enzyme immunoassay (EIA), sandwich competition assay, or using the methods described in examples herein.

"Humanized" antibody refers to a chimeric antibody comprising amino acid residues from non-human CDRs and amino acid residues from human FRs. In some embodiments, all or substantially all CDRs in the humanized antibody correspond to those of a non-human antibody, and all or substantially all FRs correspond to those of a human antibody. The humanized antibody may optionally comprise at least a portion of an antibody constant region from a human antibody. The "humanized form" of an antibody (e.g., a non-human antibody) refers to an antibody that has been humanized.

Herein, the term "half-life extension domain" and the expression "binding moiety extending the half-life" are used interchangeably and refer to a chemical structure that can endow a molecule (e.g., an antibody) to which it binds with a prolonged circulatory half-life following administration to an animal. Such chemical structures include, for example, a flexible hydrophilic molecule (e.g., a carbohydrate or PEG (polyethylene glycol)), an immunoglobulin Fc region, serum albumin, a serum albumin-binding domain, or a serum albumin-binding peptide (e.g., an anti-HSA ISVD). The half-life extension domain can be linked to the binding molecule or antibody of the present disclosure by chemical conjugation or fusion, depending on its particular properties.

In some embodiments, the EGFR-binding molecule according to the first aspect of the present disclosure, the cMet-binding molecule according to the second aspect of the present disclosure, and the EGFR- and cMet-binding molecule according to the third aspect of the present disclosure comprise an ISVD binding to human serum albumin as a half-life extension domain. In some embodiments, the ISVD binding to human serum albumin is selected from serum albumin-binding moieties of Alb-1, Alb-3, Alb-4, Alb-5, Alb-6, Alb-7, Alb-8, Alb-9, Alb-10, and Alb-23. In one embodiment, the serum albumin-binding moiety is Alb-8 or Alb-23 or a variant thereof, e.g., those shown on pages 7-9 of WO 2012/175400. In some preferred embodiments, the ISVD binding to human serum albumin comprises a CDR1 comprising or consisting of an amino acid sequence set forth in SEQ ID NO: 46, a CDR2 comprising or consisting of an amino acid sequence set forth in SEQ ID NO: 47, and a CDR3 comprising or consisting of an amino acid sequence set forth in SEQ ID NO: 48. In some embodiments, the ISVD binding to human serum albumin comprises the sequence of SEQ ID NO: 45 or a sequence having at least 90%, 91%, 92%, 93%, 94%, 95%, 96%, 97%, 98%, or 99% identity therewith.

Herein, the terms "immunoglobulin Fc region", "Fc domain", "Fc portion", and " Fc region" are used interchangeably to define a C-terminal region of an immunoglobulin heavy chain, and the region comprises at least a portion of a constant region. It is known that the heavy chain constant region of each immunoglobulin comprises four or five domains, which are named in the following order: CH1-hinge-CH2-CH3(-CH4). CH4 is present in IgM without a hinge region. In the present disclosure, the Fc domain may comprise a CH2 domain and a CH3 domain, and optionally further comprises all or part of an immunoglobulin hinge region, but does not comprise a heavy chain variable region VH and a light chain variable region VL as well as a heavy chain constant region CH1 and a light chain constant region CL of an immunoglobulin. For example, in one example, from N-terminus to C-terminus, the Fc domain may comprise or consist of a CH2 domain and a CH3 domain. In another example, from N-terminus to C-terminus, the Fc domain may comprise or consist of an immunoglobulin hinge region, a CH2 domain, and a CH3 domain. The Fc domain is often present as a dimerized form, and each Fc domain in the dimerized form is also referred to herein as an Fc subunit.

The term "Fc region" includes Fc regions of native sequences and variant Fc regions. In certain embodiments, the antibody according to the present disclosure comprises a human IgG heavy chain Fc region. In some embodiments, the human IgG heavy chain Fc region extends from Glu216, Cys226, or Pro230 to the carboxyl-terminus of the heavy chain. However, the C-terminal lysine (Lys447) of the Fc region may or may not be present. Unless otherwise stated, amino acid residues in the Fc region or constant region are numbered according to the EU numbering scheme, also known as the EU index, as described in Sequences of Proteins of Immunological Interest, 5th Ed. Public Health Service, National Institutes of Health, Bethesda, MD, 1991. In some embodiments, the Fc region is an Fc region of human IgG1, IgG2, IgG3, or IgG4. In some embodiments, the Fc region comprises a modification, relative to an Fc region of a native sequence. In some specific embodiments, the Fc region has increased or decreased effector functions. In some specific embodiments, the Fc region has increased or decreased binding to FcyR. In some specific embodiments, the Fc region comprises a heavy chain mismatch-preventing mutation, e.g., a Knob-into-hole (KIH) mutation.

In some cases, the immunoglobulin Fc region comprising a hinge region sequence is preferred, which may, for example, promote dimerization of the antibody polypeptide chains and/or provide a cysteine residue for coupling to other active molecules. Such hinge sequence may correspond substantially or partly to the hinge region of IgG1, IgG2, IgG3, or IgG4. For example, the hinge region sequence may include all or part of the core hinge region and all or part of the lower hinge region. The core hinge region has an amino acid sequence CPPC in IgG1, IgG2, and IgG3, and a CPSC sequence in IgG4. Preferably, the hinge region contains at least one disulfide bond linking the two Fc chains. In some embodiments, the hinge region sequence comprises a hinge region sequence from E216 to T225 or a hinge region sequence from D221 to T225 (according to EU numbering) of IgG1, or a corresponding hinge region sequence from other immunoglobulin isotypes. In some embodiments, the immunoglobulin single variable domain (ISVD) of the present disclosure is linked to the Fc region by a hinge sequence comprising, for example, EPKSS (SEQ ID NO: 49) or EPKSC (SEQ ID NO: 50).

The term "effector function" refers to biological activities attributed to an immunoglobulin Fc region that vary with immunoglobulin isotype. Examples of immunoglobulin effector functions include: C1q binding and complement-dependent cytotoxicity (CDC), Fc receptor binding effect, antibody-dependent cell-mediated cytotoxicity (ADCC), antibody-dependent cellular phagocytosis (ADCP), cytokine secretion, immune complex-mediated antigen uptake in antigen-presenting cells, down-regulation of cell surface receptors (such as B-cell receptors), and B-cell activation.

In the case that no effector function is required, the Fc region may comprise a mutation that reduces or eliminates the effector function. In some cases (e.g., where the antibody of the present disclosure serves as an ADC carrier), preferably the Fc region comprises a mutation that reduces or eliminates the binding of the Fc region to the Fcy receptor, e.g., a LALA mutation where the lysine (L) at positions 234 and 235 of the Fc region is mutated to alanine (A), to reduce Fcy receptor-mediated off-target cytotoxicity. Additionally or alternatively, into the Fc region, a mutation may be introduced to increase binding to FcRn and/or remove protease sites, and/or an amino acid modification that can be used for coupling to active molecules may be introduced. Additionally or alternatively, out of antibody production considerations, the Fc region may be mutated, e.g., to remove or replace amino acids that may undergo post-translational modifications (e.g., glycosylation), so as to provide therapeutic antibodies with improved potential to be manufactured as a drug and improved developability.

The term "antibody-dependent cell-mediated cytotoxicity (ADCC)" is one of the major mechanisms by which certain cytotoxic effector cells (e.g., natural killer (NK) cells) mediate the killing of target cells and foreign host cells. The Fc region of the antibody activates NK cells to exert an ADCC effect by binding to the Fc receptor FcyRIIIA (i.e., CD16a) expressed on, for example, NK cells.

The term "complement-dependent cytotoxicity (CDC)" refers to the lysis of target cells in the presence of complement. Activation of the classical complement pathway is initiated by the binding of the first component of the complement system (C1q) to an antibody (of an appropriate subclass) binding to its corresponding antigen. To assess complement activation, a CDC assay can be performed, for example, by the method described in Gazzano-Santoro et al., J. Immunol. Methods 202: 163 (1996).

"Affinity" refers to the strength of the sum of all non-covalent interactions between a single binding site of a molecule (e.g., an antibody) and its binding partner (e.g., an antigen). Unless otherwise stated, when used herein, "binding affinity" refers to the intrinsic binding affinity that reflects a 1:1 interaction between members of a binding pair (e.g., an antibody and an antigen). The affinity of a molecule X for its partner Y can generally be represented by a binding dissociation equilibrium constant (K_{D}). Affinity can be measured by common methods known in the art, including those known in the prior art and described herein.

Herein, the "percent identity (%)" of an amino acid sequence refers to the percentage of positions at which the candidate sequence has the same amino acid residues as those of the specific amino acid sequence shown in the present disclosure at the corresponding aligned positions, with gaps introduced if necessary to achieve maximum percent sequence identity and without considering any conservative replacements as part of sequence identity, while aligning the candidate sequence with the specific amino acid sequence shown in the present disclosure.

In some embodiments, the present disclosure contemplates variants of the ISVD, the binding molecule, and the antibody sequence of the present disclosure. The variants comprise amino acid alterations relative to the ISVD, the binding molecule, and the antibody sequence specifically disclosed herein. In some embodiments, in the comparison window, the variants have a considerable degree of identity relative to the ISVD, the binding molecule, and the antibody sequence specifically disclosed herein. For example, the identity is at least 80%, 85%, 90%, 95%, 97%, 98%, 99%, or higher. Herein, if no comparison window (i.e., region of interest to be compared) is designated, the alignment is performed over the full-length reference sequence.

In some embodiments of the present disclosure, the amino acid alteration described in the present disclosure includes substitutions, insertions, or deletions of amino acids. Preferably, the amino acid alteration described in the present disclosure is an amino acid substitution, preferably a conservative substitution. In a preferred embodiment, the amino acid alteration described in the present disclosure takes place in regions outside CDRs (e.g., in FRs). More preferably, the amino acid alteration described in the present disclosure takes place in a region outside the VHH. In some embodiments, the substitution is a conservative substitution. The conservative substitution refers to a substitution of an amino acid with another amino acid of the same class, for example, a substitution of an acidic amino acid with another acidic amino acid, a substitution of a basic amino acid with another basic amino acid, or a substitution of a neutral amino acid with another neutral amino acid. Exemplary substitutions are shown in Table A below:

**Table A**

| Original residue | Exemplary substitution | Preferred substitution |
|---|---|---|
| Ala (A) | Val; Leu; Ile | Val |
| Arg (R) | Lys; Gln; Asn | Lys |
| Asn (N) | Gln; His; Asp, Lys; Arg | Gln |
| Asp (D) | Glu; Asn | Glu |
| Cys (C) | Ser; Ala | Ser |
| Gln (Q) | Asn; Glu | Asn |
| Glu (E) | Asp; Gln | Asp |
| Gly (G) | Ala | Ala |
| His (H) | Asn; Gln; Lys; Arg | Arg |
| Ile (I) | Leu, Val; Met; Ala; Phe; Nle | Leu |
| Leu (L) | Nle; Ile; Val; Met; Ala; Phe | Ile |
| Lys (K) | Arg; Gln; Asn | Arg |
| Met (M) | Leu; Phe; Ile | Leu |
| Phe (F) | Trp; Leu; Val; Ile; Ala; Tyr | Tyr |
| Pro (P) | Ala | Ala |
| Ser (S) | Thr | Thr |
| Thr (T) | Val; Ser | Ser |
| Trp (W) | Tyr; Phe | Tyr |
| Tyr (Y) | Trp; Phe; Thr; Ser | Phe |
| Val (V) | Ile; Leu; Met; Phe; Ala; Nle | Leu |

Herein, an "isolated" antibody or antibody fragment refers to an artificial antibody or antibody fragment, a recombinantly produced antibody or antibody fragment, and an antibody or antibody fragment that has been at least partially separated from components in the natural environment in which it was produced. In some embodiments, the antibody (e.g., the anti-EGFR/cMet antibody) or antibody fragment (e.g., the anti-EGFR ISVD or anti-cMet ISVD) according to the present disclosure is "isolated". In some embodiments, the isolated antibody or antibody fragment is purified to a purity greater than 90%, 95%, or 99%, as determined by, e.g., electrophoresis (e.g., SDS-PAGE, isoelectric focusing (IEF), and capillary electrophoresis) or chromatography (e.g., ion exchange or reversephase HPLC).

Herein, the term "host cell" refers to a cell into which an exogenous polynucleotide has been introduced, including the progeny of such cells. Host cells include "transformants" and "transformed cells", which include primary transformed cells and progeny derived therefrom. The host cell is any type of cell system that can be used to produce the antibody molecule of the present disclosure, including eukaryotic cells, e.g., mammalian cells, insect cells, and yeast cells; and prokaryotic cells, e.g., *Escherichia coli* cells. Host cells include cultured cells, as well as cells within a transgenic animal, a transgenic plant, or a cultured plant tissue or animal tissue.

Herein, the term "expression vector" refers to a vector comprising a recombinant polynucleotide, which comprises an expression control sequence effectively linked to a nucleotide sequence to be expressed. Expression vectors contain sufficient cis-regulatory elements for expression, and other elements for expression may be provided by a host cell or in an *in vitro* expression system. Expression vectors include all those known in the art, including cosmids, plasmids (e.g., naked or encapsulated in liposomes), and viruses (e.g., lentiviruses, retroviruses, adenoviruses, and adeno-associated viruses) into which recombinant polynucleotides are incorporated.

Herein, the terms "endocytosis" and "internalization" are used interchangeably and refer to the process whereby a ligand/receptor complex is internalized and delivered into the cytosol or transferred to a suitable intracellular compartment, triggered by binding of the ligand to the corresponding receptor on the cell surface. In some embodiments, the antibody of the present disclosure triggers EGFR and/or cMet receptor-mediated endocytosis upon binding to EGFR and/or cMet expressed on the cell surface. Herein, the endocytic activity of the antibody can be characterized by measuring the endocytosis and endocytosis rate, for example, by the method described in examples. In some embodiments, the antibodies of the present disclosure with endocytic activity can be used as a tool for transporting an anti-tumor drug into cancer cells in the ADCs of the present disclosure.

Herein, the term "conjugate" refers to a molecule formed by conjugating one or more immunoglobulin-related molecules or fragments thereof to one or more other molecules. Typically, the conjugate comprises at least one nonproteinaceous chemical structural moiety, e.g., a chemical linker for achieving the conjugation. In some cases, the other molecule may be likewise an immunoglobulin-related molecule or fragment thereof. In some cases, the other molecules may be different from an immunoglobulin-related molecule or fragment thereof. The one or more other molecules may be identical or mutually different. For example, the other molecules may be target-binding elements and/or effector elements, such as chemotherapeutic agents, toxins, drugs (e.g., immunotherapeutic agents), radioelements, probes, or signaling molecules.

"Antibody-drug conjugate (ADC)" refers to a compound obtained by linking an antigen-binding molecule to a (small molecule) drug via a linker. Herein, references to an antibody-drug conjugate or an ADC include pharmaceutically acceptable salts, solvates, and other equivalent forms thereof. Herein, the drug compound moiety in an ADC may be referred to as a "payload" or a "toxin".

The term "linker" refers to a structural fragment that covalently links a drug (e.g., a small molecule drug) to an antigen-binding molecule moiety. It should be understood that the linker has a functional group that can form a bond with a functional group of the antigen-binding molecule prior to linking to the antigen-binding molecule. In some cases, the linker may also have a degradable moiety and optionally a hydrophilicity regulation module such as a PEG segment. In some embodiments of the ADC according to the present disclosure, the linker is preferably "degradable", such that it can be cleaved and release the payload upon the delivery of ADC to a target region (e.g., a target tumor tissue site). Such "degradable linkers" that may be used include, e.g., acid-labile linkers, peptidase-sensitive linkers, photolabile linkers, dimethyl linkers, or disulfide-containing linkers.

The term "linker-payload" refers to a compound formed by linking a payload to a linker. In some cases, the linker-payload is used as an intermediate for ADC synthesis.

The term "therapeutic agent" encompasses any substance effective in preventing or treating diseases such as cancer, including chemotherapeutic agents, cytotoxic agents, immunomodulatory agents (e.g., immunosuppressants), other antibodies, small molecule drugs, angiogenesis inhibitors, or cytokines.

The term "drug" refers to a compound that can modulate biological processes, particularly alter or prevent pathological processes. Herein, the drug preferably refers to an anti-tumor compound.

The term "small molecule drug" refers to a low molecular weight drug that can modulate biological processes, particularly alter or prevent pathological processes. "Small molecule" is defined as a molecule with a molecular weight of less than 10 kD, generally less than 2 kD, preferably less than 1 kD, and more preferably less than 500 D. A small molecule drug includes, but is not limited to, an organic molecule, an organic molecule containing inorganic components, a molecule containing radioactive atoms, a synthetic molecule, a peptidomimetic, and an antibody mimetic with the molecular weights as defined above. Compared to large molecules, small molecules, as therapeutic agents, penetrate cells better, are less susceptible to degradation, and are less likely to elicit an immune response.

"Anti-tumor compound" is a pharmaceutically active compound that has an effect on tumors, including but not limited to cytotoxic agents or chemotherapeutic agents, e.g., the cytotoxic agents disclosed in WO2021/173773 and US5658920; camptothecin compounds, e.g., exatecan and Dxd (an exatecan derivative); auristatin compounds, e.g., monomethyl auristatin E (MMAE) and MMAF.

The term "cytotoxic agent" used in the present disclosure refers to a substance that inhibits or prevents cell functions and/or elicits cell death or cell destruction.

"Chemotherapeutic agents" include chemical compounds useful in the treatment of cancers or immune system diseases.

The term "alkyl" described herein refers to a fully saturated branched or unbranched hydrocarbon group. The alkyl preferably contains 1-16 carbon atoms, e.g., 1-12 carbon atoms, 1-10 carbon atoms, 1-6 carbon atoms, or 1-4 carbon atoms. Representative examples of alkyl include, but are not limited to, methyl, ethyl, n-propyl, isopropyl, n-butyl, sec-butyl, isobutyl, tert-butyl, n-pentyl, isopentyl, neopentyl, n-hexyl, 3-methylhexyl, 2,2-dimethylpentyl, 2,3-dimethylpentyl, n-heptyl, n-octyl, n-nonyl, n-decyl, and the like.

The term "alkenyl" refers to a linear or branched hydrocarbon group containing 2-16 carbon atoms and containing at least one double bond and no triple bonds. The alkenyl group preferably contains 2-12 carbon atoms, 2-10 carbon atoms, 2-8 carbon atoms, 2-6 carbon atoms, or 2-4 carbon atoms. Representative examples of alkenyl include, but are not limited to, ethenyl, propenyl, butenyl, pentenyl, hexenyl, and the like.

The term "alkynyl" refers to a linear or branched hydrocarbon group containing 2-16 carbon atoms and containing at least one triple bond. The alkynyl group preferably contains 2-12 carbon atoms, 2-10 carbon atoms, 2-8 carbon atoms, 2-6 carbon atoms, or 2-4 carbon atoms. Representative examples of alkynyl include, but are not limited to, ethynyl, propynyl, butynyl, pentynyl, hexynyl, and the like.

The term "halogen" or "halo" refers to fluorine (-F), chlorine (-Cl), bromine (-Br), or iodine (-I).

The term "haloalkyl" refers to alkyl, as defined herein, substituted with one or more halogen groups as defined herein. The haloalkyl may preferably be a monohaloalkyl, a dihaloalkyl or a polyhaloalkyl (including perhaloalkyl). The monohaloalkyl group may contain one iodine, bromine, chlorine or fluorine in the alkyl. The dihaloalkyl and polyhaloalkyl groups may contain two or more identical halogen atoms in the alkyl or contain a combination of different halo groups. Preferably, the polyhaloalkyl comprises up to 12, 10 or 8 or 6 or 4 or 3 or 2 halogen groups. Non-limiting examples of haloalkyl include fluoromethyl, difluoromethyl, trifluoromethyl, chloromethyl, dichloromethyl, trichloromethyl, pentafluoroethyl, heptafluoropropyl, difluorochloromethyl, dichlorofluoromethyl, difluoroethyl, difluoropropyl, dichloroethyl, and dichloropropyl. The perhaloalkyl refers to alkyl in which all hydrogen atoms are replaced by halogen atoms.

The term "haloalkenyl" refers to alkenyl, as defined herein, substituted with one or more halogen groups as defined herein. The term "haloalkynyl" refers to alkynyl, as defined herein, substituted with one or more halogen groups as defined herein. The meaning of "halo" as defined for "haloalkyl" is applicable to "haloalkenyl" and "haloalkynyl".

The terms "alkoxy" and "alkyl-O-" are used interchangeably, and refer to the alkyl group as defined above linked via an oxygen atom. Preferably, the alkoxy has 1-8 carbon atoms (C₁₋₈ alkoxy), 1-6 carbon atoms (C₁₋₆ alkoxy), 1-4 carbon atoms (C₁₋₄ alkoxy), or 1-3 carbon atoms (C₁₋₃ alkoxy). Examples of alkoxy include, but are not limited to, methoxy, ethoxy, propoxy (including n-propoxy and isopropoxy), butoxy (including n-butoxy, sec-butoxy, isobutoxy, tert-butoxy, and the like), pentyloxy (including n-pentyloxy, isopentyloxy, neopentyloxy, and the like), hexyloxy, heptyloxy, octyloxy, and the like.

The term "amino acid" refers to naturally occurring and synthetic amino acids, amino acid analogs, and artificially modified forms thereof. The amino acids may be either L isomers or D isomers. In the present disclosure, the 20 natural amino acids are represented by single-letter and three-letter abbreviations well known in the art, e.g.: phenylalanine (Phe; F), tyrosine (Tyr; Y), leucine (Leu; L), glycine (Gly; G), alanine (Ala; A), valine (Val; V), lysine (Lys; K), serine (Ser; S), glutamic acid (Glu; E), aspartic acid (Asp; D), asparagine (Asn; N), isoleucine (Ile; I), arginine (Arg; R), proline (Pro; P), and glutamine (Gln; Q). The remaining amino acids are represented by full names or multi-letter abbreviations known in the art. For example, citrulline may be represented by Cit; cyclobutane-1,1-dicarboxamide-citrulline is represented by cBu-Cit. Unless otherwise specified, the amino acids of the present disclosure refer to L-amino acids.

The term "penturonic acid" refers to a compound formed by oxidation of the primary hydroxyl of a pentose sugar as defined above to carboxyl. Examples of penturonic acids include, but are not limited to, xyluronic acid and arabinuronic acid.

The term "hexuronic acid" refers to a compound formed by oxidation of the primary hydroxyl of a hexose sugar as defined above to carboxyl. Examples of hexuronic acid include, but are not limited to, glucuronic acid, galacturonic acid, and mannuronic acid.

The term "optional" or "optionally" means that the subsequently described event or circumstance occurs or does not occur, and the description includes instances where the event or circumstance occurs and instances where the event or circumstance does not occur. For example, when a group or structure is "optionally substituted", the group or structure may or may not be substituted.

Herein, "pharmaceutically acceptable" refers to a case where a substance can be administered to an individual or subject without eliciting biologically or otherwise undesirable side effects, e.g., those serious and intolerable ones. Herein, "pharmaceutically acceptable" and "pharmaceutical usable" are used interchangeably, provided that there is no contradiction according to the context.

The term "pharmaceutically acceptable salt" refers to salts that retain the biological effects and performances of the ADCs of the present disclosure and are not biologically or otherwise undesired. The ADCs of the present disclosure may exist in their pharmaceutically acceptable salt forms, including acid addition salts and base addition salts. In the present disclosure, pharmaceutically acceptable non-toxic acid addition salts refer to salts formed by the ADCs of the present disclosure and organic or inorganic acids including, but not limited to, hydrochloric acid, sulfuric acid, hydrobromic acid, hydroiodic acid, phosphoric acid, nitric acid, perchloric acid, acetic acid, oxalic acid, maleic acid, fumaric acid, tartaric acid, benzenesulfonic acid, methanesulfonic acid, salicylic acid, succinic acid, citric acid, lactic acid, propionic acid, benzoic acid, p-toluenesulfonic acid, malic acid, and the like. Pharmaceutically acceptable non-toxic base addition salts refer to salts formed by the ADCs of the present disclosure and organic or inorganic bases including, but not limited to, alkali metal salts (e.g., lithium, sodium, or potassium salts), alkaline earth metal salts (e.g., calcium or magnesium salts), and organic base salts (e.g., ammonium salts formed by reaction with N group-containing organic bases).

The term "solvate" refers to an association compound formed by one or more solvent molecules and the antibody-drug conjugate (ADC) of the present disclosure. Solvents for forming solvates include, but are not limited to, water, methanol, ethanol, isopropanol, ethyl acetate, tetrahydrofuran, N,N-dimethylformamide, dimethylsulfoxide, and the like.

The term "drug-to-antibody ratio" or "DAR" refers to the ratio of the drug moiety (D) conjugated to an Ab moiety described herein to the Ab moiety. In some embodiments described herein, DAR may be determined by p in formula I. For example, the DAR may be 1-16, e.g., 2-16, 4-16, 5-12, 6-10, 2-8, 3-8, 2-6, 4-6, or 6-10, e.g., 2, 3, 4, 5, 6, 7, 8, 9, 10, 11, 12, 13, 14, or 15. DAR may also be calculated as the average DAR of the molecular population in the product, i.e., the overall ratio of the drug moiety (D) conjugated to the Ab moiety described herein to the Ab moiety in the product as measured by assay methods (e.g., by conventional methods such as mass spectrometry, ELISA, electrophoresis, and/or HPLC), and such a DAR is referred to herein as average DAR. In some embodiments, the average DAR value of the conjugate of the present disclosure is 1-16, e.g., 2-16, 4-16, 5-12, 6-10, 2-8, 3-8, 2-6, 4-6, or 6-10; e.g., 1.0-8.0 or 2.0-6.0; e.g., 0.5, 0.6, 0.7, 0.8, 0.9, 1, 1.1, 1.2, 1.3, 1.4, 1.5, 1.6, 1.7, 1.8, 1.9, 2, 2.1, 2.2, 2.3, 2.4, 2.5, 2.6, 2.7, 2.8, 2.9, 3, 3.1, 3.2, 3.3, 3.4, 3.5, 3.6, 3.7, 3.8, 3.9, 4, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, 8, 8.1, 8.2, 8.3, 8.4, 8.5, 8.6, 8.7, 8.8, 8.9, 9.0, 9.1, 9.2, 9.3, 9.4, 9.5, 9.6, 9.7, 9.8, 9.9, or 10.0, and ranges with two of these values as endpoints. It should be understood that when referring to average DAR values, an ADC of the present disclosure refers to a population of ADC molecules or mixture of ADC molecules comprising ADC molecules having the same and/or different DARs.

The term "half maximal effective concentration (EC₅₀)" refers to the concentration of a drug, an antibody, an ADC, or a toxin that induces a response of 50% between the baseline and the maximum after a particular exposure time. In the context of the present application, EC₅₀ is expressed in nM.

The term "fluorescence activated cell sorting" or "FACS" refers to a specialized type of flow cytometry. It provides a method for sorting a heterogeneous mixture of biological cells, one cell at a time, into two or more containers based on the specific light scattering and fluorescence characteristics of each cell (FlowMetric. "Sorting Out Fluorescence Activated Cell Sorting". 2017-11-09). Instruments for performing FACS are known to those skilled in the art and are commercially available to the public. Examples of such instruments include FACS Star Plus, FACScan and FACSort instruments from Becton Dickinson (Foster City, CA), Epics C from Coulter Epics Division (Hialeah, FL), and MoFlo from Cytomation (Colorado Springs, Colorado).

The term "pharmaceutical composition" refers to a composition that exists in a form allowing effective biological activity of the active ingredient contained therein and does not contain additional ingredients having unacceptable toxicity to a subject to which the composition is administered.

The term "pharmaceutical excipient" refers to a diluent, an adjuvant (e.g., Freund's adjuvant (complete and incomplete)), a carrier, a stabilizer, or the like, that is administered together with an active substance.

The term "pharmaceutical combination", "combination product", "pharmaceutical union" or "united product" refers to a non-fixed combination product or a fixed combination product, including but not limited to, a kit and a pharmaceutical composition. The term "non-fixed combination" means that the active ingredients (e.g., (i) the antigen-binding molecule or ADC molecule of the present disclosure, including a pharmaceutically acceptable salt thereof,, and (ii) an additional therapeutic agent) are administered, either simultaneously or sequentially (without specific time limitation or at identical or different time intervals), to a patient as separate entities, wherein such administration provides two or more prophylactically or therapeutically effective active agents in the patient. In some embodiments, the antigen-binding molecule or ADC molecule of the present disclosure and the additional therapeutic agent used in the pharmaceutical combination are administered at levels that do not exceed their levels when used alone. The term "fixed combination" means that two or more active agents are administered to a patient simultaneously in the form of a single entity. The dose and/or time intervals of two or more active agents are preferably selected such that the combined use of the ingredients can result in a therapeutic effect on the disease or condition which is greater than that achieved by the use of any one of the ingredients alone. The ingredients may each take a separate formulation form, and such separate formulation forms may be the same or different.

The terms "individual" and "subject" are used interchangeably and include mammals. The mammals include, but are not limited to, domesticated animals (e.g., cows, sheep, cats, dogs, and horses), primates (e.g., humans and non-human primates such as monkeys), rabbits, and rodents (e.g., mice and rats). Particularly, the individual or subject is a human.

The terms "tumor" and "cancer" are used interchangeably herein and refer to a physiological disease in mammals that is characterized by unregulated cell growth, and encompass solid tumors and liquid tumors, malignant tumors and benign tumors, as well as all pre-cancerous and cancerous cells and tissues.

Herein, the term "treat", "treating" or "treatment" refers to clinical intervention intending to alter the natural progress of a disease in an individual being treated. Desired therapeutic effects include, but are not limited to, preventing occurrence or recurrence of diseases, alleviating symptoms, reducing any direct or indirect pathological outcomes of the diseases, preventing metastasis, delaying disease progression, ameliorating or alleviating conditions, and alleviating or improving prognosis. Where tumor or cancer therapy is involved, "treat", "treating" or "treatment" encompasses anti-tumor biological effects that can be caused by human intervention (e.g., by administration of drugs), including but not limited to, for example, decrease in tumor volume, decrease in number of tumor cells, decrease in tumor cell proliferation, or decrease in tumor cell viability.

As used herein, "prevention", "prevent", or "preventing" includes the inhibition of the development or progression of symptoms of a disease or condition, or a specific disease or condition. In some embodiments, subjects with family history of cancer are candidates for preventive regimens. Generally, in the context of cancer, the term "prevention", "prevent", or "preventing" refers to the administration of a drug prior to the onset of signs or symptoms of cancer, particularly in subjects at risk of cancer.

The term "effective amount" refers to an amount or a dose of the antigen-binding molecule, the ADC molecule, the composition, or the combination of the present disclosure that generates expected effects in a patient in need of treatment or prevention after being administered to the patient in a single dose or multiple doses. A "therapeutically effective amount" and a "prophylactically effective amount" may be included, depending on desired effects.

The term "therapeutically effective amount" refers to an amount effective to achieve a desired therapeutic result at a necessary dose for a necessary period of time. The therapeutically effective amount of an antibody or an ADC may vary depending on a variety of factors, such as the disease state, the age, sex, and weight of an individual, and the ability of the antibody or the ADC to elicit a desired response in the individual. The therapeutically effective amount is also such an amount that any toxic or undesired effect of the antibody or the ADC does not outweigh the therapeutically beneficial effect. "Therapeutically effective amount" preferably inhibits a measurable parameter (e.g., tumor growth rate, tumor volume, etc.) by at least about 20%, more preferably at least about 40%, even more preferably at least about 50%, 60%, or 70%, and still more preferably at least about 80% or 90%, relative to an untreated subject. The ability of a compound to inhibit a measurable parameter (e.g., cancer) can be evaluated in an animal model system that predicts efficacy in human tumors.

"Prophylactically effective amount" refers to an amount effective to achieve a desired prophylactic result at a necessary dose for a necessary period of time. Generally, since a prophylactic dose is used in subjects before or at an earlier stage of a disease, the prophylactically effective amount is less than the therapeutically effective amount.

The term "anti-tumor effect" refers to a biological effect that can be demonstrated by a variety of means, including but not limited to, for example, decrease in tumor volume, decrease in number of tumor cells, decrease in tumor cell proliferation, or decrease in tumor cell viability.

The present disclosure will be described in detail below. It will be understood by those skilled in the art that unless otherwise expressly stated to the contrary, any technical feature described in any of the following sections, subsections, or embodiments may be combined with any technical feature described in any other section, subsection, or embodiment, and these combinations are contemplated in the present disclosure.

### I. First aspect of the present disclosure: ISVD specifically binding to cMet and cMet-binding molecule comprising the ISVD

cMet (or c-Met) is the gene product of the proto-oncogene MET, which is encoded on chromosome 7. cMet recognizes only one known ligand, hepatocyte growth factor (HGF). The cMet protein is a receptor tyrosine kinase that is overexpressed or mutated in many tumor cell types and plays a key role in the proliferation, survival, invasion, and metastasis of tumor cells and tumor angiogenesis. Inhibition of cMet may induce cell death in tumor cells that overexpress the cMet protein or that express a constitutively activated cMet protein.

A first aspect of the present disclosure provides an ISVD specifically binding to cMet (i.e., an anti-cMet ISVD) and a heavy-chain antibody and a cMet-binding molecule that comprise the ISVD. In some embodiments, the anti-cMet ISVD, the heavy-chain antibody, and the cMet-binding molecule of the present disclosure bind to human cMet with moderate or high affinity. In some embodiments, the anti-cMet ISVD and the cMet-binding molecule of the present disclosure exhibit improved tissue permeability compared to conventional four-chain antibodies.

The ISVD specifically binding to cMet of the present disclosure comprises, from N-terminus to C-terminus, three complementarity-determining regions CDR1, CDR2, and CDR3.

In some embodiments, the immunoglobulin single variable domain (ISVD) specifically binding to cMet of the present disclosure comprises
(a) three CDRs contained in an amino acid sequence set forth in any one of SEQ ID NOs: 16, 39-40, and 121-130;
(b) three CDRs contained in an amino acid sequence set forth in any one of SEQ ID NO: 21and SEQ ID NOs: 42 and 134-136; or
(c) three CDRs contained in an amino acid sequence set forth in SEQ ID NO: 26. In some embodiments, the ISVD specifically binding to cMet of the present disclosure comprises a variant that, as compared to the three CDRs described in one of (a)-(c) above, has amino acid alterations in one or more CDRs, with no more than 1 to 3 amino acid alterations permitted per CDR, wherein the amino acid alterations are amino acid additions or deletions, or conservative amino acid substitutions. In some embodiments, the CDRs according to the present disclosure are defined according to AbM, Chothia, Kabat, IMGT, or any combination thereof, preferably according to Kabat or AbM, or a combination thereof.

In some embodiments, the ISVD specifically binding to cMet of the present disclosure comprises the following according to the Kabat numbering scheme:
(a) a CDR1 set forth in SEQ ID NO: 18 or a variant of the CDR1 set forth in SEQ ID NO: 18 with no more than 1 amino acid alteration (e.g., a CDR1 set forth in any one of SEQ ID NOs: 41 and 131-133), a CDR2 set forth in SEQ ID NO: 19 or a variant of the CDR2 set forth in SEQ ID NO: 19 with no more than 2 amino acid alterations, and a CDR3 set forth in SEQ ID NO: 20 or a variant of the CDR3 set forth in SEQ ID NO: 20 with no more than 2 amino acid alterations;
(b) a CDR1 set forth in SEQ ID NO: 23 or a variant of the CDR1 set forth in SEQ ID NO: 23 with no more than 1 amino acid alteration, a CDR2 set forth in SEQ ID NO: 24 or a variant of the CDR2 set forth in SEQ ID NO: 24 with no more than 2 amino acid alterations, and a CDR3 set forth in SEQ ID NO: 25 or a variant of the CDR3 set forth in SEQ ID NO: 25 with no more than 2 amino acid alterations; or
(c) a CDR1 set forth in SEQ ID NO: 28 or a variant of the CDR1 set forth in SEQ ID NO: 28 with no more than 1 amino acid alteration, a CDR2 set forth in SEQ ID NO: 29 or a variant of the CDR2 set forth in SEQ ID NO: 29 with no more than 2 amino acid alterations, and a CDR3 set forth in SEQ ID NO: 30 or a variant of the CDR3 set forth in SEQ ID NO: 30 with no more than 2 amino acid alterations;
wherein the amino acid alteration is an amino acid addition or deletion, or a conservative amino acid substitution.

In some embodiments, the present disclosure provides an immunoglobulin single variable domain (ISVD) specifically binding to cMet, wherein the ISVD comprises:
(a) a CDR1, a CDR2, and a CDR3 comprising or consisting of SEQ ID NOs: 18, 19, and 20, respectively;
(b) a CDR1, a CDR2, and a CDR3 comprising or consisting of SEQ ID NOs: 41, 19, and 20, respectively;
(c) a CDR1, a CDR2, and a CDR3 comprising or consisting of SEQ ID NOs: 131, 19, and 20, respectively;
(d) a CDR1, a CDR2, and a CDR3 comprising or consisting of SEQ ID NOs: 132, 19, and 20, respectively;
(e) a CDR1, a CDR2, and a CDR3 comprising or consisting of SEQ ID NOs: 133, 19, and 20, respectively; or
(f) a CDR1, a CDR2, and a CDR3 comprising or consisting of SEQ ID NOs: 23, 24, and 25, respectively. In some embodiments, the ISVD as defined under (b) or (f) above is preferred.

In some embodiments, the ISVD specifically binding to cMet of the present disclosure comprises or consists of a VHH. In some embodiments, the ISVD specifically binding to cMet of the present disclosure comprises or consists of the following sequence:
(a) a sequence set forth in any one of SEQ ID NOs: 16, 39-40, and 121-130, or a sequence having at least 90%, 91%, 92%, 93%, 94%, 95%, 96%, 97%, 98%, or 99% identity therewith;
(b) a sequence set forth in any one of SEQ ID NOs: 21, 42, and 134-136, or a sequence having at least 90%, 91%, 92%, 93%, 94%, 95%, 96%, 97%, 98%, or 99% identity therewith; or
(c) a sequence set forth in SEQ ID NO: 26, or a sequence having at least 90%, 91%, 92%, 93%, 94%, 95%, 96%, 97%, 98%, or 99% identity therewith;
preferably, the amino acid alterations do not occur in the CDRs. In some embodiments, the ISVD comprising the amino acid sequence of SEQ ID NO: 39, 40, or 42 is preferred.

In some embodiments, the present disclosure provides an antibody comprising the ISVD specifically binding to cMet of the present disclosure, particularly an anti-cMet heavy-chain antibody.

In some embodiments, the present disclosure provides a binding molecule comprising the ISVD specifically binding to cMet of the present disclosure. In some embodiments, the cMet-binding molecule comprises or consists of an antibody selected from: a single-domain antibody, a nanobody, a VHH antibody, and a heavy-chain antibody. In some other embodiments, the cMet-binding molecule is selected from a monospecific antibody, a bispecific antibody, and a multispecific antibody.

In some embodiments, the cMet-binding molecule of the present disclosure comprises at least one ISVD specifically binding to cMet of the present disclosure. For example, the cMet-binding molecule comprises two, three, four, or more identical or different ISVDs specifically binding to cMet of the present disclosure; preferably, the cMet-binding molecule comprises two, three, or four different ISVDs specifically binding to cMet of the present disclosure. In some embodiments, the cMet-binding molecule provided in the present disclosure comprises two ISVDs binding to different epitopes of cMet.

In some embodiments, the anti-cMet ISVD comprised in the cMet-binding molecule of the present disclosure is preferably a humanized VHH domain. Compared with a *Camelidae* VHH, the humanized VHH exhibits reduced human anti-Camelidae antibody response in human body, enhancing the safety of antibody application.

In some embodiments, the ISVD specifically binding to cMet or the cMet-binding molecule of the present disclosure exhibits one or more of the following properties:
(1) binding to human cMet with moderate or high affinity;
(2) specifically binding to cMet expressed on the cell surface;
(3) blocking binding of ligand HGF, when present, to cMet on the cell surface;
(4) being internalized by cells expressing cMet;
(5) having cross-reactivity with human cMet and cynomolgus monkey cMet;
(6) having synergistic binding mediated by different dual epitopes of cMet; and
(7) having synergistic endocytosis mediated by different dual epitopes of cMet.

In some embodiments, the cMet-binding molecule of the present disclosure is in the form of a monospecific antibody molecule, a bispecific antibody molecule, or a multispecific antibody molecule. The multispecific antibody molecule may be, for example, a trispecific antibody molecule comprising a first binding specificity for cMet and second and third binding specificities for one or more other molecules.

In some embodiments, the cMet-binding molecule of the present disclosure comprises a first ISVD and a second ISVD specifically binding to the same epitope on cMet. In some embodiments, the first ISVD and the second ISVD are the ISVDs according to the present disclosure that specifically bind to the same epitope on cMet. In some further embodiments, the first ISVD and the second ISVD comprise: (i) a CDR1 comprising or consisting of an amino acid sequence selected from SEQ ID NOs: 18 and 41, and a CDR2 and a CDR3 comprising or consisting of amino acid sequences of SEQ ID NO:19 and SEQ ID NO:20, respectively; or (ii) a CDR1, a CDR2, and a CDR3 comprising or consisting of amino acid sequences of SEQ ID NOs: 23-25, respectively. In some further embodiments, the first ISVD and the second ISVD comprise, consist essentially of, or consist of an amino acid sequence having at least 90%, 91%, 92%, 93%, 94%, 95%, 96%, 97%, 98%, or 99% identity to an amino acid sequence set forth in any one of SEQ ID NO: 16, 39 or 40 or any one of SEQ ID NO: 21 or 42. In some preferred embodiments, the first ISVD and the second ISVD comprise, consist essentially of, or consist of an amino acid sequence of SEQ ID NO: 16, 39, or 40, or an amino acid sequence of SEQ ID NO: 21 or 42.

In some embodiments, the cMet-binding molecule of the present disclosure comprises a first ISVD and a second ISVD specifically binding to different epitopes on cMet. In some embodiments, the first ISVD and the second ISVD are the ISVDs according to the present disclosure that specifically bind to different epitopes on cMet. In some embodiments, the first ISVD comprises a first anti-cMet VHH domain and the second ISVD comprises a second anti-cMet VHH domain, or vice versa, wherein the first and second cMet VHH domains are different from each other. In some embodiments, the first anti-cMet VHH domain comprises: a CDR1, a CDR2, and a CDR3 comprising or consisting of amino acid sequences of SEQ ID NOs: 23-25, respectively, and the second anti-cMet VHH domain comprises: a CDR1 comprising or consisting of an amino acid sequence selected from SEQ ID NOs: 18 and 41, and a CDR2 and a CDR3 comprising or consisting of amino acid sequences of SEQ ID NO: 19 and SEQ ID NO: 20, respectively. In some further embodiments, the first anti-cMet VHH domain comprises, consists essentially of, or consists of an amino acid sequence having at least 90%, 91%, 92%, 93%, 94%, 95%, 96%, 97%, 98%, or 99% identity to an amino acid sequence set forth in SEQ ID NO: 21 or 42, and the second anti-cMet VHH domain comprises, consists essentially of, or consists of an amino acid sequence having at least 90%, 91%, 92%, 93%, 94%, 95%, 96%, 97%, 98%, or 99% identity to an amino acid sequence set forth in SEQ ID NO: 16, 39, or 40. In some further embodiments, the first anti-cMet VHH domain comprises, consists essentially of, or consists of an amino acid sequence of SEQ ID NO: 21 or 42, and the second anti-cMet VHH domain comprises, consists essentially of, or consists of an amino acid sequence of SEQ ID NO: 16, 39, or 40. In some preferred embodiments, the first ISVD comprises the first anti-cMet VHH domain, and the second ISVD comprises the second anti-cMet VHH domain. In some embodiments, the binding molecule is an anti-cMet biepitopic antibody.

In some embodiments, the cMet-binding molecule of the present disclosure may be in single-chain or multi-chain form. In some embodiments, the binding molecule further comprises a peptide linker for linking different domains (e.g., two or more ISVDs) located on the same polypeptide chain. In some other embodiments, the binding molecule further comprises an immunoglobulin Fc region.

In some embodiments, the present disclosure provides an anti-cMet biepitopic antibody, wherein the antibody comprises a first polypeptide chain and a second polypeptide chain, wherein, from N-terminus to C-terminus,
the first polypeptide chain comprises: a first ISVD specifically binding to cMet, and an immunoglobulin Fc region;
the second polypeptide chain comprises: a second ISVD specifically binding to cMet, and an immunoglobulin Fc region;
preferably,
   - the first polypeptide chain comprises a sequence of SEQ ID NO: 88, or a sequence having at least 90%, 91%, 92%, 93%, 94%, 95%, 96%, 97%, 98%, or 99% identity therewith; and
   - the second polypeptide chain comprises a sequence of SEQ ID NO: 89, or a sequence having at least 90%, 91%, 92%, 93%, 94%, 95%, 96%, 97%, 98%, or 99% identity therewith;
more preferably, the first polypeptide chain comprises or consists of a sequence of SEQ ID NO: 88, and the second polypeptide chain comprises or consists of a sequence of SEQ ID NO: 89.

In some embodiments, the cMet-binding molecule of the present disclosure further comprises at least one ISVD specifically binding to EGFR, preferably at least one (e.g., 1) anti-EGFR ISVD according to the present disclosure.

In some embodiments, the cMet-binding molecule of the present disclosure is linked, at its N-terminus or C-terminus, to one or more other groups, residues, or moieties via one or more peptide linkers, wherein as compared to a corresponding cMet-binding molecule lacking the linked one or more other groups, residues, or moieties, the cMet-binding molecule of the present disclosure exhibits the following properties provided by the one or more other groups, residues, or moieties: an extended half-life and effector functions, such as antibody-dependent cellular cytotoxicity (ADCC) and complement-dependent cytotoxicity (CDC).

In some embodiments, the one or more other groups, residues, moieties providing the extended half-life are selected from a polyethylene glycol molecule, a serum protein or a fragment thereof, a moiety that can bind to a serum protein (e.g., serum albumin (such as human serum albumin)), a moiety that binds to a serum immunoglobulin (such as IgG), and an Fc domain.

In some embodiments, the cMet-binding molecule of the present disclosure is linked, at its N-terminus or C-terminus and via one or more peptide linkers, to an ISVD binding to human serum albumin. In some embodiments, the ISVD binding to human serum albumin comprises a CDR1 comprising or consisting of an amino acid sequence set forth in SEQ ID NO: 46, a CDR2 comprising or consisting of an amino acid sequence set forth in SEQ ID NO: 47, and a CDR3 comprising or consisting of an amino acid sequence set forth in SEQ ID NO: 48. In some embodiments, the ISVD binding to human serum albumin comprises the sequence of SEQ ID NO: 45 or a sequence having at least 90%, 91%, 92%, 93%, 94%, 95%, 96%, 97%, 98%, or 99% identity therewith.

In certain embodiments, the cMet-binding molecule provided herein is modified to increase or decrease the degree of glycosylation thereof. The addition or deletion of glycosylation sites of the cMet-binding molecule can be conveniently achieved by changing the amino acid sequence to create or remove one or more glycosylation sites. When the cMet-binding molecule comprises an Fc region, the carbohydrate attached to the Fc region can be changed. In some applications, modifications that remove undesired glycosylation sites may be useful, for example, removing fucose modules to enhance antibody-dependent cell-mediated cytotoxicity (ADCC) (see Shield et al., (2002) JBC277:26733). In other applications, galactosylation modifications can be made to regulate complement-dependent cytotoxicity (CDC). In certain embodiments, one or more amino acid modifications can be introduced into the Fc region of the cMet-binding molecule provided herein, thereby producing an Fc region variant to enhance, for example, the effectiveness of the cMet-binding molecule of the present disclosure in the treatment of cancer.

### II. Second aspect of the present disclosure: ISVD specifically binding to EGFR and EGFR-binding molecule comprising the ISVD

The epidermal growth factor receptor (EGFR, ErbB1 or HER1) is a type I transmembrane glycoprotein encoded by the c-erbBl proto-oncogene. EGFR is a member of the human epidermal growth factor receptor (HER) family of receptor tyrosine kinases (RTKs), and the family members include HER2 (ErbB2), HER3 (ErbB3), and HER4 (ErbB4). Increased expression or kinase activity of EGFR is associated with a variety of human cancers, and therefore, EGFR is an attractive target for the treatment of cancer.

In this description, "EGFR" refers to EGFR from any species and includes EGFR isotypes, fragments, variants, or homologs from any species. Human EGFR is the protein shown in UniProt P00533. Alternative splicing of the mRNA encoded by the human EGFR gene produces four isotypes: isotype 1 to isotype 4. EGFR is a transmembrane protein and comprises a large extracellular region, a single transmembrane domain, an intracellular juxtamembrane domain, a tyrosine kinase domain, and a C-terminal regulatory region. The binding of EGFR to a ligand induces receptor dimerization and autophosphorylation of several tyrosine residues (Y992, Y1045, Y1068, Y1148, and Y1173) in the C-terminal regulatory region of EGFR. Aberrant EGFR expression/activity is associated with many diseases, e.g., cancer.

In a second aspect, the present disclosure provides an ISVD specifically binding to EGFR (anti-EGFR ISVD) and an EGFR-binding molecule comprising the ISVD. In some embodiments, the anti-EGFR ISVD and the EGFR-binding molecule of the present disclosure exhibit improved tissue permeability compared to conventional four-chain antibodies. In some embodiments, the anti-EGFR ISVD and the EGFR-binding molecule of the present disclosure bind to human EGFR with moderate or low affinity. Herein, "moderate affinity" means that the binding affinity K_{D} value of an antibody for a target epitope is equal to or greater than 1 nM, but less than 50 nM, e.g. as determined by the surface plasmon resonance (SPR) technique; "low affinity" means that the binding affinity K_{D} value of an antibody for a target epitope is greater than 50 nM. In some embodiments, compared to a "high-affinity" anti-EGFR ISVD or an EGFR-binding molecule comprising the ISVD having a K_{D} value below 1 nM, particularly below 0.1 nM, the moderate- or low-affinity anti-EGFR ISVD or the EGFR-binding molecule comprising the ISVD of the present disclosure exhibits at least one of the following advantages: (i) showing increased specificity for tumor tissues; (ii) showing reduced on-target toxicity, e.g., skin toxicity, in normal tissues; (iii) having improved safety; and (iv) being more effective in treating cancer.

The immunoglobulin single variable domain (ISVD) specifically binding to EGFR of the present disclosure comprises, from N-terminus to C-terminus, three complementarity-determining regions CDR1, CDR2, and CDR3. In some embodiments, the immunoglobulin single variable domain (ISVD) specifically binding to EGFR of the present disclosure comprises
(a) three CDRs contained in an amino acid sequence set forth in any one of SEQ ID NO: 1 and SEQ ID NOs: 31 and 94-99;
(b) three CDRs contained in an amino acid sequence set forth in any one of SEQ ID NOs: 6, 32, and 100-102; or
(c) three CDRs contained in an amino acid sequence set forth in any one of SEQ ID NOs: 11, 36, 84, and 105-114.

In some embodiments, the ISVD specifically binding to EGFR of the present disclosure comprises a variant that, as compared to the three CDRs described in one of (a)-(c) above, has amino acid alterations in one or more CDRs, with no more than 1 to 3 amino acid alterations permitted per CDR, wherein the amino acid alterations are amino acid additions or deletions, or conservative amino acid substitutions. In some embodiments, the CDRs according to the present disclosure are defined according to AbM, Chothia, Kabat, IMGT, or any combination thereof, preferably according to Kabat or AbM, or a combination thereof.

In some embodiments, the ISVD specifically binding to EGFR of the present disclosure comprises the following according to the Kabat numbering scheme:
(a) a CDR1 set forth in SEQ ID NO: 3 or a variant of the CDR1 set forth in SEQ ID NO: 3 with no more than 1 amino acid alteration, a CDR2 set forth in SEQ ID NO: 4 or a variant of the CDR2 set forth in SEQ ID NO: 4 with no more than 2 amino acid alterations, and a CDR3 set forth in SEQ ID NO: 5 or a variant of the CDR3 set forth in SEQ ID NO: 5 with no more than 2 amino acid alterations;
(b) a CDR1 set forth in SEQ ID NO: 8 or a variant of the CDR1 set forth in SEQ ID NO: 8 with no more than 1 amino acid alteration, a CDR2 set forth in SEQ ID NO: 9 or a variant of the CDR2 set forth in SEQ ID NO: 9 with no more than 2 amino acid alterations (e.g., a CDR2 set forth in SEQ ID NO: 34 or 103), and a CDR3 set forth in SEQ ID NO: 10 or a variant of the CDR3 set forth in SEQ ID NO: 10 with no more than 2 amino acid alterations (e.g., a CDR3 set forth in SEQ ID NO: 35 or 104); or
(c) a CDR1 set forth in SEQ ID NO: 13 or a variant of the CDR1 set forth in SEQ ID NO: 13 with no more than 1 amino acid alteration, a CDR2 set forth in SEQ ID NO: 14 or a variant of the CDR2 set forth in SEQ ID NO: 14 with no more than 2 amino acid alterations (e.g., a CDR2 set forth in SEQ ID NO: 38 or any one of SEQ ID NOs: 85 and 115-120), and a CDR3 set forth in SEQ ID NO: 15 or a variant of the CDR3 set forth in SEQ ID NO: 15 with no more than 2 amino acid alterations;
wherein the amino acid alterations are amino acid additions or deletions, or conservative amino acid substitutions.

In some embodiments, the present disclosure provides an immunoglobulin single variable domain (ISVD) specifically binding to EGFR, wherein the ISVD comprises:
(a) a CDR1, a CDR2, and a CDR3 comprising or consisting of SEQ ID NOs: 3, 4, and 5, respectively;
(b) a CDR1, a CDR2, and a CDR3 comprising or consisting of SEQ ID NOs: 8, 9, and 10, respectively;
(c) a CDR1, a CDR2, and a CDR3 comprising or consisting of SEQ ID NOs: 8, 34, and 35, respectively;
(d) a CDR1, a CDR2, and a CDR3 comprising or consisting of SEQ ID NOs: 8, 103, and 104, respectively;
(e) a CDR1, a CDR2, and a CDR3 comprising or consisting of SEQ ID NOs: 13, 14, and 15, respectively;
(f) a CDR1 comprising or consisting of SEQ ID NO: 13, a CDR2 comprising or consisting of any one of SEQ ID NOs: 115-120, and a CDR3 comprising or consisting of SEQ ID NO: 15;
(g) a CDR1, a CDR2, and a CDR3 comprising or consisting of SEQ ID NOs: 13, 38, and 15, respectively; or
(h) a CDR1, a CDR2, and a CDR3 comprising or consisting of SEQ ID NOs: 13, 85, and 15, respectively. In some embodiments, the ISVD as defined under (a), (c), or (h) above is preferred. In some embodiments, the ISVD defined under (c) is more preferred.

In some embodiments, the ISVD specifically binding to EGFR of the present disclosure comprises or consists of a VHH. In some embodiments, the ISVD specifically binding to EGFR of the present disclosure comprises or consists of the following sequence:
(a) a sequence set forth in any one of SEQ ID NOs: 1, 31, and 94-99, or a sequence having at least 90%, 91%, 92%, 93%, 94%, 95%, 96%, 97%, 98%, or 99% identity therewith;
(b) a sequence set forth in any one of SEQ ID NOs: 6, 32, and 100-102, or a sequence having at least 90%, 91%, 92%, 93%, 94%, 95%, 96%, 97%, 98%, or 99% identity therewith; or
(c) a sequence set forth in any one of SEQ ID NOs: 11, 36, 84, and 105-114, or a sequence having at least 90%, 91%, 92%, 93%, 94%, 95%, 96%, 97%, 98%, or 99% identity therewith; preferably, the amino acid alterations do not occur in the CDRs. In some embodiments, the ISVD comprising the amino acid sequence of SEQ ID NO: 31, 32, 84, or 36 is preferred. In some other embodiments, the ISVD comprising the amino acid sequence of SEQ ID NO: 32 is more preferred.

In some embodiments, the present disclosure provides an antibody comprising the ISVD specifically binding to EGFR of the present disclosure, particularly an anti-EGFR heavy-chain antibody.

In some embodiments, the present disclosure provides a binding molecule that specifically binds to EGFR. In some embodiments, the EGFR-binding molecule comprises the ISVD specifically binding to EGFR of the present disclosure. In some embodiments, the EGFR-binding molecule comprises or consists of an antibody selected from: a single-domain antibody, a nanobody, a VHH antibody, and a heavy-chain antibody. In some other embodiments, the EGFR-binding molecule is a monospecific antibody, a bispecific antibody, or a multispecific antibody.

In some embodiments, the EGFR-binding molecule of the present disclosure comprises at least one ISVD specifically binding to EGFR of the present disclosure. For example, the ISVD comprises two, three, four, or more identical or different ISVDs specifically binding to EGFR of the present disclosure.

In some embodiments, the ISVD comprised in the EGFR-binding molecule of the present disclosure is preferably a humanized VHH. Compared with a *Camelidae* VHH, the humanized VHH exhibits reduced human anti-Camelidae antibody response in human body, enhancing the safety of antibody application.

In some embodiments, the ISVD specifically binding to EGFR or the EGFR-binding molecule of the present disclosure exhibits one or more of the following properties:
(1) binding to human EGFR with moderate or low affinity;
(2) having cross-reactivity with human EGFR and cynomolgus monkey EGFR;
(3) specifically binding to EGFR expressed on the surface of tumor cells; and
(4) being internalized by EGFR-expressing tumor cells.

In some embodiments, the EGFR-binding molecule of the present disclosure is in the form of a bispecific antibody molecule or a multispecific antibody molecule. The multispecific antibody molecule may be, for example, a trispecific antibody molecule comprising a first binding specificity for EGFR and second and third binding specificities for one or more other molecules. In some embodiments, the EGFR-binding molecule of the present disclosure further comprises at least one ISVD specifically binding to cMet, preferably at least one or two anti-cMet ISVDs according to the present disclosure.

In some embodiments, the EGFR-binding molecule of the present disclosure is linked, at its N-terminus or C-terminus, to one or more other groups, residues, or moieties via one or more peptide linkers, wherein as compared to a corresponding EGFR-binding molecule lacking the linked one or more other groups, residues, or moieties, the EGFR-binding molecule of the present disclosure exhibits the following properties provided by the one or more other groups, residues, or moieties: an extended half-life and effector functions, such as antibody-dependent cellular cytotoxicity (ADCC) and complement-dependent cytotoxicity (CDC).

In some embodiments, the one or more other groups, residues, moieties providing the extended half-life are selected from a polyethylene glycol molecule, a serum protein or a fragment thereof, a moiety that can bind to a serum protein (e.g., serum albumin (such as human serum albumin)), a moiety that binds to a serum immunoglobulin (such as IgG), and an Fc domain.

In some embodiments, the EGFR-binding molecule of the present disclosure is linked, at its N-terminus or C-terminus and via one or more peptide linkers, to an ISVD binding to human serum albumin. In some embodiments, the ISVD binding to human serum albumin comprises, e.g., a CDR1 comprising or consisting of an amino acid sequence set forth in SEQ ID NO: 46, a CDR2 comprising or consisting of an amino acid sequence set forth in SEQ ID NO: 47, and a CDR3 comprising or consisting of an amino acid sequence set forth in SEQ ID NO: 48. In some embodiments, the ISVD binding to human serum albumin comprises a sequence of SEQ ID NO: 45 or a sequence having at least 90%, 91%, 92%, 93%, 94%, 95%, 96%, 97%, 98%, or 99% identity therewith.

In certain embodiments, the EGFR-binding molecule provided herein is modified to increase or decrease the degree of glycosylation thereof. The addition or deletion of glycosylation sites of the EGFR-binding molecule can be conveniently achieved by changing the amino acid sequence to create or remove one or more glycosylation sites. When the EGFR-binding molecule comprises an Fc region, the carbohydrate attached to the Fc region can be changed. In some applications, modifications that remove undesired glycosylation sites may be useful, for example, removing fucose modules to enhance antibody-dependent cell-mediated cytotoxicity (ADCC) (see Shield et al.,

(2002) JBC277:26733). In other applications, galactosylation modifications can be made to regulate complement-dependent cytotoxicity (CDC). In certain embodiments, one or more amino acid modifications can be introduced into the Fc region of the EGFR-binding molecule provided herein, thereby producing an Fc region variant to enhance, for example, the effectiveness of the EGFR-binding molecule of the present disclosure in the treatment of cancer.

### III. Third aspect of the present disclosure: EGFR- and cMet-binding molecule

In a third aspect, the present disclosure provides an EGFR- and cMet-binding molecule. Preferably, the binding molecule is a multispecific antibody capable of binding to both EGFR and cMet. In some embodiments, the multispecific antibody according to the present disclosure exhibits one or more of the following properties:
(1) binding to EGFR, e.g., human EGFR, with moderate or low affinity;
(2) being internalized by EGFR-expressing tumor cells;
(3) specifically binding to cMet, e.g., human cMet;
(4) blocking binding of ligand HGF, when present, to cMet on the cell surface;
(5) being internalized by cMet-expressing tumor cells, particularly producing a synergistic internalization enhancement effect when binding to different epitopes of cMet;
(6) binding to tumor cells expressing both EGFR and cMet and preferably exhibiting synergistic binding activity;
(7) being endocytosed by tumor cells expressing both EGFR and cMet and preferably exhibiting synergistic endocytic activity;
(8) having cross-reactivity with human EGFR and cynomolgus monkey EGFR;
(9) having cross-reactivity with human cMet and cynomolgus monkey cMet; and
(10) reducing tumor cell proliferation and metastasis.

Monoclonal antibodies have been established as anti-tumor therapeutics in the past two decades, and a variety of monoclonal antibodies targeting EGFR and cMet have been approved or are in clinical development, but acquired resistance developed by tumors limits their long-term efficacy. Due to their ability to specifically bind to different antigenic epitopes, multispecific antibodies (e.g., bispecific antibodies or trispecific antibodies) can be designed to simultaneously act on signal transduction pathways of two or more different mediators. This design helps prevent tumors from developing acquired resistance.

In some embodiments, the multispecific antibody of the present disclosure is an EGFR- and cMet-binding molecule, which comprises at least one ISVD specifically binding to EGFR as defined according to the second aspect of the present disclosure (e.g., Section II herein), e.g., one or two ISVDs binding to EGFR of the present disclosure, and further comprises at least one ISVD binding to cMet as defined according to the first aspect of the present disclosure (e.g., Section I herein), e.g., one or two ISVDs binding to cMet of the present disclosure, optionally, wherein the ISVDs located on the same polypeptide chain are linked via one or more peptide linkers.

Generally, an EGFR- and cMet-binding molecule comprising two or more ISVDs is also referred to herein as a "multivalent" EGFR- and cMet-binding molecule. For example, a "bivalent" EGFR- and cMet-binding molecule may comprise one ISVD binding to EGFR and one ISVD binding to cMet, optionally linked via a peptide linker. A "trivalent" EGFR- and cMet-binding molecule may comprise one ISVD binding to EGFR and two ISVDs binding to cMet, optionally linked via two peptide linkers; or may comprise two ISVDs binding to EGFR and one ISVD binding to cMet, optionally linked via two peptide linkers. A "tetravalent" EGFR- and cMet-binding molecule may comprise two ISVDs binding to EGFR and two ISVDs binding to cMet, optionally linked via three peptide linkers; or may comprise one ISVD binding to EGFR and three ISVDs binding to cMet, optionally linked via three peptide linkers; or may comprise three ISVDs binding to EGFR and one ISVD binding to cMet, optionally linked via three peptide linkers, etc.

In multivalent EGFR- and cMet-binding molecules, the two or more ISVDs may be the identical or different and may target the same antigenic epitope of EGFR, the same antigenic epitope of cMet, different antigenic epitopes of EGFR, or different antigenic epitopes of cMet, or any suitable combination thereof.

In some preferred embodiments, the multispecific EGFR- and cMet-binding molecule of the present disclosure comprises at least one (preferably one) ISVD targeting EGFR and two ISVDs targeting different epitopes on cMet. In some other preferred embodiments, the multispecific EGFR- and cMet-binding molecule of the present disclosure comprises at least one (preferably one) ISVD targeting EGFR and two ISVDs targeting the same epitope on cMet.

### Structural forms of multispecific antibodies of the present disclosure

Multispecific antibodies (e.g., bispecific antibodies and trispecific antibodies) can be classified into many types based on different components and construction methods. For example, based on the substantial bilateral symmetry of multispecific antibody structures, they can be classified into a symmetric structure and an asymmetric structure; based on the presence or absence of IgG Fc region in multispecific antibodies, they can be classified into an antibody format with an Fc region and an antibody format without an Fc region; based on the number of antigen-binding sites in multispecific antibodies, they can be classified into bivalent, trivalent, or tetravalent antibodies or those of greater valencies, and the like; based on the number of polypeptide chains constituting multispecific antibodies, they can be classified into a single-chain form or a multi-chain form. See, e.g., Brinkmann U. and Kontermann R. E., The making of bispecific antibodies, Mabs, 2017, 9(2): 182-212. These structural forms of multispecific antibodies known in the art are contemplated in the present disclosure.

### Multispecific antibody in single-chain form

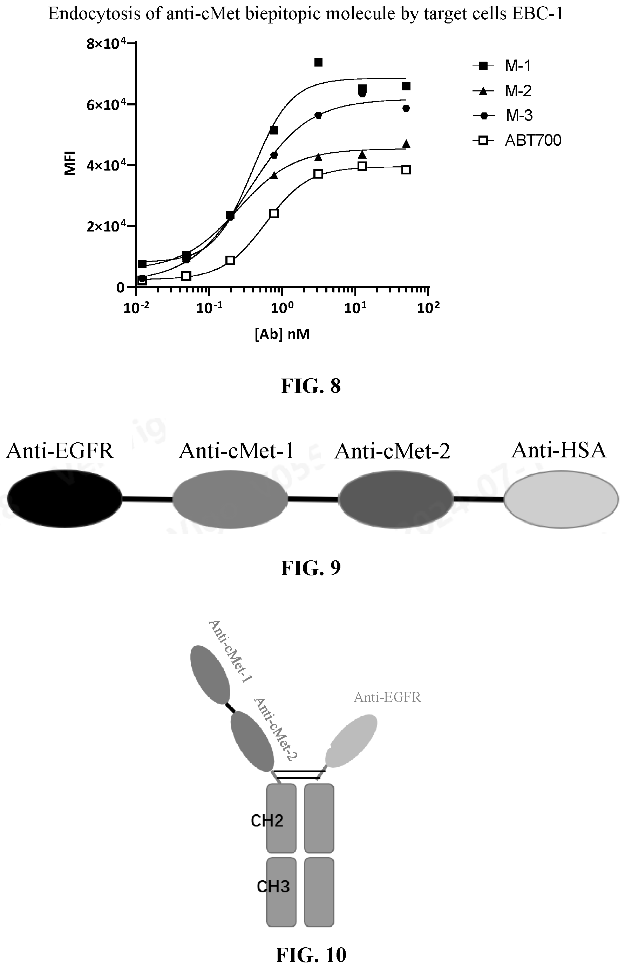

In some embodiments, the present disclosure provides an EGFR- and cMet-binding molecule in a single-chain form (e.g., see FIG. 9), wherein at least one ISVD specifically binding to EGFR and at least one ISVD specifically binding to cMet are located on one polypeptide chain, wherein the ISVDs are linked to each other via a peptide linker or directly linked.

In some embodiments, the present disclosure provides a multispecific antibody in a single-chain form, which comprises a single polypeptide chain, wherein the polypeptide chain comprises, from N-terminus to C-terminus:

(ISVD^{A})ₙ₁-(ISVD^{B})ₙ₂-(HLE)ₙ₃-(ISVD^{A})ₙ₄-(ISVD^{B})ₙ₅-(HLE)ₙ₆, (I)

wherein n1, n2, n3, n4, n5 and n6 are each independently selected from integers of 0, 1 and 2; ISVD^{A} and ISVD^{B} represent ISVD domains binding to an antigen A and an antigen B, respectively, where A and B are different from each other and are independently selected from EGFR and cMet; HLE represents a serum albumin-binding peptide as the half-life extension domain; the symbol "-" represents linkage via a peptide linker or direct linkage, preferably a peptide linker having 5 to 15 amino acids in length. The ISVD^{A}s in Formula (I) can each independently target the same epitope or different epitopes on the antigen A. The ISVD ^{B}s in Formula (I) can each independently target the same epitope or different epitopes on the antigen B. In some cases, preferably, the multispecific antibody has a valency of 2-6 (i.e., n1 + n2 + n4 + n5 = 2-6), more preferably, no more than 4 (i.e., n1 + n2 + n4 + n5 = 2-4), e.g., divalent, trivalent, or tetravalent.

The number of the anti-EGFR ISVD domains and the number of the anti-cMet ISVD domains in the multispecific antibody may be equal or different. The multispecific antibody may or may not comprise an HLE domain as required. In some embodiments, the number of the anti-EGFR ISVD domains is no more than 4, preferably no more than 3, e.g., 1. In some embodiments, the number of the anti-cMet ISVD domains is no more than 4, preferably no more than 3, e.g., 2. In some embodiments, the ratio of the number of the anti-EGFR ISVD domain to the number of the anti-cMet ISVD domain is 1:1 or 1:2. In some embodiments, the antibody comprises 0 or 1 HLE.

In some embodiments, the present disclosure provides a multispecific antibody comprising a single polypeptide chain, wherein the polypeptide chain comprises, from N-terminus to C-terminus:
(i) ISVD^{A}-ISVD^{B};
(ii) ISVD^{A}-ISVD^{B}- ISVD^{B}, wherein the ISVD^{B}s each target the same epitope or preferably different epitopes of the antigen B;
(iii) ISVD ^{B}-ISVD^{A}-ISVD^{B}, wherein the ISVD^{B}s each target the same epitope or preferably different epitopes of the antigen B;
wherein preferably A represents EGFR and B represents cMet. In some embodiments, the polypeptide chains of (i)-(iii) comprise one HLE at the N-terminus or preferably the C-terminus.

In some embodiments, the HLE is an anti-HSA ISVD that binds to human serum albumin. In some embodiments, the anti-HSA ISVD comprises or consists of a CDR1 comprising an amino acid sequence set forth in SEQ ID NO: 46 , a CDR2 comprising or consisting of an amino acid sequence set forth in SEQ ID NO: 47, and a CDR3 comprising or consisting of an amino acid sequence set forth in SEQ ID NO: 48. In some embodiments, the ISVD binding to human serum albumin comprises the sequence of SEQ ID NO: 45 or a sequence having at least 90%, 91%, 92%, 93%, 94%, 95%, 96%, 97%, 98%, or 99% identity therewith.

In some specific embodiments, the present disclosure provides a multispecific antibody comprising a single polypeptide chain, wherein the polypeptide chain comprises:
(a) an ISVD specifically binding to EGFR, preferably selected from ISVDs specifically binding to EGFR according to the second aspect of the present disclosure; and (b) a first ISVD specifically binding to cMet and a second ISVD specifically binding to cMet, preferably each selected from ISVDs specifically binding to cMet according to the first aspect of the present disclosure, wherein the first ISVD and the second ISVD are identical or different; preferably, the first ISVD and the second ISVD specifically bind to different epitopes of cMet. In some embodiments, the polypeptide chain further comprises an ISVD specifically binding to HSA, preferably located at the N-terminus or C-terminus of the polypeptide chain. In some embodiments, preferably, from N-terminus to C-terminus, the polypeptide chain comprises: an ISVD specifically binding to EGFR, a first peptide linker, a first ISVD specifically binding to cMet, a second peptide linker, a second ISVD specifically binding to cMet, and optionally a third peptide linker and an ISVD specifically binding to HSA.

### Multispecific antibody in double-chain form

In some embodiments, the present disclosure provides an EGFR- and cMet-binding molecule in a double-chain form (e.g., see FIG. 10), which comprises at least one ISVD specifically binding to EGFR, at least one ISVD specifically binding to cMet, and at least one half-life extension domain, wherein the half-life extension domain is an immunoglobulin Fc region, optionally wherein the ISVDs located on the same polypeptide chain are linked via a peptide linker or directly.

In some embodiments, the present disclosure provides a multispecific antibody comprising a first polypeptide chain and a second polypeptide chain, wherein
the first polypeptide chain comprises, from N-terminus to C-terminus: (ISVD^{A})ₙ₁-(ISVD^{B})ₙ₂-HLE-(ISVD^{A})ₙ₃-(ISVD^{B})ₙ₄, (II)
the second polypeptide chain comprises, from N-terminus to C-terminus: (ISVD^{B})ₘ₁-(ISVD^{A})ₘ₂-HLE-(ISVD^{B})ₘ₃-(ISVD^{A})m₄, (III)
wherein n1, n2, n3, and n4, as well as m1, m2, m3, and m4, are each independently selected from integers of 0, 1, and 2;
ISVD^{A} and ISVD^{B} represent ISVD domains binding to antigens A and B, respectively, where A and B are different from each other and independently selected from EGFR and cMet; HLE represents an immunoglobulin Fc region as a half-life extension domain, particularly a human IgG1 or IgG4 Fc region; the symbol "-" represents linkage via a peptide linker or direct linkage, preferably a peptide linker having 5-15 amino acids in length. The ISVD^{A}s in Formulas (II) and (III) can each independently target the same epitope or different epitopes on the antigen A. The ISVD^{B}s in Formulas (II) and (III) can each independently target the same epitope or different epitopes on the antigen B. In some cases, preferably, the multispecific antibody has a valency of 2-6 (i.e., the sum of n1, n2, n3, and n4 and m1, m2, m3, and m4 is 2-6), and more preferably, no more than 4, e.g., divalent, trivalent, or tetravalent.

The number of the anti-EGFR ISVD domains and the number of the anti-cMet ISVD domains in the multispecific antibody may be equal or different. In some embodiments, the number of the anti-EGFR ISVD domains is no more than 4, preferably no more than 3, e.g., 1. In some embodiments, the number of the anti-cMet ISVD domains is no more than 4, preferably no more than 3, e.g., 2. In some embodiments, the ratio of the number of the anti-EGFR ISVD domain to the number of the anti-cMet ISVD domain is 1:1 or 1:2.

In some preferred embodiments, the multispecific antibody comprises a first polypeptide chain and a second polypeptide chain, wherein:
the first polypeptide chain comprises, from N-terminus to C-terminus: (ISVD^{A})-HLE,
the second polypeptide chain comprises, from N-terminus to C-terminus: (ISVD^{B})-HLE;
   or
the first polypeptide chain comprises, from N-terminus to C-terminus: (ISVD^{B})-(ISVD^{A})-HLE,
the second polypeptide chain comprises, from N-terminus to C-terminus: (ISVD^{B})-HLE;
the ISVD ^{B}s each target the same epitope or preferably different epitopes of the antigen B;
   or
the first polypeptide chain comprises, from N-terminus to C-terminus: (ISVD^{A})-HLE;
the second polypeptide chain comprises, from N-terminus to C-terminus: (ISVD^{B})-(ISVD^{B})-HLE,
the ISVD ^{B}s each target the same epitope or preferably different epitopes of the antigen B;
preferably A represents EGFR and B represents cMet.

Due to dimerization of the immunoglobulin Fc region, the first and second polypeptide chains of the above multispecific antibody may associate to form a heterodimer, thereby producing a multispecific binding molecule in double-chain form. Preferably, in order to promote heterodimerization of the first and second polypeptide chains, a Knob-into-hole mutation, such as a (T366W/T366S, L368A, Y407V) mutation or a (T366Y/Y407T) mutation, may be introduced in the Fc regions of the first and second polypeptide chains. Preferably, the Fc region comprises an amino acid sequence from human IgG1 or IgG4; more preferably the Fc region further comprises a mutation that reduces or eliminates binding to Fcy receptor, such as a LALA mutation.

In some specific embodiments, the EGFR- and cMet-binding molecule of the present disclosure is in a double-chain form and comprises:
(a) an ISVD specifically binding to EGFR, wherein the ISVD is located on one polypeptide chain, and preferably the ISVD is selected from the ISVDs specifically binding to EGFR according to the second aspect of the present disclosure, wherein the ISVD is linked to an Fc region (Fc subunit) at the C-terminus; and
(b) a first ISVD specifically binding cMet and a second ISVD specifically binding cMet that are located on the other chain from the N terminus to the C terminus, preferably each selected from ISVDs specifically binding to cMet according to the first aspect of the present disclosure, wherein the first ISVD and the second ISVD are identical or different; preferably, the first ISVD and the second ISVD specifically bind to different epitopes of cMet; optionally, the first ISVD and the second ISVD are linked via one or more peptide linkers;
wherein the C-terminus of the second ISVD is linked to an Fc region (Fc subunit).

In some specific embodiments, the EGFR- and cMet-binding molecule of the present disclosure is a double-chain multispecific antibody comprising a first polypeptide chain and a second polypeptide chain, wherein from N-terminus to C-terminus, the first polypeptide chain comprises an ISVD specifically binding to EGFR and an immunoglobulin Fc region, and the second polypeptide chain comprises a first ISVD specifically binding to cMet, a peptide linker, a second ISVD specifically binding to cMet, and an immunoglobulin Fc region.

### Exemplary antigen domain combination

In some embodiments of the multispecific antibody according to the present disclosure described above, preferably, the multispecific antibody according to the present disclosure comprises at least one anti-cMet ISVD according to the first aspect of the present disclosure (e.g., as defined in Section I) and/or at least one anti-EGFR ISVD according to the second aspect of the present disclosure (e.g., as defined in Section II).

In some further preferred embodiments, the multispecific antibody according to the present disclosure comprises a first ISVD and a second ISVD that specifically bind to the same epitope on cMet. In some embodiments, the first ISVD and the second ISVD are the anti-cMet ISVDs according to the first aspect of the present disclosure that specifically bind to the same epitope on cMet. In some further embodiments, the first ISVD and the second ISVD comprise: (i) a CDR1 comprising or consisting of an amino acid sequence selected from SEQ ID NOs: 18 and 41, and a CDR2 and a CDR3 comprising or consisting of amino acid sequences of SEQ ID NO: 19 and SEQ ID NO:20, respectively; or (ii) a CDR1, a CDR2, and a CDR3 comprising or consisting of amino acid sequences of SEQ ID NOs: 23-25, respectively. In some further embodiments, the first ISVD and the second ISVD comprise, consist essentially of, or consist of an amino acid sequence having at least 90%, 91%, 92%, 93%, 94%, 95%, 96%, 97%, 98%, or 99% identity to an amino acid sequence set forth in any one of SEQ ID NO: 16, 39 or 40 or any one of SEQ ID NO: 21 or 42. In some preferred embodiments, the first ISVD and the second ISVD comprise, consist essentially of, or consist of an amino acid sequence of SEQ ID NO: 16, 39, or 40, or an amino acid sequence of SEQ ID NO: 21 or 42.

In some other further preferred embodiments, the multispecific antibody according to the present disclosure comprises a first ISVD and a second ISVD that specifically bind to different epitopes on cMet. In some embodiments, the first ISVD and the second ISVD are the anti-cMet ISVDs according to the first aspect of the present disclosure that specifically bind to different epitopes on cMet. In some embodiments, the first ISVD comprises a first anti-cMet VHH domain and the second ISVD comprises a second anti-cMet VHH domain, or vice versa, wherein the first and second cMet VHH domains are different from each other. In some embodiments, the first anti-cMet VHH domain comprises: a CDR1, a CDR2, and a CDR3 comprising or consisting of amino acid sequences of SEQ ID NOs: 23-25, respectively, and the second anti-cMet VHH domain comprises: a CDR1 comprising or consisting of an amino acid sequence selected from SEQ ID NOs: 18 and 41, and a CDR2 and a CDR3 comprising or consisting of amino acid sequences of SEQ ID NO: 19 and SEQ ID NO: 20, respectively. In some further embodiments, the first anti-cMet VHH domain comprises, consists essentially of, or consists of an amino acid sequence having at least 90%, 91%, 92%, 93%, 94%, 95%, 96%, 97%, 98%, or 99% identity to an amino acid sequence set forth in SEQ ID NO: 21 or 42, and the second anti-cMet VHH domain comprises, consists essentially of, or consists of an amino acid sequence having at least 90%, 91%, 92%, 93%, 94%, 95%, 96%, 97%, 98%, or 99% identity to an amino acid sequence set forth in SEQ ID NO: 16, 39, or 40. In some further embodiments, the first anti-cMet VHH domain comprises, consists essentially of, or consists of an amino acid sequence of SEQ ID NO: 21 or 42, and the second anti-cMet VHH domain comprises, consists essentially of, or consists of an amino acid sequence of SEQ ID NO: 16, 39, or 40. In some preferred embodiments, the first ISVD comprises the first anti-cMet VHH domain, and the second ISVD comprises the second anti-cMet VHH domain.

In some preferred embodiments of the above multispecific antibody according to the present disclosure comprising a first ISVD and a second ISVD that bind to the same cMet epitope or different cMet epitopes, the antibody further comprises at least one (preferably 1) anti-EGFR ISVD according to the second aspect of the present disclosure. In some embodiments, the anti-EGFR ISVD comprises:
(i) a CDR1, a CDR2, and a CDR3 comprising or consisting of amino acid sequences of SEQ ID NOs: 3, 4, and 5, respectively;
(ii) a CDR1, a CDR2, and a CDR3 comprising or consisting of amino acid sequences of SEQ ID NOs: 8, 9, and 10, respectively, or amino acid sequences of SEQ ID NOs: 8, 34, and 35, respectively, or amino acid sequences of SEQ ID NOs: 8, 103, and 104, respectively; or
(iii) a CDR1, a CDR2, and a CDR3 comprising or consisting of amino acid sequences of SEQ ID NOs: 13, 14, and 15, respectively, or amino acid sequences of SEQ ID NOs: 13, 85, and 15, respectively, or amino acid sequences of SEQ ID NOs: 13, 38, and 15, respectively;

preferably, the anti-EGFR ISVD:
   (a) comprises, consists essentially of, or consists of an amino acid sequence set forth in any one of SEQ ID NOs: 1, 31, and 94-99, or an amino acid sequence having at least 90%, 91%, 92%, 93%, 94%, 95%, 96%, 97%, 98%, or 99% identity therewith;
   (b) comprises, consists essentially of, or consists of an amino acid sequence set forth in any one of SEQ ID NOs: 6, 32, and 100-102, or an amino acid sequence having at least 90%, 91%, 92%, 93%, 94%, 95%, 96%, 97%, 98%, or 99% identity therewith; or
   (c) comprises, consists essentially of, or consists of an amino acid sequence set forth in any one of SEQ ID NOs: 11, 36, 84, and 105-114, or an amino acid sequence having at least 90%, 91%, 92%, 93%, 94%, 95%, 96%, 97%, 98%, or 99% identity therewith;
more preferably, the anti-EGFR ISVD:
   (a) comprises, consists essentially of, or consists of an amino acid sequence set forth in SEQ ID NO: 31;
   (b) comprises, consists essentially of, or consists of an amino acid sequence set forth in SEQ ID NO: 32; or
   (c) comprises, consists essentially of, or consists of an amino acid sequence set forth in SEQ ID NO: 84.

In some embodiments, the present disclosure provides a single-chain multispecific antibody comprising a combination of an anti-EGFR ISVD and a first anti-cMet ISVD and a second anti-cMet ISVD, wherein the combination is selected from:
(a) the anti-EGFR ISVD comprising three CDRs contained in an amino acid sequence of SEQ ID NO: 1, 6, or 11, the first anti-cMet ISVD comprising three CDRs contained in an amino acid sequence of SEQ ID NO: 21, and the second anti-cMet ISVD comprising three CDRs contained in an amino acid sequence of SEQ ID NO: 16; and
(b) the anti-EGFR ISVD comprising three CDRs contained in an amino acid sequence of SEQ ID NO: 1, 6, or 11, the first anti-cMet ISVD comprising three CDRs contained in an amino acid sequence of SEQ ID NO: 16, and the second anti-cMet ISVD comprising three CDRs contained in an amino acid sequence of SEQ ID NO: 21, wherein the CDRs are preferably defined according to Kabat;
preferably, the antibody comprises a combination of an anti-EGFR ISVD and a first anti-cMet ISVD and a second anti-cMet ISVD, wherein the combination is selected from:
(a) the anti-EGFR ISVD comprising an amino acid sequence of SEQ ID NO: 1, 6, or 11, the first anti-cMet ISVD comprising an amino acid sequence of SEQ ID NO: 21, and the second anti-cMet ISVD comprising an amino acid sequence of SEQ ID NO: 16; and
(b) the anti-EGFR ISVD comprising an amino acid sequence of SEQ ID NO: 1, 6, or 11, the first anti-cMet ISVD comprising an amino acid sequence of SEQ ID NO: 16, and the second anti-cMet ISVD comprising an amino acid sequence of SEQ ID NO: 21. In some further embodiments of this single-chain multispecific antibody, the antibody comprises, from N-terminus to C-terminus, the anti-EGFR ISVD, a peptide linker, the first anti-cMet ISVD, a peptide linker, and a second anti-cMet ISVD.

In some embodiments, the present disclosure provides a double-chain multispecific antibody comprising a combination of an anti-EGFR ISVD and a first anti-cMet ISVD and a second anti-cMet ISVD, wherein the combination is selected from:
(a) the anti-EGFR ISVD comprising three CDRs contained in an amino acid sequence of SEQ ID NO: 31, 32, or 84, the first anti-cMet ISVD comprising three CDRs contained in an amino acid sequence of SEQ ID NO: 42, and the second anti-cMet ISVD comprising three CDRs contained in an amino acid sequence of SEQ ID NO: 39;
(b) the anti-EGFR ISVD comprising three CDRs contained in an amino acid sequence of SEQ ID NO: 31, 32, or 84, the first anti-cMet ISVD comprising three CDRs contained in an amino acid sequence of SEQ ID NO: 39, and the second anti-cMet ISVD comprising three CDRs contained in an amino acid sequence of SEQ ID NO: 39;
(c) the anti-EGFR ISVD comprising three CDRs contained in an amino acid sequence of SEQ ID NO: 31, 32, or 84, the first anti-cMet ISVD comprising three CDRs contained in an amino acid sequence of SEQ ID NO: 42, and the second anti-cMet ISVD comprising three CDRs contained in an amino acid sequence of SEQ ID NO: 42; and
(d) the anti-EGFR ISVD comprising three CDRs contained in an amino acid sequence of SEQ ID NO: 31, 32, or 84, the first anti-cMet ISVD comprising three CDRs contained in an amino acid sequence of SEQ ID NO: 21, and the second anti-cMet ISVD comprising three CDRs contained in an amino acid sequence of SEQ ID NO: 40, wherein the CDRs are preferably defined according to Kabat;

preferably, the antibody comprises a combination of an anti-EGFR ISVD and a first anti-cMet ISVD and a second anti-cMet ISVD, wherein the combination is selected from:
   (a) the anti-EGFR ISVD comprising an amino acid sequence of SEQ ID NO: 31, 32, or 84, the first anti-cMet ISVD comprising an amino acid sequence of SEQ ID NO: 42, and the second anti-cMet ISVD comprising an amino acid sequence of SEQ ID NO: 39; and
   (b) the anti-EGFR ISVD comprising an amino acid sequence of SEQ ID NO: 31, 32, or 84, the first anti-cMet ISVD comprising an amino acid sequence of SEQ ID NO: 39, and the second anti-cMet ISVD comprising an amino acid sequence of SEQ ID NO: 39;
   (c) the anti-EGFR ISVD comprising an amino acid sequence of SEQ ID NO: 31, 32, or 84, the first anti-cMet ISVD comprising an amino acid sequence of SEQ ID NO: 42, and the second anti-cMet ISVD comprising an amino acid sequence of SEQ ID NO: 42; and
   (d) the anti-EGFR ISVD comprising an amino acid sequence of SEQ ID NO: 31, 32, or 84, the first anti-cMet ISVD comprising an amino acid sequence of SEQ ID NO: 21, and the second anti-cMet ISVD comprising an amino acid sequence of SEQ ID NO: 40;
more preferably, the antibody comprises a combination of an anti-EGFR ISVD and a first anti-cMet ISVD and a second anti-cMet ISVD, wherein the combination is selected from:
   (a) the anti-EGFR ISVD comprising an amino acid sequence of SEQ ID NO: 31, the first anti-cMet ISVD comprising an amino acid sequence of SEQ ID NO: 42, and the second anti-cMet ISVD comprising an amino acid sequence of SEQ ID NO: 39;
   (b) the anti-EGFR ISVD comprising an amino acid sequence of SEQ ID NO: 32, the first anti-cMet ISVD comprising an amino acid sequence of SEQ ID NO: 42, and the second anti-cMet ISVD comprising an amino acid sequence of SEQ ID NO: 39; and
   (c) the anti-EGFR ISVD comprising an amino acid sequence of SEQ ID NO: 84, the first anti-cMet ISVD comprising an amino acid sequence of SEQ ID NO: 21, and the second anti-cMet ISVD comprising an amino acid sequence of SEQ ID NO: 40;
still more preferably, the antibody comprises: an anti-EGFR ISVD comprising an amino acid sequence of SEQ ID NO: 31, a first anti-cMet ISVD comprising an amino acid sequence of SEQ ID NO: 42, and a second anti-cMet ISVD comprising an amino acid sequence of SEQ ID NO: 39.

In some further embodiments of this double-chain multispecific antibody, the antibody comprises a first polypeptide chain and a second polypeptide chain, wherein from N-terminus to C-terminus, the first polypeptide chain comprises an ISVD specifically binding to EGFR and an immunoglobulin Fc region, and the second polypeptide chain comprises a first ISVD specifically binding to cMet, a peptide linker, a second ISVD specifically binding to cMet, and an immunoglobulin Fc region.

### Exemplary peptide linker

In some embodiments of the multispecific antibody of the present disclosure, the antibody comprises a peptide linker. Herein, the "peptide linker" used in the binding molecule of the present disclosure and the antibody of the present disclosure refers to a short amino acid sequence consisting of natural amino acids. The length or flexibility of the peptide linker used in the binding molecule of the present disclosure and the antibody of the present disclosure is not particularly limited. The peptide linker used in the binding molecule and the antibody of the present disclosure may be any suitable amino acid sequence, in particular an amino acid sequence having 1 to 50, preferably 1 to 30, e.g., 1 to 10 amino acid residues. In some embodiments, the peptide linker consists essentially of glycine (G) and serine (S) residues, preferably comprises one or more repeats of a peptide motif such as a GGGGS (SEQ ID NO: 43) motif (e.g., comprising formula (Gly-Gly-Gly-Gly-Ser)n, wherein n may be 1, 2, 3, 4, 5, 6, 7, or more), for example, GGGGSGGGGS (SEQ ID NO: 44). In some embodiments, the peptide linker for linking two ISVDs in the binding molecule and the antibody of the present disclosure preferably comprises an amino acid sequence of SEQ ID NO: 44. Based on the disclosure herein, those skilled in the art will be able to determine the optimal peptide linker for use in the binding molecule of the present disclosure or the antibody of the present disclosure after limited conventional experiments.

### Exemplary immunoglobulin Fc region

In some embodiments of the multispecific antibody of the present disclosure, the antibody comprises an Fc region (Fc subunit). The Fc region that can be used in the binding molecule and the antibody of the present disclosure may be an Fc region derived from IgG1, IgG2, IgG3, or IgG4.

In some embodiments, the Fc region of the EGFR- and cMet-binding molecule of the present disclosure uses the "knobs-into-holes" technique (see, e.g., John B.B.Ridgway et al., 'Knobs-into-holes' engineering of antibody CH3 domains for heavy chain heterodimerization. Protein Engineering, 1996.9(7): p. 617-21; Shane Atwell et al., Stable heterodimers form remodeling the domain interface of a homodimer using a phage display library. J.Mol.Biol, 1997.270: p. 26-35). This technique enables the modification of the interface between the two chains of the EGFR-and cMet-binding molecule of the present disclosure, so as to promote the correct association of the two chains of the EGFR- and cMet-binding molecule of the present disclosure. Generally, this technique involves introducing a "protuberance" at the interface of one chain and introducing a corresponding "cavity" at the interface of the other chain to be paired with, such that the protuberance can be placed at the cavity. A preferred interface comprises the CH3 domain from the heavy chain constant domain of one chain and the CH3 domain from the heavy chain constant domain of the other chain to be paired with. The protuberance can be constructed by substituting small amino acid side chains at an interface of the CH3 domain from the heavy chain constant domain of one chain with relatively large side chains (such as tyrosine or tryptophan). By substituting large amino acid side chains with relatively small side chains (such as alanine or threonine), the compensating cavity of the same size as, or a similar size to, the protuberance is constructed at an interface of the CH3 domain from the heavy chain constant domain of the other chain to be paired with.

In one embodiment, the Fc region on both chains of the EGFR- and cMet-binding molecule of the present disclosure comprises a modification of binding affinity for an Fc receptor. In one embodiment, the Fc receptor is an Fcy receptor, in particular a human Fcy receptor. In one embodiment, the Fc receptor is an activating Fc receptor. In one embodiment, the modification reduces the effector function of the EGFR- and cMet-binding molecule of the present disclosure. In one specific embodiment, the effector function is antibody-dependent cell-mediated cytotoxicity (ADCC). In one embodiment, the modification is in the Fc region of the EGFR- and cMet-binding molecule of the present disclosure, particularly in the CH2 region thereof. In one embodiment, the EGFR- and cMet-binding molecule of the present disclosure comprises an amino acid replacement at position 329 (EU numbering) of the heavy chain. In one specific embodiment, the amino acid replacement is P329G. In one embodiment, the EGFR-and cMet-binding molecule of the present disclosure comprises amino acid replacements at positions 234 and 235 (EU numbering) of the heavy chain. In one specific embodiment, the amino acid replacements are L234A and L235A (LALA mutations) (Armour KL et al., Recombinant human IgG molecules lacking Fcgamma receptor I binding and monocyte triggering activities, Eur. J. Immunol., 1999, 29(8):2613-24). In one embodiment, the EGFR-and cMet-binding molecule of the present disclosure comprises amino acid replacements at positions 234, 235, and 329 (EU numbering) of the heavy chain. In one specific embodiment, the EGFR- and cMet-binding molecule of the present disclosure comprises amino acid replacements L234A, L235A, and P329G (EU numbering) in the heavy chain.

In one embodiment, the Fc region used in the binding molecule and the antibody of the present disclosure comprises a mutation YTE, i.e., a combination of mutations M252Y (Met252Tyr), S254T (Ser254Thr), and T256E (Thr256Glu) according to the EU index numbering of Kabat, to provide an extended half-life.

In some embodiments, the immunoglobulin Fc region used in the double-chain multispecific antibody of the present disclosure comprises:
an Fc chain (also referred to as a hole chain) having a Hole mutation, comprising a sequence of SEQ ID NO: 137 or a sequence having at least 90%, 91%, 92%, 93%, 94%, 95%, 96%, 97%, 98%, or 99% identity therewith; and
an Fc chain (also referred to as a knob chain) having a Knob mutation, comprising a sequence of SEQ ID NO: 138 or a sequence having at least 90%, 91%, 92%, 93%, 94%, 95%, 96%, 97%, 98%, or 99% identity therewith.

### Exemplary multispecific antibody

In some embodiments, the present disclosure provides a multispecific antibody comprising a first polypeptide chain and a second polypeptide chain, wherein:
- the first polypeptide chain comprises a sequence selected from SEQ ID NO: 70, SEQ ID NO: 71, SEQ ID NO: 72, and SEQ ID NO: 86, or a sequence having at least 90%, 91%, 92%, 93%, 94%, 95%, 96%, 97%, 98%, or 99% identity therewith; and
- the second polypeptide chain comprises a sequence selected from SEQ ID NO: 69, SEQ ID NO: 73, SEQ ID NO: 74, SEQ ID NO: 75, and SEQ ID NO: 87, or a sequence having at least 90%, 91%, 92%, 93%, 94%, 95%, 96%, 97%, 98%, or 99% identity therewith. Preferably, in some embodiments,
   (i) the first polypeptide chain comprises or consists of an amino acid sequence of SEQ ID NO: 70, or a sequence having at least 90%, 91%, 92%, 93%, 94%, 95%, 96%, 97%, 98%, or 99% identity therewith, and the second polypeptide chain comprises or consists of an amino acid sequence of SEQ ID NO: 73, 75, or 74, or a sequence having at least 90%, 91%, 92%, 93%, 94%, 95%, 96%, 97%, 98%, or 99% identity therewith;
   (ii) the first polypeptide chain comprises or consists of an amino acid sequence of SEQ ID NO: 71, or a sequence having at least 90%, 91%, 92%, 93%, 94%, 95%, 96%, 97%, 98%, or 99% identity therewith, and the second polypeptide chain comprises or consists of an amino acid sequence of SEQ ID NO: 73, 75, or 74, or a sequence having at least 90%, 91%, 92%, 93%, 94%, 95%, 96%, 97%, 98%, or 99% identity therewith;
   (iii) the first polypeptide chain comprises or consists of an amino acid sequence of SEQ ID NO: 72, or a sequence having at least 90%, 91%, 92%, 93%, 94%, 95%, 96%, 97%, 98%, or 99% identity therewith, and the second polypeptide chain comprises or consists of an amino acid sequence of SEQ ID NO: 73, 75, or 74, or a sequence having at least 90%, 91%, 92%, 93%, 94%, 95%, 96%, 97%, 98%, or 99% identity therewith;
   (iv) the first polypeptide chain comprises or consists of an amino acid sequence of SEQ ID NO: 86, or a sequence having at least 90%, 91%, 92%, 93%, 94%, 95%, 96%, 97%, 98%, or 99% identity therewith, and the second polypeptide chain comprises or consists of an amino acid sequence of SEQ ID NO: 87, or a sequence having at least 90%, 91%, 92%, 93%, 94%, 95%, 96%, 97%, 98%, or 99% identity therewith.

More preferably, in some embodiments,
(i) the first polypeptide chain comprises or consists of an amino acid sequence of SEQ ID NO: 71, and the second polypeptide chain comprises or consists of an amino acid sequence of SEQ ID NO: 73;
(ii) the first polypeptide chain comprises or consists of an amino acid sequence of SEQ ID NO: 72, and the second polypeptide chain comprises or consists of an amino acid sequence of SEQ ID NO: 73;
(iii) the first polypeptide chain comprises or consists of an amino acid sequence of SEQ ID NO: 86, and the second polypeptide chain comprises or consists of an amino acid sequence of SEQ ID NO: 87.

In some other embodiments, the present disclosure provides a multispecific antibody comprising a single polypeptide chain, wherein the polypeptide chain comprises a sequence selected from SEQ ID NOs: 51-66 or a sequence having at least 90%, 91%, 92%, 93%, 94%, 95%, 96%, 97%, 98%, or 99% identity therewith; or the polypeptide chain comprises a sequence selected from SEQ ID NOs: 67-68 and 76-83 or a sequence having at least 90%, 91%, 92%, 93%, 94%, 95%, 96%, 97%, 98%, or 99% identity therewith. Preferably, in some embodiments, the polypeptide chain comprises or consists of a sequence selected from SEQ ID NOs: 51-56.

### Properties of EGFR- and cMet-binding molecule of the present disclosure

### EGFR affinity

The EGFR- and cMet-binding molecule of the present disclosure comprises an ISVD that binds to EGFR with moderate or low affinity. EGFR is expressed at low levels in normal tissues (e.g., skin). The EGFR- and cMet-binding molecule of the present disclosure that binds EGFR with moderate or low affinity exhibits reduced on-target toxicity in normal tissues while still being able to target tumors that express EGFR at high levels, resulting in improved safety.

In some embodiments, the ISVD comprised in the binding molecule of the present disclosure and binding to human EGFR may also bind to cynomolgus monkey EGFR. For example, an ISVD binding to human EGFR may bind to cynomolgus monkey EGFR with a similar K_{D}. Herein, a similar K_{D} means that the difference between the two K_{D} values compared is no more than 10-fold, preferably no more than approximately 5-fold, or more preferably no more than 2-fold. The EGFR- and cMet-binding molecule of the present disclosure can bind to human EGFR and cynomolgus monkey EGFR. This cross-reactivity is advantageous because it allows dosing and safety testing of the EGFR- and cMet-binding molecule of the present disclosure in cynomolgus monkeys during pre-clinical development.

### cMet affinity

The EGFR- and cMet-binding molecule of the present disclosure comprises an ISVD specifically binding to cMet. In some embodiments, the EGFR- and cMet-binding molecule of the present disclosure comprises at least two ISVDs specifically binding to different epitopes on cMet, which enables the EGFR- and cMet-binding molecule of the present disclosure to have better tumor-targeting property and internalization effects.

In some embodiments, the ISVD binding to human cMet comprised in the binding molecule of the present disclosure can bind to human cMet with an affinity (K_{D}) of less than 80 nM, 50 nM, 20 nM, 15 nM, 12 nM, 11 nM, 10 nM, 9 nM, 8 nM, 7 nM, 6 nM, 5 nM, 4 nM, 3 nM, or 2.5 nM. Alternatively, the ISVD binding to human cMet may bind to human cMet with an affinity (K_{D}) of 1-20 nM, 1-15 nM, 1-10 nM, 1-9 nM, 1-8 nM, 1-7 nM, 1-6 nM, 1-5 nM, 1-4 nM, 1-3 nM, 1-2.5 nM, or 2-2.5 nM.

In some embodiments, the ISVD binding to human cMet comprised in the binding molecule of the present disclosure may also bind to cynomolgus monkey cMet. For example, an ISVD that binds to human cMet may bind to cynomolgus monkey cMet with a similar K_{D}. The EGFR- and cMet-binding molecule of the present disclosure can bind to human cMet and cynomolgus monkey cMet. This cross-reactivity is advantageous because it allows dosing and safety testing of the EGFR- and cMet-binding molecule of the present disclosure in cynomolgus monkeys during pre-clinical development.

### Specific binding to both EGFR and cMet

The EGFR- and cMet-binding molecule of the present disclosure can bind to both the EGFR target and the cMet target. A number of tumors are known to co-express both EGFR and cMet and, therefore, the EGFR- and cMet-binding molecule of the present disclosure that has the ability to bind to both EGFR and cMet is expected to be advantageous.

### Internalization

The EGFR- and cMet-binding molecule of the present disclosure can mediate effective internalization. This is particularly useful for conjugates as it ensures that the conjugates are internalized into the cell and delivered to the lysosome where the antibody molecule is subsequently degraded and the drug is released into the cell, exerting the cellular effect of the drug, e.g., cytotoxicity.

The internalization of the EGFR- and cMet-binding molecule of the present disclosure by cells can be analyzed by contacting live cells with the EGFR- and cMet-binding molecule of the present disclosure and detecting the EGFR-and cMet-binding molecule of the present disclosure after a sufficient period of internalization. An antibody molecule is determined not to be internalized by a cell when it remains on the cell surface (e.g., is detected on the cell surface, and/or is not detected within the cell). After the antibody molecule is detected inside the cell (e.g., located in the cytoplasm or organelle), it is determined that the antibody molecule has been internalized.

The internalization mediated by the EGFR- and cMet-binding molecule of the present disclosure shows greater selectivity for tumor cells that co-express these two targets when compared with that mediated by the EGFR monospecific binding molecule or the cMet monospecific binding molecule, thereby minimizing its adverse effects on normal tissues that do not show significant levels of EGFR and cMet co-expression.

### In vitro activity

The EGFR- and cMet-binding molecule of the present disclosure exhibits cytotoxic activity *in vitro.* Cytotoxic activity can be analyzed by using an *in vitro* cell viability assay, such as the CellTiter-Glo^{®} (Promega) assay. In some embodiments, the cell is a cell expressing both EGFR and cMet.

In some embodiments, the EGFR- and cMet-binding molecule of the present disclosure is capable of increasing killing of cells expressing significant amount of both EGFR and cMet, e.g., tumor cells, as compared to cells expressing low levels of EGFR and/or cMet. Cells expressing significant amount of both EGFR and cMet can be determined by measuring the relative EGFR and cMet receptor densities on the cell surface.

### In vivo activity

The EGFR- and cMet-binding molecule of the present disclosure is capable of inhibiting the development or progression of cancer *in vivo.* In some embodiments, the cancer may be a cancer that expresses both or one of EGFR and cMet. The cancer cells may express one or both of EGFR and cMet on the cell surface. The cancer may be, for example, selected from lung cancer (e.g., lung squamous cell carcinoma, lung adenocarcinoma, and non-small cell lung cancer), breast cancer (e.g., triple negative breast cancer), colon cancer, and pharyngeal squamous cell carcinoma.

### IV. Fourth aspect of the present disclosure: nucleic acid, vector, host, and production method

In a fourth aspect, the present disclosure provides encoding nucleic acids, vectors, host cells, and production methods of the ISVDs, the binding molecules, and the polypeptides according to the first to third aspects of the present disclosure.

In one embodiment, the present disclosure provides a method for preparing the ISVD, the EGFR-binding molecule, the cMet-binding molecule, or the multispecific antibody of the present disclosure, wherein the method comprises culturing a host cell comprising a nucleic acid encoding the ISVD, the EGFR-binding molecule, the cMet-binding molecule, or the multispecific antibody, or an expression vector comprising the nucleic acid, under conditions suitable for expression of the nucleic acid encoding the ISVD, the EGFR-binding molecule, the cMet-binding molecule, or the multispecific antibody, and optionally isolating the ISVD, the EGFR-binding molecule, the cMet-binding molecule, or the multispecific antibody. In a certain embodiment, the method further comprises isolating the ISVD, the EGFR-binding molecule, the cMet-binding molecule, or the multispecific antibody from the host cell (or host cell culture medium).

For recombinant production of the ISVD, the EGFR-binding molecule, the cMet-binding molecule, or the multispecific antibody of the present disclosure, a nucleic acid encoding the ISVD, the EGFR-binding molecule, the cMet-binding molecule, or the multispecific antibody of the present disclosure is first isolated and inserted into a vector for further cloning and/or expression in a host cell. Such nucleic acids can be easily isolated and sequenced by using conventional procedures, for example, by using an oligonucleotide probe capable of specifically binding to the nucleic acid encoding the ISVD, the EGFR-binding molecule, the cMet-binding molecule, or the multispecific antibody of the present disclosure.

The ISVD, the EGFR-binding molecule, the cMet-binding molecule, or the multispecific antibody of the present disclosure prepared as described herein may be purified by known technology, such as high-performance liquid chromatography, ion exchange chromatography, gel electrophoresis, affinity chromatography, and size exclusion chromatography. The actual conditions used for purifying a particular protein also depend on factors such as net charge, hydrophobicity, and hydrophilicity, and these will be apparent to those skilled in the art. The purity of the ISVD, the EGFR-binding molecule, the cMet-binding molecule, or the multispecific antibody of the present disclosure can be determined by any one of a variety of well-known analytical methods including size exclusion chromatography, gel electrophoresis, high-performance liquid chromatography, and the like.

### V. Fifth aspect of the present disclosure: immunofusion, immunoconjugate, and antibody-drug conjugate (ADC)

In a fifth aspect, the present disclosure provides immunofusions, immunoconjugates, and antibody-drug conjugates comprising the ISVD, the antibody, and the antigen-binding molecule according to the first to third aspects of the present disclosure.

### Immunofusion and immunoconjugate

In one embodiment, the present disclosure provides an immunofusion or an immunoconjugate produced by fusing or conjugating the ISVD, the antibody, and the antigen-binding molecule according to the first to third aspects of the present disclosure to a heterologous molecule.

In one embodiment, in the immunofusion, the antigen-binding molecule (e.g., antibody) of the present disclosure is linked to a heterologous peptide or polypeptide molecule, either directly or via an amino acid linker. The heterologous peptides or polypeptides that can be mentioned include, but are not limited to, proteins or polypeptides conferring additional functional activity to the fusion, or tag peptides facilitating the purification or detection of the immunofusion.

In one embodiment, in the immunoconjugate, the antigen-binding molecule (e.g., antibody) of the present disclosure is conjugated to a therapeutic agent, a diagnostic agent, or a detectable agent. In a conjugate, a chemical linker may be used to covalently link different entities of the conjugate. In some cases, advantageously, the chemical linker is a "cleavable linker" that facilitates release of the antigen-binding molecule polypeptide upon delivery to a target site. For example, acid-labile linkers, peptidase-sensitive linkers, photolabile linkers, dimethyl linkers, or disulfide-containing linkers can be used.

In embodiments of conjugation to a therapeutic agent, the therapeutic agent suitable for the conjugate includes, but is not limited to, a cytotoxin (e.g., a cytostatic agent or a cytotoxic agent), a drug, or a radioisotope.

In embodiments of conjugation to a diagnostic agent or a detectable agent, such conjugates can be used as part of a clinical testing method (e.g., to determine the efficacy of a specific therapy) for monitoring or predicting the onset, development, progression, and/or severity of a disease or condition. Such diagnosis and detection can be achieved by conjugating the antibody to the detectable agent, which includes, but is not limited to, a variety of enzymes, such as horseradish peroxidase; prosthetic groups, such as streptavidin/biotin and avidin/biotin; fluorescent substances; luminescent substances; radioactive substances; and positron-emitting metals and non-radioactive paramagnetic metal ions used in various positron emission tomography techniques.

In some embodiments, the therapeutic agent suitable for the conjugate includes, but is not limited to, a drug (e.g., an anti-tumor drug); in some other embodiments, the diagnostic agent suitable for the conjugate includes, but is not limited to, a radiodiagnostic agent, a fluorescent substance, or a luminescent substance.

### Antibody-drug conjugate (ADC)

In some preferred embodiments, the present disclosure provides an antibody-drug conjugate (ADC).

In some embodiments, the present disclosure provides an antibody-drug conjugate (ADC) having formula (I₀) or a pharmaceutically acceptable salt or a solvate thereof:

Ab-(L-D)ₚ (I₀)

wherein:
Ab is the binding molecule of the present disclosure, e.g., an antibody, such as the antibody or the fragment thereof (e.g., an antigen-binding fragment) specifically binding to EGFR and/or cMet described above;
L is a linker;
D is a drug, e.g., an anti-tumor compound; and
p is an integer selected from 1 to 16, e.g., an integer selected from 1-10, 1-9, 2-8, 4-10, 6-8, 3-7, 4-6, and 2-6, e.g., 1, 2, 3, 4, 5, 6, 7, 8, 9, 10, 11, or 12. In some embodiments, Ab is the EGFR- and cMet-binding molecule according to the third aspect of the present disclosure, particularly the multispecific antibody according to the third aspect of the present disclosure. In some particularly preferred embodiments, the multispecific antibody comprises a first polypeptide chain and a second polypeptide chain, wherein:
   (i) the first polypeptide chain comprises or consists of an amino acid sequence of SEQ ID NO: 71, and the second polypeptide chain comprises or consists of an amino acid sequence of SEQ ID NO: 73;
   (ii) the first polypeptide chain comprises or consists of an amino acid sequence of SEQ ID NO: 72, and the second polypeptide chain comprises or consists of an amino acid sequence of SEQ ID NO: 73;
   (iii) the first polypeptide chain comprises or consists of an amino acid sequence of SEQ ID NO: 86, and the second polypeptide chain comprises or consists of an amino acid sequence of SEQ ID NO: 87.

It will be understood that the -L-D moiety can be covalently linked to Ab by any means known in the art. In some embodiments, the -L-D moiety is covalently linked to Ab via a sulfur (S) atom from Ab; that is, the -L-D moiety and Ab are linked via -S-. In some embodiments, the sulfur atom is derived from the opening of an interchain disulfide bond of Ab. In some embodiments, the sulfur atom is from (engineered or natural) cysteine in Ab.

In some embodiments, the present disclosure provides an antibody-drug conjugate (ADC) having formula (I) or a pharmaceutically acceptable salt or a solvate thereof:

Ab-(S-L-D)ₚ (I)

wherein:
Ab is the binding molecule of the present disclosure, e.g., an antibody, such as the antibody or the fragment thereof (e.g., an antigen-binding fragment) specifically binding to EGFR and/or cMet described above;
L is a linker;
D is a drug, e.g., an anti-tumor compound; and
p is an integer selected from 1 to 16, e.g., an integer selected from 1-10, 1-9, 2-8, 4-10, 6-8, 3-7, 4-6, and 2-6, e.g., 1, 2, 3, 4, 5, 6, 7, 8, 9, 10, or 12. It should be understood that S in formula (I) is the sulphur from the antibody Ab.

In some embodiments, Ab is the EGFR- and cMet-binding molecule according to the third aspect of the present disclosure, particularly the multispecific antibody according to the third aspect of the present disclosure. In some particularly preferred embodiments, the multispecific antibody comprises a first polypeptide chain and a second polypeptide chain, wherein:
(i) the first polypeptide chain comprises or consists of an amino acid sequence of SEQ ID NO: 71, and the second polypeptide chain comprises or consists of an amino acid sequence of SEQ ID NO: 73;
(ii) the first polypeptide chain comprises or consists of an amino acid sequence of SEQ ID NO: 72, and the second polypeptide chain comprises or consists of an amino acid sequence of SEQ ID NO: 73;
(iii) the first polypeptide chain comprises or consists of an amino acid sequence of SEQ ID NO: 86, and the second polypeptide chain comprises or consists of an amino acid sequence of SEQ ID NO: 87.

It will be understood that p refers to the number of -L-D linked to Ab in the antibody-drug conjugate molecule of formula (I₀) or (I), and may also be referred to as DAR.

In some embodiments, D in formula (I₀) or (I) of the present disclosure may be any anti-tumor compound and is not particularly limited as long as it is a compound having an anti-tumor effect and having a structural moiety capable of linking to a linker. For example, the anti-tumor compound may be a pharmaceutically active compound that acts on a tumor. For an anti-tumor compound, it is preferable that a part or all of the linker is cleaved in a tumor cell to release the anti-tumor compound, thereby exhibiting an anti-tumor effect.

In some embodiments, the anti-tumor compound may be, for example, a cytotoxic agent, such as a camptothecin compound or an auristatin compound.

In some embodiments, D has a structure represented by formula (D-1a) or formula (D-1b): 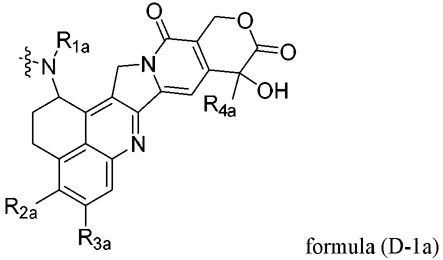
wherein R₁ₐ is selected from H and C₁-C₆ alkyl;
R₂ₐ is selected from H, halogen, C₁-C₆ alkyl, C₁-C₆ haloalkyl, -OR₅ₐ, and -SR₅ₐ;
R₃ₐ is selected from H, halogen, CN, C₁-C₆ alkyl, C₁-C₆ haloalkyl, and -OR₅ₐ; and
R₄ₐ and R₅ₐ are independently selected from H and C₁-C₄ alkyl;
in some embodiments, R₁ₐ is H; R₂ₐ is C₁-C₆ alkyl; R₃ₐ is halogen, preferably -F; R₄ₐ is C₁-C₄ alkyl, preferably ethyl; 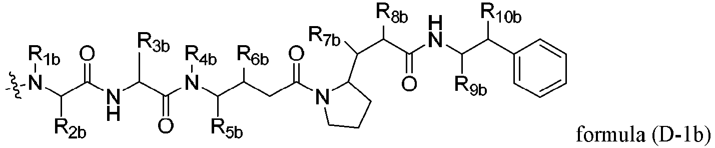
wherein R_{1b}, R_{2b}, R_{3b}, R_{4b}, R_{5b}, and R_{8b} are each independently selected from C₁₋₈ alkyl, preferably C₁₋₄ alkyl, such as methyl, ethyl, n-propyl, isopropyl, n-butyl, isobutyl, or sec-butyl;
R_{6b} and R_{7b} are each independently selected from C₁₋₈ alkoxy, such as methoxy, ethoxy, or propoxy;
R_{9b} is selected from C₁₋₈ alkyl and COOH, preferably C₁₋₄ alkyl, such as methyl, ethyl, n-propyl, isopropyl, n-butyl, isobutyl, or sec-butyl; and
R _{10b} is selected from OH and H.

In some embodiments, R_{1b}, R_{4b}, and R_{8b} are each independently selected from C₁₋₂ alkyl, preferably methyl;
R_{2b}, R_{3b}, and R_{5b} are each independently selected from C₃₋₄ alkyl;
R_{6b} and R_{7b} are each independently selected from C₁₋₂ alkoxy; and
R_{9b} is selected from C₁₋₄ alkyl and R_{10b} is OH; or R_{9b} is COOH and R_{10b} is H.

It will be understood that the wavy lines in the structural formulas indicate that the valence bond is linked to the rest of the molecule. For example, the wavy line in the structural formula D indicates that the valence bond is linked to L.

In some embodiments, D has a structure represented by formula (D-2a) or formula (D-2b): 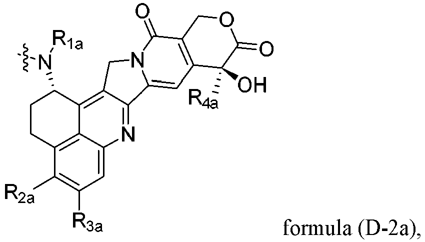
wherein R₁ₐ, R₂ₐ, R₃ₐ, and R₄ₐ are as defined above; or 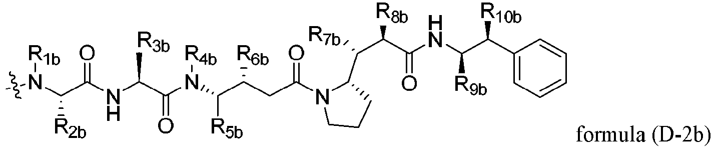
wherein R_{1b}, R_{2b}, R_{3b}, R_{ab}, R_{5b}, R_{6b}, R_{7b}, R_{8b}, R_{9b}, and R_{10b} are as defined above.

In some embodiments, D has a structure represented by formula (D-3a) or (D-3b):

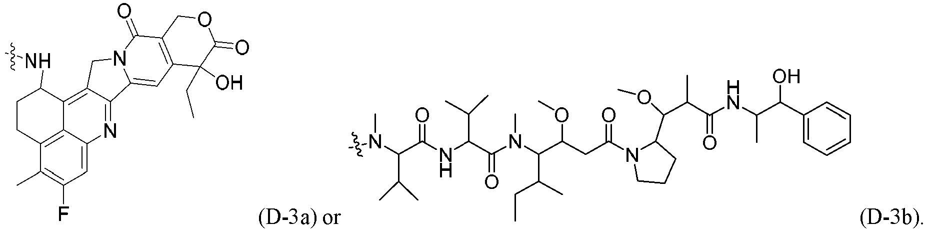

In some embodiments, D has a structure represented by formula (D-4a) or (D-4b):

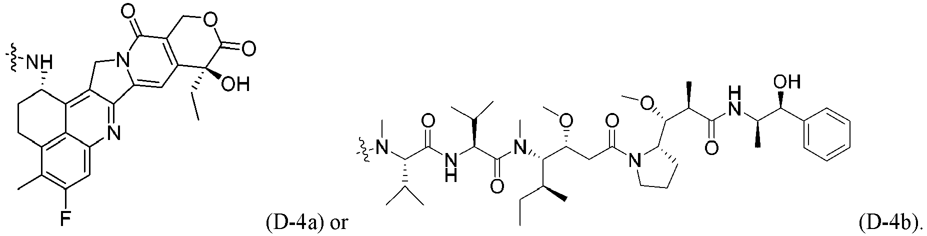

In some embodiments, in the ADC of the present disclosure, the drug is exatecan, Dxd, SN-38, monomethyl auristatin E (MMAE), or MMAF. The structural formulas are shown below:

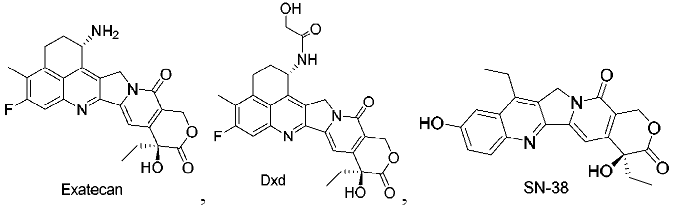

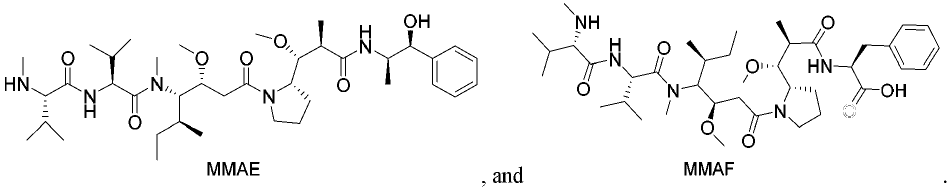

In some embodiments, -L- has the structure below: -Z-L₁-L₂-L₃-
wherein
Z is selected from 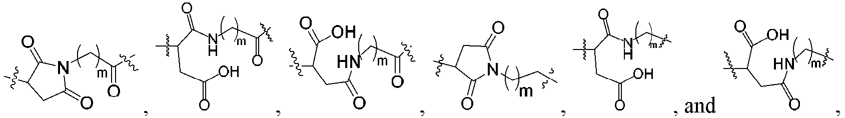
wherein m is an integer selected from 1-10, e.g., 1, 2, 3, 4, 5, 6, 7, or 8;
L₁ is selected from absent, 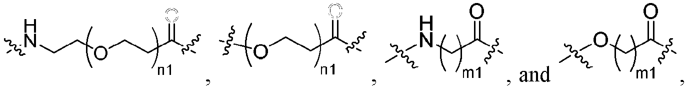 wherein n1 and m1 are each independently an integer selected from 0-20, e.g., an integer selected from 0-12, e.g., 1, 2, 3, 4, 5, 6, 7, or 8;
L₂ is an amino acid residue or a peptide consisting of 2-8 amino acids; and
L₃ is selected from 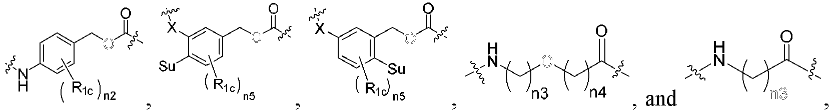 wherein X is selected from -NH-, -O-, and -S-; each R_{1c} is independently selected from C₁₋₈ alkyl, C₁₋₈ haloalkyl-, C₁₋₈ alkoxy, halogen, nitro, and cyano; each Su is independently selected from pentose, penturonic acid, hexose, and hexuronic acid; n2 is 0, 1, 2, 3, or 4; n5 is 0, 1, 2, or 3; n3 and n4 are independently 1, 2, 3, 4, 5, or 6; and wherein Z is linked to S on Ab, and L₃ is linked to D.

In some embodiments, -L- has the structure below: -Z-L₁-L₂-L₃-
wherein
Z is selected from 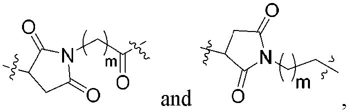 wherein m is an integer selected from 1-10, e.g., 1, 2, 3, 4, 5, 6, 7, or 8;
L₁ is selected from absent, 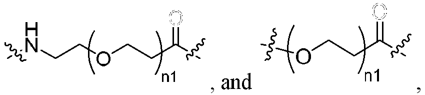 wherein n1 is independently an integer selected from 0-12, e.g., 1, 2, 3, 4, 5, 6, 7, or 8;
L₂ is a peptide consisting of 2 to 8 amino acids; and
L₃ is selected from 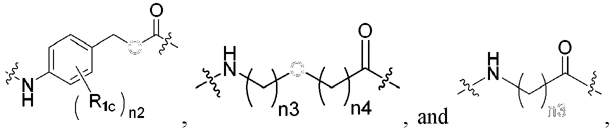 wherein R_{1c} is selected from H and C₁-C₆ alkyl; n2 is 1, 2, 3, or 4; and n3 and n4 are independently 1, 2, 3, 4, 5, or 6.

It should be understood that in -Z-L₁-L₂-L₃- described above, Z is linked to Ab, e.g., to S on Ab, and L₃ is linked to D.

In some embodiments, Z is selected from 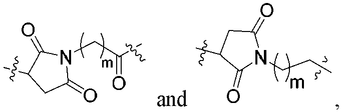 wherein m is 1, 2, 3, 4, 5, 6, 7, or 8.

In some embodiments, Z is selected from

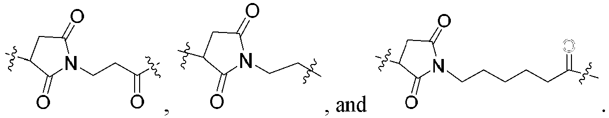

In some embodiments, L₁ is selected from absent, 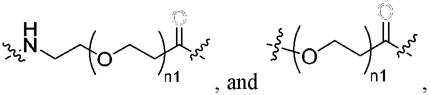 wherein n1 is independently an integer selected from 0-12, for example, 1, 2, 3, 4, 5, 6, 7, or 8.

In some embodiments, L₁ is selected from absent,

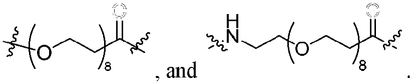

In some embodiments, L₂ is an amino acid residue or a peptide consisting of 2, 3, 4, 5, 6, or 7 amino acids. In some embodiments, L₂ is an amino acid residue. In some embodiments, L₂ is a peptide consisting of 2, 3, 4, 5, 6, or 7 amino acids.

In some embodiments, the amino acid residue or amino acid is preferably an L-amino acid. Moreover, in addition to the α-amino acid, the amino acid residue or amino acid may be an amino acid residue or amino acid having a structure such as β-alanine, ε-aminohexanoic acid, γ-aminobutyric acid, or the like, and may also be an unnatural amino acid, such as an N-methylated amino acid.

In some embodiments, said amino acid residue or amino acid is each independently selected from valine (Val), alanine (Ala), glycine (Gly), lysine (Lys), citrulline (Cit), glutamine (Gln), glutamic acid (Glu), phenylalanine (Phe), leucine (Leu), tyrosine (Tyr), serine (Ser), aspartic acid (Asp), asparagine (Asn), isoleucine (Ile), arginine (Arg), proline (Pro), methionine (Met), tryptophan (Trp), cysteine (Cys), histidine (His), and threonine (Thr). In some embodiments, said amino acid residue or amino acid is each independently selected from glycine (Gly), valine (Val), alanine (Ala), citrulline (Cit), phenylalanine (Phe), lysine (Lys), glutamic acid (Glu), and glutamine (Gln). In some embodiments, said amino acid residue or amino acid is each independently selected from glycine (Gly), valine (Val), alanine (Ala), citrulline (Cit), and glutamic acid (Glu).

In some embodiments, L₂ is selected from -Ala-, -Val-, -Gly-, -Val-Ala-, -Val-Cit-, -Glu-Val-Cit-, and -Gly-Gly-Phe-Gly-.

In some embodiments, L₂ is selected from -Gly-, -Val-Ala-, -Val-Cit-, -Glu-Val-Cit-, and -Gly-Gly-Phe-Gly-. In some embodiments, L₂ is -Gly-. In some embodiments, L₂ is selected from -Val-Ala-, -Gly-Gly-Phe-Gly-, -Val-Cit-, and -Glu-Val-Cit-.

It should be understood that L₂ is linked to L₁ or Z through the amino group of the amino acid on the left and to L₃ through the carbonyl group of the amino acid on the right, which is consistent with the explanation below.

In some embodiments, L₃ is selected from: 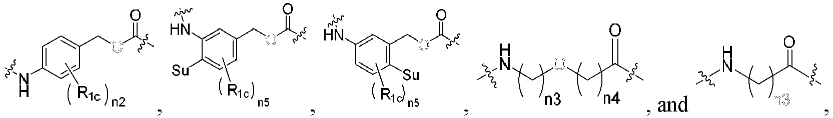
wherein each R_{1c} is independently selected from C₁₋₈ alkyl, C₁₋₈ haloalkyl-, C₁₋₈ alkoxy, halogen, nitro, and cyano;
each Su is independently selected from 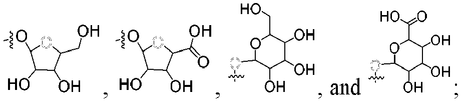 n2 is 0, 1, 2, 3, or 4; n5 is 0, 1, 2, or 3; and n3 and n4 are independently 1, 2, 3, 4, 5, or 6.

In some embodiments, L₃ is selected from: 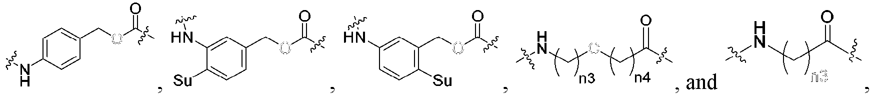 wherein the variables are as defined herein.

In some embodiments, L₃ is selected from: 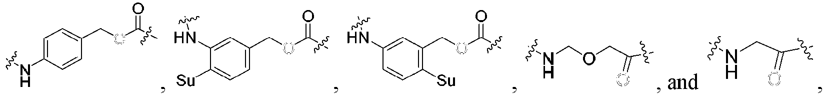 wherein the variables are as defined herein.

In some embodiments, L₃ is selected from 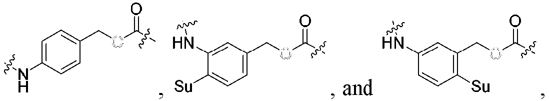 wherein the variables are as defined herein.

In some embodiments, Su is selected from xylose, arabinose, xyluronic acid, arabinuronic acid, glucose, galactose, mannose, glucuronic acid, galacturonic acid, and mannuronic acid.

In some embodiments, Su is selected from

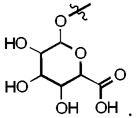

In some embodiments, each Su is independently:

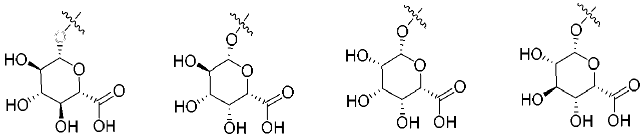

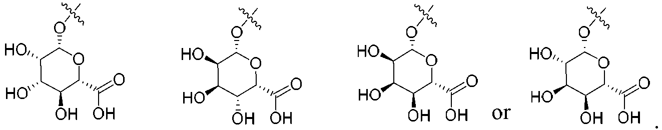

In some embodiments, each Su is independently

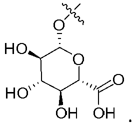

In some embodiments, each Su is independently

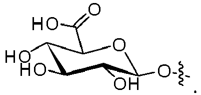

In some embodiments, L₃ is selected from 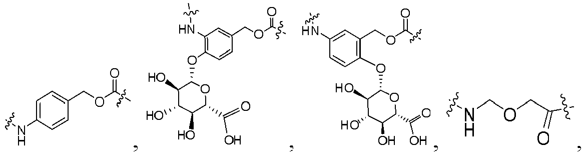 and

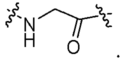

In some embodiments, L₃ is selected from

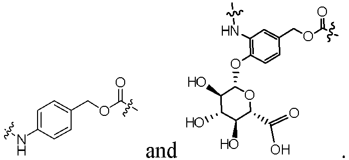

In some embodiments, L₃ is selected from

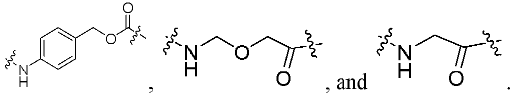

It should be understood that L₃ is linked to L₂ through the amino group on the left and to D through the carbonyl group on the right, which is consistent with the explanation below.

In some embodiments, each -Z-L₁-L₂-L₃- is independently selected from the following structures: 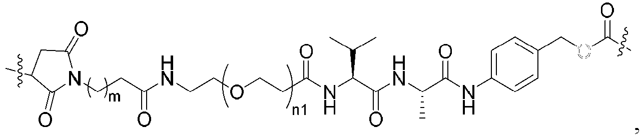 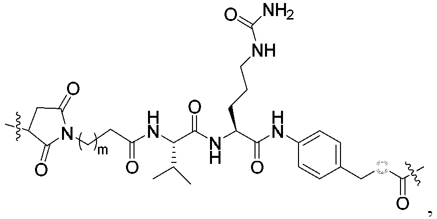 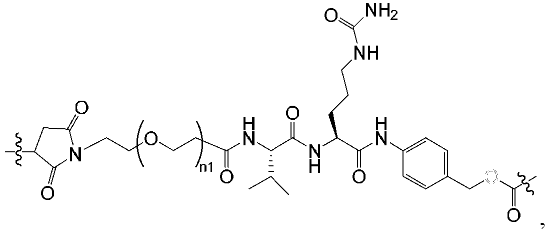 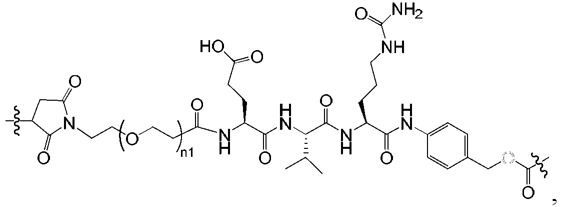 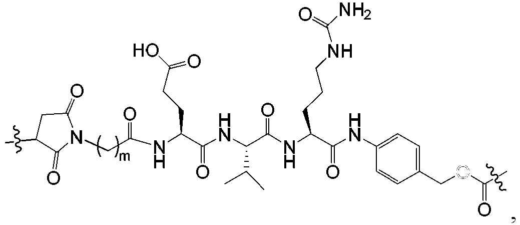 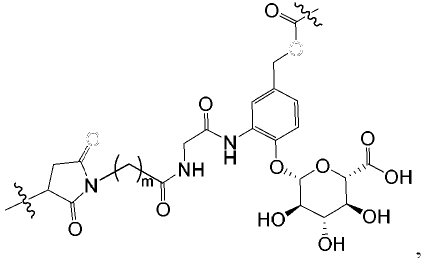 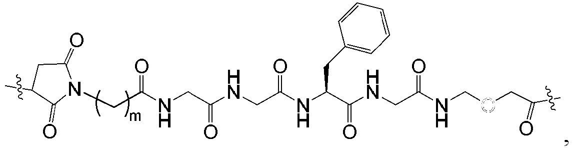 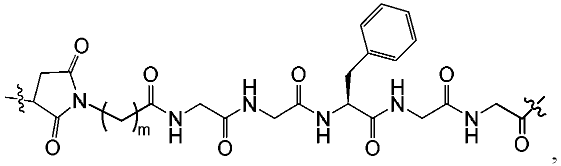 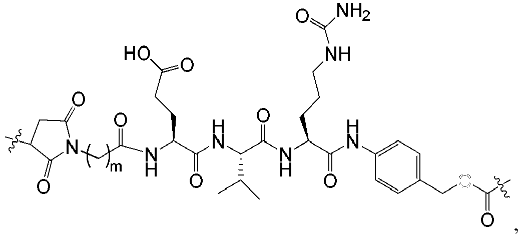 and 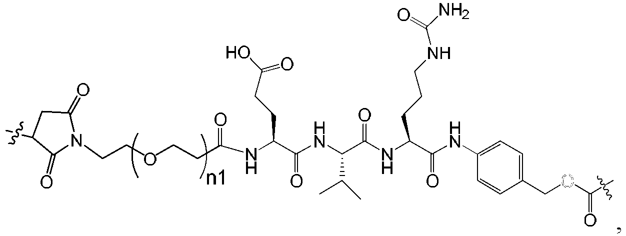
wherein each m is independently an integer selected from 1-10, for example, 1, 2, 3, 4, 5, 6, 7, or 8;
n1 is independently an integer selected from 0-12, e.g., 1, 2, 3, 4, 5, 6, 7, or 8, preferably 6 or 8; and
wherein the left side of the group is linked to S on Ab, and the right side is linked to D.

In some embodiments, each -Z-L₁-L₂-L₃- is independently selected from the following structures: 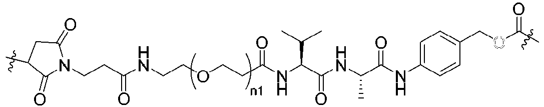 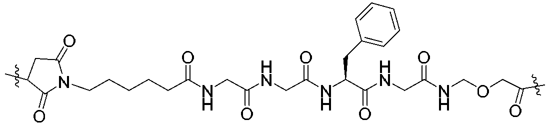 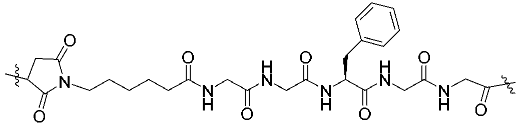 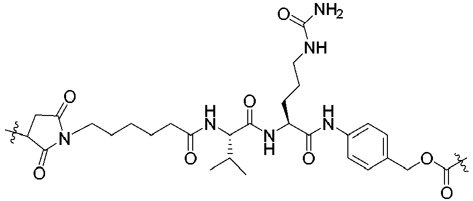 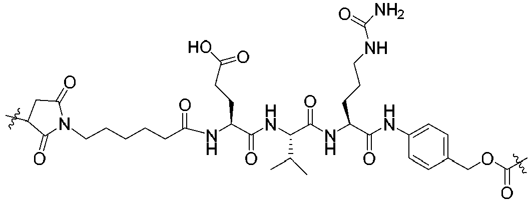 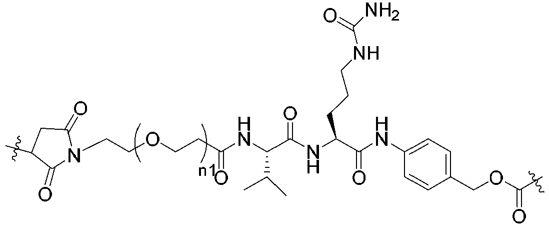 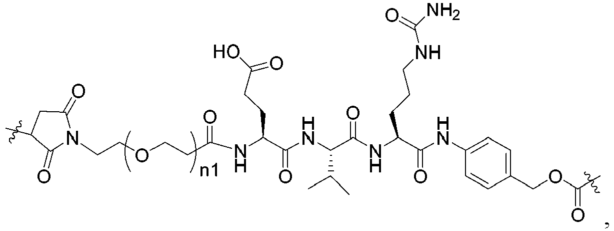 wherein n1 is independently an integer selected from 0-8, for example, 1, 2, 3, 4, 5, 6, 7, or 8, preferably 8.

It should be understood that, without particular description and without contradiction according to the context, for the ADCs of the present disclosure, the bond on the left side of the divalent group as shown herein is linked to Ab or a group near the Ab end, and the bond on the right side of the divalent group is linked to D or a group near the D end. For example, when L₂ is 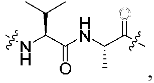 the amino group on the left is linked to L₁ and the carbonyl group on the right is linked to L₃.

In some embodiments, the antibody-drug conjugate has an average DAR of 2-10, 6-10, 4-8, 7-9, 2-4, or 2-6.

In some embodiments, the antibody-drug conjugate is selected from: 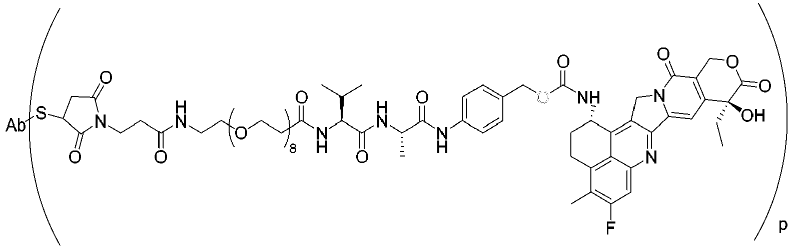 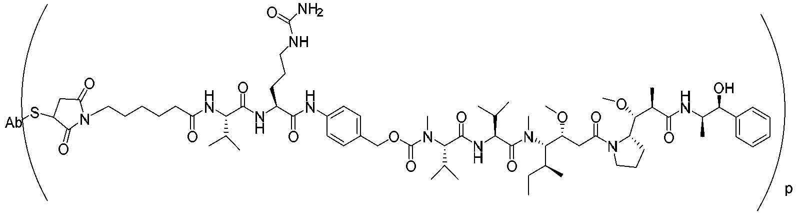 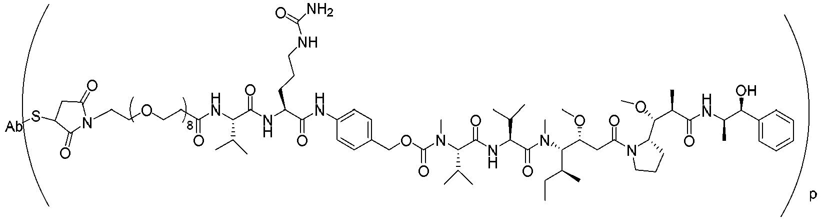 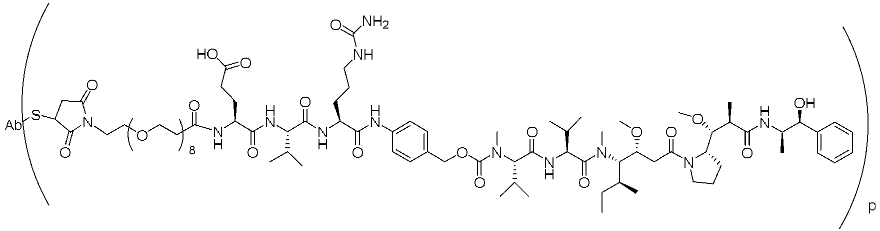 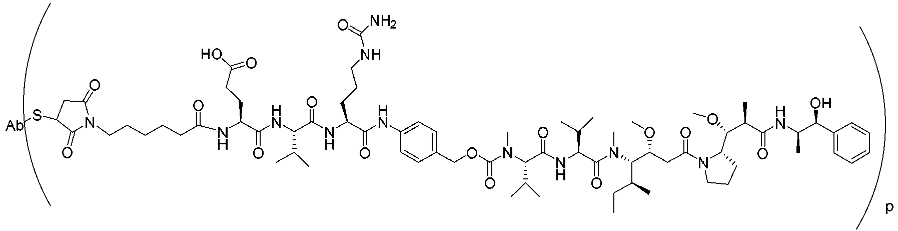 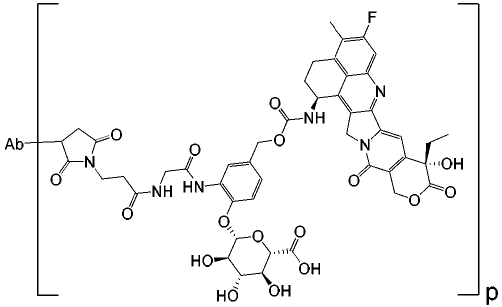 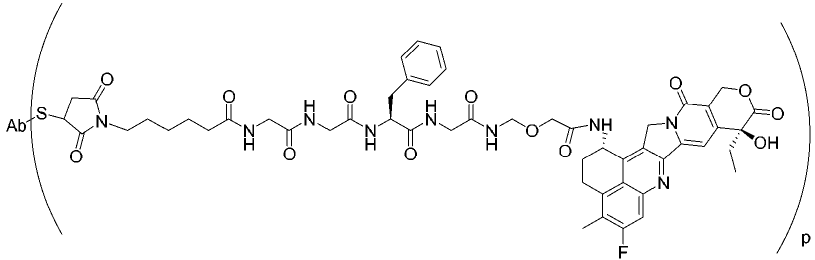 wherein Ab is the binding molecule of the present disclosure, such as a multispecific antibody, preferably V-17-Fc, V-20-Fc, V-23-Fc, and V-26-Fc; p is as defined above; for example, p is an integer selected from 1 to 16, e.g., an integer selected from 1-10, 1-9, 2-8, 4-10, 6-8, 3-7, 4-6, and 2-6, e.g., 1, 2, 3, 4, 5, 6, 7, 8, 9, 10, 11, or 12. Preferably, the antibody-drug conjugate has an average DAR of, e.g., 2-10, 6-10, 4-8, 7-9, 2-4, or 2-6. In some embodiments, the multispecific antibody is a multispecific antibody according to the third aspect of the present disclosure. In some particularly preferred embodiments, the multispecific antibody comprises a first polypeptide chain and a second polypeptide chain, wherein:
(i) the first polypeptide chain comprises or consists of an amino acid sequence of SEQ ID NO: 71, and the second polypeptide chain comprises or consists of an amino acid sequence of SEQ ID NO: 73;
(ii) the first polypeptide chain comprises or consists of an amino acid sequence of SEQ ID NO: 72, and the second polypeptide chain comprises or consists of an amino acid sequence of SEQ ID NO: 73;
(iii) the first polypeptide chain comprises or consists of an amino acid sequence of SEQ ID NO: 86, and the second polypeptide chain comprises or consists of an amino acid sequence of SEQ ID NO: 87.

It should be understood that the S atom linked to Ab in the above ADC is derived from antibody Ab. Disulfide bonds in Ab (e.g., an interchain disulfide bond) are opened by a reducing agent, such as TCEP, to generate a sulfydryl group (-SH), which is then linked to a terminal functional group of the linker, such as a maleimide moiety. In some embodiments, the S atom linked to Ab is from cysteine of Ab.

It should be noted that the above and other technical solutions of the present disclosure and one or more features thereof can be arbitrarily combined to form technical solutions not directly described herein, and these technical solutions not directly described are also encompassed within the scope of the present application.

### Preparation of the ADC molecule of the present disclosure

In another aspect, the present disclosure provides a method for preparing an ADC by using the antibody of the present disclosure. The "ADC" in the present disclosure is defined as an antibody conjugated to an active substance (D, which may also be referred to as a payload) having biological and/or pharmaceutical activity via a linker (L). The method comprises conjugating the antibody (Ab) of the present disclosure to one or more active substances D via one or more linkers (L) (e.g., as defined herein). Preferably, the linker-active substance is site-specifically conjugated to the antibody.

In some embodiments, the method comprises: preparing an Ab for an ADC, which comprises culturing a host cell comprising a nucleic acid encoding the Ab (e.g., any one and/or more polypeptide chains) or an expression vector comprising the nucleic acid, as provided above, under conditions suitable for expressing the Ab or a chain thereof; and optionally isolating the Ab from the host cell (or host cell culture medium).

In some embodiments, the method comprises the following steps:
(a) adding the antibody Ab into a buffer, adding a reducing agent, and then incubating;
(b) adding a linker-payload to the reaction solution in step (a) for conjugating to obtain a crude product; and
(C) optionally purifying the crude product to obtain the antibody-drug conjugate of the present disclosure;
wherein Ab is as defined above.

It should be understood that the linker-payload is reacted with the Ab to provide the -L-D moiety in the compound of formula I, and where -L-D is clearly defined, the structure of the linker-payload can be determined according to the prior art.

In some embodiments, the buffer in step a) is a PBS buffer, preferably with a pH of 5.0-9.0, e.g., 6.0-8.0.

In some embodiments, the reducing agent in step a) is TCEP.

In some embodiments, the linker-payload has the following structure: Z'-L₁-L₂-L₃-D, wherein L₁, L₂, L₃, and D are as defined above, Z' is 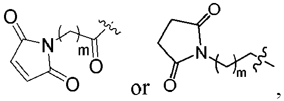 and m is as defined above and is, e.g., 1, 2, 3, 4, 5, 6, 7, or 8.

In some embodiments, Z' is selected from

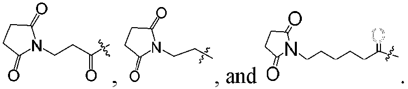

In some embodiments, the method for synthesizing an ADC in which Z is 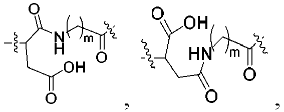 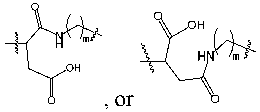 further comprises an additional hydrolysis step of opening the maleimide ring.

In some embodiments, the steps are performed under the specific reaction conditions disclosed in the examples.

It should be noted that the ranges of specific reaction conditions disclosed in the examples, or embodiments resulting from a 100%, 80%, 60%, 40%, 20%, or 10% variation of the specific values, are also contemplated by the present disclosure.

### VI. Sixth aspect of the present disclosure: pharmaceutical composition, pharmaceutical formulation, combination product, and kit

In some embodiments, the present disclosure provides a composition comprising the ISVD, the EGFR-binding molecule, the cMet-binding molecule, or the multispecific antibody or the ADC described herein; preferably, the composition is a pharmaceutical composition or a pharmaceutical formulation. In one embodiment, the composition further comprises a pharmaceutical supplementary material. In one embodiment, the composition comprises the ISVD, the EGFR-binding molecule, the cMet-binding molecule, or the multispecific antibody or ADC of the present disclosure, and a combination of one or more additional therapeutic agents (e.g., a chemotherapeutic drug, a tumor vaccine, an antibody binding to other specific antigens on a tumor cell, or other tumor cell-depleting antibodies).

In some embodiments, the composition of the present disclosure is a pharmaceutical composition or a pharmaceutical formulation, which comprises a suitable pharmaceutical supplementary material, such as a pharmaceutical carrier and a pharmaceutical excipient known in the art, including buffers. As used herein, "pharmaceutical carrier" includes any and all solvents, dispersion media, isotonic agents, absorption delaying agents, and the like that are physiologically compatible. The pharmaceutical carrier suitable for use in the present disclosure may be a sterile liquid, such as water and oil, including those of petroleum, animal, plant, or synthetic origin, such as peanut oil, soybean oil, mineral oil, or sesame oil.

The pharmaceutical composition or the formulation of the present disclosure may further comprise more than one active ingredient required by a particular indication treated, preferably those having complementarity activity without adversely affecting one another. When used in the treatment of cancers, such active ingredients include, but are not limited to, anti-cancer agents and chemotherapeutic agents; when used in the treatment of infectious diseases, such active ingredients include, but are not limited to, antiviral agents and antibiotics. The active ingredients are properly present in combination in amounts effective for the intended use.

In some embodiments, the present disclosure further provides a combination product, which comprises at least one ISVD, EGFR-binding molecule, cMet-binding molecule, or multispecific antibody or ADC of the present disclosure, or further comprises one or more additional anti-tumor agents.

In some embodiments, in the combination product, two or more ingredients may be administered to a subject in combination sequentially, separately, or simultaneously.

In some embodiments, the present disclosure further provides a kit, which comprises the ISVD, the EGFR-binding molecule, the cMet-binding molecule, the multispecific antibody, the ADC, the pharmaceutical composition, or the combination product of the present disclosure, and optionally a package insert directing the administration.

In some embodiments, the present disclosure further provides a pharmaceutical product, which comprises the ISVD, the EGFR-binding molecule, the cMet-binding molecule, the multispecific antibody, the ADC, the pharmaceutical composition, or the combination product of the present disclosure, and optionally further comprises a package insert directing the administration.

### VII. Seventh aspect of the present disclosure: use and method

The ISVD, the EGFR-binding molecules, the cMet-binding molecule, or the multispecific antibody comprising them as disclosed herein has *in vitro* and *in vivo* diagnostic as well as therapeutic use. For example, these molecules may be administered to *in vitro* or *ex vivo* cultured cells or to a subject, e.g., a human subject, to treat and/or diagnose an EGFR antigen- and/or cMet-associated disease, e.g., cancer.

In some embodiments, the present disclosure provides a diagnostic method for detecting the presence of related EGFR and/or cMet antigens in a biological sample, e.g., serum, semen, urine, or a tissue biopsy sample (e.g., from a hyperproliferative or cancerous lesion), *in vitro* or *in vivo.* The diagnostic method comprises: (i) contacting a sample (and optionally, a control sample) with the ISVD, the EGFR-binding molecule, the cMet-binding molecule, or the multispecific antibody described herein or administering the ISVD, the EGFR-binding molecule, the cMet-binding molecule, or the multispecific antibody to a subject under conditions enabling interactions, and (ii) detecting the formation of a complex between the ISVD, the EGFR-binding molecule, the cMet-binding molecule, or the multispecific antibody and the sample (and optionally, the control sample). The formation of the complex indicates the presence of the relevant antigen and may show the suitability for or requirements of the treatment described herein.

In some embodiments, the relevant antigen is detected prior to treatment, e.g., prior to an initial treatment or prior to a certain treatment after a treatment interval. Detection methods that can be used include immunohistochemistry, immunocytochemistry, FACS, ELISA assays, PCR techniques (e.g., RT-PCR), or *in vivo* imaging techniques. Generally, the ISVD, the EGFR-binding molecule, the cMet-binding molecule, or the multispecific antibody used in *in vivo* and *in vitro* detection methods is directly or indirectly labeled with a detectable substance to facilitate the detection of bound or unbound conjugates. Suitable detectable substances include various biologically active enzymes, prosthetic groups, fluorescent substances, luminescent substances, paramagnetic (e.g., nuclear magnetic resonance active) substances, and radioactive substances.

In some embodiments, the level and/or distribution of related antigens are/is determined *in vivo.* For example, the ISVD, the EGFR-binding molecule, the cMet-binding molecule, or the multispecific antibody of the present disclosure labeled with a detectable substance is detected in a non-invasive manner, e.g., by using appropriate imaging techniques such as positron emission tomography (PET) scanning. In one embodiment, for example, the level and/or distribution of related antigens are/is determined *in vivo* by detecting the ISVD, the EGFR-binding molecule, the cMet-binding molecule, or the multispecific antibody of the present disclosure that is detectably labeled with a PET reagent (e.g., ¹⁸F-fluorodeoxyglucose (FDG)).

In one embodiment, the present disclosure provides a diagnostic kit, which comprises the ISVD, the EGFR-binding molecule, the cMet-binding molecule, or the multispecific antibody described herein and an instruction for use.

In some embodiments, the present disclosure relates to use of the ISVD, the EGFR-binding molecule, the cMet-binding molecule, or the multispecific antibody or ADC of the present disclosure *in vivo* for treating cancer and inhibiting the growth or metastasis of a tumor expressing EGFR and/or cMet in a subject, thereby inhibiting or reducing the growth or metastasis of the cancer. The EGFR-binding molecule, the cMet-binding molecule, or the multispecific antibody or the ADC of the present disclosure may be used alone. Alternatively, the ISVD, the EGFR-binding molecule, the cMet-binding molecule, or the multispecific antibody or the ADC of the present disclosure may be administered in combination with other cancer therapeutics/prophylactic agents. When the ISVD, the EGFR-binding molecule, the cMet-binding molecule, or the multispecific antibody or the ADC of the present disclosure is administered in combination with one or more other drugs, the combination may be administered in any order or simultaneously.

Thus, in one embodiment, the present disclosure provides a method for treating cancer, which comprises administering to a subject a therapeutically effective amount of the ISVD, the EGFR-binding molecule, the cMet-binding molecule, the multispecific antibody, or the ADC described herein. In another embodiment, the present disclosure provides a method for preventing the development of cancer resistance in a subject, which comprises administering to the subject a therapeutically effective amount of the ISVD, the EGFR-binding molecule, the cMet-binding molecule, or the multispecific antibody or the ADC described herein.

In some embodiments, the cancer treated with the ISVD, the EGFR-binding molecule, the cMet-binding molecule, or the multispecific antibody includes, but is not limited to, a cancer expressing EGFR and/or cMet, e.g., lung cancer (e.g., lung squamous cell carcinoma, lung adenocarcinoma, and non-small cell lung cancer), breast cancer (e.g., triple negative breast cancer), colon cancer, and pharyngeal squamous cell carcinoma.

The following examples are described to facilitate the understanding of the present disclosure. The examples are not intended to be and should not be construed in any way as limiting the scope of protection of the present disclosure.

### Examples

### Example 1. Preparation and Purification of Reference Benchmark Antibody and Reference Benchmark Antibody-Drug Conjugate

Reference benchmark antibody JNJ-61186372 is a bispecific antibody targeting EGFR and cMet, and it consists of an anti-EGFR half antibody and an anti-cMET half antibody. JNJ-61186372 was generated based on the sequences of SEQ ID NOs:199-202 and preparation method in patent WO2014081954A1.

Reference benchmark antibody ABT-700 is a monospecific IgG antibody targeting cMet, and it was prepared based on the sequences of SEQ ID NOs: 86-87 in the patent CN109562189B and the method described therein.

Reference benchmark antibody RAA22/B09-57 (also referred to as BMK-AZD or AZD Ab herein) is a bispecific antibody targeting EGFR and cMet, and it consists of an anti-EGFR half antibody and an anti-cMET half antibody and was prepared based on the sequences of SEQ ID NOs: 59-62 in Patent Application No. US2023/0183358A1 and the method described therein.

The reference benchmark antibody-drug conjugate ABBV399 is formed by conjugation of a cMet-targeting monospecific antibody (ABT-700) to an MMAE molecule, and can be prepared based on the sequences set forth in SEQ ID NOs: 86-87 and the conjugation method in the patent CN109562189B.

The reference benchmark antibody-drug conjugate AZD9592 (also referred to as AZD ADC herein) is formed by conjugation of a bispecific antibody targeting EGFR and cMet (RAA22/B09-57) to a camptothecin molecule, and can be prepared based on the sequences of SEQ ID NOs: 59-62 and the conjugation method in Patent Application No. US2023/0183358A1.

### Example 2. Generation of ISVDs Specifically Binding to EGFR and cMet Respectively

### 2.1 Alpaca immunization

Recombinant his-tagged human EGFR ECD protein (iCareAb Biotechnology) and recombinant his-tagged human cMet ECD protein (iCareAb Biotechnology) were used as target antigens for alpaca immunization. The immunization process was performed as follows.

Two healthy adult alpacas (iCareAb Biotechnology) were selected, 0.5 mg of the target antigen (recombinant his-tagged human EGFR ECD protein or recombinant his-tagged human cMet ECD protein) was mixed well with Gerbu adjuvant (GERBU biochemicals GmbH) according to a ratio of 1:1, and the alpacas were immunized by subcutaneous multipoint injection into the cervical lymph nodes. The dose for the first immunization was 500 µg and the dose for the subsequent immunizations was 250 µg. A total of 3-4 immunizations were performed, and the immunization interval was 18-21 days. Seven days after the second immunization and the third immunization, 5 mL of jugular vein blood of the alpacas was collected for ELISA serum titer assay. The results showed that the criteria for blood collection for library construction were successfully met after three rounds of immunization, and the blood collection for library construction was then arranged.

### 2.2 CONSTRUCTION AND SELECTION OF PHAGE DISPLAY LIBRARY

As described in Example 2.1, after the third immunization of the alpacas with EGFR or cMet separately, 100 mL of jugular vein blood of the alpacas was collected and centrifuged to extract peripheral blood mononuclear cells (PBMCs). Total RNA was extracted from PBMCs, and cDNA was generated by reverse transcription using a PrimeScript reverse transcription kit (Takara) with RNA as a template. With cDNA as a template, nucleic acid fragments of conventional IgG (VH) and heavy chain-only IgG (comprising VHH as ISVD) lacking the CH1 domain were generated in the first round of PCR amplification. The two types of nucleic acids were separated on agarose gels, and the coding nucleic acid comprising VHH was extracted, purified, and then subjected to a second round of PCR amplification to obtain a nucleic acid fragment containing only a VHH gene fragment. The extracted and purified VHH gene fragment was inserted into a phage display vector, and the vector was electroporated into *E*. *coli* competent cells, and the bacterial solution was cryopreserved at -80 °C.

The *E. coli* ER2738 bacterial solution was thawed, and the thawed bacterial solution was inoculated into 100 mL of 2YT-A culture medium (Shanghai Sangon Biotech). A helper phage (New England Biolabs) was added for infection, and the bacterial cells were resuspended in 2× YT-AK culture medium (Shanghai Sangon Biotech) and cultured at 37 °C and 200 rpm overnight. The culture supernatant was collected, and recombinant phages were prepared using a PEG/NaCl precipitation method to prepare an anti-EGFR phage display library and an anti-cMet phage display library separately. The anti-EGFR phage display library and the anti-cMet phage display library were subjected to enrichment panning using biotin-human EGFR protein and biotin-human cMet protein for subsequent selection of positive clones by ELISA.

### 2.3 SELECTION OF POSITIVE CLONES BY ELISA AND SEQUENCING

The expression supernatants of the *E. coli* ER2738 clones obtained by enrichment panning in Example 2.2 were assayed by an ELISA binding assay. The assay process was performed as follows.

The antigen (human EGFR-his antigen or human cMet-his antigen) was diluted to 2 µg/mL with a PBS buffer and then applied to coat a 96-well ELISA plate, and the plate was incubated overnight at 4 °C. The antigen-coated plate was washed 5 times with PBST, blocked with a PBST blocking solution containing 5% skim milk at room temperature for 1 h, and then washed 6 times with PBST. The expression supernatant of the *E. coli* ER2738 obtained in Example 2.2 was added, and the plate was incubated at 37 °C for 1 h while shaking. The plate was washed 6 times with PBST, and an anti-M13-HRP secondary antibody (iCareAb Biotechnology (Suzhou) Co., Ltd.) diluted with PBS was added. The plate was shaken at 37 °C for 45 min. The plate was washed 5 times with PBST, a TMB chromogenic solution was added, and color development was performed in the dark for 5-15 min. A stop solution was then added. The plate was read using a microplate reader, and the absorbance values at OD_{450 nm}-OD _{650 nm} were measured. Bacterial clones with a reading value greater than 1 were selected for Sanger sequencing. A single clone of ER2738 strain containing the corresponding positive VHH sequence was selected, and glycerol was added. The mixture was cryopreserved in a refrigerator at -80 °C.

### 2.4 PRODUCTION OF VHH-HIS AND VHH-FC

A positive anti-EGFR VHH sequence and a positive anti-cMET VHH sequence were obtained by PCR from an anti-EGFR positive clone and an anti-cMET positive clone, respectively, and a tag (a 6×His tag or an Fc tag was added to the C-terminus: hIgG1 isotype) was added to the C-terminus. The constructs were then separately inserted into an expression vector pcDNA3.4. HEK-293F cells (hereinafter also referred to as "293F cells") were transiently transfected, and the supernatant was collected. After the primary purification of the 6×His-tagged protein using a Ni column, the protein was further subjected to polishing purification using an ion exchange column; the hFc fusion protein was purified using a Protein A column. The purities of the VHH antibodies were determined by SDS-PAGE and SEC-HPLC. Thus, two kinds of tagged VHHs targeting EGFR, anti-EGFR VHH-his and anti-EGFR VHH-Fc, were obtained; and two kinds of tagged VHHs targeting cMet, anti-cMet VHH-his and anti-cMet VHH-Fc, were obtained.

### 2.5 ASSAY ON BINDING OF ANTIBODIES TO TARGET CELLS BY FACS

The binding of antibodies expressed in the supernatant of transiently transfected HEK-293F to target cells was assayed by FACS.

Specifically, 100 µL of the anti-EGFR supernatant and 100 µL of the anti-cMet supernatant obtained by transient transfection of HEK-293F in Example 2.4 were collected as test samples. The binding of the anti-EGFR supernatant to target cells CHO-S-EGFR or CHO-S was assayed by FACS, and the binding of the anti-cMet supernatant to target cells CHO-S-cMet or CHO-S was assayed by FACS. CHO-S-EGFR cell is short for a CHO-S ENGINEERING CELL LINE EXPRESSING HUMAN EGFR. CHO-S-cMet cell is short for a CHO-S ENGINEERING CELL LINE EXPRESSING HUMAN CMET.

FACS ASSAY WAS PERFORMED AS FOLLOWS. The cells were seeded in a 96-well plate at a density of 3 × 10⁵ cells/well and centrifuged at 300 g at 4 °C for 5 min. The test supernatants (100 µL/well) were added, and the mixtures were incubated for one hour. A secondary antibody PE-anti-human IgG (invitrogen, Cat#: 12-4998-82) was added, and the mixtures were then incubated at 4 °C for half an hour. The mean fluorescence intensity (MFI) of the cells was assayed by a flow cytometer (Life Technologies) and analyzed using FlowJo. The results showed that the 23 anti-EGFR VHHs obtained after primary screening all bound to the target cell CHO-S-EGFR to different degrees. The 18 anti-cMet VHHs obtained after primary screening all bound to the target cell CHO-S-cMet to different degrees. The anti-EGFR supernatant and the anti-cMet supernatant showed little binding to CHO-S cells.

### EXAMPLE 3. Assay on In Vitro Biological Activity of EACH VHH as ISVD

### 3.1 BINDING OF VHHS TO CELLS BY FACS

The binding of the Fc-tagged anti-EGFR VHHs and Fc-tagged anti-cMet VHHs prepared in Example 2 to target cells was assayed by FACS. In the assay on anti-EGFR VHHs, the target cells used were EGFR-expressing tumor cells NCI-H1975 (human lung adenocarcinoma cell line); in the assay on anti-cMet VHHs, the target cells used were cMet-expressing tumor cells EBC-1 (human lung squamous cell carcinoma cell line). FACS assay was performed as follows.

The target cells were seeded in a 96-well plate at a density of 1.5 × 10⁵ cells/well and centrifuged at 300 g at 4 °C for 5 min. An anti-EGFR VHH-Fc (375 nM, 3-fold serially diluted) or an anti-cMet VHH-Fc (50 nM, 4-fold serially diluted) was added for resuspension, and the plate was incubated at 4 °C for 1 h. A secondary antibody PE-anti-human IgG (eBioscience, cat#: 12-4998-82) was added, and the mixtures were incubated at 4 °C for half an hour. The MFI of the cells was assayed by a flow cytometer (Life Technologies).

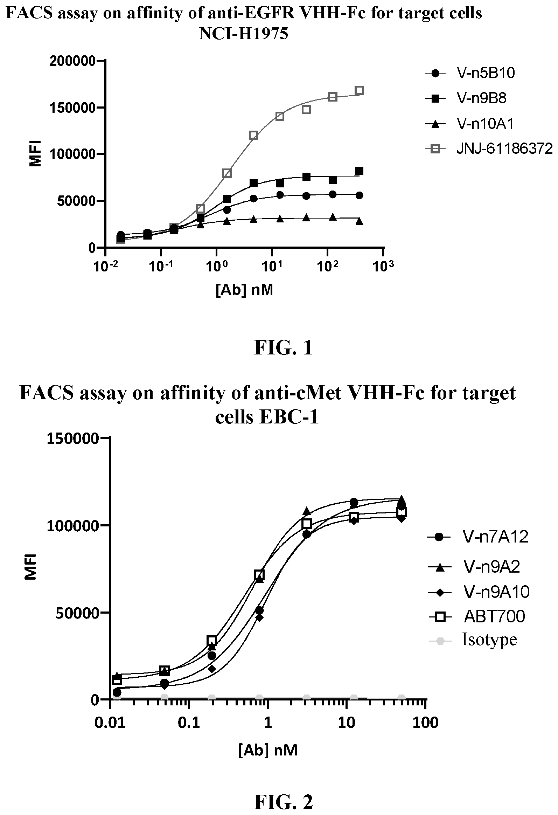

The assay results of the anti-EGFR VHH-Fc are shown in FIG. 1. As anti-EGFR VHH candidates, V-n5B10, V-n9B8, and V-n10A1 bound to target cells in a dose-dependent manner, and their binding activity is lower than that of the control antibody. For ease of description, the anti-EGFR VHHs of V-n5B10, V-n9B8, and V-n10A1 are sometimes also referred to as 5B10, 9B8, and 10A1, respectively, subsequently and elsewhere herein.

The assay results of the anti-cMet VHH-Fc are shown in FIG. 2. As anti-cMet VHH candidates, V-n7A12, V-n9A2, and V-n9A10 bound to target cells in a dose-dependent manner. For ease of description, the anti-cMET VHHs of V-n7A12, V-n9A2, and V-n9A10 are sometimes also referred to as 7A12, 9A2, and 9A10, respectively, subsequently and elsewhere herein.

### 3.2 Determination of blocking effect of anti-cMet VHH-Fc on binding of ligand HGF to target cells EBC-1

Target cells EBC-1 were seeded in a 96-well plate at a density of 1.5 × 10⁵ cells/well and centrifuged at 300 g at 4 °C for 5 min. 50 µL of ligand HGF-His (1 µg/mL) (Beijing ACROBiosystems Co., Ltd.) was added, and the mixture was incubated for 1 h. Then, 50 µL of anti-cMet VHH-Fc V-n7A12, V-n9A2, or V-n9A10 (100 nM, 5-fold serially diluted) was added, followed by mixing and incubation for 1 h. The secondary antibody iF647-anti-his (Genscript) was added. The mixtures were incubated at 4 °C for half an hour, and the MFI of the cells was assayed by a flow cytometer (Life Technologies).

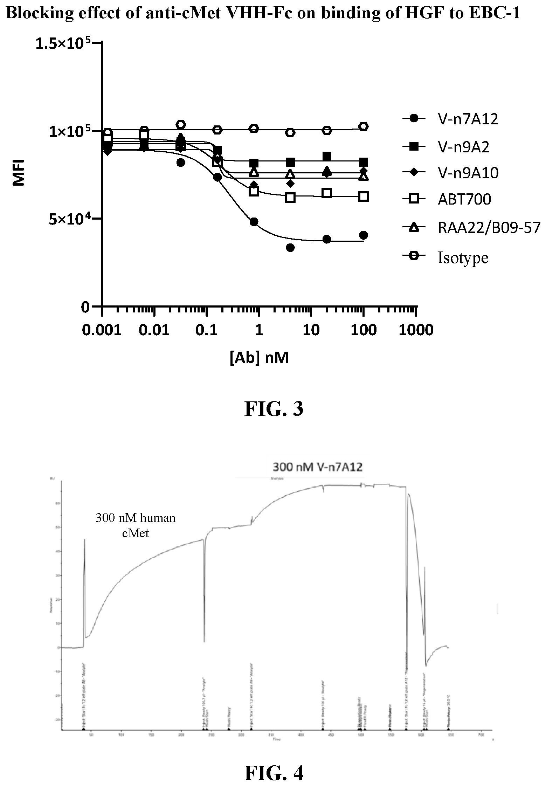

The results are shown in FIG. 3. V-n7A12, V-n9A2, and V-n9A10 blocked the binding of EBC-1 cells to the ligand HGF, and their MFI values at the highest concentration were 40640, 82172, and 77193, respectively. The ratios of MFI value at the highest concentration to that at the lowest concentration were 2.22, 1.12, and 1.14, respectively. The above results showed that the candidate molecule V-n7A12 blocked the binding of EBC-1 cells to the ligand HGF in a dose-dependent manner. V-n9A2 and V-n9A10 showed weak blocking of the binding of EBC-1 cells to the ligand HGF.

### 3.3 EPITOPE IDENTIFICATION

Whether the V-n7A12 and V-n9A2 antibodies in the anti-cMet VHH-Fc format bound to the same epitope on the antigen cMet was determined by surface plasmon resonance (SPR) technique.

The V-n9A2 antibody was immobilized with a CM5 sensor chip (Cytiva). Human cMet antigen (300 nM) was injected onto the sensor chip at a flow rate of 50 µL/min, with an association phase of 200 s. Then, the anti-cMet antibody V-n7A12 was injected onto the sensor chip at a flow rate of 50 µL/min, with an association phase of 120 s.

The results are shown in FIG. 4. V-n9A2 bound to the human cMet antigen without affecting the binding of V-n7A12 to the human cMet antigen. This indicates that the anti-cMet antibodies V-n7A12 and V-n9A2 bound to different epitopes on the antigen cMet.

### 3.4 FACS ASSAY ON ENDOCYTOSIS of each VHH-Fc BY CELLS

The internalization ability of the tumor target cells to the anti-EGFR VHH-Fc and anti-cMet VHH-Fc prepared in Example 2 was assayed by FACS.

Tumor target cells NCI-H1975 and EBC-1 were prepared and seeded in a 96-well plate at 1-1.5 × 10⁵ cells/well. An anti-EGFR VHH-Fc (125 nM, 3-fold dilution) or an anti-cMet VHH-Fc (50 nM, 4-fold dilution) prepared in Example 2 was added separately, and the plate was incubated at 4 °C for 30 min. The supernatants containing respective VHH-Fcs were removed by centrifugation. The cells were evenly divided into 2 groups and separately incubated at 4 °C and 37 °C for 4 h. After the incubation was completed, PBS chilled in ice bath was immediately added to terminate the endocytosis experiment. The secondary antibody PE-anti-human IgG (eBioscience, Cat. No. 12-4998-82) was added. The mixtures were incubated at 4 °C for 30 min, and then the MFI of the cells was assayed by a flow cytometer (Life Technologies).

The internalization level of the cell surface-bound antibody was calculated using the formula below: $endocytosis = MFI of sample incubated at 4 ° C - MFI of sample incubated at 37 ° C .$ $Endocytosis rate % = 100 % - \left(MFI of sample incubated at 37 °C/MFI of sample incubated at 4 °C\right) \times 100 % .$

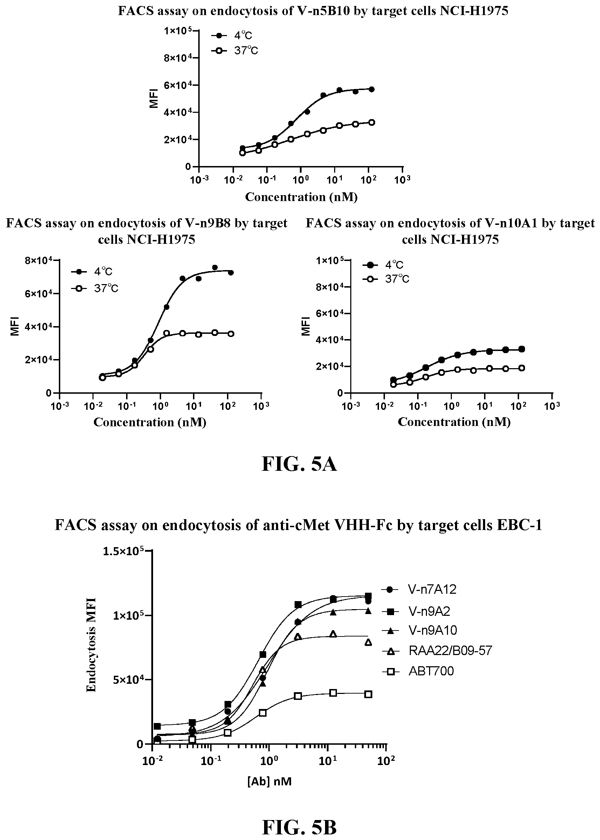

The results of the endocytosis of the candidate molecules serving as the anti-EGFR antibodies by the tumor target cell NCI-H1975 are shown in FIG. 5A, in which the MFI of the sample incubated at 4 °C and the MFI of the sample incubated at 37 °C at the test antibody concentrations are shown. The endocytosis of the candidate molecules serving as the anti-cMet antibodies by the tumor target cell EBC-1 is shown in FIG. 5B, in which the endocytosis MFIs at the test antibody concentrations determined according to the above formula were shown.

### 3.5 ELISA ASSAY ON CROSS-REACTIVITY OF each VHH-Fc WITH ANTIGENS OF DIFFERENT SPECIES

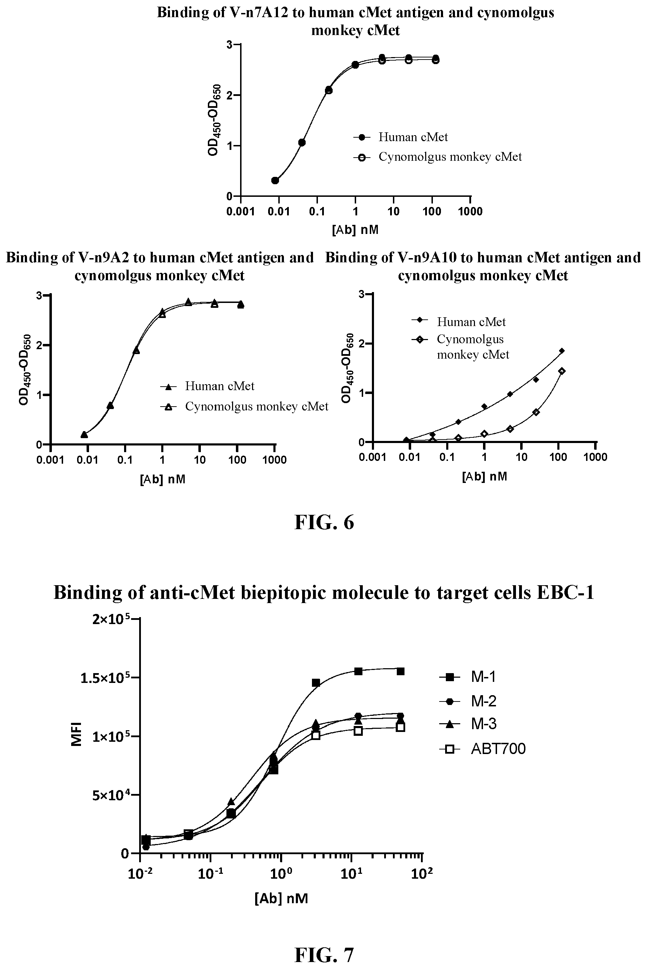

Human cMet-His antigen or cynomolgus monkey cMet-His antigen was diluted to 1 µg/mL with a PBS buffer and then applied to coat a 96-well ELISA plate, and the plate was incubated overnight at 4 °C. The antigen-coated plate was washed 3 times with PBST (300 µL/well), and PBS containing 5% skim milk was added at 200 µL/well. The plate was shaken and blocked at 37 °C for 2 h. The plate was washed 3 times with PBST, and each anti-cMet VHH-Fc 5-fold diluted was added. The plate was incubated at 37 °C for 1 h with shaking. The plate was washed 3 times with PBST, and an anti-human Fc-HRP secondary antibody (Abcam, CAT#: ab97225) diluted with PBS was added at 100 µL/well. The plate was then incubated at 37 °C for 45 min with shaking. Then, the plate was washed 3 times with PBST (300 µL/well), a TMB chromogenic solution was added at 100 µL/well, and color development was performed in the dark for 5-10 min. A stop solution was then added at 50 µL/well. The plate was read using a microplate reader, and the absorbance values at OD_{450 nm}-OD _{650 nm} were measured. The ELISA assay results are shown in FIG. 6 and Table 1.

**Table 1. Binding assay of anti-cMet antibodies to human cMet antigen or monkey cMet antigen by ELISA**

| **Antigen** | **EC₅₀ of anti-cMet antibody (nM)** | | |
|---|---|---|---|
| | **V-n7A12** | **V-n9A2** | **V-n9A10** |
| **Human cMet** | 0.066 | 0.108 | - |
| **Cynomolgus monkey cMet** | 0.063 | 0.105 | - |

| | | | |
|---|---|---|---|
| Note: "-" indicates that the EC₅₀ value could not be fitted. | | | |

The results showed that the anti-cMet candidate molecules V-n7A12 and V-n9A2 exhibited cross-reactivity with the human cMet antigen and the cynomolgus monkey cMet antigen, and exhibited similar binding affinities for human cMet and monkey cMet. However, the affinity of V-n9A10 for the human cMet antigen is significantly different from that for the monkey cMet antigen.

### Example 4. VHH Sequence Optimization and Characterization

### 4.1 VHH SEQUENCE OPTIMIZATION

The original VHH sequences prepared in Example 2 were humanized using the "best-fit method". Alignment and analysis were performed on amino acid sequences of VHH framework regions by using a human germline V gene database to select the optimal germline sequence. Based on the Kabat CDR definition, the humanized VHH sequences were generated by replacing the best-fitted human CDR sequences with VHH CDR sequences. A plurality of residues of the framework regions were back-mutated and, where applicable, post-translational modifications (PTMs) were removed. The sequences were reverse-translated and optimized, and then sent to GENEWIZ (Shanghai, China) for gene synthesis. Then, the genes were constructed into the expression vector pcDNA 3.4 to express the monovalent humanized VHHs with a His tag fused at the C-terminus. Thus, the humanized VHH clone proteins were obtained.

Immunogenicity analysis was performed on the VHH sequences that have undergone humanization and PTM removal. If the sequence is with high risk, amino acid mutations were performed on TCE (T cell epitope) of the sequence to eliminate or reduce the risk of immunogenicity. The mutated sequences were sent to GENEWIZ (Shanghai, China) for gene synthesis and then constructed into pcDNA 3.4 expression vectors to express VHHs with a His tag fused at the C-terminus. Thereby, VHH clone proteins with low immunogenicity risk were obtained.

### Sequence optimization of anti-EGFR VHHs

The parent anti-EGFR antibodies and optimized sequences thereof are shown in Tables 2A-2C below.

**Table 2A. Anti-EGFR VHH-10A1 antibodies and optimized sequences thereof**

| **Antibody name** | **Parent sequence** |
|---|---|
| V-n10A1 | SEQ ID NO: 11 |

| **Antibody name** | **Humanization** |
|---|---|
| zn10A1.m5 | SEQ ID NO: 105 |
| zn10A1.m6 | SEQ ID NO: 106 |

| **Antibody name** | **PTM removal** |
|---|---|
| pn10A1.m1 | SEQ ID NO: 107 |
| pn10A1.m2 | SEQ ID NO: 108 |
| pn10A1.m3 | SEQ ID NO: 109 |
| pn10A1.m4 | SEQ ID NO: 110 |
| pn10A1.m5 | SEQ ID NO: 111 |
| pn10A1.m6 | SEQ ID NO: 112 |

| **Antibody name** | **Humanization + PTM removal** |
|---|---|
| pzn10A1_m6m5 | SEQ ID NO: 113 |
| ppzn10A1_m6m5.m1 | SEQ ID NO: 114 |
| ppzn10A1_m6m5.m3 (also referred to as hu10A1^{b}) | SEQ ID NO: 84 |
| dppzn10A1_m6m5.m3_1.m2 (also referred to as hu10A1) | SEQ ID NO: 36 |

**Table 2B. Anti-EGFR VHH 9B8 antibodies and optimized sequences thereof**

| **Antibody name** | **Parent sequence** |
|---|---|
| V-n9B8 | SEQ ID NO: 6 |

| **Antibody name** | **Humanization** |
|---|---|
| zn9B8.m2 | SEQ ID NO: 100 |
| zn9B8.m4 | SEQ ID NO: 101 |

| **Antibody name** | **Humanization + PTM removal** |
|---|---|
| pzn9B8.m2.m11 (also referred to as hu9B8) | SEQ ID NO: 32 |
| pzn9B8.m2.m12 | SEQ ID NO: 102 |

**Table 2C. Anti-EGFR VHH 5B10 antibodies and optimized sequences thereof**

| **Antibody name** | **Parent sequence** |
|---|---|
| V-n5B10 | SEQ ID NO: 1 |

| **Antibody name** | **Humanization** |
|---|---|
| zn5B10.m16 | SEQ ID NO: 94 |
| zn5B10.m17 | SEQ ID NO: 95 |
| zn5B10.m18 | SEQ ID NO: 96 |
| zn5B10.m20 (also referred to as hu5B10) | SEQ ID NO: 31 |
| zn5B10.m21 | SEQ ID NO: 97 |
| zn5B10.m22 | SEQ ID NO: 98 |
| zn5B10.m23 | SEQ ID NO: 99 |

### Sequence optimization of anti-cMET VHHs

The parent anti-cMet antibodies and optimized sequences thereof are shown in Tables 2D-2E below.

**Table 2D. Anti-cMet VHH 9A2 antibodies and optimized sequences thereof**

| **Antibody name** | **Parent sequence** |
|---|---|
| V-n9A2 | SEQ ID NO: 21 |

| **Antibody name** | **Humanization** |
|---|---|
| VHH8 | SEQ ID NO: 134 |
| VHH9 | SEQ ID NO: 135 |
| VHH10 (also referred to as hu9A2) | SEQ ID NO: 42 |
| VHH11 | SEQ ID NO: 136 |

**Table 2E. Anti-cMet VHH 7A12 antibodies and optimized sequence thereof**

| **Antibody name** | **Parent sequence** |
|---|---|
| V-n7A12 | SEQ ID NO: 16 |

| **Antibody name** | **Humanization** |
|---|---|
| 7A12-M1 | SEQ ID NO: 121 |
| 7A12-M2 | SEQ ID NO: 122 |
| 7A12-M3 | SEQ ID NO: 123 |
| 7A12-M4 | SEQ ID NO: 124 |

| **Antibody name** | **PTM removal** |
|---|---|
| pn7A12.N32X.m1 | SEQ ID NO: 125 |
| pn7A12.N32X.m3 | SEQ ID NO: 126 |
| pn7A12.N32X.m4 | SEQ ID NO: 127 |
| pn7A12.N32X.m6 | SEQ ID NO: 128 |

| **Antibody name** | **Humanization + PTM removal** |
|---|---|
| pzn7A12m4.m1 (also referred to as hu7A12^{b}) | SEQ ID NO: 40 |

| **Antibody name** | **Humanization + PTM removal + primary immunogenicity removal** |
|---|---|
| dpzn7A12m4.m1_1 | SEQ ID NO: 129 |
| dpzn7A12m4.m1_3 | SEQ ID NO: 130 |

| **Antibody name** | **Humanization + PTM removal + secondary immunogenicity removal +** |
|---|---|
| dpzn7A12m4.ml_1.m1 (also referred to as hu7A12) | SEQ ID NO: 39 |

### 4.2. Functional characterization of optimized antibodies

The binding of the anti-EGFR VHH-His candidate antibody molecules V-n10A1, V-n9B8, and V-n5B10, the anti-cMet VHH-His candidate molecules V-n9A2 and V-n7A12, and their corresponding molecules with optimized and engineered sequences to target cells MDA-MB-468 (a triple negative breast cancer cell line), EBC-1 cells (a human non-small cell lung cancer cell line), MKN45 cell line (a human gastric cancer cell line), or CHOK1 cells overexpressing EGFR or cMet was assayed by FACS binding assay. The assay was performed as follows.

The target cells were seeded in a 96-well plate at a density of 1.5 × 10⁵ cells/well and centrifuged at 300 g at 4 °C for 5 min. The test antibody VHH-His or the reference antibody was added for resuspension, and the cells were incubated at 4 °C for 1 h. The secondary antibody iF647-anti-his (Genscript, 1:1000 diluted, Cat. No. A01802-100) or APC-anti-his (BioLegend, 1:200, Cat. No. 362605) was added, and the mixture was incubated at 4 °C for half an hour or 45 min. The MFI of the cells was assayed by a flow cytometer (Life Technologies).

### 1) Sequence optimization engineering of anti-EGFR antibodies

After the parent antibodies V-n10A1, V-n9B8, and V-n5B10 were subjected to sequence optimization engineering, the binding activity of each optimized anti-EGFR antibody for target cells was assayed by FACS binding assay, and the results are shown in Tables 3A and 3B.

**Table 3A. Binding activity of humanized anti-EGFR antibodies for target cells**

| **Antibody name** | **EC₅₀(nM)** | **Maximum MFI value** | **Target cell** |
|---|---|---|---|
| V-n10A1 | - | 5829 | |
| zn10A1.m5 | - | 3485 | |
| zn10A1.m6 | - | 9675 | |
| V-n9B8 | 2.04 | 94621 | |
| zn9B8.m2 | 2.49 | 95829 | |
| zn9B8.m4 | 1.43 | 57113 | |
| V-n5B10 | 15.03 | 110773.6 | MDA-MB-468 |
| zn5B10.m16 | 3.132 | 76781.3 | |
| zn5B10.m17 | 23.16 | 93566.7 | |
| zn5B10.m18 | 1.303 | 71122.4 | |
| zn5B10.m20 | 0.2192 | 59191.5 | |
| zn5B10.m21 | 0.429 | 58747 | |
| zn5B10.m22 | 1.149 | 86869.5 | |
| zn5B10.m23 | 1.936 | 74208.6 | |

| | | | |
|---|---|---|---|
| Note: "-" indicates that the EC₅₀ value could not be fitted. | | | |

**Table 3B. Binding activity of anti-EGFR antibodies after PTM removal sequence optimization for target cells**

| **Antibody name** | **EC₅₀(nM)** | **Maximum MFI value** | **Target cell** |
|---|---|---|---|
| **V-n10A1 First round of PTM removal sequence optimization** | | | |
| V-n10A1 | - | 5157 | MDA-MB-468 |
| pn10A1.m1 | - | 10255.7 | |
| pn10A1.m2 | - | 3773.5 | |
| pnl0A1.m3 | - | 5477.6 | |
| pn10A1.m4 | - | 5265 | |
| pn10A1.m5 | - | 27433.9 | |
| pn10A1.m6 | - | 5787 | |

| V-n10A1 **Second round of PTM removal sequence optimization** | | | |
|---|---|---|---|
| V-n10A1 | | 2759 | CHOK1-huEGFR cells |
| pzn10A1_m6m5 | - | 3573 | |
| ppzn10A1_m6m5.m1 | - | 2470 | |
| ppzn10A1_m6m5.m3 | - | 4767 | |

| zn9B8.m2 **PTM removal sequence optimization** | | | |
|---|---|---|---|
| zn9B8.m2 | 3.940 | 84343 | CHOK1-huEGFR cells |
| pzn9B8.m2.m11 | 3.39 | 80145 | |
| pzn9B8.m2.m12 | 4.963 | 85741 | |

| | | | |
|---|---|---|---|
| Note: "-" indicates that the EC₅₀ value could not be fitted. | | | |

### 2) Sequence optimization engineering of anti-cMet antibodies:

After the parent antibodies V-n7A12 and V-n9A2 were subjected to sequence optimization engineering, the binding activity of each optimized anti-cMet antibody for target cells was assayed by FACS binding assay, and the results are shown in Tables 3C-3F.

**Table 3C. Binding activity of humanized anti-cMet antibodies for target cells**

| **Antibody name** | **EC₅₀(nM)** | **Maximum MFI value** | **Target cell** |
|---|---|---|---|
| V-n9A2 | 1.564 | 290877.6 | EBC-1 cells |
| VHH8 | 2.505 | 143281 | |
| VHH9 | 1.626 | 193984.2 | |
| VHH10 | 1.632 | 233386.4 | |
| VHH11 | 2.107 | 150778.6 | |
| V-n7A12 | 3.759 | 35568.6 | MKN45 cells |
| 7A12-M1 | 88.1 | 56591.3 | |
| 7A12-M2 | - | 36691.1 | |
| 7A12-M3 | - | 57350.4 | |
| 7A12-M4 | 4.112 | 44671.9 | |

| | | | |
|---|---|---|---|
| Note: "-" indicates that the EC₅₀ value could not be fitted. | | | |

**Table 3D. Binding activity of anti-cMet antibodies after PTM removal sequence optimization for target cells**

| **Antibody name** | **EC₅₀(nM)** | **Maximum MFI value** | **Target cell** |
|---|---|---|---|
| V-n7A12 | 3.759 | 35568.6 | MKN45 cells |
| pn7A12.N32X.m1 | 5.098 | 40412.3 | |
| pn7A12.N32X.m3 | 4.157 | 49561.8 | |
| pn7A12.N32X.m4 | 4.965 | 38029.9 | |
| pn7A12.N32X.m6 | 4.454 | 44729.5 | |

**Table 3E. Binding activity of anti-cMet antibodies after primary immunogenicity removal for target cells**

| **Antibody name** | **EC₅₀(nM)** | **Maximum MFI value** | **Target cell** |
|---|---|---|---|
| V-n7A12 | 1.008 | 74188 | CHOK1-hucMet cells |
| dpzn7A12m4.m1_1 | 0.7158 | 73854 | |
| dpzn7A12m4.m1_3 | 0.8094 | 63423 | |

**Table 3F. Binding activity of anti-cMet antibody after secondary immunogenicity removal for target cells**

| **Antibody name** | **EC₅₀(nM)** | **Maximum MFI value** | **Target cell** |
|---|---|---|---|
| V-n7A12 | 1.937 | 145845 | CHOK1-hucMet cells |
| dpzn7A12m4.ml_1.m1 | 2.098 | 135258 | |

### 4.4. SPR assay on anti-cMet VHHs and anti-EGFR VHHs

The binding affinity of the anti-cMet VHHs and the anti-EGFR VHHs for the target antigen was characterized using the SPR method.

The VHH_His-tagged monovalent antibodies were assayed using method I below. 10 µg/mL antigen (EGFR antigens or cMet antigens of different species) was immobilized on a CM5 chip, and then each test antibody (serially diluted) was injected at a flow rate of 30 µL/min. The association time was set to 120 s, and the dissociation time was set to 200 s. After dissociation was completed, regeneration was performed with 10 mM glycine (pH 2.0). The experimental data were analyzed using a 1:1 binding model. The results are shown in Table 4A below.

The VHH_Fc-tagged bivalent antibodies were assayed using method II below. Each test antibody (at a concentration of 10 µg/mL) was captured by a Protein A chip, and then an antigen (EGFR antigens or cMet antigens of different species) was injected at a flow rate of 30 µL/min. The association time was set to 120 s, and the dissociation time was set to 200 s. After dissociation was completed, regeneration was performed with 10 mM glycine (pH 2.0). The experimental data were analyzed using a 1:1 binding model. The results are shown in Table 4B below.

**Table 4A. SPR data for VHH_His-tagged monovalent anti-cMet antibodies and anti-EGFR antibodies**

| **Antibody name** | **Antigen** | **ka (1/Ms)** | **kd (1/s)** | **KD (M)** |
|---|---|---|---|---|
| V-n5B10 | Human EGFR | 2.32E+05 | 5.36E-03 | 2.32E-08 |
| V-n5B10 | Cyno EGFR | 5.22E+05 | 3.84E-03 | 7.36E-09 |
| V-n10A1 | Human EGFR | 6.91E+05 | 2.58E-02 | 3.74E-08 |
| V-n10A1 | Cyno EGFR | 5.81E+05 | 1.00E-02 | 1.72E-08 |
| V-n7A12 | Human cMet | 5.09E+05 | 1.53E-04 | 3.01E-10 |
| V-n7A12 | Cyno cMet | 4.58E+04 | 4.39E-03 | 9.60E-08 |
| V-n9A2 | Human cMet | 1.81E+05 | 2.90E-03 | 1.60E-08 |
| V-n9A2 | Cyno cMet | 1.02E+05 | 7.20E-03 | 7.03E-08 |
| ABT700 | Human cMet | 1.08E+05 | 7.38E-05 | 6.81E-10 |
| ABT700 | Cyno cMet | 1.22E+05 | 7.14E-05 | 5.84E-10 |
| BMK-AZD | Human EGFR | 1.90E+05 | 3.10E-03 | 1.63E-08 |
| BMK-AZD | Cyno EGFR | 5.91E+05 | 2.63E-02 | 4.45E-08 |
| BMK-AZD | Human cMet | 2.88E+05 | 6.23E-04 | 2.16E-09 |
| BMK-AZD | Cyno cMet | 3.58E+05 | 1.15E-03 | 3.22E-09 |

| | | | | |
|---|---|---|---|---|
| Note: All the tested V-n series antibodies in the table are in the format of a monovalent VHH with a His tag at the C-terminus and were assayed by method I; the control antibodies in the table were assayed using method II. | | | | |

**Table 4B. SPR data for VHH_Fc-tagged bivalent anti-EGFR antibodies**

| **Antibody name** | **Antigen** | **ka (1/Ms)** | **kd (1/s)** | **KD (M)** |
|---|---|---|---|---|
| V-F42 (hu9B8_Fc)₂ | Human EGFR | 3.30E+04 | 3.92E-04 | 1.19E-08 |
| V-F42 | Cyno EGFR | 3.92E+04 | 8.04E-05 | 2.05E-09 |

| | | | | |
|---|---|---|---|---|
| Note: All the antibodies in the table are in the format of a dimeric VHH with an Fc tag at the C-terminus and were assayed using method II. | | | | |

### Example 5. Construction and Characterization of Anti-cMet Biepitopic Antibody

### 5.1 CONSTRUCTION AND PREPARATION of anti-cMet biepitopic antibody

The anti-cMet biepitopic M-1 molecule of the present disclosure is in an asymmetric double-chain form and has a first polypeptide chain (9A2-Fc chain) set forth in SEQ ID NO: 88 and a second polypeptide chain (hu7A12^{b}-Fc chain) set forth in SEQ ID NO: 89.

The anti-cMet monoepitopic M-2 molecule of the present disclosure is in a symmetrical double-chain form and has a first polypeptide chain and a second polypeptide chain (9A2-Fc chain) set forth in SEQ ID NO: 90.

The anti-cMet monoepitopic M-3 molecule of the present disclosure is in a symmetrical double-chain form and has a first polypeptide chain and a second polypeptide chain (hu7A12^{b}-Fc chain) set forth in SEQ ID NO: 91.

The first/second polypeptide chains of the M-1, M-2 and M-3 described above were constructed into pcDNA 3.4 expression vectors (if the antibody was of a symmetric structure, one type of vector was generated; if the antibody was of an asymmetric structure, two types of vectors respectively containing coding genes of the first and second polypeptide chains were generated). HEK293F cells were transfected with the vectors. The cells were cultured for 3 days, and culture supernatants of the transfected cells were collected and loaded onto a Protein A column (MabSelect PrismA, Cytiva) for purification. The antibodies were eluted with an acetic acid-sodium acetate solution (pH 3.5) and immediately neutralized with 2 M Tris. The concentration of the antibodies was measured with Nano Drop. Protein purity was determined by SDS-PAGE and analytical HPLC-SEC.

### 5.2 FACS ASSAY ON BINDING OF BIEPITOPIC ANTIBODIES TO TARGET CELLS

Binding of the tumor target cells EBC-1 to anti-cMet molecules was assayed by FACS.

Tumor target cells EBC-1 were prepared and seeded in a 96-well plate at 1.5 × 10⁵ cells/well. Anti-cMet molecules M-1, M-2, and M-3 (50 nM, 4-fold diluted) were separately added. The mixtures were incubated at 4 °C for 1 h and centrifuged, and the supernatants containing respective VHH-Fcs were removed. The secondary antibody PE-anti-human IgG (eBioscience, Cat. No. 12-4998-82) was added. The mixtures were incubated at 4 °C for 30 min, and then the MFI of the cells was assayed by a flow cytometer (Life Technologies).

As shown in FIG. 7, the results showed that: compared to the anti-cMet monoepitopic molecules M-2 and M-3, the tumor target cells EBC-1 exhibited stronger binding to the anti-cMet biepitopic molecule M-1, and the binding activity of the biepitopic molecule for the cells was also better than that of the control group ABT700.

### 5.3 FACS ASSAY ON SYNERGISTIC ENDOCYTOSIS EFFECT OF BIEPITOPIC ANTIBODY

The synergistic endocytosis effect of the anti-cMet biepitopic molecule M-1 was assayed by the FACS method.

Tumor target cells EBC-1 were prepared and seeded in a 96-well plate at 1.5 × 10⁵ cells/well. Anti-cMet molecules M-1, M-2, and M-3 (50 nM, 4-fold diluted) were separately added. The mixtures were incubated at 4 °C for 30 min and centrifuged, and the supernatants containing respective VHH-Fcs were removed. The cells were evenly divided into 2 groups and separately incubated at 4 °C and 37 °C for 4 h. After the incubation was completed, PBS chilled in ice bath was immediately added to terminate the endocytosis experiment. Subsequently, the secondary antibody PE-anti-human IgG (eBioscience, Cat. No. 12-4998-82) was added. The mixtures were incubated at 4 °C for 30 min, and then the MFI of the cells was assayed by a flow cytometer (Life Technologies).

The internalization level of the cell surface-bound antibody was calculated using the formula below:${\text{endocytosis = MFI of sample incubated at 4}}^{∘} {\text{C - MFI of sample incubated at 37}}^{∘} \text{C} \text{.}$

As shown in FIG. 8, the above experimental results showed that: the tumor target cells EBC-1 exhibited relatively high endocytosis for the anti-cMet biepitopic molecule M-1 (as compared to the anti-cMet monoepitopic molecules M-2 and M-3), and the anti-cMet biepitopic molecule M-1 exhibited a better endocytosis effect than ABT700.

### EXAMPLE 6. GENERATION AND ASSAY OF MULTISPECIFIC ANTI-EGFR/CMet ANTIBODY MOLECULES

### 6.1 GENERATION OF MULTISPECIFIC ANTI-EGFR/CMetANTIBODY MOLECULES

According to the analysis and assay results of Example 1-Example 5 described above, an anti-EGFR VHH and an anti-cMet VHH were combined together to form single-chain or multi-chain multispecific antibodies.

### Single-chain multispecific antibodies

An anti-EGFR VHH sequence and an anti-cMet VHH sequence were combined together to form a single-chain antibody format. Specifically, one anti-EGFR VHH sequence (V-n5B10, V-n9B8, or V-n10A1) or a humanized VHH sequence thereof and two VHH sequences (V-n7A12 and V-n9A2) targeting the same or different epitopes on cMet or humanized VHH sequences thereof were combined to form a specific anti-EGFR/cMet antibody molecule. A GGGGS peptide linker (SEQ ID NO: 43) was added to the C-terminus of the anti-EGFR VHH, and what were then sequentially added were: an anti-cMet VHH (a first anti-cMet ISVD), a GGGGSGGGGS peptide linker (SEQ ID NO: 44), and another anti-cMet VHH (a second anti-cMet ISVD). Exemplary multispecific antibody molecules resulting from this combination and amino acid sequences thereof are shown in Table 5.

**Table 5: Exemplary single-chain multispecific antibody molecules**

| **Antibody name** | **Assembly format of antibody** | **Sequence No.** |
|---|---|---|
| V-1 | 5B10-9A2-7A12 | SEQ ID NO: 51 |
| V-3 | 5B10-7A12-9A2 | SEQ ID NO: 52 |
| V-7 | 9B8-9A2-7A12 | SEQ ID NO: 53 |
| V-9 | 9B8-7A12-9A2 | SEQ ID NO: 54 |
| V-13 | 10A1-9A2-7A12 | SEQ ID NO: 55 |
| V-15 | 10A1-7A12-9A2 | SEQ ID NO: 56 |
| V-16 | hu10A1-hu7A12-9A2 | SEQ ID NO: 57 |
| V-17 | hu10A1-hu7A12-hu9A2 | SEQ ID NO: 58 |
| V-18 | hu10A1-hu7A12-hu7A12 | SEQ ID NO: 59 |
| V-19 | hu10A1-hu9A2-hu9A2 | SEQ ID NO: 60 |
| V-20 | hu5B10-hu7A12-hu9A2 | SEQ ID NO: 61 |
| V-21 | hu5B10-hu7A12-hu7A12 | SEQ ID NO: 62 |
| V-22 | hu5B10-hu9A2-hu9A2 | SEQ ID NO: 63 |
| V-23 | hu9B8-hu7A12-hu9A2 | SEQ ID NO: 64 |
| V-24 | hu9B8-hu7A12-hu7A12 | SEQ ID NO: 65 |
| V-25 | hu9B8-hu9A2-hu9A2 | SEQ ID NO: 66 |

| | | |
|---|---|---|
| Note: In Table 5, V-n5B10, V-n9B8, and V-n10A1 as anti-EGFR VHHs were abbreviated as 5B10, 9B8, and 10A1, respectively; V-n7A12, V-n9A2, and V-n9A10 as anti-cMet VHHs were abbreviated as 7A12, 9A2, and 9A10, respectively. | | |

Optionally, the specific antibody molecules were linked to an ISVD binding to human serum albumin, e.g., Alb8, to extend the half-life of the specific anti-EGFR/cMet antibody molecules. For example, the C-terminus of the specific antibody molecule was linked to an ISVD binding to human serum albumin, e.g., Alb8 (SEQ ID NO: 45), via a peptide linker (e.g., GGGGS set forth in SEQ ID NO: 43).

Specifically, taking V-17 as an example, as exemplified in FIG. 9, a GGGGS peptide linker (SEQ ID NO: 43) was linked to the C-terminus of V-17, followed by an anti-HSA-Alb8 (SEQ ID NO: 45). The combination forms hu10A1-hu7A12-hu9A2-anti-HSA-A1b8 (also referred to as "V-17-anti-HSA-Alb8"). Similarly, for the rest specific antibody molecules described in Table 5, a GGGGS peptide linker (SEQ ID NO: 43) was linked to the C-termini, followed by an anti-HSA-Alb8 (SEQ ID NO: 45), to separately form V-1-anti-HSA-Alb8, V-3-anti-HSA-Alb8, V-7-anti-HSA-Alb8, V-9-anti-HSA-Alb8, V-13-anti-HSA-Alb8, V-15-anti-HSA-Alb8, V-16-anti-HSA-Alb8 (SEQ ID NO: 67), V-17-anti-HSA-Alb8 (SEQ ID NO: 68), V-18-anti-HSA-Alb8 (SEQ ID NO: 76), V-19-anti-HSA-Alb8 (SEQ ID NO: 77), V-20-anti-HSA-Alb8 (SEQ ID NO:78), V-21-anti-HSA-Alb8 (SEQ ID NO: 79), V-22-anti-HSA-Alb8 (SEQ ID NO:80), V-23-anti-HSA-Alb8 (SEQ ID NO: 81), V-24-anti-HSA-Alb8 (SEQ ID NO: 82), and V-25-anti-HSA-Alb8 (SEQ ID NO: 83).

The single-chain multispecific anti-EGFR/cMet antibodies described in the present disclosure were constructed into pcDNA 3.4 expression vectors. HEK293F cells were transfected with the vectors and cultured for 3 days, and the culture supernatants of the transfected cells were collected and loaded into Cytiva PrismA packing (Cytiva) for purification. The antibodies were eluted with an acetic acid-sodium acetate solution (pH 3.5) and immediately neutralized with 2 M Tris. The concentration of the antibodies was measured with Nano Drop. The protein purity was determined by SDS-PAGE and analytical HPLC-SEC, and the proteins were then stored at -80 °C.

### Multi-chain multispecific antibodies

An anti-EGFR VHH sequence and a VHH sequence targeting the same or different epitopes on cMet were each subjected to combination to form a polypeptide chain comprising an Fc subunit, and the polypeptide chains may be further associated to form a homodimer or a heterodimer, thereby forming a multispecific EGFR- and cMet-binding molecule in a double-chain form.

FIG. 10 shows an exemplary multi-chain multispecific antibody structure. Specifically, the anti-EGFR VHH sequence and an Fc subunit were combined to constitute a polypeptide chain (anti-EGFR-Fc), where optionally, the Fc subunit contained an LALA mutation and/or a Knob-into-hole structure. The prepared polypeptide chains include the sequences set forth in Table 6A:

**Table 6A. Exemplary sequences of anti-EGFR-Fc**

| Assembly format of domain | Sequence No. |
|---|---|
| hu10A1-Fc | SEQ ID NO: 70 |
| hu5B10-Fc | SEQ ID NO: 71 |
| hu9B8-Fc | SEQ ID NO: 72 |
| hu10A1^{b}-Fc | SEQ ID NO: 86 |

The anti-cMet VHH sequence and an Fc subunit were combined to constitute a polypeptide chain (anti-cMet1-anti-cMet2-Fc). Specifically, the C-terminus of a first anti-cMet VHH sequence was linked to the N-terminus of a second anti-cMet VHH sequence that is identical or different via a peptide linker (GGGGSGGGGS set forth in SEQ ID NO: 42), and then the C-terminus of the second anti-cMet VHH was linked to the Fc subunit via a hinge region, thus constituting a polypeptide chain, where optionally, the Fc subunit contained an LALA mutation and/or a Knob-into-hole structure. The prepared polypeptide chains include the sequences set forth in Table 6B:

**Table 6B. Exemplary sequences of anti-cMet1-anti-cMet2-Fc**

| Assembly format of domain | Sequence No. |
|---|---|
| hu9A2-hu7A12-Fc | SEQ ID NO: 73 |
| hu9A2-hu9A2-Fc | SEQ ID NO: 74 |
| hu7A12-hu7A12-Fc | SEQ ID NO: 75 |
| 9A2-hu7A12-Fc | SEQ ID NO: 69 |
| 9A2-hu7A12^{b}-Fc | SEQ ID NO: 87 |

Any one of the anti-EGFR-Fc polypeptide chains described above may be associated with any one of the anti-cMet1-anti-cMet2-Fc polypeptide chains to form a dimer. For example, multispecific anti-EGFR/cMet antibodies in a double-chain form set forth in Table 7 were synthesized:

**Table 7. Examples of multispecific anti-EGFR/cMet antibodies in a double-chain form**

| Name of anti-EGFR/cMet antibody in a double-chain form | Polypeptide chain 1 constituting double chains | Polypeptide chain 2 constituting double chains |
|---|---|---|
| V-17-Fc | hu10A1-Fc (SEQ ID NO: 70) | hu9A2-hu7A12-Fc (SEQ ID NO: 73) |
| V-18-Fc | hu10A1-Fc (SEQ ID NO: 70) | hu7A12-hu7A12-Fc (SEQ ID NO: 75) |
| V-19-Fc | hu10A1-Fc (SEQ ID NO: 70) | hu9A2-hu9A2-Fc (SEQ ID NO: 74) |
| V-20-Fc | hu5B10-Fc (SEQ ID NO: 71) | hu9A2-hu7A12-Fc (SEQ ID NO: 73) |
| V-21-Fc | hu5B10-Fc (SEQ ID NO: 71) | hu7A12-hu7A12-Fc (SEQ ID NO: 75) |
| V-22-Fc | hu5B10-Fc (SEQ ID NO: 71) | hu9A2-hu9A2-Fc (SEQ ID NO: 74) |
| V-23-Fc | hu9B8-Fc (SEQ ID NO: 72) | hu9A2-hu7A12-Fc (SEQ ID NO: 73) |
| V-24-Fc | hu9B8-Fc (SEQ ID NO: 72) | hu7A12-hu7A12-Fc (SEQ ID NO: 75) |
| V-25-Fc | hu9B8-Fc (SEQ ID NO: 72) | hu9A2-hu9A2-Fc (SEQ ID NO: 74) |
| V-26-Fc | hu10A1^{b}-Fc (SEQ ID NO: 86) | 9A2-hu7A12^{b}-Fc (SEQ ID NO: 87) |

The polypeptide chain 1 and the polypeptide chain 2 of the multispecific anti-EGFR/cMet antibody in a double-chain form described in the present disclosure were separately constructed into pcDNA 3.4 expression vectors. HEK293F cells were transfected with the vectors and cultured for 3 days, and the culture supernatants of the transfected cells were collected and loaded into a Protein A column (MabSelect PrismA, Cytiva) for purification. The antibodies were eluted with an acetic acid-sodium acetate solution (pH 3.5) and immediately neutralized with 2 M Tris. The concentration of the antibodies was measured with Nano Drop. The protein purity was determined by SDS-PAGE and analytical HPLC-SEC, and the proteins were then stored at -80 °C.

### 6.2 FACS BINDING ASSAY

Binding of the trispecific anti-EGFR/cMet antibody molecules described above to target cells EBC-1 and NCI-H1975 (Cell Bank of the Chinese Academy of Sciences) was assayed by the FACS method.

Target cells were separately prepared and seeded in a 96-wellplate at 1.5 × 10⁵ cells/well. The trispecific antibodies prepared in Example 6.1 (250 nM, 3-fold diluted) were added. The mixtures were incubated at 4 °C for 1 h and then centrifuged at a centrifugal speed of 300 g for 4 min. The supernatants were removed. The plate was washed 2 times with FACS buffer (1% BSA) by repeated centrifugation. The secondary antibody PE-anti-human IgG (eBioscience, Cat. No. 12-4998-82) was added. The mixtures were incubated at 4 °C for 1 h, and then the plate was washed 2 times by centrifugation. Finally, the cells were resuspended in FACS buffer (1% BSA), and the MFI of the cells was assayed by a flow cytometer (Life Technologies).

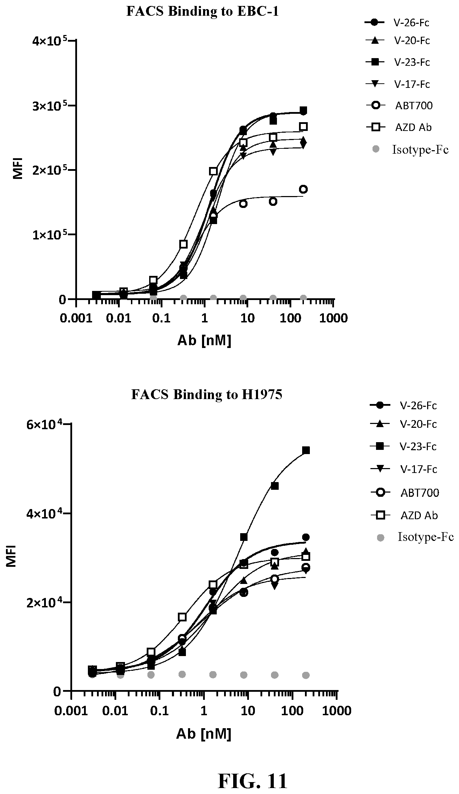

As shown in FIG. 11, the trispecific antibodies V-17-Fc, V-20-Fc, V-23-Fc, and V-26-Fc were able to bind to EBC-1 and NCI-H1975 cells.

### 6.3. FACS ASSAY ON ENDOCYTOSIS

The internalization ability of the trispecific anti-EGFR/cMet antibody molecules described above in the target cells EBC-1 and NCI-H1975 (Cell Bank of the Chinese Academy of Sciences) was assayed by the FACS method.

Target cells were separately prepared and seeded in a 96-well plate at 3 × 10⁵ cells/well. The trispecific antibodies prepared in Example 6.1 (250 nM, 3-fold diluted) were added. The mixtures were incubated at 4 °C for 30 min and centrifuged. The supernatants containing the trispecific antibodies were removed. The cells were evenly divided into 2 groups and separately incubated at 4 °C and 37 °C for 4 h. After the incubation was completed, PBS chilled in ice bath was immediately added to terminate the endocytosis experiment. The secondary antibody was added. The mixtures were incubated at 4 °C for 30 min, and then the MFI of the cells was assayed by a flow cytometer (Life Technologies). For the VHH-Fc format molecules, PE-anti-human IgG (eBioscience, Cat. No. 12-4998-82) was used as the secondary antibody; for the his-tagged VHH molecules, APC-anti-his (BioLegend, Cat. No. 362605) was used as the secondary antibody.

The internalization level of the cell surface-bound antibody was calculated using the formula below:${\text{Endocytosis = MFI of sample incubated at 4}}^{∘} {\text{C - MFI of sample incubated at 37}}^{∘} \text{C} \text{.}$

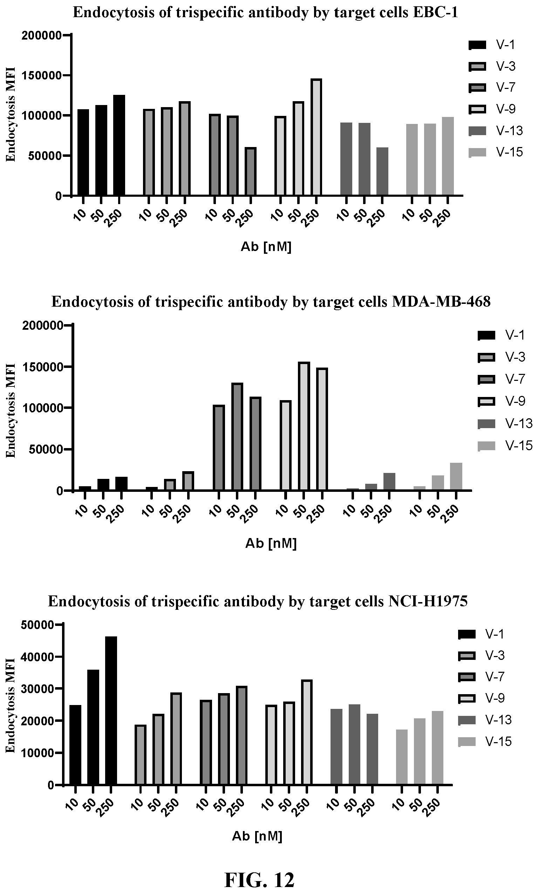

The endocytosis results of the candidate trispecific antibody molecules V-1, V-3, V-7, V-9, V-13, V-15, V-17-Fc, V-20-Fc, V-23-Fc, and V-26-Fc in target cells MDA-MB-468, EBC-1 and NCI-H1975 are shown in FIG. 12 to FIG. 13.

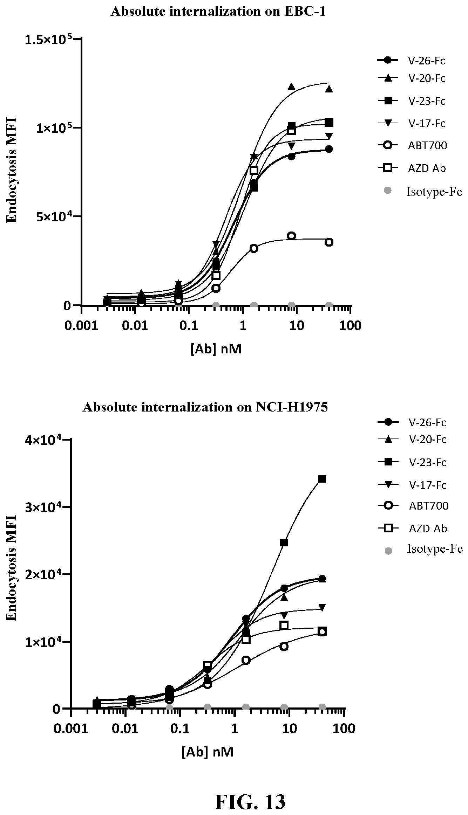

As shown in FIG. 12 and FIG. 13, the trispecific anti-EGFR/cMet antibody molecules V-1, V-3, V-7, V-9, V-13, V-15, V-17-Fc, V-20-Fc, V-23-Fc, and V-26-Fc were able to be endocytosed by the target cells.

### 6.4 SPR ASSAY ON AFFINITY OF TRISPECIFIC ANTIBODIES FOR HUMAN AND CYNOMOLGUS MONKEY ANTIGENS

### 6.4.1 SPR assay on affinity of trispecific antibodies for human and cynomolgus monkey EGFR antigens

The affinity of the trispecific anti-EGFR/cMet antibody molecules for human and cynomolgus monkey EGFRs (Sino Biological Inc.) was assayed by SPR using Cytiva.

Antigens human EGFR-his (ECD, Met1-Ser645) and cynomolgus monkey EGFR-his (ECD, Met1-Ser645) (Sino Biological Inc.) (20 µg/mL) were coupled to amino groups immobilized on a GLM sensor chip (Biorad). The proportionally diluted trispecific anti-EGFR/cMet antibody molecules of Example 6.1 were injected onto the sensor chip at a flow rate of 100 µL/min, with an association phase of 100 s followed by dissociation for 180 s. After each dissociation phase, a 10 mM glycine (pH 1.5) regeneration buffer was used. The sensorgrams for the blank surface and the buffer channel were subtracted from the test sensorgrams. The experimental data were subjected to Langmuir analysis using a 1:1 binding model. The affinity results of the candidate molecules for human and cynomolgus monkey EGFRs are shown in Table 8.

The results showed that the candidate molecules exhibited similar association and dissociation rates for the human EGFR antigen and the cynomolgus monkey EGFR antigen, and these trispecific anti-EGFR/cMet antibody molecules had cross-reactions with the cynomolgus monkey EGFR.

**Table 8. SPR assay on affinity of trispecific antibodies for human and cynomolgus monkey EGFR antigens**

| Antibody name | Antigen | **Kon (1/Ms)** | **Koff (1/s)** | **K_{D} (M)** |
|---|---|---|---|---|
| **V-1** | **Human EGFR** | 1.12E+05 | 3.54E-03 | 3.17E-08 |
| | **Cynomolgus monkey EGFR** | 7.41E+04 | 2.06E-03 | 2.78E-08 |
| **V-3** | **Human EGFR** | 1.19E+05 | 3.00E-03 | 2.51E-08 |
| | **Cynomolgus monkey EGFR** | 1.12E+05 | 2.46E-03 | 2.18E-08 |
| **V-7** | **Human EGFR** | 9.16E+04 | 1.17E-06 | 1.28E-11 |
| | **Cynomolgus monkey EGFR** | 9.32E+04 | 1.25E-07 | 1.35E-12 |
| **V-9** | **Human EGFR** | 8.69E+04 | 2.35E-07 | 2.70E-12 |
| | **Cynomolgus monkey EGFR** | 7.64E+04 | 1.95E-06 | 2.55E-11 |
| **V-13** | **Human EGFR** | 2.76E+05 | 1.35E-02 | 4.89E-08 |
| | **Cynomolgus monkey EGFR** | 4.33E+05 | 1.62E-02 | 3.74E-08 |
| **V-15** | **Human EGFR** | 3.34E+05 | 9.43E-03 | 2.83E-08 |
| | **Cynomolgus monkey EGFR** | 3.35E+05 | 7.64E-03 | 2.28E-08 |
| **V-23-Fc** | **Human EGFR** | 2.10E+04 | 2.02E-04 | 9.63E-09 |
| | **Cynomolgus monkey EGFR** | 2.82E+04 | 3.14E-04 | 1.11E-08 |
| **V-26-Fc** | **Human EGFR** | 4.09E+02 | 9.88E-02 | 2.42E-04 |
| | **Cynomolgus monkey EGFR** | 5.87E+04 | 2.19E-01 | 3.74E-06 |

### 6.4.2 SPR assay on affinity of trispecific antibodies for human and cynomolgus monkey cMet antigens

Antigens human cMet-his (ECD, Met1-Thr932) and cynomolgus monkey cMet-his (ECD, Met1-Thr932) (Sino Biological Inc.) (20 µg/mL) were coupled to amino groups immobilized on a GLM sensor chip (Biorad). The proportionally diluted trispecific anti-EGFR/cMet antibody molecules (500 nM) of Example 6.1 were injected onto the sensor chip at a flow rate of 30 µL/min, with an association phase of 100 s followed by dissociation for 180 s. After each dissociation phase, a 10 mM glycine (pH 1.5) regeneration buffer was used. The sensorgrams for the blank surface and the buffer channel were subtracted from the test sensorgrams. The experimental data were analyzed using a 1:1 binding model. The affinity results of the candidate molecules for human cMet and cynomolgus monkey cMet are shown in Table 9.

The results showed that the candidate molecules exhibited similar association and dissociation rates for the human cMet antigen and the cynomolgus monkey cMet antigen, and these trispecific anti-EGFR/cMet antibody molecules had cross-reactions with the cynomolgus monkey cMet.

**Table 9. SPR assay on affinity of trispecific antibodies for human and cynomolgus monkey cMet antigens**

| | | **Kon (1/Ms)** | **Koff (1/s)** | **KD (M)** |
|---|---|---|---|---|
| **V-1** | **Human cMet** | 1.35E+05 | 1.32E-03 | 9.81E-09 |
| | **Cynomolgus monkey cMet** | 1.33E+05 | 8.55E-04 | 6.43E-09 |
| **V-3** | **Human cMet** | 1.83E+05 | 1.58E-03 | 8.61E-09 |
| | **Cynomolgus monkey cMet** | 1.76E+05 | 1.06E-03 | 5.99E-09 |
| **V-7** | **Human cMet** | 1.30E+05 | 1.33E-03 | 1.02E-08 |
| | **Cynomolgus monkey cMet** | 1.20E+05 | 7.89E-04 | 6.59E-09 |
| **V-9** | **Human cMet** | 1.16E+05 | 1.67E-03 | 1.44E-08 |
| | **Cynomolgus monkey cMet** | 1.08E+05 | 1.10E-03 | 1.02E-08 |
| **V-13** | **Human cMet** | 3.04E+05 | 8.25E-04 | 2.72E-09 |
| | **Cynomolgus monkey cMet** | 2.00E+05 | 3.81E-04 | 1.91E-09 |
| **V-15** | **Human cMet** | 1.89E+05 | 1.25E-03 | 6.61E-09 |
| | **Cynomolgus monkey cMet** | 1.85E+05 | 8.31E-04 | 4.49E-09 |
| **V-17-Fc** | **Human cMet** | 9.78E+04 | 1.37E-04 | 1.40E-09 |
| | **Cynomolgus monkey cMet** | 7.93E+04 | 1.54E-04 | 1.95E-09 |
| **V-20-Fc** | **Human cMet** | 1.54E+05 | 3.65E-04 | 2.37E-09 |
| | **Cynomolgus monkey cMet** | 4.30E+04 | 1.99E-04 | 4.62E-09 |
| **V-23-Fc** | **Human cMet** | 2.86E+04 | 2.15E-03 | 7.52E-08 |
| | **Cynomolgus monkey cMet** | 9.68E+04 | 2.25E-04 | 2.33E-09 |
| **V-26-Fc** | **Human cMet** | 7.26E+04 | 9.70E-05 | 1.34E-09 |
| | **Cynomolgus monkey cMet** | 1.68E+05 | 1.23E-04 | 7.30E-10 |

### 6.5. Determination of blocking effect of trispecific anti-EGFR/cMet antibody molecules on binding of ligand HGF to target cells EBC-1

Target cells EBC-1 were seeded in a 96-well plate at a density of 1.5 × 10⁵ cells/well and centrifuged at 300 g at 4 °C for 5 min. Trispecific anti-EGFR/cMet antibodies (200 nM, 4-fold serially diluted) were first added and incubated with the cells for 30 min, and then the ligand HGF-His (2 µg/mL) (Beijing Sino Biological Inc.) was added. The mixtures were co-incubated at 4 °C for 1 h. The secondary antibody iF647-anti-his (Genscript) was added. The mixtures were incubated at 4 °C for half an hour, and the MFI of the cells was assayed by a flow cytometer (Life Technologies).

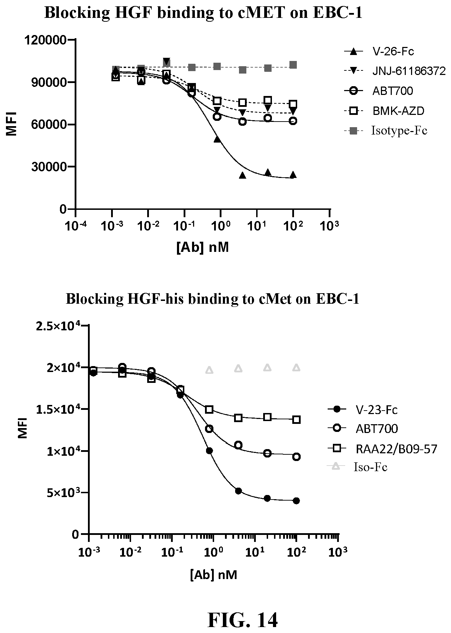

As shown in FIG. 14, the results showed that the trispecific anti-EGFR/cMet antibodies of the present disclosure were able to block the binding of EBC-1 cells to the ligand HGF, with a blocking activity better than that of ABT700 and BMK-AZD.

### 6.6 FACS ASSAY ON SYNERGISTIC ENDOCYTOSIS OF TRISPECIFIC ANTIBODIES IN TARGET CELLS

The molecule M-4, serving as a control molecule, is in the form of an asymmetric double-chain Fc and has a first polypeptide chain (hu9A2-hu7A12-Fc) set forth in SEQ ID NO: 73 and a second polypeptide chain (anti-Covid-19 chain) set forth in SEQ ID NO: 92, where the anti-Covid-19 chain did not bind to target cell EGFR or cMET.

The molecule M-5, serving as a control molecule, is in the form of an asymmetric double-chain Fc and has a first polypeptide chain (anti-Covid-19 chain) set forth in SEQ ID NO: 93 and a second polypeptide chain (hu5B10-Fc) set forth in SEQ ID NO: 71, where the anti-Covid-19 chain did not bind to target cell EGFR or cMET.

The molecule M-6, serving as a control molecule, is in the form of an asymmetric double-chain Fc and has a first polypeptide chain (anti-Covid-19 chain) set forth in SEQ ID NO: 93 and a second polypeptide chain (hu9B8-Fc) set forth in SEQ ID NO: 72, where the anti-Covid-19 chain did not bind to target cell EGFR or cMET.

The molecule M-7, serving as a control molecule, is in the form of an asymmetric double-chain Fc and has a first polypeptide chain (anti-Covid-19 chain) set forth in SEQ ID NO: 93 and a second polypeptide chain (hu10A1-Fc) set forth in SEQ ID NO: 70, where the anti-Covid-19 chain did not bind to target cell EGFR or cMET.

The first/second polypeptide chains of the molecules M-4, M-5, M-6 and M-7 described above were constructed into pcDNA 3.4 expression vectors (if the antibody was of a symmetric structure, one type of vector was generated; if the antibody was of an asymmetric structure, two types of vectors respectively containing coding genes of the first and second polypeptide chains were generated). HEK293F cells were transfected with the vectors. The cells were cultured for 3 days, and culture supernatants of the transfected cells were collected and loaded onto a Protein A column (MabSelect PrismA, Cytiva) for purification. The antibodies were eluted with an acetic acid-sodium acetate solution (pH 3.5) and immediately neutralized with 2 M Tris. The concentration of the antibodies was measured with Nano Drop. Protein purity was determined by SDS-PAGE and analytical HPLC-SEC.

The enhanced internalization ability of the trispecific anti-EGFR/cMet antibodies in the tumor target cells EBC-1 was assayed by the FACS method.

Tumor target cells EBC-1 were prepared and seeded in a 96-well plate at 3 × 10⁵ cells/well. The trispecific anti-EGFR/cMet antibody molecules V-20-Fc and V-23-Fc, anti-cMet M-4, and anti-EGFR molecules M-5, M-6, and M-7 (40 nM, 5-fold diluted) were separately added. The mixtures were incubated at 4 °C for 30 min and centrifuged, and the supernatants containing respective VHH-Fcs were removed. The cells were evenly divided into 2 groups and separately incubated at 4 °C and 37 °C for 4 h. After the incubation was completed, PBS chilled in ice bath was immediately added to terminate the endocytosis experiment. The secondary antibody PE-anti-human IgG (eBioscience, Cat. No. 12-4998-82) was added. The mixtures were incubated at 4 °C for 30 min, and then the MFI of the cells was assayed by a flow cytometer (Life Technologies).

The internalization level of the cell surface-bound antibody was calculated using the formula below:${\text{endocytosis = MFI of sample incubated at 4}}^{∘} {\text{C - MFI of sample incubated at 37}}^{∘} \text{C} \text{.}$

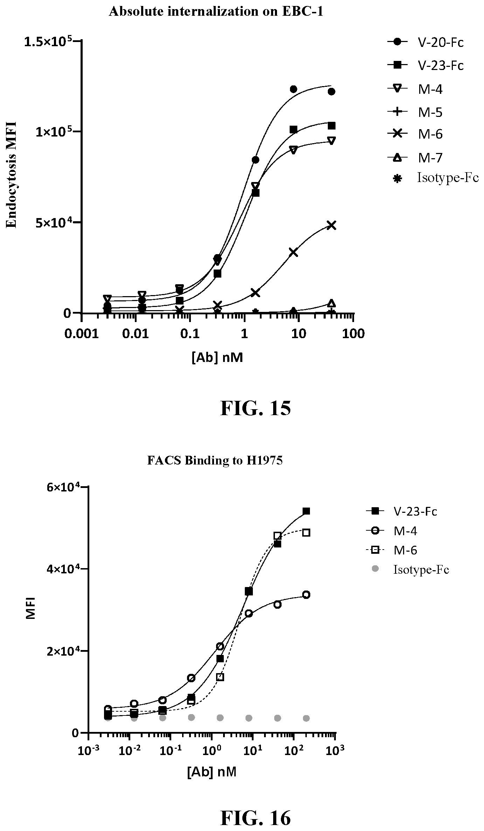

As shown in FIG. 15, the tumor target cells EBC-1 exhibited relatively high endocytosis for the trispecific antibody molecules V-20-Fc and V-23-Fc (as compared to anti-cMet M-4 and anti-EGFR molecules M-5, M-6, and M-7).

### 6.7 FACS ASSAY ON SYNERGISTIC BINDING OF TRISPECIFIC ANTIBODIES TO TARGET CELLS

NCI-H1975 cells were seeded in a 96-well plate at 1.5 × 10⁵ cells/well. The trispecific antibodies of the present disclosure (200 nM, 5-fold diluted) were added. The mixtures were incubated at 4 °C for 1 h and then centrifuged at a centrifugal speed of 300 g for 4 min. The supernatants were removed. The plate was washed 2 times with FACS buffer (1% BSA) by repeated centrifugation. The secondary antibody PE-anti-human IgG (eBioscience, Cat. No. 12-4998-82) was added. The mixtures were incubated at 4 °C for 1 h, and then the plate was washed 2 times by centrifugation. Finally, the cells were resuspended in FACS buffer (1% BSA), and the MFI of the cells was assayed by a flow cytometer (Life Technologies).

As shown in FIG. 16, V-23-Fc had stronger binding ability compared to its corresponding control antibodies (M4 and M6), indicating that these two targets have a synergistic binding effect.

### EXAMPLE 7. PREPARATION, CHARACTERIZATION, AND ASSAY OF ANTIBODY-DRUG CONJUGATES (ADCS)

Linker-payloads used in the example section of the present disclosure are known in the art and/or commercially available, or may be prepared as described herein. When the drawn structure is inconsistent with the actual situation, the structure should be allowed to be modified or corrected according to the actual situation.

### General synthetic method A

The antibodies of the present disclosure (5.45 mg/mL) in 20 mM His-hacn, 150 mM NaCl, pH 5.5 were placed in Eppendorf tubes. Six molar equivalents of TCEP (Tris(2-carboxyethyl)phosphine hydrochloride, 5 mM) were added to the antibody buffer (TCEP:antibody = 6:1). The Eppendorf tubes containing the reaction mixtures were placed on a shaker (×500 rpm), and the mixtures were reacted at 37 °C for 2 h. Another 6 molar equivalents of TCEP (5 mM) were added to the mixtures. The reaction mixtures were placed on a shaker (500 rpm) and then shaken at 37 °C for 2 h. Then, ultrafiltration (MWCO 30 kd) was performed to remove TCEP, and the buffer was supplemented with 20 mM His-hac buffer. Six molar equivalents of a linker-payload (5 mg/mL in DMA) were added dropwise to the fully reduced antibodies, while the DMA concentration was kept below 20% (v/v). The reaction was maintained on a shaker at room temperature for 2 h (linker-payload:antibody = 6:1). The conversion rate was determined by HIC-HLPC. Purification was performed when the conversion was completed. The reaction mixtures were transferred to ultrafiltration tubes (MWCO 30 kd) and the samples were centrifuged at a rotation speed of 10,000 rpm for 5 min until the volumes of the solutions were reduced by half, and His-hac buffer (containing 10% DMA) was then added to replenish to the original volumes. The flow-through substances were discarded. The washing step was repeated 10 times. Then, solution replacement was performed by repeated washing 10 times with 10 mM His-hac pH = 5.0, and finally the remaining solutions were transferred and adjusted to an appropriate concentration.

When another method, Pro A, was used for purification (the ProA product was labeled with a capacity of 40 g/L), washing was performed with 10 CV of 10 mM His-hac before use. Then, the samples were loaded onto gravity columns packed with Pro A resin. Free payload was completely removed by washing with at least 10 CV of 10 mM His-hac buffer containing 10% DMSO and 50 CV of 10 mM His-hac buffer containing no DMSO. Elution was then performed with a pH 3 acetic acid solution (at a concentration of 50 mM), and the eluates were immediately neutralized to pH 5.5 to pH 6.0 with 2 M Tris buffer (pH 12.0). The conjugate solutions were combined in Eppendorf tubes. The concentration was measured (Nanodrop, A280). 10 mM His-Hac (pH 6) was used as a blank. If the final concentration was lower than the expected concentration, the solutions were concentrated to the required concentration by the centrifugal ultrafiltration method (MWCO 30 kd ultrafiltration membrane).

The purity was determined by HIC and SEC-HPLC methods. Free linker-payload was determined by the RP-HPLC method.

### General methods and/or parameters for determining or detecting ADCs

### ◆ Size exclusion chromatography (SEC) method (for total ADC detection)

### Parameter of SEC-HPLC method

| Chromatographic condition | |
|---|---|
| Column compartment | 25±1°C |
| Flow rate | 0.8 mL/mL |
| Run time | 20 min |
| Injection volume | At least 40 µg |
| Elution type | Isocratic elution |
| Column | Zenix-C SEC-150, 7.8*300mm 150* |
| Mobile phase | 50mM PB +300 mM NaCl, pH 6.8 ±0.1 |

### ◆ Reverse-phase HPLC (RP HPLC) method (for free drug detection)

### Parameter of RP HPLC method

| | |
|---|---|
| Column | Agilent ZORBAX StableBond 300 C3 column, 4.6 × 150 mm, 5 µm |
| Detection wavelength | 280 nm; 214 nm (optional) |
| Temperature of column compartment | 60 °C |
| Temperature of sampler | 15 °C |
| Flow rate | 1.0 mL/min |
| Injection volume | 2µg |
| Mobile phase | Mobile phase A: a solution of 0.1% TFA in H₂O |
| | Mobile phase B: a solution of 0.1% TFA in ACN |

Elution was performed according to the following table.

| Time (min) | Mobile phase A (%) | Mobile phase B (%) |
|---|---|---|
| 0.0 | 75.0 | 25.0 |
| 2.0 | 75.0 | 25.0 |
| 10.0 | 5.0 | 95.0 |
| 12.0 | 5.0 | 95.0 |
| 14.0 | 5.0 | 95.0 |
| 16.0 | 80.0 | 20.0 |
| 18.0 | 80.0 | 20.0 |

### ◆ HIC-HPLC method (for detection of free antibody and DAR distribution)

### HIC-HPLC condition.

| | |
|---|---|
| Column | MAbPac^{™} HIC-Butyl HPLC column, 5 µm, 4.6 × 100 mm, Thermo SCIENTIFIC |
| Detection wavelength | 280 nm; 214 nm (optional) |
| Temperature of column compartment | 25°C |
| Temperature of sampler | 15°C |
| Flow rate | 0.6 mL/min |
| Injection volume | 30-50µg |
| Mobile phase | Mobile phase A: 1.5 M (NH₄)₂SO₄ and 50 mM PB, pH 7.4 |
| | Mobile phase B: 50 mM PB, 25% IPA, pH 7.4 |

Elution was performed according to the following table.

| Time (min) | Mobile phase A (%) | Mobile phase B (%) |
|---|---|---|
| 0.0 | 100.0 | 0.0 |
| 7.0 | 100.0 | 0.0 |
| 23.0 | 0.0 | 100.0 |
| 26.0 | 0.0 | 100.0 |
| 26.5 | 100.0 | 0.0 |
| 35.0 | 100.0 | 0.0 |

### 7.1 Preparation and characterization of ADC molecules

### a) Preparation of Mal-PEG8-VA-PAB-Exatecan-conjugated ADC molecules

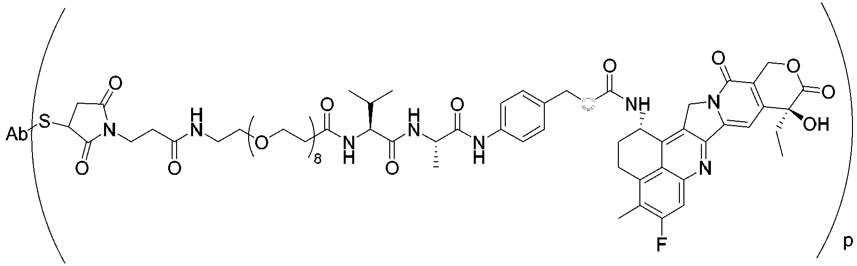

Ab was the antibody V-26-Fc prepared in the present application, and p was mainly 4. It will be understood that p may also be another positive integer, e.g., 1, 2, 3, 5, 6, 7, 8, 9, or 10.

According to the synthetic method A described above, V-26-Fc-VA-Exd (MW 96.37, average Dar 4.0, yield: 63%, purity: 91.73%) was prepared using the antibody V-26-Fc and the linker-payload Mal-PEG8-VA-PAB-Exatecan (CAS No.: 2679821-39-5; MedChemExpress, HY-147271).

According to the preparation method for V-26-Fc-VA-Exd, the antibody V-26-Fc was separately replaced by V-17-Fc, V-20-Fc, and V-23-Fc to prepare V-17-Fc-VA-Exd, V-20-Fc-VA-Exd, and V-23-Fc-VA-Exd, respectively.

### b) Preparation of MC-VC-PAB-MMAE-conjugated ADC molecules

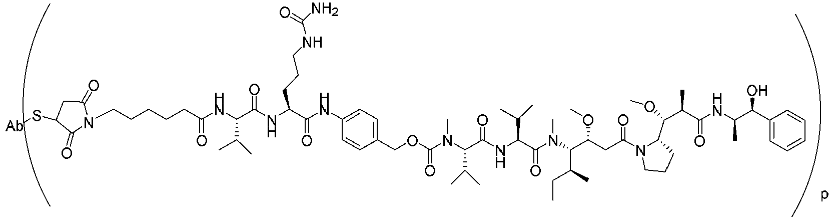

Ab was the antibody V-26-Fc prepared in the present application, and p was mainly 4. It will be understood that p may also be another positive integer, e.g., 1, 2, 3, 5, 6, 7, 8, 9, or 10.

According to the synthetic method A described above, V-26-Fc-VC-MMAE was prepared using the antibody V-26-Fc and the linker-payload MC-VC-PAB-MMAE (CAS No.: 646502-53-6; MedChemExpress, HY-15575).

According to the preparation method for V-26-Fc-VC-MMAE, the antibody V-26-Fc was separately replaced by V-17-Fc, V-20-Fc, and V-23-Fc to prepare V-17-Fc-VC-MMAE, V-20-Fc-VC-MMAE, and V-23-Fc-VC-MMAE, respectively.

### c) Preparation of Mal-PEG8-VC-PAB-MMAE-conjugated ADC molecules

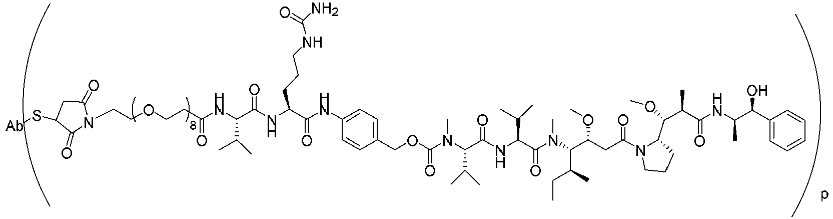

Ab was the antibody V-26-Fc prepared in the present application, and p was mainly 4. It will be understood that p may also be another positive integer, e.g., 1, 2, 3, 5, 6, 7, 8, 9, or 10.

According to the synthetic method A described above, V-26-Fc-PEG-VC-MMAE was prepared using the antibody V-26-Fc and the linker-payload Mal-PEG8-VC-PAB-MMAE (CAS No.: 2353409-69-3; MedChemExpress, HY-141156).

According to the preparation method for V-26-Fc-PEG-VC-MMAE, the antibody V-26-Fc was separately replaced by V-17-Fc, V-20-Fc, and V-23-Fc to prepare V-17-Fc-PEG-VC-MMAE, V-20-Fc-PEG-VC-MMAE, and V-23-Fc-PEG-VC-MMAE, respectively.

### d) Preparation of Mal-PEG8-EVC-PAB-MMAE-conjugated ADC molecules

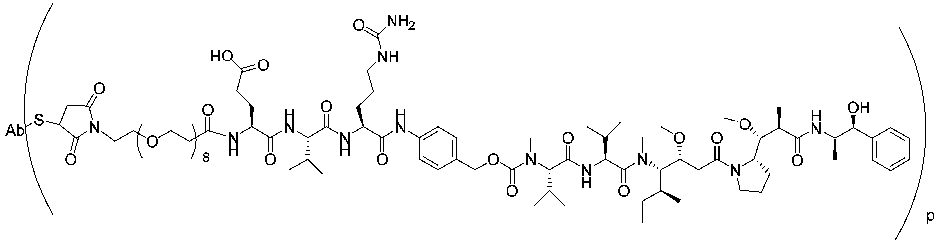

Ab was the antibody V-26-Fc prepared in the present application, and p was mainly 4. It will be understood that p may also be another positive integer, e.g., 1, 2, 3, 5, 6, 7, 8, 9, or 10.

According to the synthetic method A described above, V-26-Fc-PEG-EVC-MMAE was prepared using the antibody V-26-Fc and the linker-payload Mal-PEG8-EVC-PAB-MMAE (prepared according to the preparation method in the present application).

According to the preparation method for V-26-Fc-PEG-EVC-MMAE, the antibody V-26-Fc was separately replaced by V-17-Fc, V-20-Fc, and V-23-Fc to prepare V-17-Fc-PEG-EVC-MMAE, V-20-Fc-PEG-EVC-MMAE, and V-23-Fc-PEG-EVC-MMAE, respectively.

### e) Preparation of MC-EVC-PAB-MMAE-conjugated ADC molecules

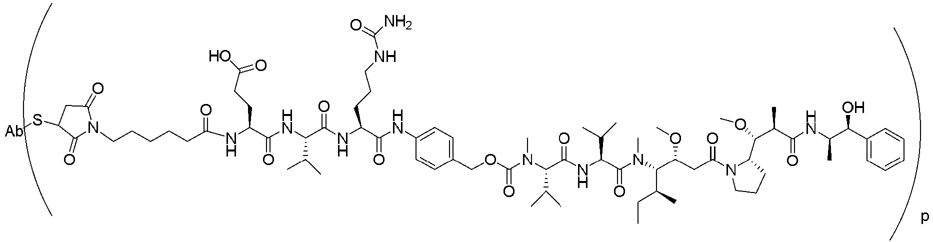

Ab was the antibody V-26-Fc prepared in the present application, and p was mainly 4. It will be understood that p may also be another positive integer, e.g., 1, 2, 3, 5, 6, 7, 8, 9, or 10.

According to the synthetic method A described above, V-26-Fc-EVC-MMAE was prepared using the antibody V-26-Fc and the linker-payload MC-EVC-PAB-MMAE (CAS No.: 2873452-49-2; MedChemExpress, HY-154915).

According to the preparation method for V-26-Fc-EVC-MMAE, the antibody V-26-Fc was separately replaced by V-17-Fc, V-20-Fc, and V-23-Fc to prepare V-17-Fc-EVC-MMAE, V-20-Fc-EVC-MMAE, and V-23-Fc-EVC-MMAE, respectively.

### f) Preparation of Mal-Gly-Exatecan-D-glucuronic acid-conjugated ADC molecules:

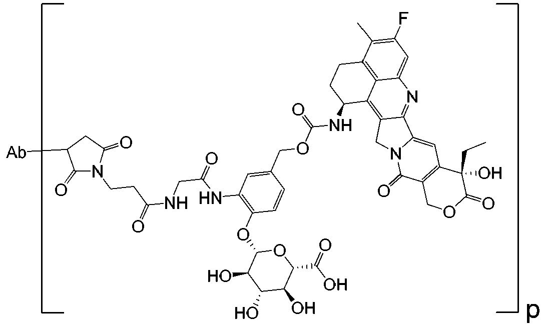

Ab was the antibody V-23-Fc prepared in the present application, and p was mainly 4. It will be understood that p may also be another positive integer, e.g., 1, 2, 3, 5, 6, 7, 8, 9, or 10.

According to the synthetic method A described above, V-23-Fc-Gluc-Exd (or referred to as V-23-Fc-Glu-Exd; MW 96, average Dar 4.0, yield 50%, purity 84.31%) was prepared using the antibody V-23-Fc and the linker-payload Mal-Gly-Exatecan-D-glucuronic acid (CA No.: 2763252-25-9; MedChemExpress, HY-153179).

According to the preparation method for V-23-Fc-Gluc-Exd, the antibody V-23-Fc was separately replaced by V-26-Fc, V-17-Fc, and V-20-Fc to prepare V-26-Fc-Gluc-Exd, V-17-Fc-Gluc-Exd, and V-23-Fc-Gluc-Exd, respectively.

Characterization data for exemplary ADCs prepared in the present application are as follows:

| **Molecule ID** | **Conjugation means** | **MW** | **Free payload** | **Average Dar value** | **Yield (%)** | **Purity (%)** |
|---|---|---|---|---|---|---|
| V-26-Fc-VA-Exd | Cysteine | 96.37 | <1% | Average Dar 4.0 | 63% | 91.73% |
| V-20-Fc-VA-Exd | Cysteine | 96.36 | <1% | Average Dar 4.0 | 69% | 87.2% |
| V-23-Fc-VA-Exd | Cysteine | 97.51 | <1% | Average Dar 4.0 | 62% | 92.7% |
| V-26-Fc-VC-MMAE | Cysteine | 96.31 | <1% | Average Dar 4.0 | 66.6% | 99.67% |
| V-20-Fc-VC-MMAE | Cysteine | 96.07 | <1% | Average Dar 4.0 | 68.2% | 98.3% |
| V-23-Fc-VC-MMAE | Cysteine | 97.51 | <1% | Average Dar 4.0 | 64.7% | 93.5% |
| V-26-Fc-PEG-VC-MMAE | Cysteine | 97.56 | <1% | Average Dar 3.7 | 66% | 89.3% |
| V-20-Fc-PEG-EVC-MMAE | Cysteine | 97.84 | <1% | Average Dar 4.0 | 62.8% | 86.94% |
| V-23-Fc-PEG-EVC-MMAE | Cysteine | 99.21 | <1% | Average Dar 3.7 | 50% | 89.54% |
| V-26- Fc-PEG- EVC-MMAE | Cysteine | 98.07 | <1% | Average Dar 4.0 | 65% | 94.92% |
| V-23-Fc-Gluc-Exd | Cysteine | 96 | <1% | Average Dar 4.0 | 50% | 84.31% |

### Preparation of reference benchmark antibody-drug conjugate AZD9592

A TCEP solution (15 equivalents) was added to an antibody **(RAA22/B09-57, also referred to as BMK-AZD or AZD Ab herein)** solution. The mixture was mixed well and left to react at 37 °C for 2 h. DMA and 16 equivalents of a 10 mM linker-payload (Mal-PEG8-amide-Val-Ala-(4-NH2)-Exatecan) stock solution prepared by dissolving in DMA were added to the reaction liquid. The organic solvent in the final reaction system accounted for 10%. The reaction liquid was mixed well and placed in a thermostatic shaker at 22 °C to react for another 1 hour. Then, N-acetylcysteine was added to quench the reaction. The sample obtained after conjugation was purified using an ultrafiltration concentration tube and buffer exchanged into a storage buffer (30 mM histidine, 30 mM arginine-HCl, containing 0.02% PS 80, pH 6.8) for the final ADCs. The sample was sterilized by filtration through a 0.22 µm syringe filter.

The characterization data for the reference benchmark antibody-drug conjugate of the present application are as follows:

| **Molecule ID** | **MW** | **Free payload** | **Average Dar value** | **Yield (%)** | **Purity (%)** |
|---|---|---|---|---|---|
| AZD9592 | 96.37 | 1.2% | Average Dar 5.9 | 95.82% | 98.57% |
| ABBV399 | 96.36 | <1% | Average Dar 3.0 | 27.6% | 100% |

### Preparation of Mal-PEG8-EVC-PAB-MMAE of the present application

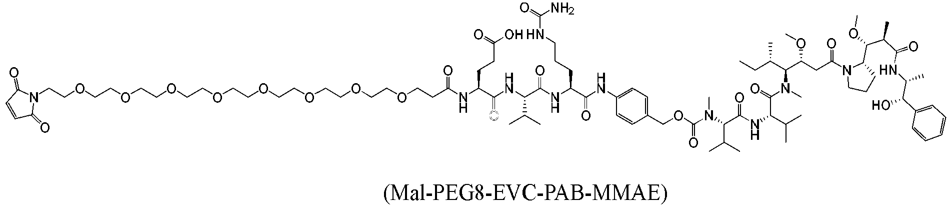

### Step 1:

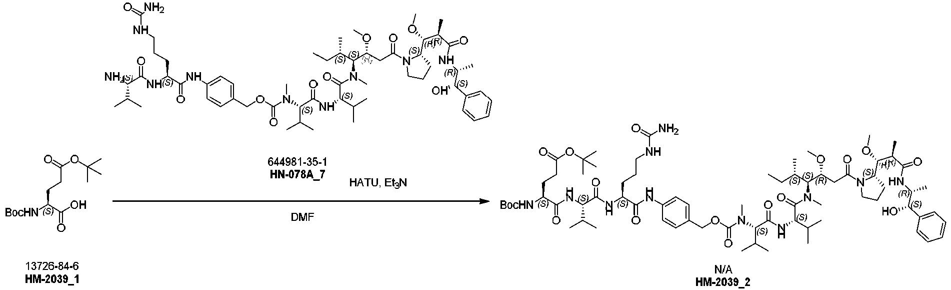

(1) Compound HM-2039_1 (CAS: 13726-84-6) (100 mg, 0.33 mmol, 1 eq) and HN-078A_7 (Catalog: HY-100374, CAS: 644981-35-1) (388.86 mg, 0.346, 1.05 eq) were added to DMF (6 mL), and HATU (188 mg) and triethylamine (66.7 mg) were added. The mixture was reacted for 4 h under N₂ atmosphere.
(2) Upon reaction completion, as monitored by LCMS, the reaction liquid was added dropwise to water. The mixture was extracted twice with EA (30 mL × 2). The organic phases were combined, washed three times with saturated brine (30 mL × 3), dried over anhydrous sodium sulfate, and concentrated to give a white solid (410 mg, yield: 88.29%).

### Step 2:

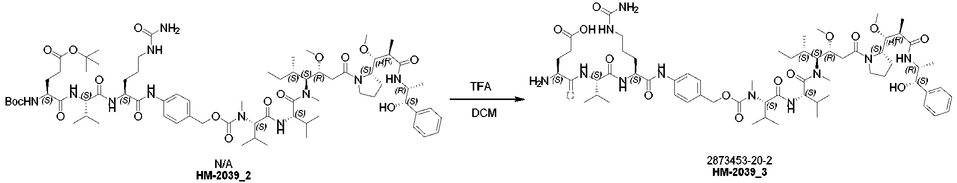

(1) Compound HM-2039_2 (410 mg, 0.291 mmol, 1 eq) was added to DCM (4 mL), and TFA (1 mL) was added. The mixture was reacted (at 0-10 °C) for 1 h under nitrogen atmosphere.
(2) Upon reaction completion, as monitored by LCMS, DCM was removed by concentration at a low temperature to give a crude product, which was purified by reverse-phase column chromatography to give a product (200 mg, yield: 54.86%) as a white solid.

### Step 3:

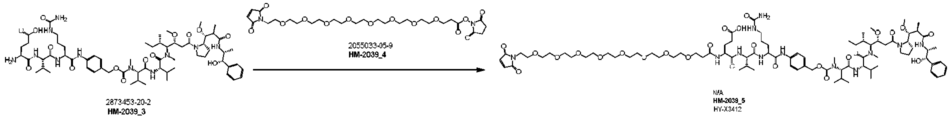

(1) HM-2039 _3 (0.1 g, 79.84 µmol, 1 eq) and HM-2039_4 (CAS: 1818294-46-0) (51.86 mg, 83.83 µmol, 1.05 eq) were dissolved in DMF. The reaction liquid was stirred for 2 h.
(2) Upon reaction completion, as monitored by LCMS, the reaction liquid was directly purified by preparative chromatography (TFAin water, ACN) to give a target product (30 mg, yield: 23.54%, MW 1756.08, purity: 99.18%) as a white solid.

¹H NMR (400 MHz, DMSO) δ 10.04 (s, 1H), 8.35-8.25 (m, 0.28H), 8.25-8.15 (m, 1H), 8.13-8.03 (m, 1.5H), 7.94-7.87 (m, 0.4H), 7.76-7.70 (m, 1H), 7.67-7.63 (m, 0.4H), 7.62-7.57 (m, 1.7H), 7.38-7.25 (m, 7H), 7.22-7.14 (m, 1H), 7.04 (s, 2H), 5.99 (s, 1H), 5.66 - 5.20 (m, 2H), 5.15-4.95 (s, 2H), 4.80 - 4.15 (m, 8H), 4.10-3.90 (m, 2H), 3.63-3.56 (m, 5H), 3.52-3.48 (m, 28H), 3.28-3.24 (m, 4H), 3.22-3.18 (m, 5H), 3.16-3.10 (m, 2H), 3.10 - 2.84 (m, 9H), 2.50-2.40 (m, 2H), 2.30-2.20 (m, 3H), 2.20-1.69 (m, 10H), 1.67-1.30 (m, 7H), 1.08 - 0.98 (m, 7H), 0.89 - 0.76 (m, 26H).

### 7.2 FACS binding assay on ADCs of the present disclosure

Binding of the ADC molecules formed by conjugating the trispecific anti-EGFR/cMet antibody molecules described above to a toxin molecule (i.e., payload) to target cells MDA-MB-468, EBC-1, and NCI-H1975 (Cell Bank of the Chinese Academy of Sciences) was assayed by the FACS method.

The experimental method is as described in Example 6.2. Target cells were separately prepared and seeded in a 96-well plate at 1.5 × 10⁵ cells/well. Test ADCs (200 nM or 40 nM, 5-fold diluted) were added, and the mixtures were incubated at 4 °C for 1 h and then centrifuged at a centrifugal speed of 300 g for 4 min. The supernatants were removed. The plate was washed 2 times with FACS buffer (1% BSA) by repeated centrifugation. The secondary antibody (PE-anti-human IgG (eBioscience, Cat. No. 12-4998-82)) was added. The mixtures were incubated at 4 °C for 1 h, and then the plate was washed 2 times by centrifugation. Finally, the cells were resuspended in FACS buffer (1% BSA), and the MFI of the cells was assayed by a flow cytometer (Life Technologies).

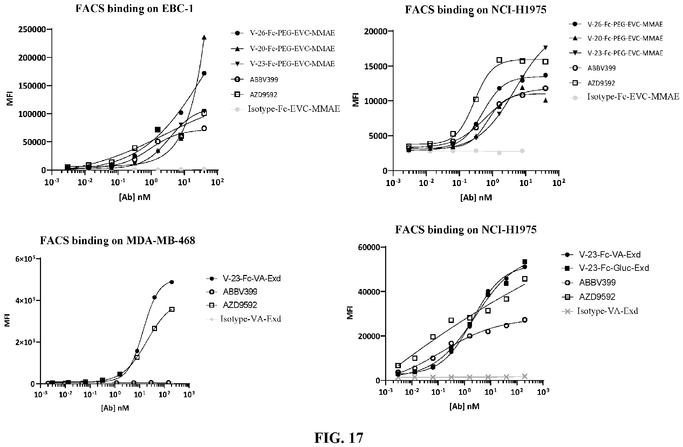

As shown in FIG. 17, V-26-Fc-PEG-EVC-MMAE, V-20-Fc-PEG-EVC-MMAE, and V-23-Fc-PEG-EVC-MMAE were able to bind to EBC-1 and NCI-H1975 cells. Likewise, V-23-Fc-VA-Exd and V-23-Fc-Gluc-Exd were able to bind to MDA-MB-468 cells and NCI-H1975 cells, and the binding activity was better than that of the control groups AZD9592 and ABBV399.

### 7.3. Cell killing assay of ADCs of the present disclosure

Killing of target cells MDA-MB-468, EBC-1, and NCI-H1975 cells (Cell Bank of the Chinese Academy of Sciences) by the ADC molecules formed by conjugating the trispecific anti-EGFR/cMet antibody molecules described above to a toxin was assayed by the FACS method.

The cells (100 µL, 1 × 10⁴) resuspended in a cell culture medium were seeded into each well of a 96-well plate and cultured in an incubator at 37 °C with 5% CO₂ overnight. The next day, 100 µL of serially diluted V-20-Fc-PEG-EVC-MMAE, V-23-Fc-PEG-EVC-MMAE, and V-26-Fc-PEG-EVC-MMAE, as well as positive control ADCs ABBV399 and AZD9592 were added to corresponding wells. The cells were cultured for another 3-5 days. Then, 20 µL of CCK8 (Shanghai Life iLab Biotech, Cat. No. AC11L054) was added to each well. The mixtures were cultured in an incubator at 37 °C with 5% CO₂ until color development. The absorbance at 450 nm was recorded using a microplate reader (Molecular Device, SpectraMax M5).

The cell viability was calculated by the following formula: percentage cell viability = [(As - Ab) / (Ac - Ab)] × 100. As = experimental well absorbance (cells, culture medium, CCK8, and ADCs); Ab = blank well absorbance (culture medium and CCK8); Ac = control well absorbance (cells, culture medium, and CCK8).

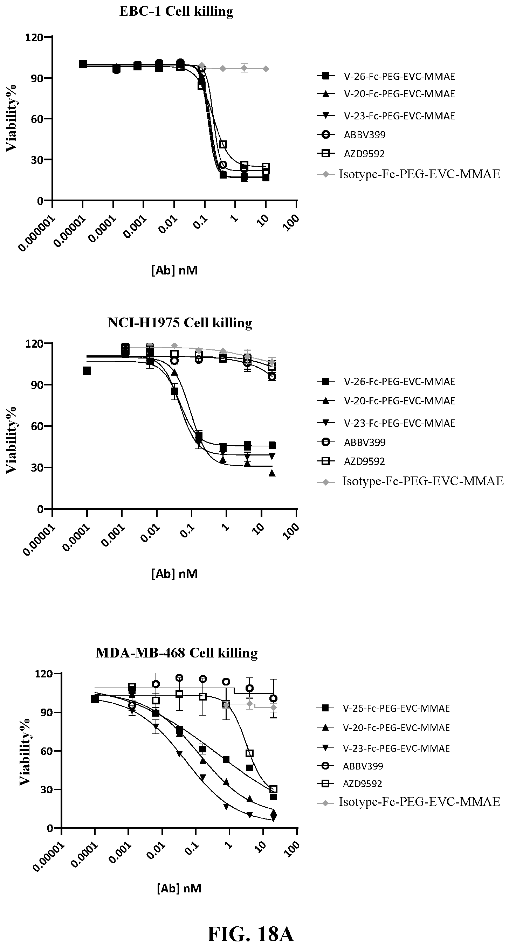

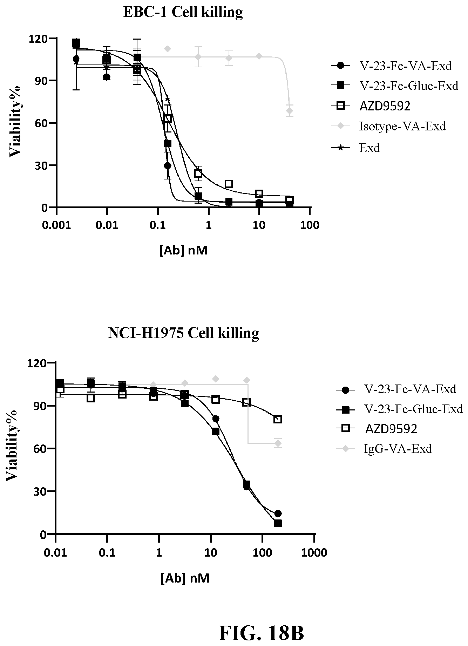

The results of the killing effect of V-20-Fc-PEG-EVC-MMAE, V-23-Fc-PEG-EVC-MMAE, and V-26-Fc-PEG-EVC-MMAE on EBC-1, NCI-H1975, and MDA-MB-468 are shown in Table 10A below and FIG. 18A. The results of the killing effect of V-23-Fc-VA-Exd and V-23-Fc-Gluc-Exd on EBC-1 and NCI-H1975 are shown in Table 10B below and FIG. 18B. The results showed that the killing effect of each ADC molecule on target cells was substantially equal to or better than that of the positive control drugs (ABBV399 and AZD9592).

**Table 10A. Killing of target cells by V-20-Fc-PEG-EVC-MMAE, V-23-Fc-PEG-EVC-MMAE, and V-26-Fc-PEG-EVC-MMAE**

| **Name of ADC** | **IC₅₀ value** |
|---|---|
| Target cell: EBC-1 | |
| V-20-Fc-PEG-EVC-MMAE | 0.1546 nM |
| V-23-Fc-PEG-EVC-MMAE | 0.1414 nM |
| V-26-Fc-PEG-EVC-MMAE | 0.1315 nM |
| AZD9592 | 0.1893 nM |
| ABBV399 | 0.1937 nM |

| Target cell: NCI-H1975 | |
|---|---|
| V-20-Fc-PEG-EVC-MMAE | 0.09336 nM |
| V-23-Fc-PEG-EVC-MMAE | 0.04603 nM |
| V-26-Fc-PEG-EVC-MMAE | 0.04696 nM |
| AZD9592 | 11.3 nM |
| ABBV399 | - |

| Target cell: MDA-MB-468 | |
|---|---|
| V-20-Fc-PEG-EVC-MMAE | 0.1282 nM |
| V-23-Fc-PEG-EVC-MMAE | 0.04861 nM |
| V-26-Fc-PEG-EVC-MMAE | 0.4841 nM |
| AZD9592 | 3.231 nM |
| ABBV399 | - |

| | |
|---|---|
| Note: "-" indicates that the IC₅₀ value could not be fitted. | |

**Table 10B. Killing of target cells by V-23-Fc-VA-Exd and V-23-Fc-Gluc-Exd**

| **Name of ADC** | **IC₅₀ value** |
|---|---|
| Target cell: EBC-1 | |
| V-23-Fc-VA-Exd | 0.1381 nM |
| V-23-Fc-Gluc-Exd | 0.1290 nM |
| AZD9592 | 0.1641 nM |

| Target cell: NCI-H1975 | |
|---|---|
| V-23-Fc-VA-Exd | 25.29 nM |
| V-23-Fc-Gluc-Exd | 33.99 nM |
| AZD9592 | - |

| | |
|---|---|
| Note: "-" indicates that the IC₅₀ value could not be fitted. | |

### 7.4. Assay on cell binding of and cell killing by PEG-modified and/or EVC-modified ADCs

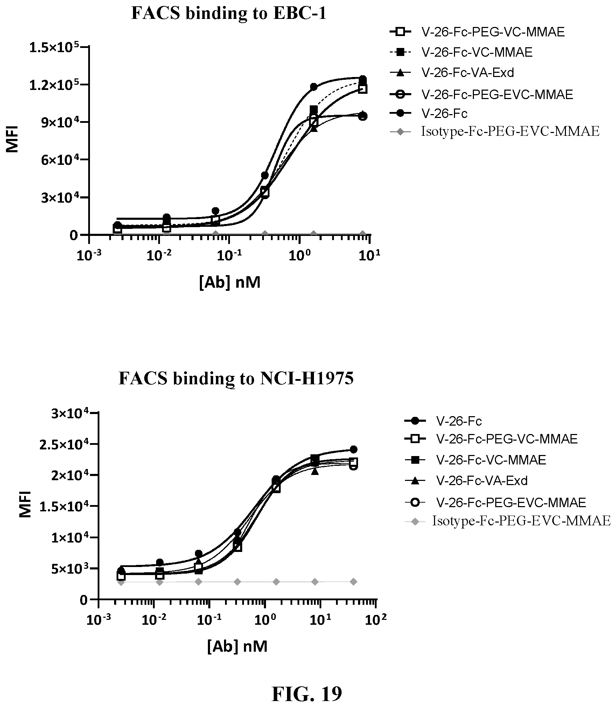

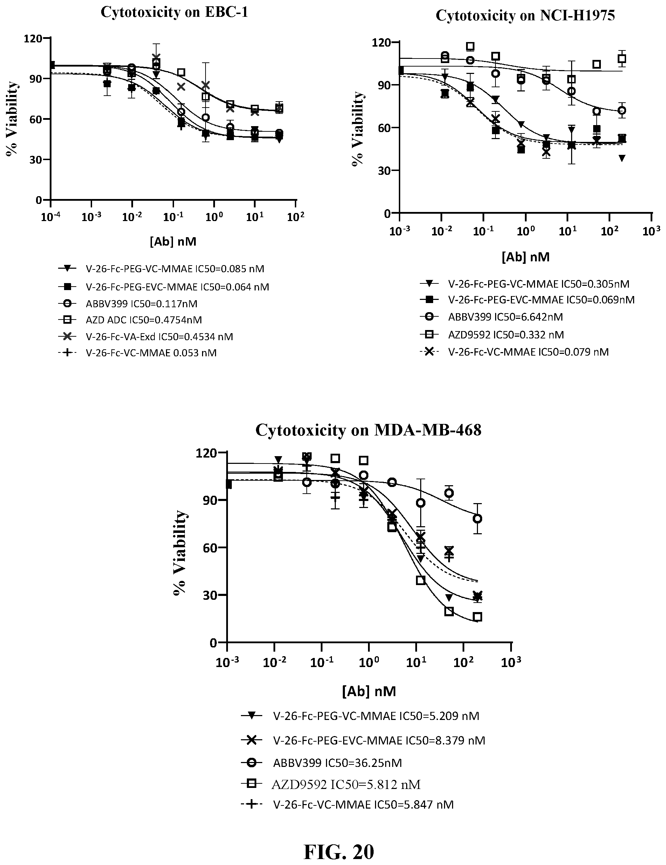

V-26-Fc, a representative of the antibody moiety, was separately conjugated to Mal-PEG8-EVC-PAB-MMAE (V-26-Fc-PEG-EVC-MMAE), Mal-PEG8-VC-PAB-MMAE (V-26-Fc-PEG-VC-MMAE), and MC-VC-PAB-MMAE (V-26-Fc-VC-MMAE), as well as Mal-PEG8-VA-PAB-Exatecan (V-26-Fc-VA-Exd). The FACS binding assay is as described above (e.g., Example 6.2 or 7.2). The experimental results are shown in FIG. 19. The experimental procedures of the cell killing assay are as described in section 7.3, and the experimental results are shown in FIG. 20.

### Example 8. Evaluation of In Vivo Efficacy of Multispecific Anti-EGFR/cMet Antibody-Drug Conjugates (ADCs)

*In vivo* efficacy of ADC molecules formed by conjugating trispecific anti-EGFR/cMet antibody molecules to a toxin molecule was evaluated using the EBC-1, NCI-H1975, NCI-H441 (lung adenocarcinoma cells), MDA-MB-468, FADU (head and neck cancer cells), and SW48 (colon adenocarcinoma cells) CDX mouse models. Tumor cells were thawed using a fresh culture medium. Cells in the logarithmic growth phase were collected. During cell collection, the culture medium was removed, and the cells were washed twice and then resuspended in DPBS. The cells were then inoculated into BALB/c nu/nu mice by subcutaneous injection. The inoculation amounts for the EBC-1, NCI-H1975, NCI-H441, MDA-MB-468, FADU, and SW48 CDX models were 3 × 10⁶/100 µL, 3 × 10⁶/100 µL, 1 × 10⁷/100 µL, 1 × 10⁷/100 pL, 5 × 10⁶/100 pL, and 5 × 10⁶/100 µL, respectively. When the average tumor volume of the mice reached 110-250 mm³, the mice were randomly grouped according to tumor volume. The day of grouping was defined as day 0. All the CDX mouse models were dosed with a test sample on D0 by means of a single intravenous bolus injection. After the start of administration, till the study endpoint, the mice were weighed 1-2 times weekly, the tumor volume was measured 1-2 times weekly, and the animals were observed 2 times daily. The tumor volume was calculated by the following formula: tumor volume (mm³) = 0.5 × tumor length × tumor width². Tumor inhibition was represented by the change in tumor size (based on baseline), so as to evaluate the antitumor efficacy of the test sample. The change in tumor size (based on baseline) was calculated by the following formula: $change in tumor size = \left({V}_{t}-{V}_{0}\right) / {V}_{0} \times 100 %$
*Vₜ*: average tumor volume of the mice in the test sample treatment group on day t;
*V*₀: average tumor volume of the mice in the test sample treatment group on day 0;

### 8.1 In vivo efficacy of V-20-Fc-PEG-EVC-MMAE, V-23-Fc-PEG-EVC-MMAE, and V-26-PEG-EVC-MMAE in CDX models

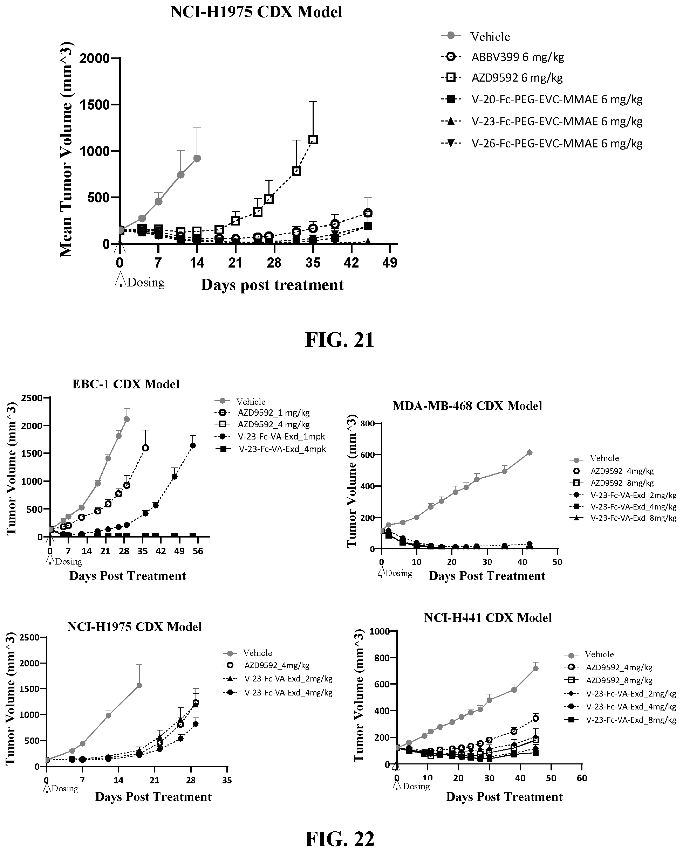

As shown in FIG. 21, the experimental results demonstrated that V-20-Fc-PEG-EVC-MMAE, V-23-Fc-PEG-EVC-MMAE, and V-26-Fc-PEG-EVC-MMAE all exhibited strong tumor inhibition effects. After intravenous injection of the drugs, the tumor volume significantly shrank, and the efficacy was better than that of positive control drugs ABBV399 and AZD9592.

### 8.2 In vivo efficacy of V-23-Fc-VA-Exd in CDX models

*In vivo* efficacy of V-23-Fc-VA-Exd was evaluated using EBC-1, NCI-H1975, NCI-H441, and MDA-MB-468 CDX mouse models. As shown in FIG. 22, the experimental results demonstrated that V-23-Fc-VA-Exd exhibited a better *in vivo* tumor inhibition effect than that of the positive control drug AZD9592.

In the EBC-1 model, on day 54 after administration, high-dose V-23-Fc-VA-Exd and AZD9592 achieved complete tumor elimination; at a lower dose, V-23-Fc-VA-Exd had better efficacy than AZD9592.

In the NCI-H1975 model, on day 29 after administration, the tumor inhibition effect of 2 mg/kg V-23-Fc-VA-Exd was comparable to that of 4 mg/kg AZD9592; V-23-Fc-VA-Exd had better efficacy than AZD9592 at the same dose.

In the NCI-H441 model, on day 45 after administration, V-23-Fc-VA-Exd had better efficacy than AZD9592 at the same dose; the tumor inhibition effect of 2 mg/kg V-23-Fc-VA-Exd was comparable to that of 8 mg/kg AZD9592.

In the MDA-MB-468 model, on day 42 after administration, the tumors were completely eliminated by V-23-Fc-VA-Exd and AZD9592 at 4 mg/kg and 8 mg/kg; the tumor volume change rate resulting from 2 mg/kg V-23-Fc-VA-Exd was -72%.

### 8.3 In vivo efficacy of V-23-Fc-Gluc-Exd in CDX models

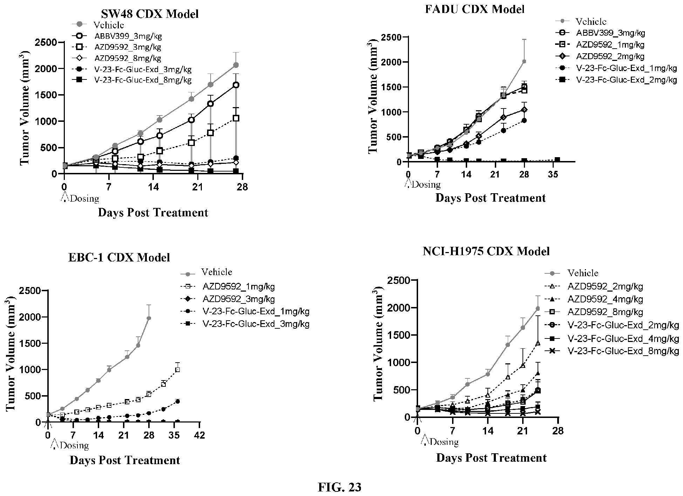

The *in vivo* efficacy of V-23-Fc-Gluc-Exd was evaluated using SW48 CDX (colorectal cancer model, EGFR⁺⁺ & cMet⁺), FADU CDX (head and neck cancer model, EGFR⁺⁺⁺ & cMet^{+/-}), EBC-1 CDX (non-small cell lung cancer model, EGFR⁺⁺ & cMet⁺⁺⁺), and NCI-H1975 CDX (non-small cell lung cancer model, EGFR⁺ & cMet⁺) mouse models. As shown in FIG. 23, the experimental results demonstrated that V-23-Fc-Gluc-Exd exhibited a better *in vivo* tumor inhibition effect than the positive control drug.

In the SW48 model, the FADU model, and the NCI-H1975 model, V-23-Fc-Gluc-Exd had better efficacy than the positive control drugs, including AZD9592 and ABBV399, at the same dose.

In the EBC-1 CDX model, on day 36 after administration, at a low dose (1 mg/kg), V-23-Fc-Gluc-Exd had better efficacy than AZD9592, and the tumors in the two groups of animals were completely eliminated at a dose of 3 mg/kg.

The exemplary examples of the present disclosure have been described above, and it should be understood by those skilled in the art that these disclosed content is merely exemplary and that various other substitutions, adaptations, and modifications may be made within the scope of the present disclosure. Therefore, the present disclosure is not limited to the specific examples listed herein.

### Brief Description of Sequence Listing:

| **Sequence No.** | **Description** | **Sequence** |
|---|---|---|
| **Anti-EGFR antibodies; CDRs defined according to Kabat numbering scheme, underlined** | | |
| | | **V-n5B10** |
| 1 | Amino acid sequence of VHH | AVQLVDSGGGLVQPGGSLRLDCSASGTIIGTYTMAWYRQAPG KQREMVAIITPGGGSHHADSVKGRFTISRDNGKSTVYLQMSSL KFEDTAVYYCNAKARWGTTEYWGQGTQVTVSS |
| 2 | Nucleotide sequence of VHH | GCCGTGCAGCTGGTGGATTCTGGGGGAGGCTTGGTGCAGCC TGGGGGGTCTCTGAGACTCGACTGTTCAGCCTCTGGAACCA TCATCGGCACCTATACCATGGCCTGGTACCGCCAGGCTCCAG GGAAGCAGCGCGAGATGGTCGCAATTATTACGCCTGGTGGT GGCTCACACCATGCAGACTCCGTGAAGGGCCGATTCACCAT CTCCAGAGACAACGGCAAGTCTACGGTGTATCTCCAAATGA GCAGCCTGAAATTCGAGGACACGGCCGTCTATTACTGTAAT GCCAAGGCCCGTTGGGGCACTACTGAGTACTGGGGCCAGG GGACCCAGGTCACCGTCTCCTCA |
| 3 | CDR1 | TYTMA |
| 4 | CDR2 | IITPGGGSHHADSVKG |
| 5 | CDR3 | KARWGTTEY |
| | | **V-n5B10 sequence optimization** |
| | | Humanization, with same CDRs 1-3 as V-n5B10 |
| 31 | zn5B10.m20 (also referred to as hu5B10) | EVQLVESGGGLVQPGGSLRLSCSASGTIIGTYTMAWYRQAPG KQREMVAIITPGGGSHHADSVKGRFTISRDNSKNTLYLQMSSL RAEDTAVYYCNAKARWGTTEYWGQGTLVTVSS |
| 94 | zn5B10.m16 | EVQLVESGGGLVQPGGSLRLSCAASGTIIGTYTMAWYRQAPG KQREMVAIITPGGGSHHADSVKGRFTISRDNSKNTLYLQMNSL RAEDTAVYYCNAKARWGTTEYWGQGTMVTVSS |
| 95 | zn5B10.m17 | EVQLVESGGGLVQPGGSLRLSCAASGTIIGTYTMAWYRQAPG KQREMVAIITPGGGSHHADSVKGRFTISRDNSKNTVYLQMNS LRAEDTAVYYCNAKARWGTTEYWGQGTMVTVSS |
| 96 | zn5B10.m18 | EVQLVESGGGLVQPGGSLRLSCSASGTIIGTYTMAWYRQAPG KQREMVAIITPGGGSHHADSVKGRFTISRDNSKSTVYLQMSSL RAEDTAVYYCNAKARWGTTEYWGQGTLVTVSS |
| 97 | zn5B10.m21 | EVQLVESGGGLVQPGGSLRLSCSASGTIIGTYTMAWYRQAPG KQREMVAIITPGGGSHHADSVKGRFTISRDNGKNTLYLQMSSL RAEDTAVYYCNAKARWGTTEYWGQGTLVTVSS |
| 98 | zn5B10.m22 | EVQLVESGGGLVQPGGSLRLSCSASGTIIGTYTMAWYRQAPG KQREMVAIITPGGGSHHADSVKGRFTISRDNSKNTLYLQMSSL RFEDTAVYYCNAKARWGTTEYWGQGTLVTVSS |
| 99 | zn5B10.m23 | EVQLVESGGGLVQPGGSLRLDCSASGTIIGTYTMAWYRQAPG KQREMVAIITPGGGSHHADSVKGRFTISRDNSKSTVYLQMSSL RFEDTAVYYCNAKARWGTTEYWGQGTLVTVSS |
| | | **V-n9B8** |
| 6 | Amino acid sequence of VHH | QVQLVESGGGLVQPGGSLRLSCAASGLTFSYYAMAWFRQAPG KEREFVAAINWNGGNTYNTDSVKGRFTISRDNAKNTVYLQM NSLKPEDTAVYYCAVRYNSASYLVARSSDYNYWGQGTLVTVS S |
| 7 | Nucleotide sequence of VHH | CAGGTGCAGCTGGTAGAGTCTGGGGGAGGCTTGGTGCAAC CTGGGGGCTCTCTGAGACTCTCCTGTGCAGCCTCTGGACTC ACCTTCAGTTACTATGCCATGGCCTGGTTCCGCCAGGCTCCA GGGAAGGAGCGTGAGTTTGTAGCAGCTATTAACTGGAATGG TGGTAACACATACAATACAGACTCCGTGAAGGGCCGATTCA CCATCTCCAGAGACAACGCCAAGAATACGGTGTATCTGCAA ATGAACAGCCTGAAACCTGAGGACACGGCCGTTTATTACTG TGCAGTTAGATACAATAGTGCTAGTTACTTAGTCGCGAGATC GTCAGATTATAACTACTGGGGCCAGGGGACCCTGGTCACCG TCTCCTCA |
| 8 | CDR1 | YYAMA |
| 9 | CDR2 | AINWNGGNTYNTDSVKG |
| 10 | CDR3 | RYNSASYLVARSSDYNY |
| | | **V-n9B8 sequence optimization** |
| | | Humanization, with same CDRs 1-3 as V-n9B8 |
| 100 | zn9B8.m2 | EVQLLESGGGLVQPGGSLRLSCAASGLTFSYYAMAWFRQAPG KEREFVAAINWNGGNTYNTDSVKGRFTISRDNSKNTVYLQM NSLTPEDTAVYYCAVRYNSASYLVARSSDYNYWGQGTLVTVS S |
| 101 | zn9B8.m4 | EVQLLESGGGLVQPGGSLRLSCAASGLTFSYYAMAWFRQAPG KEREFVAAINWNGGNTYNTDSVKGRFTISRDNSKNTVYLQM NSLRSEDTAVYYCAVRYNSASYLVARSSDYNYWGQGTLVTVS S |
| | | Humanization + PTM removal, with same CDR1 as V-n9B8 |
| | | pzn9B8.m2.m11 (also referred to as hu9B8) |
| 32 | hu9B8_VHH | EVQLLESGGGLVQPGGSLRLSCAASGLTFSYYAMAWFRQAPG KEREFVAAINWNQGNTYNTDSVKGRFTISRDNSKNTVYLQM NSLTPEDTAVYYCAVRYNQASYLVARSSDYNYWGQGTLVTVS S |
| 33 | Nucleotide sequence of hu9B8 VHH | GAAGTGCAGCTGCTCGAGTCCGGGGGCGGGCTGGTGCAGC CCGGCGGCAGCCTCAGACTGAGCTGCGCCGCTAGCGGCCT GACCTTCAGCTACTACGCCATGGCCTGGTTCAGACAAGCCC CCGGCAAGGAGAGAGAGTTCGTGGCCGCCATCAACTGGAA CCAAGGCAACACCTACAACACCGACAGCGTGAAGGGCAGA TTCACCATCAGCAGAGACAACAGCAAGAACACCGTGTACC TGCAGATGAACAGCCTGACCCCCGAGGACACCGCCGTGTA CTACTGCGCCGTGAGATACAACCAAGCTAGCTACCTGGTGG CTAGAAGCAGCGACTACAACTACTGGGGCCAAGGCACCCT GGTGACCGTGAGCAGC |
| 8 | hu9B8_CDR1 | YYAMA |
| 34 | hu9B8_CDR2 | AINWNQGNTYNTDSVKG |
| 35 | hu9B8_CDR3 | RYNQASYLVARSSDYNY |
| | | pzn9B8.m2.m12 |
| 102 | pzn9B8.m2.m12_VHH | EVQLLESGGGLVQPGGSLRLSCAASGLTFSYYAMAWFRQAPG KEREFVAAINWNAGNTYNTDSVKGRFTISRDNSKNTVYLQM NSLTPEDTAVYYCAVRYNAASYLVARSSDYNYWGQGTLVTVS S |
| 8 | pzn9B8.m2.m12_ CDR1 | YYAMA |
| 103 | pzn9B8.m2.m12_ CDR2 | AINWNAGNTYNTDSVKG |
| 104 | pzn9B8.m2.m12_ CDR3 | RYNAASYLVARSSDYNY |
| | | **V-n10A1** |
| 11 | Amino acid sequence of VHH | QVQLVESGGGLVQAGGSLRLSCAASGRTGSLHAMGWFRQAP GKERERVATLNLSDGSAVTADSVKGRFTISTDNTKNSVYLQM NSLKPEDTAVYYCTASRSPSGIRYYKPDYWGQGTQVTVSS |
| 12 | Nucleotide sequence of VHH | CAGGTGCAGCTGGTAGAATCTGGGGGAGGATTGGTGCAGG CTGGGGGCTCTCTGAGACTCTCCTGTGCAGCCTCTGGACGC ACCGGCAGTCTACATGCCATGGGCTGGTTCCGCCAGGCTCC AGGGAAGGAGCGTGAAAGAGTAGCAACGCTTAACCTAAGT GATGGTAGCGCAGTTACTGCAGATTCCGTGAAGGGCCGATT CACCATCTCCACGGACAACACCAAGAACTCGGTGTATCTGC AAATGAACAGCCTGAAACCTGAGGACACGGCCGTTTATTAC TGTACAGCGTCTCGTTCCCCCTCGGGTATTAGATACTATAAG CCTGACTACTGGGGCCAGGGGACCCAGGTCACCGTTTCCTC G |
| 13 | CDR1 | LHAMG |
| 14 | CDR2 | TLNLSDGSAVTADSVKG |
| 15 | CDR3 | SRSPSGIRYYKPDY |
| | | **V-n10A1 sequence optimization** |
| | | Humanization, with same CDRs 1-3 as V-n10A1 |
| 105 | zn10A1.m5 | QVQLVQSGPEVKKPGTSVKVSCKASGRTGSLHAMGWFRQAR GQERERVATLNLSDGSAVTADSVKGRFTITTDNSKNSVYMELS SLRSEDTAVYYCTASRSPSGIRYYKPDYWGQGTMVTVSS |
| 106 | zn10A1.m6 | QVQLVESGGGLVQPGGSLRLSCAASGRTGSLHAMGWFRQAP GKERERVATLNLSDGSAVTADSVKGRFTISTDNSKNSVYLQMN SLRAEDTAVYYCTASRSPSGIRYYKPDYWGQGTLVTVSS |
| | | PTM removal |
| 107 | pn10A1.m1 | QVQLVESGGGLVQAGGSLRLSCAASGRTGSLHAMGWFRQAP GKERERVATLQLSDGSAVTADSVKGRFTISTDNTKNSVYLQM |
| | | NSLKPEDTAVYYCTASRSPSGIRYYKPDYWGQGTQVTVSS |
| 108 | pn10A1.m2 | QVQLVESGGGLVQAGGSLRLSCAASGRTGSLHAMGWFRQAP GKERERVATLNLADGSAVTADSVKGRFTISTDNTKNSVYLQM NSLKPEDTAVYYCTASRSPSGIRYYKPDYWGQGTQVTVSS |
| 109 | pn10A1.m3 | QVQLVESGGGLVQAGGSLRLSCAASGRTGSLHAMGWFRQAP GKERERVATLQLTDGSAVTADSVKGRFTISTDNTKNSVYLQMN SLKPEDTAVYYCTASRSPSGIRYYKPDYWGQGTQVTVSS |
| 110 | pn10A1.m4 | QVQLVESGGGLVQAGGSLRLSCAASGRTGSLHAMGWFRQAP GKERERVATLNLSEGSAVTADSVKGRFTISTDNTKNSVYLQMN SLKPEDTAVYYCTASRSPSGIRYYKPDYWGQGTQVTVSS |
| 111 | pn10A1.m5 | QVQLVESGGGLVQAGGSLRLSCAASGRTGSLHAMGWFRQAP GKERERVATLNLSDVSAVTADSVKGRFTISTDNTKNSVYLQM NSLKPEDTAVYYCTASRSPSGIRYYKPDYWGQGTQVTVSS |
| 112 | pn10A1.m6 | QVQLVESGGGLVQAGGSLRLSCAASGRTGSLHAMGWFRQAP GKERERVATLNLSDASAVTADSVKGRFTISTDNTKNSVYLQM NSLKPEDTAVYYCTASRSPSGIRYYKPDYWGQGTQVTVSS |
| | | Humanization + PTM removal |
| 113 | pzn10A1_m6m5 | QVQLVESGGGLVQPGGSLRLSCAASGRTGSLHAMGWFRQAP GKERERVATLNLSDVSAVTADSVKGRFTISTDNSKNSVYLQMN SLRAEDTAVYYCTASRSPSGIRYYKPDYWGQGTLVTVSS |
| 114 | ppzn10A1_m6m5.m1 | QVQLVESGGGLVQPGGSLRLSCAASGRTGSLHAMGWFRQAP GKERERVATLRLSDVSAVTADSVKGRFTISTDNSKNSVYLQMN SLRAEDTAVYYCTASRSPSGIRYYKPDYWGQGTLVTVSS |
| 84 | ppzn10A1_m6m5.m3 (also referred to as hu10A1^{b}) | QVQLVESGGGLVQPGGSLRLSCAASGRTGSLHAMGWFRQAP GKERERVATLQLSDVSAVTADSVKGRFTISTDNSKNSVYLQMN SLRAEDTAVYYCTASRSPSGIRYYKPDYWGQGTLVTVSS |
| 36 | dppzn10A1m6m5. m3_1.m2 (also referred to as hu10A1) | QVQLVESGGGLVQPGGSLRLSCAASGRTGSLHAMGWFRQAP GKERERVATLRLSDVSAVTADSVKGRFTISTDNSKNTLYLQMN SLRAEDTAVYYCTASRSPSGIRYYKPDYWGQGTLVTVSS |
| 37 | Nucleotide sequence of hu10A1 | CAGGTGCAGCTCGTGGAATCAGGAGGTGGACTGGTGCAGC CAGGTGGATCTCTGAGGCTGTCTTGTGCAGCTAGCGGAAGA ACAGGCTCTCTGCACGCCATGGGTTGGTTCAGACAGGCTCC AGGCAAGGAGAGAGAAAGAGTGGCCACACTGCgGCTGAGC GACGTGTCAGCAGTGACAGCAGACAGCGTGAAGGGTAGAT TCACCATCAGTACCGACAACAGCAAGAACACCCTGTACCTG CAGATGAACAGCCTGAGAGCTGAGGATACCGCAGTGTACTA TTGCACCGCTTCTCGAAGCCCTAGCGGCATTCGGTACTACA AGCCTGACTATTGGGGTCAAGGCACACTGGTCACAGTGTCA TCT |
| | | CDR2 of the PTM-removal antibody, the rest CDRs being the same as those of V-n10A1 |
| 115 | pn10A1.m1 | TLQLSDGSAVTADSVKG |
| 116 | pn10A1.m2 | TLNLADGSAVTADSVKG |
| 117 | pn10A1.m3 | TLQLTDGSAVTADSVKG |
| 118 | pn10A1.m4 | TLNLSEGSAVTADSVKG |
| 119 | pn10A1.m5 | TLNLSDVSAVTADSVKG |
| 120 | pn10A1.m6 | TLNLSDASAVTADSVKG |
| 38 | CDR2 of hu10A1 m6m5.m1 | TLRLSDVSAVTADSVKG |
| 85 | CDR2 of hu10A1^{b} m6m5.m3 | TLQLSDVSAVTADSVKG |

| **Anti-c-Met antibodies; CDRs defined according to Kabat numbering scheme, bold and underlined** | | |
|---|---|---|
| | | **V-n7A12** |
| 16 | Amino acid sequence of VHH | EVQLVESGGGLVQAGGSLRLSCVASGNIFSSNHMGWFRQAPG KQREFIAYITTREITNYADSVKGRFTISRDNTKNTVYLQMNVL KPEDTAVYYCIADQSREIRRSWGQGTQVTVSS |
| 17 | Nucleotide sequence of VHH | GAGGTGCAGCTGGTGGAGTCTGGGGGAGGCTTGGTGCAGG CTGGGGGGTCTCTGAGACTCTCCTGTGTAGCCTCTGGAAAC ATCTTCAGTAGCAATCACATGGGCTGGTTCCGCCAGGCTCC AGGGAAGCAGCGCGAGTTCATCGCATATATTACTACTCGTGA AATCACAAACTATGCAGACTCCGTGAAGGGCCGATTCACCA TCTCCAGAGACAACACCAAGAACACGGTGTATCTGCAAATG AACGTACTGAAACCTGAGGACACAGCCGTCTATTACTGTAT AGCAGATCAATCTAGAGAAATCCGCCGTTCCTGGGGCCAGG GGACCCAGGTCACCGTCTCCTCG |
| 18 | CDR1 | SNHMG |
| 19 | CDR2 | YITTREITNYADSVKG |
| 20 | CDR3 | DQSREIRRS |
| | | **V-n7A12 sequence optimization** |
| | | Humanization, with same CDRs 1-3 as V-n7A12 |
| 121 | 7A12-M1 | EVQLVESGGGLVQPGGSLRLSCAASGNIFSSNHMGWVRQAPG KGLEWVAYITTREITNYADSVKGRFTISRDNSKNTVYLQMNSL RAEDTAVYYCIADQSREIRRSWGQGTLVTVSS |
| 122 | 7A12-M2 | EVQLVESGGGLIQPGGSLRLSCKASGNIFSSNHMGWFRQAPG KQLEWVAYITTREITNYADSVKGRFTISRDNSKNTVYLQMNNL RSEDTAVYYCIADQSREIRRSWGQGTLVTVSS |
| 123 | 7A12-M3 | EVQLVESGGGLIQPGGSLRLSCTASGNIFSSNHMGWFRQAPGK QLEWIAYITTREITNYADSVKGRFTISRDNSKNTVYLQMNNLR SEDTAVYYCIADQSREIRRSWGQGTQVTVSS |
| 124 | 7A12-M4 | EVQLVESGGGLIQAGGSLRLSCTASGNIFSSNHMGWFRQAPG KQREFIAYITTREITNYADSVKGRFTISRDNSKNTVYLQMNNL RSEDTAVYYCIADQSREIRRSWGQGTLVTVSS |
| | | PTM removal |
| 125 | pn7A12.N32X.m1 | EVQLVESGGGLVQAGGSLRLSCVASGNIFSSYHMGWFRQAPG KQREFIAYITTREITNYADSVKGRFTISRDNTKNTVYLQMNVL KPEDTAVYYCIADQSREIRRSWGQGTQVTVSS |
| 126 | pn7A12.N32X.m3 | EVQLVESGGGLVQAGGSLRLSCVASGNIFSSHHMGWFRQAPG KQREFIAYITTREITNYADSVKGRFTISRDNTKNTVYLQMNVL KPEDTAVYYCIADQSREIRRSWGQGTQVTVSS |
| 127 | pn7A12.N32X.m4 | EVQLVESGGGLVQAGGSLRLSCVASGNIFSSFHMGWFRQAPG KQREFIAYITTREITNYADSVKGRFTISRDNTKNTVYLQMNVL KPEDTAVYYCIADQSREIRRSWGQGTQVTVSS |
| 128 | pn7A12.N32X.m6 | EVQLVESGGGLVQAGGSLRLSCVASGNIFSSDHMGWFRQAPG KQREFIAYITTREITNYADSVKGRFTISRDNTKNTVYLQMNVL KPEDTAVYYCIADQSREIRRSWGQGTQVTVSS |
| | | Humanization + PTM removal |
| 40 | pzn7A12m4.m1 (also referred to as hu7A12^{b}) | EVQLVESGGGLIQAGGSLRLSCTASGNIFSSYHMGWFRQAPG KQREFIAYITTREITNYADSVKGRFTISRDNSKNTVYLQMNNL RSEDTAVYYCIADQSREIRRSWGQGTLVTVSS |
| | | Humanization + PTM removal + primary immunogenicity removal |
| 129 | dpzn7A12m4.m1_1 | EVQLVESGGGLIQAGGSLRLSCTASGNIFSSYHMGWFRQAPG KQREFIAYITTREITNYADSVKGRFTISRDNSKNTLYLQMNSLR AEDTAVYYCIADQSREIRRSWGQGTLVTVSS |
| 130 | dpzn7A12m4.m1_3 | EVQLVESGGGLIQAGGSLRLSCTASGNIFSSYHMGWFRQAPG KQREFIAYITTREITNYADSVKGRFTISRDNAKNTVYLQMNSL RPEDTAVYYCIADQSREIRRSWGQGTLVTVSS |
| | | Humanization + PTM removal + secondary immunogenicity removal |
| 39 | dpzn7A12m4.ml_1.m1 (also referred to as hu7A12) | EVQLVESGGGLVQPGGSLRLSCTASGNIFSSYHMGWFRQAPG KQREFIAYITTREITNYADSVKGRFTISRDNSKNTLYLQMNSLR AEDTAVYYCIADQSREIRRSWGQGTLVTVSS |
| | | CDR1 of the PTM-removal antibody, the rest CDRs being the same as those of V-n7A12 |
| 41 | pn7A12.N32X.m1 (CDR1 of hu7A12 and hu7A12^{b}) | SYHMG |
| 131 | pn7A12.N32X.m3 | SHHMG |
| 132 | pn7A12.N32X.m4 | SFHMG |
| 133 | pn7A12.N32X.m6 | SDHMG |
| | | **V-n9A2** |
| 21 | Amino acid sequence of VHH | QSQLAESGGGLVQSGGSLGLSCVASGFTISRSIMSWYRQPPGK ERDWVASIGSAGNTNYVDSVKGRFTISRDVAKNTVYLQMNSL KPEDTAVYYCKTTLAVSFSEYWGQGTQVTVSS |
| 22 | Nucleotide sequence of VHH | CAAAGCCAGCTGGCCGAAAGCGGCGGCGGACTGGTGCAGA GCGGAGGATCTCTGGGCCTGTCTTGTGTGGCTTCTGGCTTTA CAATCAGCCGGTCCATCATGAGCTGGTATAGACAGCCTCCT GGCAAGGAACGGGACTGGGTCGCCAGCATCGGCAGCGCCG GCAATACAAACTACGTGGACAGCGTGAAAGGCAGATTCAC CATCTCCAGAGATGTGGCCAAGAACACCGTGTACCTGCAGA TGAACAGCCTGAAGCCCGAGGACACCGCCGTGTACTACTG CAAGACCACCCTGGCTGTGTCCTTCAGCGAGTACTGGGGCC AGGGCACACAGGTGACCGTTTCTAGC |
| 23 | CDR1 | RSIMS |
| 24 | CDR2 | SIGSAGNTNYVDSVKG |
| 25 | CDR3 | TLAVSFSEY |
| | | **V-n9A2 sequence optimization** |
| | | Humanization, with same CDRs 1-3 as V-n9A2 |
| 42 | VHH10 (also referred to as hu9A2) | QSQLVESGGGLVKPGGSLRLSCVASGFTISRSIMSWYRQAPGK ERDWVASIGSAGNTNYVDSVKGRFTISRDNAKNSLYLQMNSL RAEDTAVYYCKTTLAVSFSEYWGQGTLVTVSS |
| 134 | VHH8 | ESQLLESGGGLVQPGGSLRLSCVASGFTISRSIMSWYRQAPGK ERDWVASIGSAGNTNYVDSVKGRFTISRDNSKNTVYLQMNSL RAEDTAVYYCKTTLAVSFSEYWGQGTLVTVSS |
| 135 | VHH9 | ESQLLESGGGLVQPGGSLRLSCVASGFTISRSIMSWYRQAPGK ERDWVASIGSAGNTNYVDSVKGRFTISRDVSKNTLYLQMNSL RAEDTAVYYCKTTLAVSFSEYWGQGTLVTVSS |
| 136 | VHH11 | QSQLVESGGGLVKPGGSLRLSCVASGFTISRSIMSWYRQPPGK ERDWVASIGSAGNTNYVDSVKGRFTISRDNAKNSLYLQMNSL RAEDTAVYYCKTTLAVSFSEYWGQGTLVTVSS |
| | | **V-n9A10** |
| 26 | Amino acid sequence of VHH | QVQLVESGGGLVQPGGSLRLSCEASGFTFSSYVMSWYRQAPG KERELVALISSTTGSSTKYGDSVKGRFTISRDNAKNTVYLQMN SLKPEDTAVYYCNAMIGWNNHWGQGTHVTVSS |
| 27 | Nucleotide sequence of VHH | CAAGTGCAGCTGGTGGAAAGCGGCGGCGGACTGGTTCAGC CTGGAGGATCTCTCAGACTGAGCTGCGAGGCCAGCGGCTTT ACCTTCAGCTCCTACGTGATGAGCTGGTATAGACAGGCCCC TGGCAAAGAGCGGGAACTGGTCGCCCTGATCTCTAGCACCA CCGGCAGCTCTACAAAGTACGGCGATAGCGTGAAGGGCAG ATTCACAATCAGCCGGGACAACGCCAAGAACACCGTGTACC TGCAGATGAACAGCCTGAAGCCCGAGGACACCGCCGTGTA CTACTGTAATGCTATGATCGGCTGGAACAACCACTGGGGCC AGGGCACCCACGTGACAGTGTCCTCC |
| 28 | CDR1 | SYVMS |
| 29 | CDR2 | LISSTTGSSTKYGDSVKG |
| 30 | CDR3 | MIGWNNH |

| **Antibody component** | | |
|---|---|---|
| | | **Linker peptide** |
| 43 | Peptide linker 1 | GGGGS |
| 44 | Peptide linker 2 | GGGGSGGGGS |
| | | **Anti-HSA** |
| 45 | Anti-HSA-Alb8 | |
| 46 | Anti-HSA-Alb8 CDR1 (Kabat numbering scheme) | SFGMS |
| 47 | Anti-HSA-Alb8 CDR2 (Kabat numbering scheme) | SISGSGSDTLYADSVKG |
| 48 | Anti-HSA-Alb8 CDR3 (Kabat numbering scheme) | GGSLSR |
| | | **Hinge sequence** |
| 49 | Hinge sequence 1 | EPKSS |
| 50 | Hinge sequence 2 | EPKSC |
| | | Immunoglobulin Fc region sequence |
| 137 | Hole chain | EPKSSDKTHTCPPCPAPEAAGGPSVFLFPPKPKDTLMISRTPEV TCVVVDVSHEDPEVKFNWYVDGVEVHNAKTKPREEQYNSTY RVVSVLTVLHQDWLNGKEYKCKVSNKALPAPIEKTISKAKGQ PREPQVYTLPPSRDELTKNQVSLSCAVKGFYPSDIAVEWESNG QPENNYKTTPPVLDSDGSFFLVSKLTVDKSRWQQGNVFSCSV MHEALHNHYTQKSLSLSPGK |
| 138 | Knob chain | EPKSSDKTHTCPPCPAPEAAGGPSVFLFPPKPKDTLMISRTPEV TCVVVDVSHEDPEVKFNWYVDGVEVHNAKTKPREEQYNSTY RVVSVLTVLHQDWLNGKEYKCKVSNKALPAPIEKTISKAKGQ PREPQVYTLPPSRDELTKNQVSLWCLVKGFYPSDIAVEWESNG QPENNYKTTPPVLDSDGSFFLYSKLTVDKSRWQQGNVFSCSV MHEALHNHYTQKSLSLSPGK |

| **Trispecific anti-EGFR/cMet antibody** | | |
|---|---|---|
| **Single-chain multispecific** | | |
| 51 | Assembly format of each ISVD in | AVQLVDSGGGLVQPGGSLRLDCSASGTIIGTYTMAWYRQAPG KQREMVAIITPGGGSHHADSVKGRFTISRDNGKSTVYLQMSSL |
| | antibody V-1: 5B10-9A2-7A12 | KFEDTAVYYCNAKARWGTTEYWGQGTQVTVSSGGGGSQSQL AESGGGLVQSGGSLGLSCVASGFTISRSIMSWYRQPPGKERDW VASIGSAGNTNYVDSVKGRFTISRDVAKNTVYLQMNSLKPED TAVYYCKTTLAVSFSEYWGQGTQVTVSSGGGGSGGGGSEVQL VESGGGLVQAGGSLRLSCVASGNIFSSNHMGWFRQAPGKQRE FIAYITTREITNYADSVKGRFTISRDNTKNTVYLQMNVLKPEDT AVYYCIADQSREIRRSWGQGTQVTVSS |
| 52 | Assembly format of each ISVD in antibody V-3: 5B10-7A12-9A2 | AVQLVDSGGGLVQPGGSLRLDCSASGTIIGTYTMAWYRQAPG KQREMVAIITPGGGSHHADSVKGRFTISRDNGKSTVYLQMSSL KFEDTAVYYCNAKARWGTTEYWGQGTQVTVSSGGGGSEVQ LVESGGGLVQAGGSLRLSCVASGNIFSSNHMGWFRQAPGKQR EFIAYITTREITNYADSVKGRFTISRDNTKNTVYLQMNVLKPED TAVYYCIADQSREIRRSWGQGTQVTVSSGGGGSGGGGSQSQL AESGGGLVQSGGSLGLSCVASGFTISRSIMSWYRQPPGKERDW VASIGSAGNTNYVDSVKGRFTISRDVAKNTVYLQMNSLKPED TAVYYCKTTLAVSFSEYWGQGTQVTVSS |
| 53 | Assembly format of each ISVD in antibody V-7: 9B8-9A2-7A12 | QVQLVESGGGLVQPGGSLRLSCAASGLTFSYYAMAWFRQAPG KEREFVAAINWNGGNTYNTDSVKGRFTISRDNAKNTVYLQM NSLKPEDTAVYYCAVRYNSASYLVARSSDYNYWGQGTLVTVS SGGGGSQSQLAESGGGLVQSGGSLGLSCVASGFTISRSIMSWY RQPPGKERDWVASIGSAGNTNYVDSVKGRFTISRDVAKNTVY LQMNSLKPEDTAVYYCKTTLAVSFSEYWGQGTQVTVSSGGG GSGGGGSEVQLVESGGGLVQAGGSLRLSCVASGNIFSSNHMG WFRQAPGKQREFIAYITTREITNYADSVKGRFTISRDNTKNTV YLQMNVLKPEDTAVYYCIADQSREIRRSWGQGTQVTVSS |
| 54 | Assembly format of each ISVD in antibody V-9: 9B8-7A12-9A2 | QVQLVESGGGLVQPGGSLRLSCAASGLTFSYYAMAWFRQAPG KEREFVAAINWNGGNTYNTDSVKGRFTISRDNAKNTVYLQM NSLKPEDTAVYYCAVRYNSASYLVARSSDYNYWGQGTLVTVS SGGGGSEVQLVESGGGLVQAGGSLRLSCVASGNIFSSNHMGW FRQAPGKQREFIAYITTREITNYADSVKGRFTISRDNTKNTVYL QMNVLKPEDTAVYYCIADQSREIRRSWGQGTQVTVSSGGGGS GGGGSQSQLAESGGGLVQSGGSLGLSCVASGFTISRSIMSWYR QPPGKERDWVASIGSAGNTNYVDSVKGRFTISRDVAKNTVYL QMNSLKPEDTAVYYCKTTLAVSFSEYWGQGTQVTVSS |
| 55 | Assembly format of each ISVD in antibody V-13: 10A1-9A2-7A12 | QVQLVESGGGLVQAGGSLRLSCAASGRTGSLHAMGWFRQAP GKERERVATLNLSDGSAVTADSVKGRFTISTDNTKNSVYLQM NSLKPEDTAVYYCTASRSPSGIRYYKPDYWGQGTQVTVSSGG GGSQSQLAESGGGLVQSGGSLGLSCVASGFTISRSIMSWYRQP PGKERDWVASIGSAGNTNYVDSVKGRFTISRDVAKNTVYLQM NSLKPEDTAVYYCKTTLAVSFSEYWGQGTQVTVSSGGGGSGG GGSEVQLVESGGGLVQAGGSLRLSCVASGNIFSSNHMGWFRQ APGKQREFIAYITTREITNYADSVKGRFTISRDNTKNTVYLQM NVLKPEDTAVYYCIADQSREIRRSWGQGTQVTVSS |
| 56 | Assembly format of each ISVD in antibody V-15: 10A1-7A12-9A2 | QVQLVESGGGLVQAGGSLRLSCAASGRTGSLHAMGWFRQAP GKERERVATLNLSDGSAVTADSVKGRFTISTDNTKNSVYLQM NSLKPEDTAVYYCTASRSPSGIRYYKPDYWGQGTQVTVSSGG GGSEVQLVESGGGLVQAGGSLRLSCVASGNIFSSNHMGWFRQ APGKQREFIAYITTREITNYADSVKGRFTISRDNTKNTVYLQM NVLKPEDTAVYYCIADQSREIRRSWGQGTQVTVSSGGGGSGG GGSQSQLAESGGGLVQSGGSLGLSCVASGFTISRSIMSWYRQP PGKERDWVASIGSAGNTNYVDSVKGRFTISRDVAKNTVYLQM NSLKPEDTAVYYCKTTLAVSFSEYWGQGTQVTVSS |
| 57 | Assembly format of each ISVD in antibody V-16: hu10A1-hu7A12-9A2 | QVQLVESGGGLVQPGGSLRLSCAASGRTGSLHAMGWFRQAP GKERERVATLRLSDVSAVTADSVKGRFTISTDNSKNTLYLQMN SLRAEDTAVYYCTASRSPSGIRYYKPDYWGQGTLVTVSSGGG GSEVQLVESGGGLVQPGGSLRLSCTASGNIFSSYHMGWFRQA PGKQREFIAYITTREITNYADSVKGRFTISRDNSKNTLYLQMNS LRAEDTAVYYCIADQSREIRRSWGQGTLVTVSSGGGGSGGGG SQSQLAESGGGLVQSGGSLGLSCVASGFTISRSIMSWYRQPPG KERDWVASIGSAGNTNYVDSVKGRFTISRDVAKNTVYLQMNS LKPEDTAVYYCKTTLAVSFSEYWGQGTQVTVSS |
| 58 | Assembly format of each ISVD in antibody V-17: hu10A1-hu7A12-hu9A2 | QVQLVESGGGLVQPGGSLRLSCAASGRTGSLHAMGWFRQAP GKERERVATLRLSDVSAVTADSVKGRFTISTDNSKNTLYLQMN SLRAEDTAVYYCTASRSPSGIRYYKPDYWGQGTLVTVSSGGG GSEVQLVESGGGLVQPGGSLRLSCTASGNIFSSYHMGWFRQA PGKQREFIAYITTREITNYADSVKGRFTISRDNSKNTLYLQMNS LRAEDTAVYYCIADQSREIRRSWGQGTLVTVSSGGGGSGGGG SQSQLVESGGGLVKPGGSLRLSCVASGFTISRSIMSWYRQAPG KERDWVASIGSAGNTNYVDSVKGRFTISRDNAKNSLYLQMNS LRAEDTAVYYCKTTLAVSFSEYWGQGTLVTVSS |
| 59 | Assembly format of each ISVD in antibody V-18: hu10A1-hu7A12-hu7A12 | QVQLVESGGGLVQPGGSLRLSCAASGRTGSLHAMGWFRQAP GKERERVATLRLSDVSAVTADSVKGRFTISTDNSKNTLYLQMN SLRAEDTAVYYCTASRSPSGIRYYKPDYWGQGTLVTVSSGGG GSEVQLVESGGGLVQPGGSLRLSCTASGNIFSSYHMGWFRQA PGKQREFIAYITTREITNYADSVKGRFTISRDNSKNTLYLQMNS LRAEDTAVYYCIADQSREIRRSWGQGTLVTVSSGGGGSGGGG SEVQLVESGGGLVQPGGSLRLSCTASGNIFSSYHMGWFRQAP GKQREFIAYITTREITNYADSVKGRFTISRDNSKNTLYLQMNSL RAEDTAVYYCIADQSREIRRSWGQGTLVTVSS |
| 60 | Assembly format of each ISVD in antibody V-19: hu10A1-hu9A2-hu9A2 | QVQLVESGGGLVQPGGSLRLSCAASGRTGSLHAMGWFRQAP GKERERVATLRLSDVSAVTADSVKGRFTISTDNSKNTLYLQMN SLRAEDTAVYYCTASRSPSGIRYYKPDYWGQGTLVTVSSGGG GSQSQLVESGGGLVKPGGSLRLSCVASGFTISRSIMSWYRQAP GKERDWVASIGSAGNTNYVDSVKGRFTISRDNAKNSLYLQMN SLRAEDTAVYYCKTTLAVSFSEYWGQGTLVTVSSGGGGSGGG GSQSQLVESGGGLVKPGGSLRLSCVASGFTISRSIMSWYRQAP GKERDWVASIGSAGNTNYVDSVKGRFTISRDNAKNSLYLQMN SLRAEDTAVYYCKTTLAVSFSEYWGQGTLVTVSS |
| 61 | Assembly format of each ISVD in antibody V-20: hu5B10-hu7A12-hu9A2 | EVQLVESGGGLVQPGGSLRLSCSASGTIIGTYTMAWYRQAPG KQREMVAIITPGGGSHHADSVKGRFTISRDNSKNTLYLQMSSL RAEDTAVYYCNAKARWGTTEYWGQGTLVTVSSGGGGSEVQL VESGGGLVQPGGSLRLSCTASGNIFSSYHMGWFRQAPGKQRE FIAYITTREITNYADSVKGRFTISRDNSKNTLYLQMNSLRAEDT AVYYCIADQSREIRRSWGQGTLVTVSSGGGGSGGGGSQSQLV ESGGGLVKPGGSLRLSCVASGFTISRSIMSWYRQAPGKERDW VASIGSAGNTNYVDSVKGRFTISRDNAKNSLYLQMNSLRAED TAVYYCKTTLAVSFSEYWGQGTLVTVSS |
| 62 | Assembly format of each ISVD in antibody V-21: hu5B10-hu7A12-hu7A12 | EVQLVESGGGLVQPGGSLRLSCSASGTIIGTYTMAWYRQAPG KQREMVAIITPGGGSHHADSVKGRFTISRDNSKNTLYLQMSSL RAEDTAVYYCNAKARWGTTEYWGQGTLVTVSSGGGGSEVQL VESGGGLVQPGGSLRLSCTASGNIFSSYHMGWFRQAPGKQRE FIAYITTREITNYADSVKGRFTISRDNSKNTLYLQMNSLRAEDT AVYYCIADQSREIRRSWGQGTLVTVSSGGGGSGGGGSEVQLV ESGGGLVQPGGSLRLSCTASGNIFSSYHMGWFRQAPGKQREFI AYITTREITNYADSVKGRFTISRDNSKNTLYLQMNSLRAEDTAV YYCIADQSREIRRSWGQGTLVTVSS |
| 63 | Assembly format of each ISVD in antibody V-22: hu5B10-hu9A2-hu9A2 | EVQLVESGGGLVQPGGSLRLSCSASGTIIGTYTMAWYRQAPG KQREMVAIITPGGGSHHADSVKGRFTISRDNSKNTLYLQMSSL RAEDTAVYYCNAKARWGTTEYWGQGTLVTVSSGGGGSQSQL VESGGGLVKPGGSLRLSCVASGFTISRSIMSWYRQAPGKERD WVASIGSAGNTNYVDSVKGRFTISRDNAKNSLYLQMNSLRAE DTAVYYCKTTLAVSFSEYWGQGTLVTVSSGGGGSGGGGSQSQ LVESGGGLVKPGGSLRLSCVASGFTISRSIMSWYRQAPGKERD WVASIGSAGNTNYVDSVKGRFTISRDNAKNSLYLQMNSLRAE DTAVYYCKTTLAVSFSEYWGQGTLVTVSS |
| 64 | Assembly format of each ISVD in antibody V-23: hu9B8-hu7A12-hu9A2 | EVQLLESGGGLVQPGGSLRLSCAASGLTFSYYAMAWFRQAPG KEREFVAAINWNQGNTYNTDSVKGRFTISRDNSKNTVYLQM NSLTPEDTAVYYCAVRYNQASYLVARSSDYNYWGQGTLVTVS SGGGGSEVQLVESGGGLVQPGGSLRLSCTASGNIFSSYHMGW FRQAPGKQREFIAYITTREITNYADSVKGRFTISRDNSKNTLYL QMNSLRAEDTAVYYCIADQSREIRRSWGQGTLVTVSSGGGGS GGGGSQSQLVESGGGLVKPGGSLRLSCVASGFTISRSIMSWYR QAPGKERDWVASIGSAGNTNYVDSVKGRFTISRDNAKNSLYL QMNSLRAEDTAVYYCKTTLAVSFSEYWGQGTLVTVSS |
| 65 | Assembly format of each ISVD in antibody V-24: hu9B8-hu7A12-hu7A12 | EVQLLESGGGLVQPGGSLRLSCAASGLTFSYYAMAWFRQAPG KEREFVAAINWNQGNTYNTDSVKGRFTISRDNSKNTVYLQM NSLTPEDTAVYYCAVRYNQASYLVARSSDYNYWGQGTLVTVS SGGGGSEVQLVESGGGLVQPGGSLRLSCTASGNIFSSYHMGW FRQAPGKQREFIAYITTREITNYADSVKGRFTISRDNSKNTLYL QMNSLRAEDTAVYYCIADQSREIRRSWGQGTLVTVSSGGGGS GGGGSEVQLVESGGGLVQPGGSLRLSCTASGNIFSSYHMGWF RQAPGKQREFIAYITTREITNYADSVKGRFTISRDNSKNTLYLQ MNSLRAEDTAVYYCIADQSREIRRSWGQGTLVTVSS |
| 66 | Assembly format of each ISVD in antibody V-25: hu9B8-hu9A2-hu9A2 | EVQLLESGGGLVQPGGSLRLSCAASGLTFSYYAMAWFRQAPG KEREFVAAINWNQGNTYNTDSVKGRFTISRDNSKNTVYLQM NSLTPEDTAVYYCAVRYNQASYLVARSSDYNYWGQGTLVTVS SGGGGSQSQLVESGGGLVKPGGSLRLSCVASGFTISRSIMSWY RQAPGKERDWVASIGSAGNTNYVDSVKGRFTISRDNAKNSLY LQMNSLRAEDTAVYYCKTTLAVSFSEYWGQGTLVTVSSGGGG SGGGGSQSQLVESGGGLVKPGGSLRLSCVASGFTISRSIMSWY RQAPGKERDWVASIGSAGNTNYVDSVKGRFTISRDNAKNSLY LQMNSLRAEDTAVYYCKTTLAVSFSEYWGQGTLVTVSS |

| Multi-chain multispecific | | |
|---|---|---|
| | | **Anti-EGFR arm** |
| 70 | hu10A1-Fc hole chain | QVQLVESGGGLVQPGGSLRLSCAASGRTGSLHAMGWFRQAP GKERERVATLRLSDVSAVTADSVKGRFTISTDNSKNTLYLQMN SLRAEDTAVYYCTASRSPSGIRYYKPDYWGQGTLVTVSSEPKS SDKTHTCPPCPAPEAAGGPSVFLFPPKPKDTLMISRTPEVTCVV VDVSHEDPEVKFNWYVDGVEVHNAKTKPREEQYNSTYRVVS VLTVLHQDWLNGKEYKCKVSNKALPAPIEKTISKAKGQPREP QVYTLPPSRDELTKNQVSLSCAVKGFYPSDIAVEWESNGQPEN NYKTTPPVLDSDGSFFLVSKLTVDKSRWQQGNVFSCSVMHEA LHNHYTQKSLSLSPGK* |
| 71 | hu5B10-Fc hole chain | EVQLVESGGGLVQPGGSLRLSCSASGTIIGTYTMAWYRQAPG KQREMVAIITPGGGSHHADSVKGRFTISRDNSKNTLYLQMSSL RAEDTAVYYCNAKARWGTTEYWGQGTLVTVSSEPKSSDKTH TCPPCPAPEAAGGPSVFLFPPKPKDTLMISRTPEVTCVVVDVSH EDPEVKFNWYVDGVEVHNAKTKPREEQYNSTYRVVSVLTVL HQDWLNGKEYKCKVSNKALPAPIEKTISKAKGQPREPQVYTL PPSRDELTKNQVSLSCAVKGFYPSDIAVEWESNGQPENNYKTT PPVLDSDGSFFLVSKLTVDKSRWQQGNVFSCSVMHEALHNHY TQKSLSLSPGK* |
| 72 | hu9B8-Fc hole chain | EVQLLESGGGLVQPGGSLRLSCAASGLTFSYYAMAWFRQAPG KEREFVAAINWNQGNTYNTDSVKGRFTISRDNSKNTVYLQM NSLTPEDTAVYYCAVRYNQASYLVARSSDYNYWGQGTLVTVS SEPKSSDKTHTCPPCPAPEAAGGPSVFLFPPKPKDTLMISRTPE VTCVVVDVSHEDPEVKFNWYVDGVEVHNAKTKPREEQYNST YRVVSVLTVLHQDWLNGKEYKCKVSNKALPAPIEKTISKAKG QPREPQVYTLPPSRDELTKNQVSLSCAVKGFYPSDIAVEWESN GQPENNYKTTPPVLDSDGSFFLVSKLTVDKSRWQQGNVFSCS VMHEALHNHYTQKSLSLSPGK* |
| 86 | hu10A1^{b}-Fc hole chain | QVQLVESGGGLVQPGGSLRLSCAASGRTGSLHAMGWFRQAP GKERERVATLQLSDVSAVTADSVKGRFTISTDNSKNSVYLQMN SLRAEDTAVYYCTASRSPSGIRYYKPDYWGQGTLVTVSSEPKS SDKTHTCPPCPAPEAAGGPSVFLFPPKPKDTLMISRTPEVTCVV VDVSHEDPEVKFNWYVDGVEVHNAKTKPREEQYNSTYRVVS VLTVLHQDWLNGKEYKCKVSNKALPAPIEKTISKAKGQPREP QVYTLPPSRDELTKNQVSLSCAVKGFYPSDIAVEWESNGQPEN NYKTTPPVLDSDGSFFLVSKLTVDKSRWQQGNVFSCSVMHEA LHNHYTQKSLSLSPGK* |
| | | Anti-cMet Arm |
| 69 | 9A2-hu7A12-Fc knob chain | QSQLAESGGGLVQSGGSLGLSCVASGFTISRSIMSWYRQPPGK ERDWVASIGSAGNTNYVDSVKGRFTISRDVAKNTVYLQMNSL KPEDTAVYYCKTTLAVSFSEYWGQGTQVTVSSGGGGSGGGGS EVQLVESGGGLVQPGGSLRLSCTASGNIFSSYHMGWFRQAPG KQREFIAYITTREITNYADSVKGRFTISRDNSKNTLYLQMNSLR AEDTAVYYCIADQSREIRRSWGQGTLVTVSSEPKSSDKTHTCP PCPAPEAAGGPSVFLFPPKPKDTLMISRTPEVTCVVVDVSHED PEVKFNWYVDGVEVHNAKTKPREEQYNSTYRVVSVLTVLHQ DWLNGKEYKCKVSNKALPAPIEKTISKAKGQPREPQVYTLPPS RDELTKNQVSLWCLVKGFYPSDIAVEWESNGQPENNYKTTPP VLDSDGSFFLYSKLTVDKSRWQQGNVFSCSVMHEALHNHYT QKSLSLSPGK* |
| 73 | hu9A2-hu7A12-Fc knob chain | QSQLVESGGGLVKPGGSLRLSCVASGFTISRSIMSWYRQAPGK ERDWVASIGSAGNTNYVDSVKGRFTISRDNAKNSLYLQMNSL RAEDTAVYYCKTTLAVSFSEYWGQGTLVTVSSGGGGSGGGGS EVQLVESGGGLVQPGGSLRLSCTASGNIFSSYHMGWFRQAPG KQREFIAYITTREITNYADSVKGRFTISRDNSKNTLYLQMNSLR AEDTAVYYCIADQSREIRRSWGQGTLVTVSSEPKSSDKTHTCP PCPAPEAAGGPSVFLFPPKPKDTLMISRTPEVTCVVVDVSHED PEVKFNWYVDGVEVHNAKTKPREEQYNSTYRVVSVLTVLHQ DWLNGKEYKCKVSNKALPAPIEKTISKAKGQPREPQVYTLPPS RDELTKNQVSLWCLVKGFYPSDIAVEWESNGQPENNYKTTPP VLDSDGSFFLYSKLTVDKSRWQQGNVFSCSVMHEALHNHYT QKSLSLSPGK* |
| 74 | hu9A2-hu9A2-Fc knob chain | QSQLVESGGGLVKPGGSLRLSCVASGFTISRSIMSWYRQAPGK ERDWVASIGSAGNTNYVDSVKGRFTISRDNAKNSLYLQMNSL RAEDTAVYYCKTTLAVSFSEYWGQGTLVTVSSGGGGSGGGGS QSQLVESGGGLVKPGGSLRLSCVASGFTISRSIMSWYRQAPGK ERDWVASIGSAGNTNYVDSVKGRFTISRDNAKNSLYLQMNSL RAEDTAVYYCKTTLAVSFSEYWGQGTLVTVSSEPKSSDKTHT CPPCPAPEAAGGPSVFLFPPKPKDTLMISRTPEVTCVVVDVSHE DPEVKFNWYVDGVEVHNAKTKPREEQYNSTYRVVSVLTVLH QDWLNGKEYKCKVSNKALPAPIEKTISKAKGQPREPQVYTLP PSRDELTKNQVSLWCLVKGFYPSDIAVEWESNGQPENNYKTT PPVLDSDGSFFLYSKLTVDKSRWQQGNVFSCSVMHEALHNHY TQKSLSLSPGK* |
| 75 | hu7A12-hu7A12-Fc knob chain | EVQLVESGGGLVQPGGSLRLSCTASGNIFSSYHMGWFRQAPG KQREFIAYITTREITNYADSVKGRFTISRDNSKNTLYLQMNSLR AEDTAVYYCIADQSREIRRSWGQGTLVTVSSGGGGSGGGGSE VQLVESGGGLVQPGGSLRLSCTASGNIFSSYHMGWFRQAPGK QREFIAYITTREITNYADSVKGRFTISRDNSKNTLYLQMNSLRA EDTAVYYCIADQSREIRRSWGQGTLVTVSSEPKSSDKTHTCPP CPAPEAAGGPSVFLFPPKPKDTLMISRTPEVTCVVVDVSHEDP EVKFNWYVDGVEVHNAKTKPREEQYNSTYRVVSVLTVLHQD WLNGKEYKCKVSNKALPAPIEKTISKAKGQPREPQVYTLPPSR DELTKNQVSLWCLVKGFYPSDIAVEWESNGQPENNYKTTPPV LDSDGSFFLYSKLTVDKSRWQQGNVFSCSVMHEALHNHYTQ KSLSLSPGK* |
| 87 | 9A2-hu7A12^{b}-Fc knob chain | QSQLAESGGGLVQSGGSLGLSCVASGFTISRSIMSWYRQPPGK ERDWVASIGSAGNTNYVDSVKGRFTISRDVAKNTVYLQMNSL KPEDTAVYYCKTTLAVSFSEYWGQGTQVTVSSGGGGSGGGGS EVQLVESGGGLIQAGGSLRLSCTASGNIFSSYHMGWFRQAPG KQREFIAYITTREITNYADSVKGRFTISRDNSKNTVYLQMNNL RSEDTAVYYCIADQSREIRRSWGQGTLVTVSSEPKSSDKTHTC PPCPAPEAAGGPSVFLFPPKPKDTLMISRTPEVTCVVVDVSHE DPEVKFNWYVDGVEVHNAKTKPREEQYNSTYRVVSVLTVLH QDWLNGKEYKCKVSNKALPAPIEKTISKAKGQPREPQVYTLP PSRDELTKNQVSLWCLVKGFYPSDIAVEWESNGQPENNYKTT PPVLDSDGSFFLYSKLTVDKSRWQQGNVFSCSVMHEALHNHY TQKSLSLSPGK* |

| **Single-chain multispecific antibody format, with anti-HSA VHH at C-terminus** | | |
|---|---|---|
| 67 | hu10A1-hu7A12-9A2-anti-HSA-Alb8 | QVQLVESGGGLVQPGGSLRLSCAASGRTGSLHAMGWFRQAP GKERERVATLRLSDVSAVTADSVKGRFTISTDNSKNTLYLQMN SLRAEDTAVYYCTASRSPSGIRYYKPDYWGQGTLVTVSSGGG GSEVQLVESGGGLVQPGGSLRLSCTASGNIFSSYHMGWFRQA PGKQREFIAYITTREITNYADSVKGRFTISRDNSKNTLYLQMNS LRAEDTAVYYCIADQSREIRRSWGQGTLVTVSSGGGGSGGGG SQSQLAESGGGLVQSGGSLGLSCVASGFTISRSIMSWYRQPPG KERDWVASIGSAGNTNYVDSVKGRFTISRDVAKNTVYLQMNS LKPEDTAVYYCKTTLAVSFSEYWGQGTQVTVSSGGGGSEVQL VESGGGLVQPGNSLRLSCAASGFTFSSFGMSWVRQAPGKGLE WVSSISGSGSDTLYADSVKGRFTISRDNAKTTLYLQMNSLRPE DTAVYYCTIGGSLSRSSQGTLVTVSS |
| 68 | hu10A1-hu7A12-hu9A2-anti-HSA-Alb8 | QVQLVESGGGLVQPGGSLRLSCAASGRTGSLHAMGWFRQAP GKERERVATLRLSDVSAVTADSVKGRFTISTDNSKNTLYLQMN SLRAEDTAVYYCTASRSPSGIRYYKPDYWGQGTLVTVSSGGG GSEVQLVESGGGLVQPGGSLRLSCTASGNIFSSYHMGWFRQA PGKQREFIAYITTREITNYADSVKGRFTISRDNSKNTLYLQMNS LRAEDTAVYYCIADQSREIRRSWGQGTLVTVSSGGGGSGGGG SQSQLVESGGGLVKPGGSLRLSCVASGFTISRSIMSWYRQAPG KERDWVASIGSAGNTNYVDSVKGRFTISRDNAKNSLYLQMNS LRAEDTAVYYCKTTLAVSFSEYWGQGTLVTVSSGGGGSEVQL VESGGGLVQPGNSLRLSCAASGFTFSSFGMSWVRQAPGKGLE WVSSISGSGSDTLYADSVKGRFTISRDNAKTTLYLQMNSLRPE DTAVYYCTIGGSLSRSSQGTLVTVSS |
| 76 | hu10A1-hu7A12-hu7A12-anti-HSA-Alb8 | QVQLVESGGGLVQPGGSLRLSCAASGRTGSLHAMGWFRQAP GKERERVATLRLSDVSAVTADSVKGRFTISTDNSKNTLYLQMN SLRAEDTAVYYCTASRSPSGIRYYKPDYWGQGTLVTVSSGGG GSEVQLVESGGGLVQPGGSLRLSCTASGNIFSSYHMGWFRQA PGKQREFIAYITTREITNYADSVKGRFTISRDNSKNTLYLQMNS LRAEDTAVYYCIADQSREIRRSWGQGTLVTVSSGGGGSGGGG SEVQLVESGGGLVQPGGSLRLSCTASGNIFSSYHMGWFRQAP GKQREFIAYITTREITNYADSVKGRFTISRDNSKNTLYLQMNSL RAEDTAVYYCIADQSREIRRSWGQGTLVTVSSGGGGSEVQLVE SGGGLVQPGNSLRLSCAASGFTFSSFGMSWVRQAPGKGLEW VSSISGSGSDTLYADSVKGRFTISRDNAKTTLYLQMNSLRPEDT AVYYCTIGGSLSRSSQGTLVTVSS |
| 77 | hu10A1-hu9A2-hu9A2-anti-HSA-Alb8 | QVQLVESGGGLVQPGGSLRLSCAASGRTGSLHAMGWFRQAP GKERERVATLRLSDVSAVTADSVKGRFTISTDNSKNTLYLQMN SLRAEDTAVYYCTASRSPSGIRYYKPDYWGQGTLVTVSSGGG GSQSQLVESGGGLVKPGGSLRLSCVASGFTISRSIMSWYRQAP GKERDWVASIGSAGNTNYVDSVKGRFTISRDNAKNSLYLQMN SLRAEDTAVYYCKTTLAVSFSEYWGQGTLVTVSSGGGGSGGG GSQSQLVESGGGLVKPGGSLRLSCVASGFTISRSIMSWYRQAP GKERDWVASIGSAGNTNYVDSVKGRFTISRDNAKNSLYLQMN SLRAEDTAVYYCKTTLAVSFSEYWGQGTLVTVSSGGGGSEVQ LVESGGGLVQPGNSLRLSCAASGFTFSSFGMSWVRQAPGKGL EWVSSISGSGSDTLYADSVKGRFTISRDNAKTTLYLQMNSLRP EDTAVYYCTIGGSLSRSSQGTLVTVSS |
| 78 | hu5B10-hu7A12-hu9A2-anti-HSA-Alb8 | EVQLVESGGGLVQPGGSLRLSCSASGTIIGTYTMAWYRQAPG KQREMVAIITPGGGSHHADSVKGRFTISRDNSKNTLYLQMSSL RAEDTAVYYCNAKARWGTTEYWGQGTLVTVSSGGGGSEVQL VESGGGLVQPGGSLRLSCTASGNIFSSYHMGWFRQAPGKQRE FIAYITTREITNYADSVKGRFTISRDNSKNTLYLQMNSLRAEDT AVYYCIADQSREIRRSWGQGTLVTVSSGGGGSGGGGSQSQLV ESGGGLVKPGGSLRLSCVASGFTISRSIMSWYRQAPGKERDW VASIGSAGNTNYVDSVKGRFTISRDNAKNSLYLQMNSLRAED TAVYYCKTTLAVSFSEYWGQGTLVTVSSGGGGSEVQLVESGG GLVQPGNSLRLSCAASGFTFSSFGMSWVRQAPGKGLEWVSSI SGSGSDTLYADSVKGRFTISRDNAKTTLYLQMNSLRPEDTAVY YCTIGGSLSRSSQGTLVTVSS |
| 79 | hu5B10-hu7A12-hu7A12-anti-HSA-Alb8 | EVQLVESGGGLVQPGGSLRLSCSASGTIIGTYTMAWYRQAPG KQREMVAIITPGGGSHHADSVKGRFTISRDNSKNTLYLQMSSL RAEDTAVYYCNAKARWGTTEYWGQGTLVTVSSGGGGSEVQL VESGGGLVQPGGSLRLSCTASGNIFSSYHMGWFRQAPGKQRE FIAYITTREITNYADSVKGRFTISRDNSKNTLYLQMNSLRAEDT AVYYCIADQSREIRRSWGQGTLVTVSSGGGGSGGGGSEVQLV ESGGGLVQPGGSLRLSCTASGNIFSSYHMGWFRQAPGKQREFI AYITTREITNYADSVKGRFTISRDNSKNTLYLQMNSLRAEDTAV YYCIADQSREIRRSWGQGTLVTVSSGGGGSEVQLVESGGGLV QPGNSLRLSCAASGFTFSSFGMSWVRQAPGKGLEWVSSISGS GSDTLYADSVKGRFTISRDNAKTTLYLQMNSLRPEDTAVYYCT IGGSLSRSSQGTLVTVSS |
| 80 | hu5B10-hu9A2-hu9A2-anti-HSA-Alb8 | EVQLVESGGGLVQPGGSLRLSCSASGTIIGTYTMAWYRQAPG KQREMVAIITPGGGSHHADSVKGRFTISRDNSKNTLYLQMSSL RAEDTAVYYCNAKARWGTTEYWGQGTLVTVSSGGGGSQSQL VESGGGLVKPGGSLRLSCVASGFTISRSIMSWYRQAPGKERD WVASIGSAGNTNYVDSVKGRFTISRDNAKNSLYLQMNSLRAE DTAVYYCKTTLAVSFSEYWGQGTLVTVSSGGGGSGGGGSQSQ LVESGGGLVKPGGSLRLSCVASGFTISRSIMSWYRQAPGKERD WVASIGSAGNTNYVDSVKGRFTISRDNAKNSLYLQMNSLRAE DTAVYYCKTTLAVSFSEYWGQGTLVTVSSGGGGSEVQLVESG GGLVQPGNSLRLSCAASGFTFSSFGMSWVRQAPGKGLEWVSS ISGSGSDTLYADSVKGRFTISRDNAKTTLYLQMNSLRPEDTAV YYCTIGGSLSRSSQGTLVTVSS |
| 81 | hu9B8-hu7A12-hu9A2-anti-HSA-Alb8 | EVQLLESGGGLVQPGGSLRLSCAASGLTFSYYAMAWFRQAPG KEREFVAAINWNQGNTYNTDSVKGRFTISRDNSKNTVYLQM NSLTPEDTAVYYCAVRYNQASYLVARSSDYNYWGQGTLVTVS SGGGGSEVQLVESGGGLVQPGGSLRLSCTASGNIFSSYHMGW FRQAPGKQREFIAYITTREITNYADSVKGRFTISRDNSKNTLYL QMNSLRAEDTAVYYCIADQSREIRRSWGQGTLVTVSSGGGGS GGGGSQSQLVESGGGLVKPGGSLRLSCVASGFTISRSIMSWYR QAPGKERDWVASIGSAGNTNYVDSVKGRFTISRDNAKNSLYL QMNSLRAEDTAVYYCKTTLAVSFSEYWGQGTLVTVSSGGGGS EVQLVESGGGLVQPGNSLRLSCAASGFTFSSFGMSWVRQAPG KGLEWVSSISGSGSDTLYADSVKGRFTISRDNAKTTLYLQMNS LRPEDTAVYYCTIGGSLSRSSQGTLVTVSS |
| 82 | hu9B8-hu7A12-hu7A12-anti-HSA-Alb8 | EVQLLESGGGLVQPGGSLRLSCAASGLTFSYYAMAWFRQAPG KEREFVAAINWNQGNTYNTDSVKGRFTISRDNSKNTVYLQM NSLTPEDTAVYYCAVRYNQASYLVARSSDYNYWGQGTLVTVS SGGGGSEVQLVESGGGLVQPGGSLRLSCTASGNIFSSYHMGW FRQAPGKQREFIAYITTREITNYADSVKGRFTISRDNSKNTLYL QMNSLRAEDTAVYYCIADQSREIRRSWGQGTLVTVSSGGGGS GGGGSEVQLVESGGGLVQPGGSLRLSCTASGNIFSSYHMGWF RQAPGKQREFIAYITTREITNYADSVKGRFTISRDNSKNTLYLQ MNSLRAEDTAVYYCIADQSREIRRSWGQGTLVTVSSGGGGSE VQLVESGGGLVQPGNSLRLSCAASGFTFSSFGMSWVRQAPGK GLEWVSSISGSGSDTLYADSVKGRFTISRDNAKTTLYLQMNSL RPEDTAVYYCTIGGSLSRSSQGTLVTVSS |
| 83 | hu9B8-hu9A2-hu9A2-anti-HSA-Alb8 | EVQLLESGGGLVQPGGSLRLSCAASGLTFSYYAMAWFRQAPG KEREFVAAINWNQGNTYNTDSVKGRFTISRDNSKNTVYLQM NSLTPEDTAVYYCAVRYNQASYLVARSSDYNYWGQGTLVTVS SGGGGSQSQLVESGGGLVKPGGSLRLSCVASGFTISRSIMSWY RQAPGKERDWVASIGSAGNTNYVDSVKGRFTISRDNAKNSLY LQMNSLRAEDTAVYYCKTTLAVSFSEYWGQGTLVTVSSGGGG SGGGGSQSQLVESGGGLVKPGGSLRLSCVASGFTISRSIMSWY RQAPGKERDWVASIGSAGNTNYVDSVKGRFTISRDNAKNSLY LQMNSLRAEDTAVYYCKTTLAVSFSEYWGQGTLVTVSSGGGG SEVQLVESGGGLVQPGNSLRLSCAASGFTFSSFGMSWVRQAP GKGLEWVSSISGSGSDTLYADSVKGRFTISRDNAKTTLYLQMN SLRPEDTAVYYCTIGGSLSRSSQGTLVTVSS |

| **cMet biepitopic antibody, molecule M1** | | |
|---|---|---|
| 88 | 9A2-Fc hole chain: | QSQLAESGGGLVQSGGSLGLSCVASGFTISRSIMSWYRQPPGK ERDWVASIGSAGNTNYVDSVKGRFTISRDVAKNTVYLQMNSL KPEDTAVYYCKTTLAVSFSEYWGQGTQVTVSSEPKSSDKTHT CPPCPAPELLGGPSVFLFPPKPKDTLMISRTPEVTCVVVDVSHE DPEVKFNWYVDGVEVHNAKTKPREEQYNSTYRVVSVLTVLH QDWLNGKEYKCKVSNKALPAPIEKTISKAKGQPREPQVCTLP PSRDELTKNQVSLSCAVKGFYPSDIAVEWESNGQPENNYKTTP PVLDSDGSFFLVSKLTVDKSRWQQGNVFSCSVMHEALHNHYT QKSLSLSPGK* |
| 89 | hu7A 12^{b}-Fc knob chain: | EVQLVESGGGLIQAGGSLRLSCTASGNIFSSYHMGWFRQAPG KQREFIAYITTREITNYADSVKGRFTISRDNSKNTVYLQMNNL RSEDTAVYYCIADQSREIRRSWGQGTLVTVSSEPKSSDKTHTC PPCPAPELLGGPSVFLFPPKPKDTLMISRTPEVTCVVVDVSHED PEVKFNWYVDGVEVHNAKTKPREEQYNSTYRVVSVLTVLHQ DWLNGKEYKCKVSNKALPAPIEKTISKAKGQPREPQVYTLPP CRDELTKNQVSLWCLVKGFYPSDIAVEWESNGQPENNYKTTP PVLDSDGSFFLYSKLTVDKSRWQQGNVFSCSVMHEALHNHYT QKSLSLSPGK* |

| **cMet monoepitopic antibody** | | |
|---|---|---|
| 90 | 9A2-Fc chain in molecule M-2: | QSQLAESGGGLVQSGGSLGLSCVASGFTISRSIMSWYRQPPGK ERDWVASIGSAGNTNYVDSVKGRFTISRDVAKNTVYLQMNSL KPEDTAVYYCKTTLAVSFSEYWGQGTQVTVSSEPKSSDKTHT CPPCPAPELLGGPSVFLFPPKPKDTLMISRTPEVTCVVVDVSHE DPEVKFNWYVDGVEVHNAKTKPREEQYNSTYRVVSVLTVLH QDWLNGKEYKCKVSNKALPAPIEKTISKAKGQPREPQVYTLP PSRDELTKNQVSLTCLVKGFYPSDIAVEWESNGQPENNYKTTP PVLDSDGSFFLYSKLTVDKSRWQQGNVFSCSVMHEALHNHYT QKSLSLSPGK* |
| 91 | hu7A12^{b}-Fc chain in molecule M-3: | EVQLVESGGGLIQAGGSLRLSCTASGNIFSSYHMGWFRQAPG KQREFIAYITTREITNYADSVKGRFTISRDNSKNTVYLQMNNL RSEDTAVYYCIADQSREIRRSWGQGTLVTVSSEPKSSDKTHTC PPCPAPELLGGPSVFLFPPKPKDTLMISRTPEVTCVVVDVSHED PEVKFNWYVDGVEVHNAKTKPREEQYNSTYRVVSVLTVLHQ DWLNGKEYKCKVSNKALPAPIEKTISKAKGQPREPQVYTLPPS RDELTKNQVSLTCLVKGFYPSDIAVEWESNGQPENNYKTTPPV LDSDGSFFLYSKLTVDKSRWQQGNVFSCSVMHEALHNHYTQ KSLSLSPGK* |

| **Control antibody molecules M4-M7** | | |
|---|---|---|
| 92 | Anti-Covid-19 chain in molecule M-4 | AVQLVDSGGGLVQPGGSLRLSCAASGFAFNIYNMGWYRQAP GKQRELVATIAADGSTGYADSVKGRFTISRDNVKNKVDLQMN SLKPEDTAVYYCKRELGPFSSWGQGTQVTVSSEPKSSDKTHTC PPCPAPEAAGGPSVFLFPPKPKDTLMISRTPEVTCVVVDVSHE DPEVKFNWYVDGVEVHNAKTKPREEQYNSTYRVVSVLTVLH QDWLNGKEYKCKVSNKALPAPIEKTISKAKGQPREPQVYTLP PSRDELTKNQVSLSCAVKGFYPSDIAVEWESNGQPENNYKTTP PVLDSDGSFFLVSKLTVDKSRWQQGNVFSCSVMHEALHNHYT QKSLSLSPGK* |
| 93 | Anti-Covid-19 chain in molecule M-5, M-6, or M-7 | AVQLVDSGGGLVQPGGSLRLSCAASGFAFNIYNMGWYRQAP GKQRELVATIAADGSTGYADSVKGRFTISRDNVKNKVDLQMN SLKPEDTAVYYCKRELGPFSSWGQGTQVTVSSGGGGSGGGGS AVQLVDSGGGLVQPGGSLRLSCAASGFAFNIYNMGWYRQAP GKQRELVATIAADGSTGYADSVKGRFTISRDNVKNKVDLQMN SLKPEDTAVYYCKRELGPFSSWGQGTQVTVSSEPKSSDKTHTC PPCPAPEAAGGPSVFLFPPKPKDTLMISRTPEVTCVVVDVSHE DPEVKFNWYVDGVEVHNAKTKPREEQYNSTYRVVSVLTVLH QDWLNGKEYKCKVSNKALPAPIEKTISKAKGQPREPQVYTLP PSRDELTKNQVSLWCLVKGFYPSDIAVEWESNGQPENNYKTT PPVLDSDGSFFLYSKLTVDKSRWQQGNVFSCSVMHEALHNHY TQKSLSLSPGK* |

## Claims

1. An immunoglobulin single variable domain (ISVD) that specifically binds to cMet, wherein the ISVD comprises or consists of a VHH domain, wherein the VHH domain comprises:
(a) three CDRs contained in an amino acid sequence set forth in any one of SEQ ID NOs: 16, 39-40, and 121-130;
(b) three CDRs contained in an amino acid sequence set forth in any one of SEQ ID NOs: 21, 42, and 134-136; or
(c) three CDRs contained in an amino acid sequence set forth in SEQ ID NO: 26;
preferably, the VHH domain comprises:
(i) a CDR1 comprising or consisting of an amino acid sequence selected from any one of SEQ ID NOs: 18, 41, and 131-133, and a CDR2 and a CDR3 comprising or consisting of amino acid sequences of SEQ ID NO:19 and SEQ ID NO:20, respectively; particularly, a CDR1, a CDR2, and a CDR3 comprising or consisting of amino acid sequences of SEQ ID NOs: 41, 19, and 20, respectively;
(ii) a CDR1, a CDR2, and a CDR3 comprising or consisting of amino acid sequences of SEQ ID NOs: 23-25, respectively; or
(iii) a CDR1, a CDR2, and a CDR3 comprising or consisting of amino acid sequences of SEQ ID NOs: 28-30, respectively;
more preferably, the VHH domain comprises:
(a) a sequence set forth in any one of SEQ ID NOs: 16, 39-40, and 121-130, or a sequence having at least 90%, 91%, 92%, 93%, 94%, 95%, 96%, 97%, 98%, or 99% identity therewith;
(b) a sequence set forth in any one of SEQ ID NOs: 21, 42, and 134-136, or a sequence having at least 90%, 91%, 92%, 93%, 94%, 95%, 96%, 97%, 98%, or 99% identity therewith; or
(c) a sequence set forth in SEQ ID NO: 26, or a sequence having at least 90%, 91%, 92%, 93%, 94%, 95%, 96%, 97%, 98%, or 99% identity therewith;
even more preferably, the VHH domain comprises:
(a) an amino acid sequence set forth in any one of SEQ ID NOs: 16, 39-40, and 121-130;
(b) an amino acid sequence set forth in any one of SEQ ID NOs: 21, 42, and 134-136; or
(c) an amino acid sequence set forth in SEQ ID NO: 26;
particularly, the VHH domain comprises an amino acid sequence of SEQ ID NO: 39 or 40, or comprises an amino acid sequence of SEQ ID NO: 42.

2. An immunoglobulin single variable domain (ISVD) that specifically binds to EGFR, wherein the ISVD comprises or consists of a VHH domain, wherein the VHH domain comprises:
(a) three CDRs contained in an amino acid sequence set forth in any one of SEQ ID NOs: 1, 31, and 94-99;
(b) three CDRs contained in an amino acid sequence set forth in any one of SEQ ID NOs: 6, 32, and 100-102; or
(c) three CDRs contained in an amino acid sequence set forth in any one of SEQ ID NOs: 11, 36, 84, and 105-114;
preferably, the VHH domain comprises:
(i) a CDR1, a CDR2, and a CDR3 comprising or consisting of amino acid sequences of SEQ ID NOs: 3-5, respectively;
(ii) a CDR1 comprising or consisting of an amino acid sequence of SEQ ID NO: 8, a CDR2 comprising or consisting of an amino acid sequence of SEQ ID NO: 9, 34, or 103, and a CDR3 comprising or consisting of an amino acid sequence of SEQ ID NO: 10, 35, or 104; particularly, a CDR1, a CDR2, and a CDR3 comprising or consisting of amino acid sequences of SEQ ID NOs: 8, 34, and 35, respectively; or
(iii) a CDR1 comprising or consisting of an amino acid sequence of SEQ ID NO: 13, a CDR2 comprising or consisting of an amino acid sequence of any one of SEQ ID NOs: 14, 38, 85, and 115-120, and a CDR3 comprising or consisting of an amino acid sequence of SEQ ID NO:15; particularly, a CDR1, a CDR2, and a CDR3 comprising or consisting of amino acid sequences of SEQ ID NOs: 13, 85, and 15, respectively, or amino acid sequences of SEQ ID NOs: 13, 38, and 15, respectively;
more preferably, the VHH domain comprises:
(a) a sequence set forth in any one of SEQ ID NOs: 1, 31, and 94-99, or a sequence having at least 90%, 91%, 92%, 93%, 94%, 95%, 96%, 97%, 98%, or 99% identity therewith;
(b) a sequence set forth in any one of SEQ ID NOs: 6, 32, and 100-102, or a sequence having at least 90%, 91%, 92%, 93%, 94%, 95%, 96%, 97%, 98%, or 99% identity therewith; or
(c) a sequence set forth in any one of SEQ ID NOs: 11, 36, 84, and 105-114, or a sequence having at least 90%, 91%, 92%, 93%, 94%, 95%, 96%, 97%, 98%, or 99% identity therewith;
even more preferably, the VHH domain comprises:
(a) an amino acid sequence set forth in any one of SEQ ID NOs: 1, 31, and 94-99;
(b) an amino acid sequence set forth in any one of SEQ ID NOs: 6, 32, and 100-102; or
(c) an amino acid sequence set forth in any one of SEQ ID NOs: 11, 36, 84, and 105-114;
particularly, the VHH domain comprises an amino acid sequence of SEQ ID NO: 31, or comprises an amino acid sequence of SEQ ID NO: 32, or comprises an amino acid sequence of SEQ ID NO: 36, or comprises an amino acid sequence of SEQ ID NO: 84.

3. A binding molecule, comprising the ISVD according to claim 1 or 2.

4. The binding molecule according to claim 3, wherein the binding molecule comprises or consists of an antibody selected from the following: a single-domain antibody, a nanobody, a heavy-chain antibody, a monospecific antibody, and a multispecific antibody.

5. An antibody, comprising at least one (e.g., 1, 2, 3, 4, or more) ISVD that specifically binds to cMet according to claim 1.

6. The antibody according to claim 5, further comprising at least one (e.g., 1, 2, 3, 4, or more ) ISVD that specifically binds to EGFR, wherein preferably, the ISVD is an ISVD according to claim 2.

7. The antibody according to any one of claims 5-6, wherein:
(a) the antibody specifically binds to cMet and comprises two ISVDs specifically binding to the same epitope on cMet; or
(b) the antibody specifically binds to cMet and comprises two ISVDs specifically binding to different epitopes on cMet.

8. The antibody according to any one of claims 5-7, wherein:
(a) the antibody specifically binds to EGFR and cMet and comprises at least one (preferably 1) ISVD specifically binding to EGFR and two ISVDs specifically binding to the same epitope on cMet, or
(b) the antibody specifically binds to EGFR and cMet and comprises at least one (preferably 1) ISVD specifically binding to EGFR and two ISVDs specifically binding to different epitopes on cMet.

9. The antibody according to any one of claims 5-8, comprising a first ISVD and a second ISVD that specifically bind to cMet, wherein the first ISVD and the second ISVD are each an ISVD according to claim 1 that specifically binds to the same epitope on cMet;
preferably, the first ISVD and the second ISVD comprise: (i) a CDR1 comprising or consisting of an amino acid sequence selected from SEQ ID NOs: 18 and 41, and a CDR2 and a CDR3 comprising or consisting of amino acid sequences of SEQ ID NO:19 and SEQ ID NO:20, respectively; or (ii) a CDR1, a CDR2, and a CDR3 comprising or consisting of amino acid sequences of SEQ ID NOs: 23-25, respectively;
more preferably, the first ISVD and the second ISVD comprise, consist essentially of, or consist of an amino acid sequence having at least 90%, 91%, 92%, 93%, 94%, 95%, 96%, 97%, 98%, or 99% identity to an amino acid sequence set forth in any one of SEQ ID NO: 16, 39, or 40, or set forth in any one of SEQ ID NO: 21 or 42;
even more preferably, the first ISVD and the second ISVD comprise, consist essentially of, or consist of an amino acid sequence of SEQ ID NO: 16, 39, or 40, or an amino acid sequence of SEQ ID NO: 21 or 42.

10. The antibody according to any one of claims 5-9, comprising a first ISVD and a second ISVD that specifically bind to cMet, wherein the first ISVD and the second ISVD are each an ISVD according to claim 1 that specifically binds to a different epitope on cMet,
preferably, wherein: the first ISVD comprises a first anti-cMet VHH domain, and the second ISVD comprises a second anti-cMet VHH domain; or the first ISVD comprises the second anti-cMet VHH domain, and the second ISVD comprises the first anti-cMet VHH domain,
wherein the first anti-cMet VHH domain comprises: a CDR1, a CDR2, and a CDR3 comprising or consisting of amino acid sequences of SEQ ID NOs: 23-25, respectively, and the second anti-cMet VHH domain comprises: a CDR1 comprising or consisting of an amino acid sequence selected from SEQ ID NOs: 18 and 41, and a CDR2 and a CDR3 comprising or consisting of amino acid sequences of SEQ ID NO: 19 and SEQ ID NO: 20, respectively;
preferably, the first anti-cMet VHH domain comprises, consists essentially of, or consists of an amino acid sequence having at least 90%, 91%, 92%, 93%, 94%, 95%, 96%, 97%, 98%, or 99% identity to an amino acid sequence set forth in SEQ ID NO: 21 or 42, and the second anti-cMet VHH domain comprises, consists essentially of, or consists of an amino acid sequence having at least 90%, 91%, 92%, 93%, 94%, 95%, 96%, 97%, 98%, or 99% identity to an amino acid sequence set forth in SEQ ID NO: 16, 39, or 40;
more preferably, the first anti-cMet VHH domain comprises, consists essentially of, or consists of an amino acid sequence of SEQ ID NO: 21 or 42, and the second anti-cMet VHH domain comprises, consists essentially of, or consists of an amino acid sequence of SEQ ID NO: 16, 39, or 40;
preferably, the first ISVD comprises the first anti-cMet VHH domain, and the second ISVD comprises the second anti-cMet VHH domain.

11. The antibody according to any one of claims 5-10, further comprising an anti-EGFR ISVD specifically binding to EGFR, wherein the anti-EGFR ISVD is an ISVD according to claim 2,
wherein preferably, the anti-EGFR ISVD comprises:
(i) a CDR1, a CDR2, and a CDR3 comprising or consisting of amino acid sequences of SEQ ID NOs: 3, 4, and 5, respectively;
(ii) a CDR1, a CDR2, and a CDR3 comprising or consisting of amino acid sequences of SEQ ID NOs: 8, 9, and 10, respectively, or amino acid sequences of SEQ ID NOs: 8, 34, and 35, respectively, or amino acid sequences of SEQ ID NOs: 8, 103, and 104, respectively; or
(iii) a CDR1, a CDR2, and a CDR3 comprising or consisting of amino acid sequences of SEQ ID NOs: 13, 14, and 15, respectively, or amino acid sequences of SEQ ID NOs: 13, 85, and 15, respectively, or amino acid sequences of SEQ ID NOs: 13, 38, and 15, respectively;
more preferably, the anti-EGFR ISVD:
(a) comprises, consists essentially of, or consists of an amino acid sequence set forth in any one of SEQ ID NOs: 1, 31, and 94-99, or an amino acid sequence having at least 90%, 91%, 92%, 93%, 94%, 95%, 96%, 97%, 98%, or 99% identity therewith;
(b) comprises, consists essentially of, or consists of an amino acid sequence set forth in any one of SEQ ID NOs: 6, 32, and 100-102, or an amino acid sequence having at least 90%, 91%, 92%, 93%, 94%, 95%, 96%, 97%, 98%, or 99% identity therewith; or
(c) comprises, consists essentially of, or consists of an amino acid sequence set forth in any one of SEQ ID NOs: 11, 36, 84, and 105-114, or an amino acid sequence having at least 90%, 91%, 92%, 93%, 94%, 95%, 96%, 97%, 98%, or 99% identity therewith;
more preferably, the anti-EGFR ISVD:
(a) comprises, consists essentially of, or consists of an amino acid sequence set forth in SEQ ID NO: 31;
(b) comprises, consists essentially of, or consists of an amino acid sequence set forth in SEQ ID NO: 32; or
(c) comprises, consists essentially of, or consists of an amino acid sequence set forth in SEQ ID NO: 84.

12. The antibody according to any one of claims 5-11, wherein the antibody is in a single-chain form or a double-chain form.

13. The antibody according to any one of claims 5-12, wherein ISVDs on the same polypeptide chain are linked via one or more peptide linkers; preferably, the peptide linker comprises (GGGGS)n, wherein n is 1, 2, 3, 4, 5, 6, or 7, e.g., GGGGS (SEQ ID NO: 43) or GGGGSGGGGS (SEQ ID NO: 44).

14. The antibody according to any one of claims 5-13, further comprising a half-life extension moiety, preferably an immunoglobulin Fc region or an ISVD binding to human serum albumin, wherein optionally:
- the immunoglobulin Fc region is an Fc region of human IgG1, IgG2, IgG3, or IgG4 isotype;
- the ISVD binding to human serum albumin is an anti-HSA ISVD comprising or consisting of a VHH domain, wherein the VHH domain comprises CDRs 1-3 of SEQ ID NOs: 46-48.

15. The antibody according to any one of claims 5-14, comprising a first polypeptide chain and a second polypeptide chain, wherein, from N-terminus to C-terminus,
the first polypeptide chain comprises: a first ISVD specifically binding to cMET and an immunoglobulin Fc region;
the second polypeptide chain comprises: a second ISVD specifically binding to cMET and an immunoglobulin Fc region,
preferably,
- the first polypeptide chain comprises a sequence of SEQ ID NO: 88, or a sequence having at least 90%, 91%, 92%, 93%, 94%, 95%, 96%, 97%, 98%, or 99% identity therewith; and
- the second polypeptide chain comprises a sequence of SEQ ID NO: 89, or a sequence having at least 90%, 91%, 92%, 93%, 94%, 95%, 96%, 97%, 98%, or 99% identity therewith;
more preferably, the first polypeptide chain comprises or consists of a sequence of SEQ ID NO: 88, and the second polypeptide chain comprises or consists of a sequence of SEQ ID NO: 89.

16. The antibody according to any one of claims 5-14, comprising a first polypeptide chain and a second polypeptide chain, wherein, from N-terminus to C-terminus,
the first polypeptide chain comprises: an ISVD specifically binding to EGFR and an immunoglobulin Fc region, and
the second polypeptide chain comprises: a first ISVD specifically binding to cMET, a peptide linker, a second ISVD specifically binding to cMET, and an immunoglobulin Fc region.

17. The antibody according to any one of claims 5-14, comprising a single polypeptide chain,
wherein the polypeptide chain comprises: a first ISVD specifically binding to cMET, a second ISVD specifically binding to cMET, and an ISVD specifically binding to EGFR, and optionally an ISVD specifically binding to HSA;
preferably, from N-terminus to C-terminus, the polypeptide chain comprises:
an ISVD specifically binding to EGFR, a first peptide linker, a first ISVD specifically binding to cMET, a second peptide linker, a second ISVD specifically binding to cMET, and optionally a third peptide linker and an ISVD specifically binding to HSA.

18. The antibody according to claim 16, comprising a first polypeptide chain and a second polypeptide chain, wherein:
- the first polypeptide chain comprises a sequence selected from SEQ ID NO: 70, SEQ ID NO: 71, SEQ ID NO: 72, and SEQ ID NO: 86, or a sequence having at least 90%, 91%, 92%, 93%, 94%, 95%, 96%, 97%, 98%, or 99% identity therewith;
- the second polypeptide chain comprises a sequence selected from SEQ ID NO: 69, SEQ ID NO: 73, SEQ ID NO: 74, SEQ ID NO: 75, and SEQ ID NO: 87, or a sequence having at least 90%, 91%, 92%, 93%, 94%, 95%, 96%, 97%, 98%, or 99% identity therewith; and
preferably,
(i) the first polypeptide chain comprises or consists of an amino acid sequence of SEQ ID NO: 70, or a sequence having at least 90%, 91%, 92%, 93%, 94%, 95%, 96%, 97%, 98%, or 99% identity therewith, and the second polypeptide chain comprises or consists of an amino acid sequence of SEQ ID NO: 73, 75, or 74, or a sequence having at least 90%, 91%, 92%, 93%, 94%, 95%, 96%, 97%, 98%, or 99% identity therewith;
(ii) the first polypeptide chain comprises or consists of an amino acid sequence of SEQ ID NO: 71, or a sequence having at least 90%, 91%, 92%, 93%, 94%, 95%, 96%, 97%, 98%, or 99% identity therewith, and the second polypeptide chain comprises or consists of an amino acid sequence of SEQ ID NO: 73, 75, or 74, or a sequence having at least 90%, 91%, 92%, 93%, 94%, 95%, 96%, 97%, 98% or 99% identity therewith;
(iii) the first polypeptide chain comprises or consists of an amino acid sequence of SEQ ID NO: 72, or a sequence having at least 90%, 91%, 92%, 93%, 94%, 95%, 96%, 97%, 98%, or 99% identity therewith, and the second polypeptide chain comprises or consists of an amino acid sequence of SEQ ID NO: 73, 75, or 74, or a sequence having at least 90%, 91%, 92%, 93%, 94%, 95%, 96%, 97%, 98%, or 99% identity therewith;
(iv) the first polypeptide chain comprises or consists of an amino acid sequence of SEQ ID NO: 86, or a sequence having at least 90%, 91%, 92%, 93%, 94%, 95%, 96%, 97%, 98%, or 99% identity therewith, and the second polypeptide chain comprises or consists of an amino acid sequence of SEQ ID NO: 87, or a sequence having at least 90%, 91%, 92%, 93%, 94%, 95%, 96%, 97%, 98%, or 99% identity therewith;
more preferably,
(i) the first polypeptide chain comprises or consists of an amino acid sequence of SEQ ID NO: 71, and the second polypeptide chain comprises or consists of an amino acid sequence of SEQ ID NO: 73;
(ii) the first polypeptide chain comprises or consists of an amino acid sequence of SEQ ID NO: 72, and the second polypeptide chain comprises or consists of an amino acid sequence of SEQ ID NO: 73;
(iii) the first polypeptide chain comprises or consists of an amino acid sequence of SEQ ID NO: 86, and the second polypeptide chain comprises or consists of an amino acid sequence of SEQ ID NO: 87.

19. The antibody according to claim 17, comprising a single polypeptide chain, wherein the polypeptide chain comprises a sequence selected from SEQ ID NOs: 51-66 or a sequence having at least 90%, 91%, 92%, 93%, 94%, 95%, 96%, 97%, 98%, or 99% identity therewith; or the polypeptide chain comprises a sequence selected from SEQ ID NOs: 67-68 and 76-83 or a sequence having at least 90%, 91%, 92%, 93%, 94%, 95%, 96%, 97%, 98%, or 99% identity therewith;
preferably, the polypeptide chain comprises or consists of a sequence selected from SEQ ID NOs: 51-56.

20. The antibody according to any one of claims 5-19, wherein the antibody is a multispecific antibody specifically binding to EGFR and cMet and has one or more of the following properties:
(1) binding to EGFR, e.g., human EGFR, with moderate or relatively low affinity, and specifically binding to cMet, e.g., human cMet;
(2) binding to EGFR expressed on the cell surface at a relatively low level, and specifically binding to cMet expressed on the cell surface;
(3) blocking binding of ligand HGF, when present, to cMet on the cell surface;
(4) being internalized by cells expressing cMet;
(5) being internalized by cells expressing EGFR; and
(6) having cross-reactivity with human EGFR and cynomolgus monkey EGFR, and having cross-reactivity with human cMet and cynomolgus monkey cMet.

21. An antibody, comprising at least one ISVD specifically binding to EGFR according to claim 2, and optionally, further comprising at least one ISVD specifically binding to cMet, wherein preferably, the ISVD specifically binding to cMet is an ISVD according to claim 1;
preferably, the antibody has one or more of the following properties:
(1) binding to EGFR, e.g., human EGFR, with low affinity; and
(2) having cross-reactivity with human EGFR and cynomolgus monkey EGFR.

22. An isolated nucleic acid, wherein the isolated nucleic acid encodes the ISVD according to claim 1 or 2, the binding molecule according to claim 3 or 4, or the antibody according to any one of claims 5-21.

23. A vector, comprising the nucleic acid according to claim 22, wherein preferably, the vector is an expression vector.

24. A host cell, comprising the nucleic acid according to claim 22 or the vector according to claim 23, wherein, preferably, the host cell is prokaryotic or eukaryotic; more preferably, the host cell is selected from an *E. coli* cell, a yeast cell, a mammalian cell, and other cells suitable for preparing ISVDs; most preferably, the host cell is an HEK 293 cell or a CHO cell.

25. A preparation method, comprising: culturing the host cell according to claim 24, and optionally isolating the ISVD according to claim 1 or 2, the binding molecule according to claim 3 or 4, or the antibody according to any one of claims 5-21 from the host cell or from a culture medium.

26. An immunoconjugate or immunofusion, comprising the ISVD according to claim 1 or 2, the binding molecule according to claim 3 or 4, or the antibody according to any one of claims 5-21.

27. An antibody-drug conjugate having formula (I₀) or a pharmaceutically acceptable salt or a solvate thereof:
Ab-(L-D)ₚ (I₀)
wherein:
Ab is the ISVD according to claim 1 or 2, the binding molecule according to claim 3 or 4, or the antibody according to any one of claims 5-21;
L is a linker;
D is a drug, e.g., an anti-tumor compound;
p is an integer selected from 1 to 16, e.g., an integer selected from 1-10, 1-9, 2-8, 4-10, 6-8, 3-7, 4-6, and 2-6, e.g., 1, 2, 3, 4, 5, 6, 7, 8, 9, 10, 11, or 12.

28. The antibody-drug conjugate or a pharmaceutically acceptable salt or a solvate thereof according to claim 27, wherein the antibody-drug conjugate has formula (I):
Ab-(S-L-D)ₚ (I)
wherein:
Ab is the ISVD according to claim 1 or 2, the binding molecule according to claim 3 or 4, or the antibody according to any one of claims 5-21;
L is a linker;
D is a drug, e.g., an anti-tumor compound;
p is an integer selected from 1 to 16, e.g., an integer selected from 1-10, 1-9, 2-8, 4-10, 6-8, 3-7, 4-6, and 2-6, e.g., 1, 2, 3, 4, 5, 6, 7, 8, 9, 10, or 12; and
S in formula (I) is sulfur from Ab.

29. The antibody-drug conjugate or a pharmaceutically acceptable salt or a solvate thereof according to claim 27 or 28, wherein the drug is a cytotoxic agent, e.g., a camptothecin compound or an auristatin compound.

30. The antibody-drug conjugate or a pharmaceutically acceptable salt or a solvate thereof according to any one of claims 27-29, wherein D has a structure represented by formula (D-1a) or formula (D-1b): 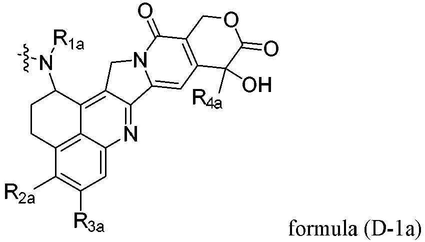
wherein R₁ₐ is selected from H and C₁-C₆ alkyl;
R₂ₐ is selected from H, halogen, C₁-C₆ alkyl, C₁-C₆ haloalkyl, -OR₅ₐ, and -SR₅ₐ;
R₃ₐ is selected from H, halogen, CN, C₁-C₆ alkyl, C₁-C₆ haloalkyl, and -OR₅ₐ; and
R₄ₐ and R₅ₐ are independently selected from H and C₁-C₄ alkyl;
or 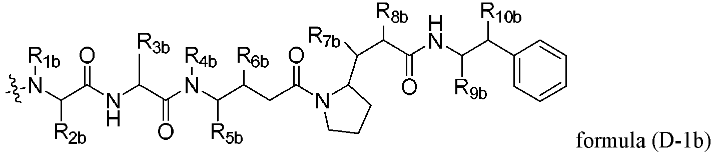
wherein R_{1b}, R_{2b}, R_{3b}, R_{4b}, R_{5b}, and R_{8b} are each independently selected from C₁₋₈ alkyl, preferably C₁₋₄ alkyl, such as methyl, ethyl, n-propyl, isopropyl, n-butyl, isobutyl, or sec-butyl;
R_{6b} and R_{7b} are each independently selected from C₁₋₈ alkoxy, such as methoxy, ethoxy, or propoxy;
R_{9b} is selected from C₁₋₈ alkyl and COOH, preferably C₁₋₄ alkyl, such as methyl, ethyl, n-propyl, isopropyl, n-butyl, isobutyl, or sec-butyl; and
R_{10b} is selected from OH and H;
the wavy line in the structural formula D indicates that the valence bond is linked to L.

31. The antibody-drug conjugate or a pharmaceutically acceptable salt or a solvate thereof according to claim 30, wherein D has a structure of formula (D-1a), wherein R₁ₐ is H; R₂ₐ is C₁-C₆ alkyl; R₃ₐ is halogen, preferably -F; R₄ₐ is C₁-C₄ alkyl, preferably ethyl.

32. The antibody-drug conjugate or a pharmaceutically acceptable salt or a solvate thereof according to claim 30, wherein D has a structure of formula (D-1b), wherein
R_{1b}, R_{4b} and R_{8b} are each independently selected from C₁₋₂ alkyl, preferably methyl;
R_{2b}, R_{3b}, and R_{5b} are each independently selected from C₃₋₄ alkyl;
R_{6b} and R_{7b} are each independently selected from C₁₋₂ alkoxy; and
R_{9b} is selected from C₁₋₄ alkyl and R_{10b} is OH; or R_{9b} is COOH and R_{10b} is H.

33. The antibody-drug conjugate or a pharmaceutically acceptable salt or a solvate thereof according to any one of claims 27-32, wherein D has a structure represented by formula (D-2a) or formula (D-2b): 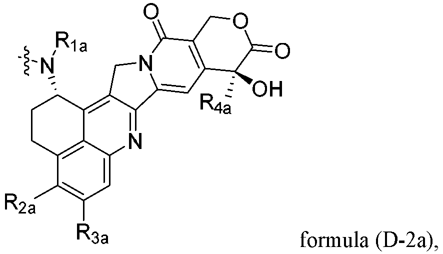
wherein R₁ₐ, R₂ₐ, R₃ₐ, and R₄ₐ are as defined in formula (D-1a); or 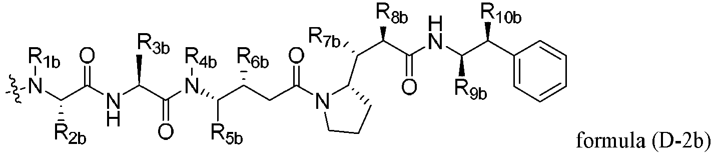
wherein R_{1b}, R_{2b}, R_{3b}, R_{4b}, R_{5b}, R_{6b}, R_{7b}, R_{8b}, R_{9b}, and R_{10b} are as defined in formula (D-1b).

34. The antibody-drug conjugate or a pharmaceutically acceptable salt or a solvate thereof according to any one of claims 27-29, wherein D has a structure represented by formula (D-3a) or (D-3b): 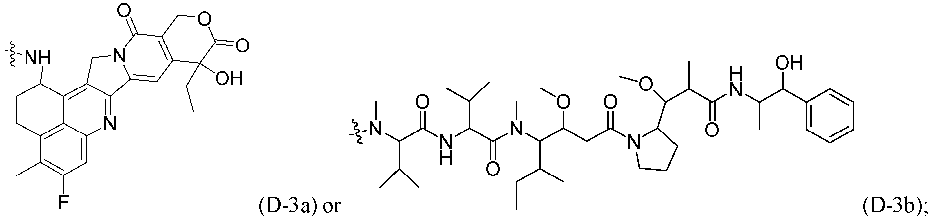 preferably, D has a structure represented by formula (D-4a) or (D-4b):

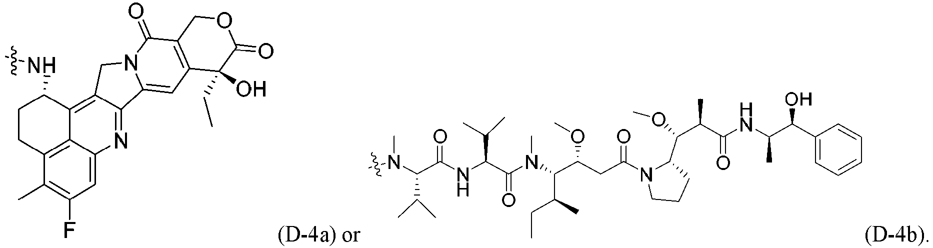

35. The antibody-drug conjugate or a pharmaceutically acceptable salt or a solvate thereof according to any one of claims 27-29, wherein the drug is exatecan, Dxd, SN-38, monomethyl auristatin E (MMAE), or MMAF.

36. The antibody-drug conjugate or a pharmaceutically acceptable salt or a solvate thereof according to any one of claims 27-35, wherein -L- has the following structure:
-Z-L₁-L₂-L₃-
wherein
Z is selected from 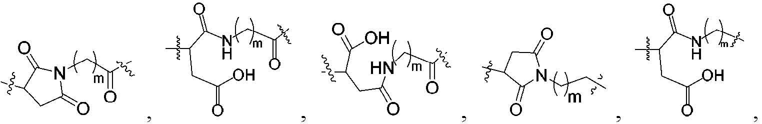 and 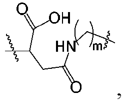 wherein m is an integer selected from 1-10, e.g., 1, 2, 3, 4, 5, 6, 7, or 8;
L₁ is selected from absent, 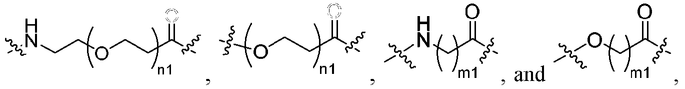 wherein n1 and m1 are each independently an integer selected from 0-20, e.g., an integer selected from 0-12, e.g., 1, 2, 3, 4, 5, 6, 7, or 8;
L₂ is an amino acid residue or a peptide consisting of 2-8 amino acids; and
L₃ is selected from 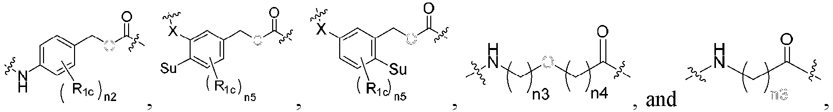 wherein X is selected from -NH-, -O-, and -S-; each R_{1c} is independently selected from C₁₋₈ alkyl, C₁₋₈ haloalkyl-, C₁₋₈ alkoxy, halogen, nitro, and cyano; each Su is independently selected from pentose, penturonic acid, hexose, and hexuronic acid; n2 is 0, 1, 2, 3, or 4; n5 is 0, 1, 2, or 3; n3 and n4 are independently 1, 2, 3, 4, 5, or 6; and
wherein Z is linked to S on Ab, and L₃ is linked to D.

37. The antibody-drug conjugate or a pharmaceutically acceptable salt or a solvate thereof according to claim 36, wherein
Z is selected from 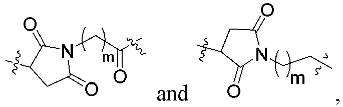 wherein m is 1, 2, 3, 4, 5, 6, 7, or 8;
preferably, Z is selected from

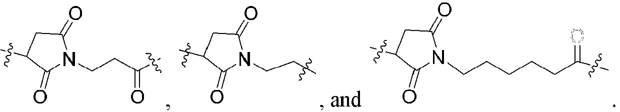

38. The antibody-drug conjugate or a pharmaceutically acceptable salt or a solvate thereof according to claim 36 or 37, wherein L₁ is selected from absent, 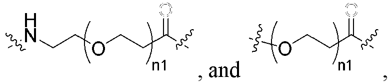 wherein n1 is independently an integer selected from 0-12, e.g., 1, 2, 3, 4, 5, 6, 7, or 8;
preferably, L₁ is selected from absent,

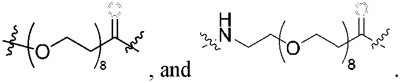

39. The antibody-drug conjugate or a pharmaceutically acceptable salt or a solvate thereof according to any one of claims 36-38, wherein L₂ is an amino acid residue or a peptide consisting of 2, 3, 4, 5, 6, or 7 amino acids; preferably, said amino acid residue or amino acid is each independently selected from valine (Val), alanine (Ala), glycine (Gly), lysine (Lys), citrulline (Cit), glutamine (Gln), glutamic acid (Glu), phenylalanine (Phe), leucine (Leu), tyrosine (Tyr), serine (Ser), aspartic acid (Asp), asparagine (Asn), isoleucine (Ile), arginine (Arg), proline (Pro), methionine (Met), tryptophan (Trp), cysteine (Cys), histidine (His), and threonine (Thr);
more preferably, said amino acid residue or amino acid is each independently selected from glycine (Gly), valine (Val), alanine (Ala), citrulline (Cit), phenylalanine (Phe), lysine (Lys), glutamic acid (Glu), and glutamine (Gln);
even more preferably, said amino acid residue or amino acid is each independently selected from glycine (Gly), valine (Val), alanine (Ala), citrulline (Cit), and glutamic acid (Glu).

40. The antibody-drug conjugate or a pharmaceutically acceptable salt or a solvate thereof according to any one of claims 36-38, wherein L₂ is selected from -Ala-, -Val-, -Gly-, -Val-Ala-, -Val-Cit-, -Glu-Val-Cit-, and -Gly-Gly-Phe-Gly-;
preferably, L₂ is selected from -Gly-, -Val-Ala-, -Val-Cit-, -Glu-Val-Cit-, and -Gly-Gly-Phe-Gly-.

41. The antibody-drug conjugate or a pharmaceutically acceptable salt or a solvate thereof according to any one of claims 36-40, wherein L₃ is selected from: 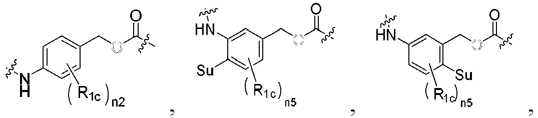 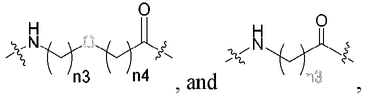
wherein each R_{1c} is independently selected from C₁₋₈ alkyl, C₁₋₈ haloalkyl-, C₁₋₈ alkoxy, halogen, nitro, and cyano;
each Su is independently selected from 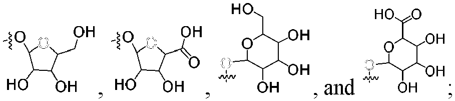 n2 is 0, 1, 2, 3, or 4; n5 is 0, 1, 2, or 3; and n3 and n4 are independently 1, 2, 3, 4, 5, or 6.

42. The antibody-drug conjugate or a pharmaceutically acceptable salt or a solvate thereof according to any one of claims 36-41, wherein L₃ is selected from: 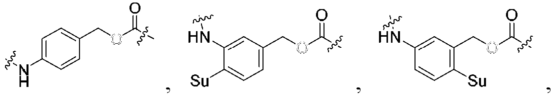 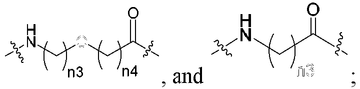
preferably, L₃ is selected from: 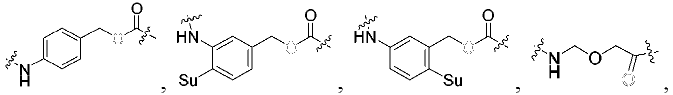 and 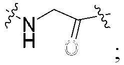
more preferably, L₃ is selected from:

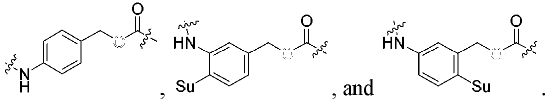

43. The antibody-drug conjugate or a pharmaceutically acceptable salt or a solvate thereof according to any one of claims 36-42, wherein each Su is independently: 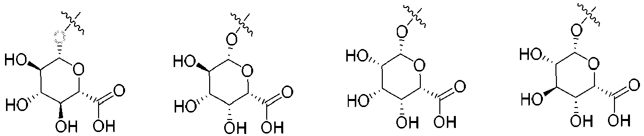 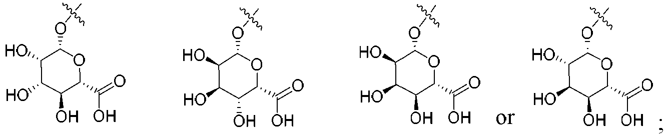
preferably, each Su is independently 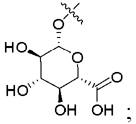
alternatively preferably, each Su is independently

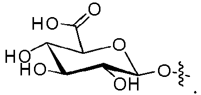

44. The antibody-drug conjugate or a pharmaceutically acceptable salt or a solvate thereof according to any one of claims 36-43, wherein L₃ is selected from: 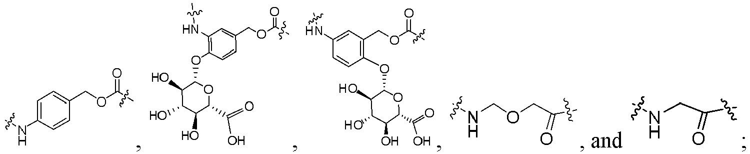 preferably, L₃ is selected from

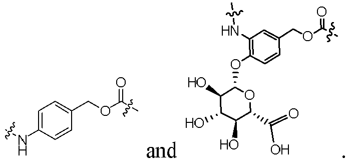

45. The antibody-drug conjugate or a pharmaceutically acceptable salt or a solvate thereof according to claim 36, wherein
each -Z-L₁-L₂-L₃- is independently selected from the following structures: 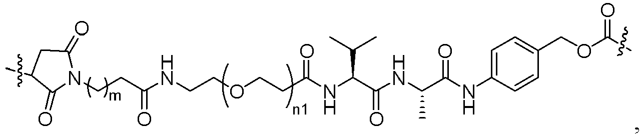 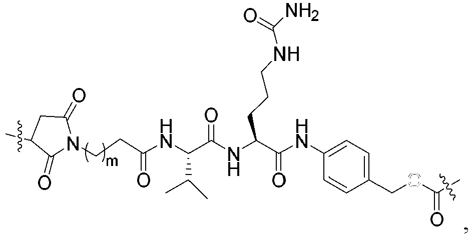 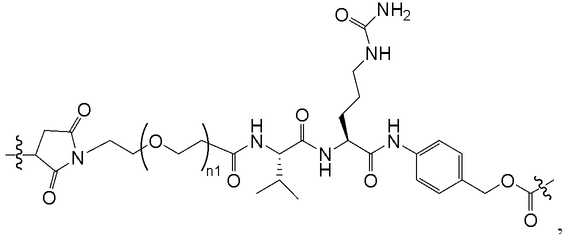 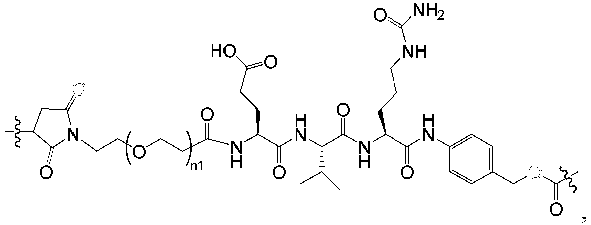 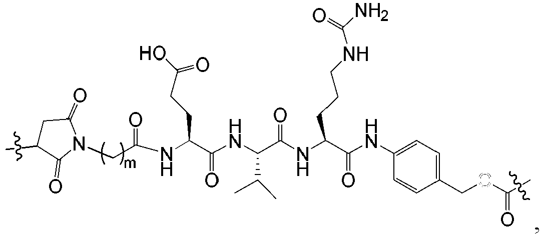 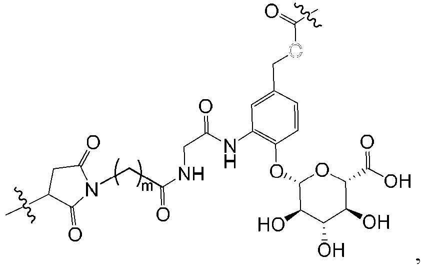 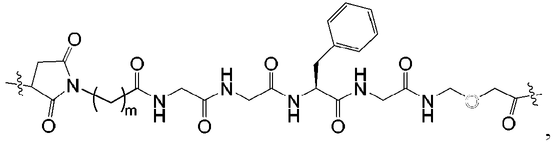 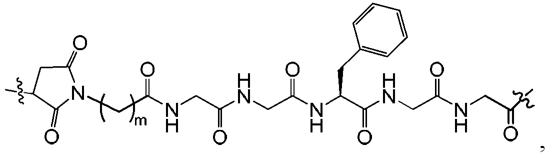 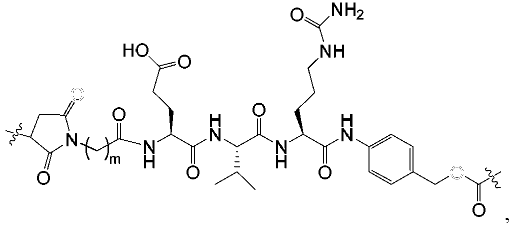
and 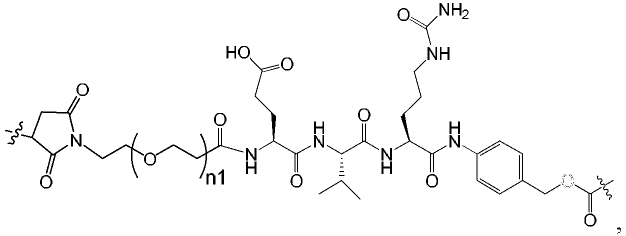
wherein each m is independently an integer selected from 1-10, for example, 1, 2, 3, 4, 5, 6, 7, or 8;
n1 is independently an integer selected from 0-12, e.g., 1, 2, 3, 4, 5, 6, 7, or 8, preferably 6 or 8; and
wherein the left side of the group is linked to S on Ab, and the right side is linked to D.

46. The antibody-drug conjugate or a pharmaceutically acceptable salt or a solvate thereof according to claim 27 or 28, wherein the antibody-drug conjugate is an antibody-drug conjugate having a structure selected from the following: 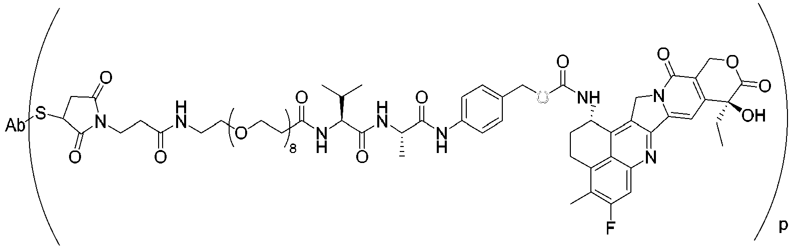 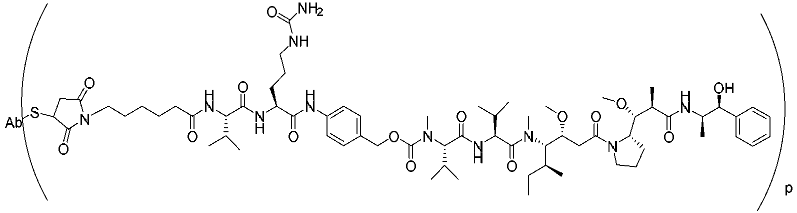 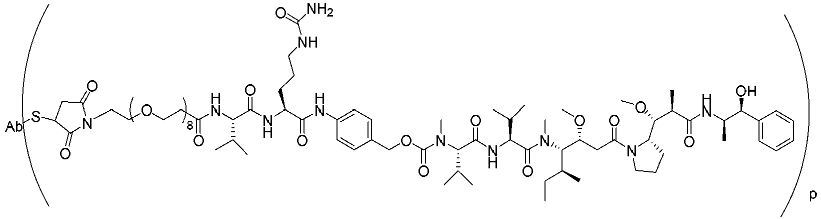 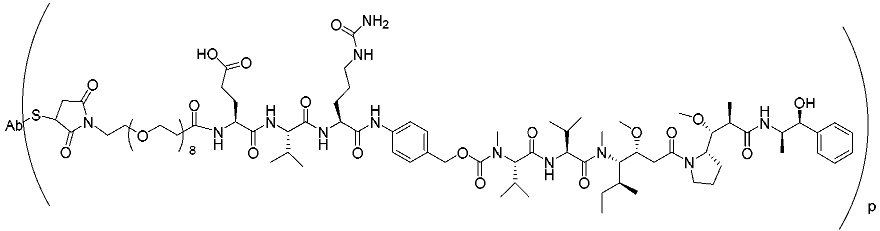 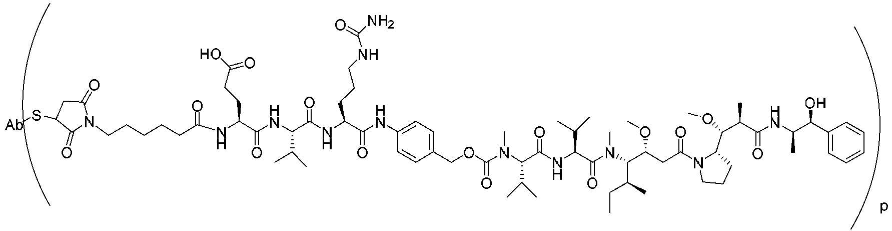 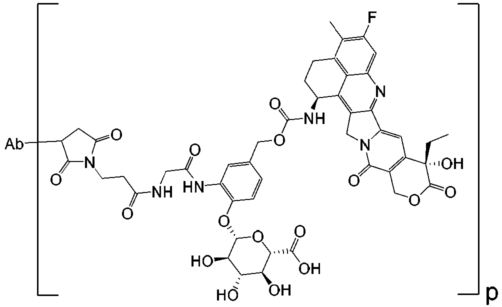 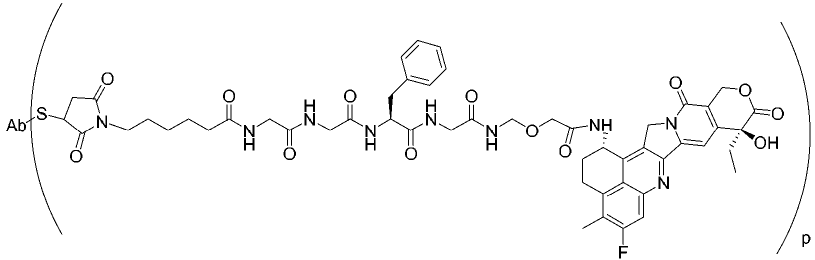
wherein Ab is the ISVD according to claim 1 or 2, the binding molecule according to claim 3 or 4, or the antibody according to any one of claims 5-21; and
p is an integer selected from 1 to 16, e.g., an integer selected from 1-10, 1-9, 2-8, 4-10, 6-8, 3-7, 4-6, and 2-6, e.g., 1, 2, 3, 4, 5, 6, 7, 8, 9, 10, 11, or 12.

47. The antibody-drug conjugate or a pharmaceutically acceptable salt or a solvate thereof according to any one of claims 27-46, wherein the antibody-drug conjugate has an average DAR of 2-10, 6-10, 4-8, 7-9, or 2-4, or 2-6.

48. A pharmaceutical composition, wherein the pharmaceutical composition comprises the ISVD according to claim 1 or 2, the binding molecule according to claim 3 or 4, the antibody according to any one of claims 5-21, the immunoconjugate or immunofusion according to claim 26, or the antibody-drug conjugate or a pharmaceutically acceptable salt or a solvate thereof according to any one of claims 27-47, and a pharmaceutically acceptable carrier; optionally, the pharmaceutical composition further comprises one or more additional pharmaceutically active polypeptides and/or compounds; for example, the pharmaceutical composition further comprises an additional therapeutic agent selected from an oncolytic drug, a cytotoxic agent, a cytokine, and an inhibitor of an immune checkpoint molecule.

49. Use of the ISVD according to claim 1 or 2, the binding molecule according to claim 3 or 4, the antibody according to any one of claims 5-21, or the immunoconjugate or immunofusion according to claim 26, or the antibody-drug conjugate or a pharmaceutically acceptable salt or a solvate thereof according to any one of claims **27-47,** for use as a medicament or for use in the manufacture of a medicament, wherein preferably, the medicament is for treating a cancer, e.g., a cancer selected from lung cancer (e.g., lung squamous cell carcinoma, lung adenocarcinoma, and non-small cell lung cancer), breast cancer (e.g., triple negative breast cancer), gastric cancer, colon cancer, and head and neck cancer (e.g., pharyngeal squamous cell carcinoma).
